(19) Europäisches Patentamt
European Patent Office
Office européen des brevets

(11) **EP 2 569 333 B1**

(12) **FASCICULE DE BREVET EUROPEEN**

(45) Date de publication et mention de la délivrance du brevet:
**13.12.2017  Bulletin 2017/50**

(21) Numéro de dépôt: **11718450.7**

(22) Date de dépôt: **01.04.2011**

(51) Int Cl.:
*C07K 16/00* (2006.01)    *C07K 16/28* (2006.01)
*C07K 16/30* (2006.01)    *A61K 31/282* (2006.01)
*A61K 39/395* (2006.01)    *A61K 31/337* (2006.01)
*A61K 31/7088* (2006.01)    *A61K 33/24* (2006.01)
*C07K 16/46* (2006.01)    *A61K 39/00* (2006.01)

(86) Numéro de dépôt international:
**PCT/FR2011/050745**

(87) Numéro de publication internationale:
**WO 2011/141653 (17.11.2011 Gazette 2011/46)**

(54) **NOUVEAUX ANTICORPS HUMANISES 12G4 MUTES ET LEURS FRAGMENTS DIRIGES CONTRE LE RECEPTEUR HUMAIN DE L'HORMONE ANTI-MULLERIENNE DE TYPE II**

NEUE MUTIERTE HUMANISIERTE 12G4-ANTIKÖRPER UND FRAGMENTE DARAUS GEGEN DEN MENSCHLICHEN ANTI-MÜLLER-HORMONREZEPTOR TYP II

NOVEL MUTATED HUMANIZED 12G4 ANTIBODIES AND THE FRAGMENTS THEREOF AGAINST THE HUMAN ANTI-MÜLLERIAN HORMONE RECEPTOR TYPE II

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.05.2010  FR 1053712**

(43) Date de publication de la demande:
**20.03.2013  Bulletin 2013/12**

(73) Titulaires:
- **Laboratoire Français du Fractionnement et des Biotechnologies**
  **91958 Les Ulis (FR)**
- **Université Montpellier I**
  **34067 Montpellier Cedex 2 (FR)**
- **Centre Régional de Lutte contre le Cancer**
  **34298 Montpellier Cedex 5 (FR)**
- **INSERM (Institut National de la Santé et de la Recherche Médicale)**
  **75654 Paris Cedex 13 (FR)**

(72) Inventeurs:
- **BEHRENS, Christian**
  **91430 Vauhallan (FR)**
- **NAVARRO-TEULON, Isabelle**
  **34980 Saint Gely du Fesc (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A2-2008/053330    US-A1- 2006 188 440**

- **QING-AN YUAN ET AL: "Isolation of anti-MISIIR scFv molecules from a phage display library by cell sorter biopanning", CANCER IMMUNOLOGY, IMMUNOTHERAPY, SPRINGER, BERLIN, DE, vol. 57, no. 3, 4 août 2007 (2007-08-04), pages 367-378, XP019586690, ISSN: 1432-0851, DOI: DOI:10.1007/S00262-007-0376-2**
- **JUAREZ-GONZALEZ V R ET AL: "Directed Evolution, Phage Display and Combination of Evolved Mutants: A Strategy to Recover the Neutralization Properties of the scFv Version of BCF2 a Neutralizing Monoclonal Antibody Specific to Scorpion Toxin Cn2", JOURNAL OF MOLECULAR BIOLOGY, LONDON, GB, vol. 346, no. 5, 11 mars 2005 (2005-03-11) , pages 1287-1297, XP004745232, ISSN: 0022-2836, DOI: DOI:10.1016/J.JMB.2004.12.060**

EP 2 569 333 B1

- IRVING R ET AL: "Phage-display library selection and mutation for the engineering of antibody affinity", IMMUNOTECHNOLOGY, ELSEVIER SCIENCE PUBLISHERS BV, NL, vol. 2, no. 1, 1 février 1996 (1996-02-01) , page 69, XP004052702, ISSN: 1380-2933, DOI: DOI:10.1016/1380-2933(96)80677-4
- BACA M ET AL: "Antibody humanization using monovalent phage display", JOURNAL OF BIOLOGICAL CHEMISTRY, AMERICAN SOCIETY FOR BIOCHEMISTRY AND MOLECULAR BIOLOGY, US, vol. 272, no. 16, 1 January 1997 (1997-01-01), pages 10678-10684, XP002308888, ISSN: 0021-9258, DOI: 10.1074/JBC.272.16.10678
- Holger E Thie: "Affinity maturation by random mutagenesis and phage display" In: "Antibody Engineering Vol. 1", 1 January 2010 (2010-01-01), Springer, Berlin, Heidelberg, DE, XP055310998, vol. 1, pages 397-409,
- Juan C. Almagro ET AL: "Antibody engineering: Humanization, affinity maturation, and selection techniques" In: "Therapeutic Monoclonal Antibodies : From Bench to Clinic", 1 October 2009 (2009-10-01), Wiley, Hoboken, US, XP055311028, pages 311-334,
- Michael Hust ET AL: "Antibody phage display" In: "Therapeutic Monoclonal Antibodies : From Bench to Clinic", 1 October 2009 (2009-10-01), Wiley, Hoboken, US, XP055311033, pages 191-211,
- IRVING R A ET AL: "Affinity maturation of recombinant antibodies using E.coli mutator cells", IMMUNOTECHNOLOGY, ELSEVIER SCIENCE PUBLISHERS BV, NL, vol. 2, no. 2, 1 June 1996 (1996-06-01), pages 127-143, XP004052677, ISSN: 1380-2933, DOI: 10.1016/1380-2933(96)00044-9
- MONDON P ET AL: "Method for generation of human hyperdiversified antibody fragment library", BIOTECHNOLOGY JOURNAL, WILEY-VCH VERLAG, WEINHEIM, DE, vol. 2, no. 1, 1 January 2007 (2007-01-01) , pages 76-82, XP002635180, ISSN: 1860-6768, DOI: 10.1002/BIOT.200600205 [retrieved on 2007-01-16]
- WINTER G ET AL: "HUMANIZED ANTIBODIES", IMMUNOLOGY TODAY, ELSEVIER PUBLICATIONS, CAMBRIDGE, GB, vol. 14, no. 6, 1 January 1996 (1996-01-01), pages 243-246, XP001005438, ISSN: 0167-4919, DOI: 10.1016/0167-5699(93)90039-N

Remarques:

Le dossier contient des informations techniques présentées postérieurement au dépôt de la demande et ne figurant pas dans le présent fascicule.

**Description**

La présente description concerne de nouveaux anticorps humanisés 12G4 mutés, et leurs fragments, dirigés contre le récepteur de l'hormone anti-mullérienne de type II.

Le cancer ovarien est la cause principale des cancers gynécologiques et est la cinquième cause de mortalité par cancer chez la femme dont les trois origines histologiques sont les suivantes :

- l'épithélium de surface (tumeur épithéliale avec différents sous types) qui représente 85-90 % des cancers ovariens,
- les cordons sexuels / stroma (tumeur de la granulosa (3% des cancers ovariens totaux)), qui représentent environ 10 % des tumeurs ovariennes,
- les cellules germinales qui représentent 5 % des cancers ovariens.

Il est généralement asymptomatique pendant les premiers stades ce qui lui vaut le surnom de « silent killer » (La Marca A., Volpe A. The Anti-Mullerian hormone and ovarian cancer. Human Reproduction Update, Vol.13, No.3 pp. 265-273, 2007).

Il existe quatre stades et pronostic (classification FIGO : Fédération internationale de Gynécologie et d' Obstétrique) pour lesquels le taux de survie diminue fortement dès le stade 2:

**Stade I:** Tumeur limitée aux ovaires (taux de survie à 5ans: 90-70%),
**Stade II:** Tumeur dans un ou deux ovaires avec extension pelvienne (taux de survie à 5ans : 70-40%),
**Stade III:** Tumeur dans un ou deux ovaires avec extension extra pelvienne (taux de survie à 5ans : 20%),
**Stade IV:** Métastases à distance à l'exclusion des métastases péritonéales (taux de survie à 5ans : <10%),

(Fauci, Braunwald et al. Principles of internal medicine. Harrison' s 17th edition / National Cancer Institute cancer.gov / CNGOF (collèges national des gynécologues et obstétriciens français).

Les principales stratégies utilisées pour le traitement du cancer ovarien sont la chirurgie et la chimiothérapie, en particulier en première ligne, tel qu'un mélange carboplatine et paclitaxel.

Des anticorps monoclonaux ont également été récemment développés tel que le cetuximab, qui est dirigé contre le récepteur du facteur de croissance épidermique (EGFR, Ozols R. F. et al., Focus on epithelial ovarian cancer, Cancer Cell. 2004, Jan ; 5(1) :19-24).

D'autres anticorps monoclonaux sont actuellement en phase III, tels que l'abagovomab dirigé contre le CA-125, l'avastin dirigé contre le facteur de croissance endothéliale vasculaire (VEGF-A), ou le farletuzumab, dirigé contre le récepteur alpha folique (FRA).

L'hormone anti-mullérienne humaine est une glycoprotéine de 560 acides aminés, membre de la famille des TGF-β. C'est une hormone émise par les cellules de Sertoli du testicule foetal, qui provoque la dégénérescence du canal de Müller.

Elle est exprimée chez l'adulte dans les cellules de Sertoli et de Leydig (testicule) et les cellules de la granulosa (ovaire).

Elle joue un rôle dans l'activité de l'ovaire adulte de régulation de la folliculogenèse.

Le récepteur de l'hormone anti-mulérienne de type II (AMHR-II) est un peptide de 573 acides aminés et possède une activité sérine-thréonine kinase.

Il est impliqué dans la régression du canal de Müller associé au développement du système de reproduction de l'homme. Il s'atrophie chez l'homme où il ne forme que l'utricule prostatique et l'hydatide sessile, mais persiste chez la femme où il est à l'origine des trompes, de l'utérus et de la plus grande partie du vagin.

Ce récepteur est fréquemment exprimé sur les cellules épithéliales tumorales d'ovaires humaines.

La demande internationale WO 2008/053330 décrit un anticorps monoclonal murin 12G4 dirigé contre AMHR-II pour le traitement des cancers ovariens. Cependant, il est bien connu de l'homme du métier que l'administration d'anticorps monoclonaux murins à l'homme provoque une réaction immune.

Cette demande internationale mentionne également que l'anticorps peut être chimérique ou humanisé, mais ne les décrit pas en tant que tels.

Cependant, les anticorps chimériques déclenchent également des réactions immunes et les anticorps humanisés, faiblement immunogènes, possèdent le défaut d'avoir une affinité de liaison à l'antigène pouvant être diminuée et d'être par conséquent moins actifs.

Il est possible selon cette demande d'augmenter ladite affinité par mutation des acides aminés présents dans l'anticorps humanisé, par modification notamment de la séquence peptidique de l'anticorps humanisé mais en respectant l'index hydropathique, c'est-à-dire leur hydrophobicité et leur charge, par exemple par substitution des acides aminés suivants: substitution arginine-lysine ou glutamate-aspartate ou sérine-thréonine ou glutamine-asparagine ou valine-leucine-isoleucine.

Un des buts de la description est de fournir des anticorps humanisés 12G4 mutés, ou des fragments de ceux-ci, possédant une affinité au moins égale à celle de l'anticorps chimérique correspondant non muté et une spécificité vis-à-vis du récepteur AMHR-II et ne déclenchant pas de réaction immune.

Un autre but est également de fournir des moyens de production desdits anticorps spécifiques du récepteur AMHR-II.

Un autre objet concerne l'utilisation de ces anticorps en tant que médicaments pour le traitement des cancers ovariens.

La présente description décrit un anticorps monoclonal humanisé 12G4 comprenant ou constitué:

a) d'une chaîne légère comprenant ou constituée :

- d'une région variable dont la séquence en acides aminés est représentée par SEQ ID NO:2 (sans leader) ou SEQ ID NO:4 (avec leader), et
- d'une région constante dont la séquence en acides aminés est représentée par SEQ ID NO:6 ou par une séquence présentant au moins 80% d'homologie avec la SEQ ID NO:6,

b) d'une chaîne lourde comprenant ou constituée :

- d'une région variable dont la séquence en acides aminés est représentée par SEQ ID NO: 8 (sans leader), ou SEQ ID NO: 10 (avec leader), et
- d'une région constante dont la séquence en acides aminés est représentée par SEQ ID NO: 12 ou par une séquence présentant au moins 80% d'homologie avec la SEQ ID NO:12,

lequel anticorps monoclonal humanisé 12G4 est muté, comprend au moins une mutation dans la chaîne légère et/ou lourde, et présente un $K_D$ pour le récepteur de type II de l'hormone anti-mullérienne humaine (AMHRII) au moins égale à celui de l'anticorps monoclonal chimérique 12G4 comprenant ou constitué :

- d'une région variable dont la séquence en acides aminés est représentée par SEQ ID NO:14 (sans leader), et
- d'une région constante dont la séquence en acides aminés est représentée par SEQ ID NO:6,

b) d'une chaîne lourde constituée :

- d'une région variable dont la séquence en acides aminés est représentée par SEQ ID NO: 18 (sans leader) ou SEQ ID NO: 10 (avec leader), et
- d'une région constante dont la séquence en acides aminés est représentée par SEQ ID NO: 12,

pour ledit récepteur, préférentiellement inférieur à $10^{-7}$M, notamment inférieur à $10^{-8}$M, en particulier compris de $10^{-9}$M à $10^{-11}$M.

La présente invention concerne un anticorps monoclonal muté liant spécifiquement le récepteur de type II de l'hormone anti-mullérienne humaine, possédant :

a) une chaîne légère constituée de la séquence en acides aminés représentée par:

- SEQ ID NO: 82 (sans leader) ou SEQ ID NO: 84 (avec leader), et

b) une chaîne lourde constituée de la séquence en acides aminés représentée par:

- SEQ ID NO: 86 (sans leader), ou SEQ ID NO: 88 (avec leader), (anticorps 3C_23K)

Les anticorps de la demande présentent aussi une affinité au moins égale à un tiers ou à la moitié de celle de l'anticorps 12G4 murin.

Dans toute la description, l'expression entre parenthèses « avec leader » après le numéro de séquence signifie que ladite séquence comporte le peptide signal ou la séquence codant pour le peptide signal, c'est-à-dire le peptide qui définit que la protéine va être secrétée.

Inversement, l'expression entre parenthèses « sans leader » signifie que ladite séquence ne comporte pas le peptide signal ou la séquence codant pour le peptide signal.

L'invention repose sur la constatation faite par les Inventeurs que des anticorps humanisés de 12G4 mutés de l'invention, bien que possédant au moins une mutation dans le CDR (trois régions déterminant la reconnaissance de l'antigène) dont l'index hydropathique n'est pas respecté, c'est-à-dire dans une région cruciale pour l'affinité et la liaison à l'antigène, et qui en règle générale ne supporte que des substitutions d'acides aminés de même index hydropathique (par exemple arginine-lysine, glutamate-aspartate, sérine-thréonine, glutamine-asparagine ou valine-leucine-isoleucine), possède toujours les propriétés suivantes:

- il présente pour un $K_D$ pour le récepteur AMHR-II (déterminé selon l'exemple
- 1, au moins similaire voire inférieur à celui de l'anticorps chimérique 12G4 correspondant et donc une affinité supérieure ou égale à celle de l'anticorps chimérique 12G4 correspondant, ou par rapport aux anticorps ou fragments d'anticorps de l'art antérieur.
- il présente une spécificité pour le récepteur AMHR-II,
- il ne déclenche pas de réaction immune ou une réaction moins importante que l'anticorps murin

A titre d'exemple, l'exemple 1 présente le $K_D$ obtenu avec des anticorps de l'invention produits dans des cellules CHO ou YB2/0,
Dans toute la description, le terme « 12G4 » et le terme « LFB112 » également utilisé, désignent la même chose et représentent le même anticorps.

L'affinité dudit anticorps peut être déterminée par un test BIAcore bien connu de l'homme du métier.

Dans l'invention, le terme « anticorps » se réfère à une immunoglobuline, protéine multimérique constituée de 4 chaînes participant à la réponse immunitaire acquise.

Les immunoglobulines sont bien connues de l'homme de métier et sont constituées d'un assemblage de deux dimères constitués chacun d'une chaîne lourde et d'une chaîne légère. Le complexe multimérique assemblé par la liaison d'une chaîne légère et d'une chaîne lourde par un pont disulfure entre deux cystéines, les deux chaînes lourdes étant elle-même également reliées entre elles par deux ponts disulfures.

Chacune des chaînes lourdes et des chaînes légères est constituée d'une région constante et d'une région variable. L'assemblage des chaînes qui composent un anticorps permet de définir une structure tridimensionnelle caractéristique en Y, où

- la base du Y correspond à la région constante Fc qui est reconnue par le complément et les récepteurs Fc, et
- l'extrémité des bras du Y correspondent à l'assemblage respectif des régions variables de la chaîne légère et variable de la chaîne lourde.

Plus précisément, chaque chaîne légère est constituée d'une région variable ($V_L$) et d'une région constante ($C_L$). Chaque chaîne lourde est constituée d'une région variable ($V_H$) et d'une région constante constituée de trois domaines constants $C_{H1}$, $C_{H2}$ et $C_{H3}$. Les domaines $C_{H2}$ et $C_{H3}$ composent le domaine Fc.

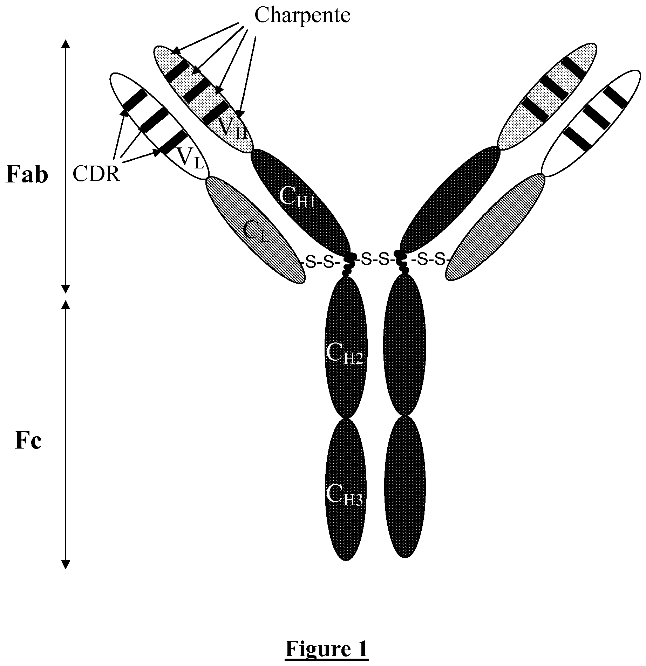

La structure d'un anticorps est représentée schématiquement dans la figure 1.

La région variable de la chaîne légère est constituée de trois régions déterminant la reconnaissance de l'antigène (CDR) entourées de quatre domaines charpentes. Le repliement tridimensionnel de la région variable est tel que les 3 CDR sont exposés du même coté de la protéine et permettent la formation d'une structure spécifique reconnaissant un antigène déterminé.

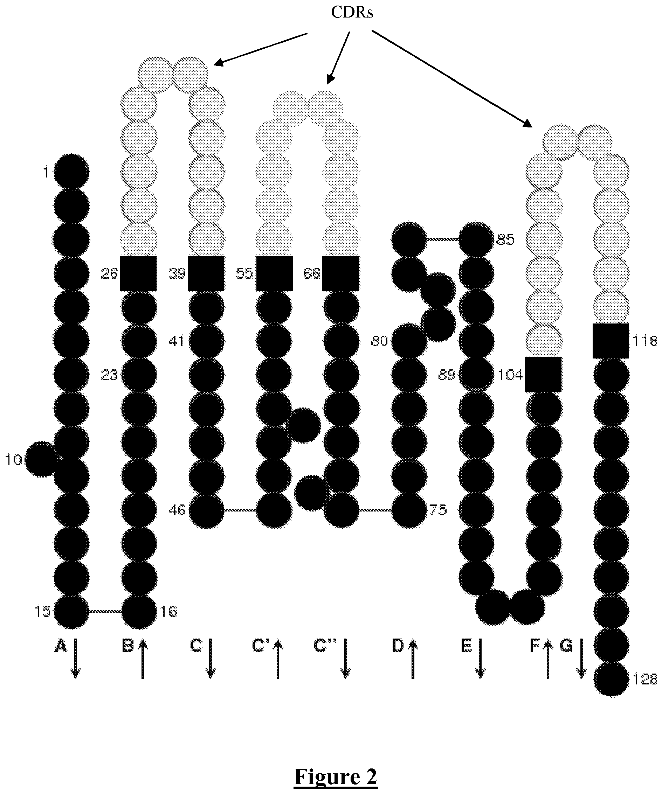

La structure en collier de perle d'une région variable d'une chaîne légère ou lourde d'un anticorps est représentée dans la figure 2.

Les anticorps décrits sont isolés et purifiés, et sont différents des anticorps naturels du fait qu'ils sont humanisés. Ces anticorps sont matures, c'est-à-dire qu'ils possèdent une structure tridimensionnelle ad hoc leur permettant de reconnaître l'antigène, et possèdent toutes les modifications post-traductionnelles essentielles à leur reconnaissance antigénique, notamment la glycosylation et la formation de ponts disulfures intra et inter moléculaires.

Il s'agit d'anticorps monoclonaux, c'est-à-dire qu'ils ne reconnaissent qu'un seul déterminant antigénique dans le récepteur AMHR-II, contrairement aux anticorps polyclonaux qui correspondent à un mélange d'anticorps, et donc peuvent reconnaître plusieurs déterminants antigéniques dans une même protéine.

Par « anticorps monoclonal chimérique », on entend un anticorps isolé dans lequel la séquence de chaque chaîne légère et/ou de chaque chaîne lourde de l'anticorps qui le constitue comprend ou consiste en une séquence hybride issue d'au moins deux animaux (ou homme) distincts.

Notamment l'anticorps chimérique 12G4 est un hybride souris/homme, ce qui signifie qu'une région de la séquence des chaînes légères et des chaînes lourdes est issue de la séquence d'une immunoglobuline de souris 12G4, et que le reste de la séquence desdites chaînes lourdes et des dites chaînes légères est issue de la séquence d'une, ou éventuellement plusieurs, immunoglobuline humaine.

La figure 18 présente la carte du vecteur d'expression permettant de produire l'anticorps chimérique 12G4.

Par «anticorps monoclonal humanisé 12G4», on entend un anticorps isolé dans lequel seuls les CDR de chaque chaîne légère et lourde de l'anticorps 12G4, notamment murin, ont été greffés dans les chaînes légère et lourdes d'un anticorps humain.

La figure 19 présente la carte du vecteur d'expression permettant de produire l'anticorps humanisé 12G4.

Dans toute la suite de la description, l'expression « anticorps chimérique 12G4» et « anticorps chimérique 12G4 non muté» désignent le même anticorps.

Par « anticorps monoclonal humanisé 12G4 muté», on entend un anticorps monoclonal humanisé 12G4 dans lequel au moins une mutation a été effectuée dans la région variable de la chaîne légère et/ou la région constante de la chaîne légère et/ou la région variable de la chaîne lourde ou la région constante de la chaîne lourde.

Ainsi, la définition de l'anticorps monoclonal humanisé muté de l'invention couvre à la fois :

- le précurseur de l'anticorps humanisé muté, notamment l'anticorps homme/souris tel que défini ci-dessus, et
- l'anticorps humanisé muté, notamment homme/souris, défini ci-dessus.

Dans un mode de réalisation avantageux, la présente demande concerne un anticorps monoclonal humanisé 12G4 muté tel que défini ci-dessus, comprenant au moins une mutation dans au moins un CDR de la région variable de la chaîne légère, et ayant une affinité pour ledit récepteur au moins égale à celle dudit anticorps monoclonal chimérique 12G4.

Dans ce mode de réalisation, lorsqu'une seule mutation est présente, elle se situe dans le CDR1 ou le CDR2 ou le CDR3.

Lorsque plus d'une mutation est présente, la deuxième ainsi que les autres peuvent se situer dans le CDR1 et/ou le CDR2 et/ou le CDR3 et/ou toute autre région de l'anticorps.

Dans un mode de réalisation avantageux, la présente demande concerne un anticorps monoclonal humanisé 12G4 muté tel que défini ci-dessus, comprenant de plus au moins une mutation dans les régions FR de la chaîne légère (VL). Les Inventeurs ont découvert que lorsque l'on effectue au moins une mutation dans le CDR1 ou le CDR2 ou le CDR3 de l'anticorps humanisé 12G4, et au moins une mutation dans la région variable, en particulier FR de la chaîne légère, lesdites au moins une mutation ne respectant pas nécessairement l'index hydropathique des acides aminés, permettent quand même, non seulement de conserver l'activité de l'anticorps humanisé mais encore d'obtenir un anticorps humanisé muté possédant une affinité au moins égale à celle de l'anticorps chimérique non muté, et ne provoquent pas de réaction immune ou moins importante

Dans un mode de réalisation avantageux, la présente demande concerne un anticorps monoclonal humanisé 12G4 muté tel que défini ci-dessus, comprenant au moins une mutation dans le CDR1 et au moins une mutation dans les régions FR de la chaîne légère (VL). Les Inventeurs ont découvert que lorsque l'on effectue au moins une mutation dans le CDR1 de l'anticorps humanisé 12G4, et au moins une mutation dans la région variable, en particulier FR de la chaîne légère, lesdites au moins une mutation ne respectant pas nécessairement l'index hydropathique des acides aminés, permettent quand même, non seulement de conserver l'activité de l'anticorps humanisé mais encore d'obtenir un anticorps humanisé muté possédant une affinité au moins égale à celle de l'anticorps chimérique non muté, et ne provoquent pas de réaction immune.

Dans un mode de réalisation avantageux, la présente demande concerne un anticorps monoclonal humanisé 12G4 muté tel que défini ci-dessus, comprenant au moins une mutation dans le CDR2 et au moins une mutation dans les régions FR de la chaîne légère (VL). Les Inventeurs ont découvert que lorsque l'on effectue au moins une mutation dans le CDR2 de l'anticorps humanisé 12G4, et au moins une mutation dans la région variable, en particulier FR de la chaîne légère, lesdites au moins une mutation ne respectant pas nécessairement l'index hydropathique des acides aminés, permettent quand même, non seulement de conserver l'activité de l'anticorps humanisé mais encore d'obtenir un anticorps humanisé muté possédant une affinité au moins égale à celle de l'anticorps chimérique non muté, et ne provoquent pas de réaction immune.

Dans un mode de réalisation avantageux, la présente demande concerne un anticorps monoclonal humanisé 12G4 muté tel que défini ci-dessus, comprenant au moins une mutation dans le CDR3 et au moins une mutation dans les régions FR de la chaîne légère (VL). Les Inventeurs ont découvert que lorsque l'on effectue au moins une mutation dans le CDR3 de l'anticorps humanisé 12G4, et au moins une mutation dans la région variable, en particulier FR de la chaîne légère, lesdites au moins une mutation ne respectant pas nécessairement l'index hydropathique des acides aminés, permettent quand même, non seulement de conserver l'activité de l'anticorps humanisé mais encore d'obtenir un anticorps humanisé muté possédant une affinité au moins égale à celle de l'anticorps chimérique non muté, et ne provoquent pas de réaction immune.

Dans un mode de réalisation avantageux, la présente demande concerne un anticorps monoclonal humanisé 12G4 muté tel que défini ci-dessus, ayant une ADCC contre des cellules, notammment Cov434, Asc 1 et META 2815, exprimant le récepteur AMHR II, notamment supérieure à l'ADCC contre les mêmes cellules. dudit anticorps monoclonal humanisé 12G4 non muté

L'ADCC (cytotoxicité cellulaire dépendante de l'anticorps) est un mécanisme dans lequel lorsque l'anticorps a reconnu un antigène, la partie Fc de l'anticorps est reconnue par un récepteur Fcγ d'une cellule tueuse qui, après liaison, est capable de tuer la cellule portant l'antigène. Les Inventeurs ont découvert que lorsque l'on effectue au moins une mutation dans l'un ou plus des CDR de l'anticorps humanisé 12G4, dont l'affinité est particulièrement diminuée par rapport à l'anticorps chimérique ou murin correspondant (exemple 2), et bien que le CDR corresponde à une région particulièrement importante de reconnaissance de l'antigène, ladite au moins une mutation permet non seulement de conserver l'activité de l'anticorps humanisé mais encore d'obtenir un anticorps humanisé muté possédant une affinité au moins égale à celle de l'anticorps chimérique non muté, et ne provoquent pas de réaction immune ou une réaction moins importante.

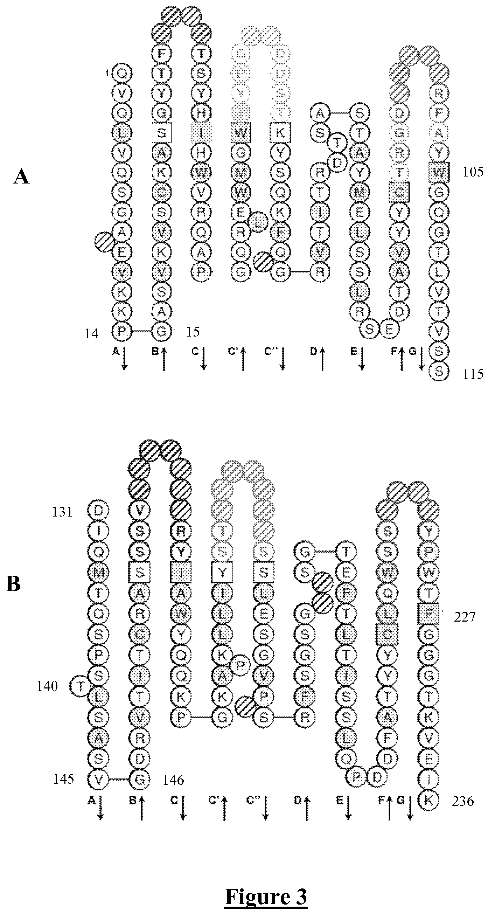

Dans le cadre de la présente demande la numérotation utilisée est basée sur la numérotation d'un fragment ScFv, la chaine lourde étant numérotée de 1 à 115 et la chaine légère de 131 à 236, telle que représentée dans les figures 3A et 3B pour l'anticorps humanisé 12G4 dans laquelle les perles grisées rayées correspondent à des acides aminés étant absents dans ladite séquence.

Les deux chaînes sont reliées entre elles par un linker comprenant les acides aminés 116 à 130.

La présente description décrit un anticorps monoclonal humanisé 12G4 muté tel que défini ci-dessus, dans lequel l'une au moins desdites mutations dans au moins un CDR de la région variable de la chaîne légère, est située dans le CDR compris dans la région contenant l'acide aminé 179 à l'acide aminé 184 de la région variable de la chaîne légère, dont la séquence en acides aminés est représentée par SEQ ID NO:2.

La région contenant l'acide aminé 179 à l'acide aminé 184 ne correspond pas au CDR complet.

Dans ce mode de réalisation, si l'anticorps ne possède qu'une mutation, elle est située dans la région du CDR de la chaîne légère contenant l'acide aminé 179 à l'acide aminé 184.

Il peut bien entendu posséder d'autres mutations dans d'autres CDR. Les Inventeurs ont découvert que lorsque l'on effectue au moins une mutation dans la région comprenant l'acide aminé 179 à 184 du CDR de l'anticorps humanisé 12G4, ladite au moins une mutation permet non seulement de conserver l'activité de l' anticorps humanisé mais encore d'obtenir un anticorps humanisé muté possédant une affinité au moins égale à celle de l'anticorps chimérique non muté et ne provoquent pas de réaction immune.

La présente description décrit un anticorps monoclonal humanisé 12G4 muté tel que défini ci-dessus, dans lequel l'une au moins desdites mutations située dans le CDR compris dans la région contenant l'acide aminé 179 à l'acide aminé 184 correspond à la substitution de l'un au moins des acides aminés suivants : S179P, E184K, E184G, E184D, S182F.

La notation utilisée ci correspond au code à une lettre bien connu de l'homme du métier.

La notation S179P signifie par exemple que l'acide aminé Sérine en position 179 est substitué par une Proline. Les Inventeurs ont découvert que lorsque l'on effectue au moins une mutation dans la région comprenant l'acide aminé 179 à 184 du CDR de l'anticorps humanisé 12G4, ladite au moins une mutation ne respectant pas nécessairement l'index hydropathique des acides aminés substitués permet quand même, non seulement de conserver l'activité de l' anticorps humanisé mais encore d'obtenir un anticorps humanisé muté possédant une affinité au moins égale à celle de l'anticorps chimérique non muté.

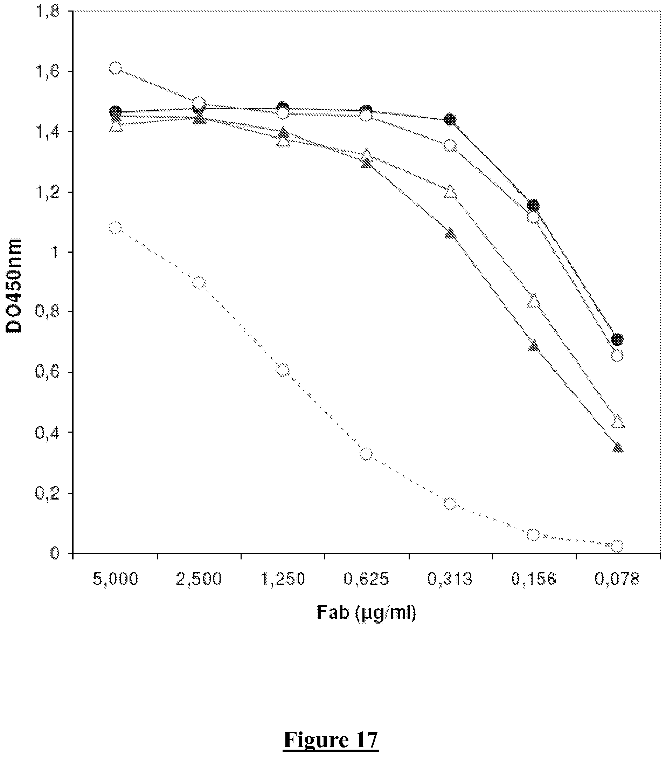

A titre d'exemple, la figure 17 présente l'affinité de liaison au récepteur AMHR-II d'anticorps humanisés mutés selon l'invention. L'anticorps 6B_78 ne possède qu'une mutation située dans le CDR de la région variable de la chaîne légère (E184K) dans laquelle un acide glutamique est remplacé par une lysine, soit le remplacement d'un acide aminé acide par un acide aminé basique, ayant par conséquent une charge totalement différente puisque opposée, et présentant pourtant toujours une activité mais surtout une affinité sensiblement meilleure que l'anticorps humanisé 12G4 non muté, et égale à celle de l'anticorps chimérique 12G4 non muté, et ne provoquent pas de réaction immune.

La présente description décrit un anticorps monoclonal humanisé 12G4 muté tel que défini ci-dessus, comprenant de plus au moins une mutation dans les régions FR de la chaîne légère (VL). Les Inventeurs ont découvert que lorsque l'on effectue au moins une mutation dans la région comprenant l'acide aminé 179 à 184 du CDR de l'anticorps humanisé 12G4, et au moins une mutation dans la région variable, en particulier FR de la chaîne légère, lesdites au moins une mutation ne respectant pas nécessairement l'index hydropathique des acides aminés permettent quand même, non seulement de conserver l'activité de l'anticorps humanisé mais encore d'obtenir un anticorps humanisé muté possédant une affinité au moins égale à celle de l'anticorps chimérique non muté, et ne provoquent pas de réaction immune.

Dans un mode de réalisation avantageux, la présente demande concerne un anticorps monoclonal humanisé 12G4 muté tel que défini ci-dessus, comprenant de plus au moins une mutation dans la chaîne lourde. Les Inventeurs ont découvert que lorsque l'on effectue au moins une mutation dans la région comprenant l'acide aminé 179 à 184 du CDR de l'anticorps humanisé 12G4, au moins une mutation dans la région variable, en particulier FR de la chaîne légère, et au moins une mutation dans la chaîne lourde, lesdites au moins une mutation ne respectant pas nécessairement l'index hydropathique des acides aminés, permettent quand même, non seulement de conserver l'activité de l' anticorps humanisé mais encore d'obtenir un anticorps humanisé muté possédant une affinité au moins égale à celle de l'anticorps chimérique non muté, et ne provoquent pas de réaction immune.

La présente description décrit un anticorps monoclonal humanisé 12G4 muté tel que défini ci-dessus, dans lequel l'une au moins desdites mutations dans les régions FR de la chaîne légère (VL) est située dans la région FR adjacente à la région contenant l'acide aminé 179 à l'acide aminé 184.

A titre d'exemple, la figure 17 et les tableaux I et VII (fixation à la cible AMHRII-Fc déterminée par Elisa) présentent l'affinité de liaison au récepteur AMHR-II d'anticorps humanisés mutés selon l'invention.

Ainsi l'anticorps 3C_23 possède trois mutations :

- une mutation dans le CDR de la région variable de la chaîne légère (S179P) dans laquelle une sérine est remplacée par une proline, soit le remplacement d'un acide aminé hydrophile par un acide aminé hydrophobe,
- une mutation dans la région variable, en particulier FR de la chaîne légère (I177T), soit le remplacement d'un acide aminé hydrophobe par un acide aminé hydrophile, possédant de plus une valeur d'index hydropathique, selon la demande internationale WO 2008/053330, totalement différente (+4,5 pour l'isoleucine et -0,7 pour la thréonine), et
- une mutation dans la chaîne lourde (Q3R), soit le remplacement d'une glutamine par une arginine dont la valeur d'index hydropathique, selon la demande internationale WO 2008/053330, varie de -3,5 pour la glutamine à -4,5 pour l'arginine,

et présentant pourtant une affinité sensiblement meilleure que celle de l'anticorps humanisé 12G4 non muté, et supérieure à celle de l'anticorps chimérique 12G4 non muté.

De même, l'anticorps 3C_23K qui, hormis les mutations de l'anticorps 3C_23 possède de plus une deuxième mutation dans le CDR de la région variable de la chaîne légère (E184K) dans laquelle un acide glutamique est remplacé par une lysine, c'est-à-dire le remplacement d'un acide aminé acide par un acide aminé basique ayant par conséquent une charge totalement différente puisque de signe opposé, présente pourtant toujours une activité mais surtout une affinité sensiblement meilleure que celle de l'anticorps humanisé 12G4 non muté, et supérieure à celle de l'anticorps chimérique 12G4 non muté, et ne provoque pas de réaction immune.

La présente description décrit un anticorps monoclonal humanisé 12G4 muté tel que défini ci-dessus, dans lequel l'une au moins desdites mutations dans les régions FR de la chaîne légère (VL) correspond à la substitution de l'un au moins des acides aminés suivants : I132T, A143T, T150A, S158P, L175Q, I177T, Y178H, V187A, S192T, G197D, F212S.

La présente description décrit un anticorps monoclonal humanisé 12G4 muté tel que défini ci-dessus, dans lequel l'une au moins desdites mutations dans la chaîne lourde correspond à la substitution de l'un au moins des acides aminés suivants:Q1E, Q3E, Q3R, Q6E, A9T, V11A, K12R, K13R, K19E, V20A, A24G, A24V, A24T, Q39E, A40V, S31G, L45P, D56N, A76T, A79T, R87G, T58A, Q62R, V67M, I70N, T74A, S77P, A79T, S88P, E89D, F102S, A103T, L110P, S114T.

La présente description décrit un anticorps monoclonal humanisé 12G4 muté tel que défini ci-dessus, possédant une chaîne légère et une chaîne lourde choisies parmi les suivantes:

a) une chaîne légère comprenant ou constituée d'une région variable dont la séquence en acides aminés est représentée par SEQ ID NO:2 dans laquelle une au moins substitution d'acides aminés suivante située dans l'un des CDR a été effectuée : S179P, E184K, E184G, E184D, S182F, ou

une chaîne légère comprenant ou constituée d'une région variable dont la séquence en acides aminés est représentée par SEQ ID NO:2 dans laquelle une au moins substitution d'acides aminés suivante située dans l'un des CDR a été effectuée : S179P, E184K, E184G, E184D, S182F, et une au moins substitution d'acides aminés suivante située dans les régions FR a été effectuée : I132T, A143T, T150A, S158P, L175Q, Y178H, V187A, S192T, G197D, F212S,

et d'une région constante dont la séquence en acides aminés est représentée par SEQ ID NO:6,

b) une chaîne lourde dont la séquence en acides aminés est représentée par SEQ ID NO:58 dans laquelle une substitution de l'un au moins des acides aminés suivants: Q1E, Q3E, Q3R, Q6E, A9T, V11A, K12R, K13R, K19E, V20A, A24G, A24V, A24T, Q39E, A40V, S31G, L45P, D56N, A76T, A79T, R87G, T58A, Q62R, V67M, I70N, T74A, S77P, A79T, S88P, E89D, F102S, A103T, L110P, S114T a été effectuée.

Le tableau VII de l'exemple 3 présente les différents clones obtenus et leur substitution. Il montre également que l'index hydropathique varie de manière importante en fonction des mutations, sans pour autant conduire à une perte d'activité et/ou d'affinité pour l'antigène et permet même pour certains clones d'obtenir une augmentation de l'affinité par rapport à l'anticorps chimérique correspondant (ratio Ac de l'invention/anticorps chimérique égal ou supérieur à 1).

Dans ce mode de réalisation, il est possible de constituer un anticorps qui est issu de la combinaison de deux anticorps préalablement obtenus et d'augmenter encore l'activité et l'affinité pour le récepteur AMHR-II par rapport à l'anticorps chimérique 12G4 non muté.

La présente description décrit un anticorps monoclonal humanisé 12G4 muté tel que défini ci-dessus, possédant une chaîne légère et une chaîne lourde choisies parmi les suivantes:

a) une chaîne légère comprenant ou constituée d'une région variable dont la séquence en acides aminés est représentée par:

- SEQ ID NO:22 (sans leader) ou SEQ ID NO:24 (avec leader), ou
- SEQ ID NO:30 (sans leader) ou SEQ ID NO:32 (avec leader), ou
- SEQ ID NO:34 (sans leader) ou SEQ ID NO:36 (avec leader), ou
- SEQ ID NO:46 (sans leader) ou SEQ ID NO:48 (avec leader),

et d'une région constante dont la séquence en acides aminés est représentée par SEQ ID NO:6,
b) une chaîne lourde comprenant ou constituée d'une région variable dont la séquence en acides aminés est représentée par:

- SEQ ID NO: 38 (sans leader), ou SEQ ID NO: 40 (avec leader),
- SEQ ID NO: 26 (sans leader), ou SEQ ID NO: 28 (avec leader),
- SEQ ID NO: 8 (sans leader), ou SEQ ID NO: 10 (avec leader),
- SEQ I NO: 42 (sans leader), ou SEQ ID NO: 44 (avec leader),
- SEQ ID NO: 50 (sans leader), ou SEQ ID NO: 52 (avec leader),

et d'une région constante dont la séquence en acides aminés est représentée par SEQ ID NO: 12.

La présente description décrit un anticorps monoclonal humanisé 12G4 muté, possédant :

a) une chaîne légère constituée de la séquence en acides aminés représentée par:

- SEQ ID NO: 70 (sans leader) ou SEQ ID NO: 72 (avec leader), et

b) une chaîne lourde constituée de la séquence en acides aminés représentée par:

- SEQ ID NO: 74 (sans leader), ou SEQ ID NO: 76 (avec leader), (anticorps 3C_23)

ou,

a) une chaîne légère constituée de la séquence en acides aminés représentée par:

- SEQ ID NO: 78 (sans leader) ou SEQ ID NO: 80 (avec leader), et

b) une chaîne lourde constituée de la séquence en acides aminés représentée par:

- SEQ ID NO: 58 (sans leader), ou SEQ ID NO: 60 (avec leader), (anticorps 6B_78)

ou,

a) une chaîne légère constituée de la séquence en acides aminés représentée par:

- SEQ ID NO: 78 (sans leader) ou SEQ ID NO: 80 (avec leader), et

b) une chaîne lourde constituée de la séquence en acides aminés représentée par:

- SEQ ID NO: 90 (sans leader), ou SEQ ID NO: 92 (avec leader), (anticorps 4C_35)

ou,

a) une chaîne légère constituée de la séquence en acides aminés représentée par:

- SEQ ID NO: 94 (sans leader) ou SEQ ID NO: 96 (avec leader), et

b) une chaîne lourde constituée de la séquence en acides aminés représentée par:

- SEQ ID NO: 98 (sans leader), ou SEQ ID NO: 100 (avec leader), (anticorps 5B_42)

Selon un autre aspect, la présente description décrit un fragment d'un anticorps monoclonal 12G4 humanisé muté tel que défini ci-dessus, choisi parmi le groupe de fragments constitué de : Fv, Fab, F(ab')2, Fab', dsFv, scFv, sc(Fv)$_2$, "diabodies".

Selon un autre aspect, la demande concerne un acide nucléique comprenant ou constitué d'une séquence codant la chaîne légère d'un anticorps monoclonal défini ci-dessus et/ou comprenant ou constitué d'une séquence codant la chaîne lourde de l'anticorps monoclonal défini ci-dessus.

La présente description décrit un acide nucléique défini ci-dessus, dans lequel la séquence codant la chaîne légère comprend ou est constituée des séquences suivantes :

a) une séquence codant la région variable de la chaîne légère représentée par SEQ ID NO : 53 dans laquelle une substitution d'au moins un codon permettant la substitution, dans l'un des CDR, d'un ou plus acides aminés suivants: S179P, E184K, E184G, E184D, S182F a été effectuée,

ou,

b) une séquence codant la région variable de la chaîne légère représentée par SEQ ID NO : 53 dans laquelle :

- au moins une substitution d'un codon permettant la substitution, dans l'un des CDR, d'un ou plus acides aminés suivants: S179P, E184K, E184G, E184D, S182F a été effectuée, et
- au moins une substitution d'au moins un codon permettant la substitution, dans l'un des FR, d'un ou plus acides aminés suivants: I132T, A143T, T150A, S158P, L175Q, Y178H, V187A, S192T, G197D, F212S, a été effectuée,

et une séquence codant la région constante représentée par SEQ ID NO : 5,

La présente description décrit un acide nucléique défini ci-dessus, dans lequel la séquence codant la chaîne lourde comprend ou est constituée des séquences suivantes :

a) SEQ ID NO : 57 dans laquelle une substitution d'au moins un codon permettant la substitution d'un ou plus acides aminés suivants: Q1E, Q3E, Q3R, Q6E, A9T, V11A, K12R, K13R, K19E, V20A, A24G, A24V, A24T, Q39E, A40V, S31G, L45P, D56N, A76T, A79T, R87G, T58A, Q62R, V67M, I70N, T74A, S77P, A79T, S88P, E89D, F102S, A103T, L110P, S114T a été effectuée.

Dans un mode de réalisation avantageux, la demande concerne un acide nucléique défini ci-dessus, comprenant une chaîne légère définie ci-dessus et une chaîne lourde définie ci-dessus.

La présente description décrit un acide nucléique défini ci-dessus, dans lequel la séquence codant la chaîne légère comprend ou est constituée d'une séquence codant une région variable et d'une séquence codant une région constante choisies parmi les suivantes:

a) région variable :

- SEQ ID NO:21 (sans leader) ou SEQ ID NO:23 (avec leader), ou
- SEQ ID NO:29 (sans leader) ou SEQ ID NO:31 (avec leader), ou
- SEQ ID NO:33 (sans leader) ou SEQ ID NO:35 (avec leader), ou
- SEQ ID NO:45 (sans leader) ou SEQ ID NO:47 (avec leader), ou

b) région constante

- SEQ ID NO: 5.

La présente description décrit un acide nucléique défini ci-dessus, dans lequel la séquence codant la chaîne lourde comprend ou est constituée d'une séquence codant une région variable et d'une séquence codant une région constante choisies parmi les suivantes:

a) région variable :

- SEQ ID NO: 25 (sans leader) ou SEQ ID NO: 27 (avec leader), ou

- SEQ ID NO: 7 (sans leader) ou SEQ ID NO: 9 (avec leader), ou
- SEQ ID NO: 37 (sans leader) ou SEQ ID NO: 39 (avec leader), ou
- SEQ ID NO: 41 (sans leader) ou SEQ ID NO: 43 (avec leader), ou
- SEQ ID NO: 49 (sans leader) ou SEQ ID NO: 51 (avec leader),

b) région constante

- SEQ ID NO: 11.

Dans un mode de réalisation avantageux, l'invention concerne un acide nucléique défini ci-dessus, dans lequel la séquence codant la chaîne légère est choisie parmi les séquences suivantes :

a) séquence codant la chaîne légère SEQ ID NO: 81 (sans leader) ou SEQ ID NO: 83 (avec leader), et
b) séquence codant la chaîne lourde SEQ ID NO: 85 (sans leader) ou SEQ ID NO: 87 (avec leader),
(anticorps 3C_23K)

La présente description décrit un acide nucléique défini ci-dessus, dans lequel la séquence codant la chaîne légère est choisie parmi les séquences suivantes :

- SEQ ID NO: 69 (sans leader) ou SEQ ID NO: 71 (avec leader), ou
- SEQ ID NO: 77 (sans leader) ou SEQ ID NO: 79 (avec leader), ou
- SEQ ID NO:93 (sans leader) ou SEQ ID NO:95 (avec leader),

et la séquence codant la chaîne lourde est choisie parmi les séquences suivantes :

- SEQ ID NO: 73 (sans leader) ou SEQ ID NO:75 (avec leader), ou
- SEQ ID NO: 57 (sans leader) ou SEQ ID NO:59 (avec leader), ou
- SEQ ID NO: 89 (sans leader) ou SEQ ID NO:91 (avec leader), ou
- SEQ ID NO: 97 (sans leader) ou SEQ ID NO:99 (avec leader),

La présente description décrit un acide nucléique défini ci-dessus, dans lequel la séquence codant la chaîne légère et la séquence codant la chaîne lourde sont les suivantes :

a) séquence codant la chaîne légère SEQ ID NO: 69 (sans leader) ou SEQ ID NO: 71 (avec leader), et
b) séquence codant la chaîne lourde SEQ ID NO: 73 (sans leader) ou SEQ ID NO: 75 (avec leader),
(anticorps 3C_23)

ou,

a) séquence codant la chaîne légère SEQ ID NO: 77 (sans leader) ou SEQ ID NO: 79 (avec leader), et
b) séquence codant la chaîne lourde SEQ ID NO: 57 (sans leader) ou SEQ ID NO: 59 (avec leader),
(anticorps 6B_78)

ou,

a) séquence codant la chaîne légère SEQ ID NO: 77 (sans leader) ou SEQ ID NO: 79 (avec leader), et
b) séquence codant la chaîne lourde SEQ ID NO: 89 (sans leader) ou SEQ ID NO: 91 (avec leader),
(anticorps 4C_35)

ou,

a) séquence codant la chaîne légère SEQ ID NO: 93 (sans leader) ou SEQ ID NO: 95 (avec leader), et
b) séquence codant la chaîne lourde SEQ ID NO: 97 (sans leader) ou SEQ ID NO: 99 (avec leader),
(anticorps 5B_42)

Selon un autre aspect, la demande concerne un vecteur d'expression comprenant au moins un acide nucléique défini ci-dessus, ledit acide nucléique étant sous contrôle des éléments permettant son expression.
Par « vecteur d'expression », est défini une molécule d'ADN qui possède des éléments permettant sa réplication (duplication) dans au moins un organisme vivant. Ces éléments permettant la réplication sont notamment des origines de réplication de levure ou de bactéries, ou encore des éléments de contrôle de la réplication d'un virus.

Les vecteurs selon la demande sont notamment des plasmides, des phages, des chromosomes artificiels de levure (YAC), des chromosomes artificiels de bactéries (BAC), les génomes modifiés de virus réplicatifs ou de virus intégratifs ...

Ces vecteurs sont dits « d'expression » car ils disposent de séquences nucléotidiques qui permettent l'expression, c'est-à-dire la transcription en ARN, des séquences nucléotidiques qu'elles contrôlent.

Dans la demande ladite séquence d'acide nucléique contenue dans ledit vecteur est placée « sous contrôle des éléments permettant son expression ». Cela signifie que ledit vecteur d'expression possède au moins une séquence d'initiation de la transcription tel qu'un promoteur d'un virus comme le promoteur précoce du virus simien SV40, ou du Cytomégalovirus (CMV) ou encore les séquences promotrices du virus du sarcome de Rous (RSV), et notamment une séquence ou promoteur comprenant une boite TATAA. De plus, ledit vecteur possède également au moins une séquence de terminaison de la transcription, et en particulier une séquence de polyadénylation issues d'un gène de mammifère, notamment humain.

A ces séquences indispensables pour l'expression de la séquence nucléotidique contenue dans ledit vecteur peuvent s'ajouter d'autres séquences permettant de réguler ou de moduler l'expression de la dite séquence. Une liste non exhaustive comprend : des introns de gènes de mammifères, et notamment humains, des séquences de régulation de transcription de type amplificateur (« enhancers ») ou encore des séquences transcrites mais non traduites de gènes de mammifères, et notamment humains.

Un mode de réalisation avantageux de l'invention concerne un vecteur d'expression tel que défini ci-dessus, codant pour l'anticorps tel que revendiqué et comprenant au moins un acide nucléique choisi parmi les acides nucléiques comprenant les séquences suivantes SEQ ID NO 83, 87

Dans un autre mode de réalisation avantageux, l'invention concerne un vecteur d'expression tel que défini ci-dessus, comprenant

- un premier acide nucléique choisi parmi les acides nucléiques de séquences suivantes : SEQ ID NO 83, ledit premier acide nucléique étant sous contrôle des éléments permettant son expression, et
- un second acide nucléique choisi parmi les acides nucléiques de séquences suivantes : SEQ ID NO 87, ledit second acide nucléique étant sous contrôle des éléments permettant son expression.

Ce vecteur d'expression comporte donc deux séquences d'acides nucléiques susmentionnées, et plus particulièrement comporte une séquence d'acide nucléique codant la chaîne légère de l'anticorps monoclonal défini ci-dessus, et une séquence d'acide nucléique codant la chaîne lourde de l'anticorps monoclonal défini ci-dessus.

Préférentiellement, ledit vecteur d'expression contient un premier élément permettant l'expression de la séquence d'acide nucléique codant la chaîne légère de l'anticorps monoclonal défini ci-dessus et un second élément permettant l'expression de la séquence d'acide nucléique codant la chaîne lourde de l'anticorps monoclonal défini ci-dessus, ledit premier et ledit second élément permettant l'expression desdites séquences d'acides nucléiques étant identiques ou différents, et préférentiellement identiques. Ces éléments de contrôle sont notamment les séquences terminales longues répétées (LTR) du virus RSV.

Un autre mode de réalisation concerne un vecteur d'expression défini ci dessus, comprenant au moins un gène de résistance à un antibiotique.

Par « au moins un gène de résistance » on entend que ledit vecteur d'expression peut contenir 1 ou 2, ou 3 ou 4 ou 5 ou 6 gènes de résistance à un antibiotique.

Par « gène de résistance à un antibiotique », on définit un gène dont le produit d'expression exerce un effet cytostatique (inhibition de la croissance) ou cytolytique (mort cellulaire) de cellules. Les antibiotiques concernés par l'invention ont notamment un effet sur les cellules procaryotes, mais peuvent également avoir un effet sur les cellules eucaryotes, qu'il s'agisse de levures, de plantes, d'insectes, d'amphibiens ou de mammifères.

Plus particulièrement, le vecteur d'expression susmentionné possède un gène de résistance à un antibiotique spécifique des cellules procaryotes et au moins un gène, préférentiellement 2 gènes, de résistance à un antibiotique spécifique des cellules eucaryotes.

On peut citer comme antibiotiques spécifiques des cellules procaryotes : l'Ampiciline, la Tétracycline et ses dérives, l'Hygromycine, la Canamycine... On peut citer comme antibiotiques spécifiques des cellules eucaryotes : le G418, la Généticine (sels de G418), la Puromycine, le Méthotrexate, la Blasticidine...

Les Unités transcriptionnelles (UT) d'intérêt codant pour la chaîne lourde et la chaîne légère sont clonés sous la forme d'ADNc et sous la dépendance du promoteur RSV. Ce promoteur correspond au LTR (Long Terminal Repeat) du virus du sarcome de Rous qui contient un élément enhancer dans sa région 5'.

Est cloné immédiatement en 3' du promoteur un intron artificiel optimisé pour l'épissage alternatif et composé d'une séquence donneuse en 5' isolé de la beta-globine humaine et en a 3' d'une séquence acceptrice dérivée du gène de variable de la chaîne lourde d'immunoglobuline. Les UT d'intérêt se terminent par des séquences de polyadénylation dérivées du gène de l'hormone de croissance (GH) d'origine humaine (hGH) pour la chaîne lourde et bovine (bGH) pour la chaîne légère. Cette différence d'origine dans le choix des polyA est réalisée dans un but de limiter les recombinaisons entre les gènes d'intérêt. Cette association promoteur LTRRSV, intron chimérique, ADNc et séquence polyA a été sélectionné car elle confère une haute activité transcriptionnelle et traductionnelle dans la lignée cellulaire YB2/0.

Le vecteur d'expression contient en plus des UT d'intérêt plusieurs UT de résistances à des molécules chimiques:

gene Bla: Ce gène (nommé Amp dans les cartes de restriction des vecteurs) exprime l'enzyme beta-lactamase chez la bactérie (promoteur procaryote) et confère une résistance à l'ampicilline.

gene Neo: Ce gène code pour l'enzyme npt II (neomycin-phosphotransferase II) sous le contrôle du promoteur SV40 et confère une résistance à divers antibiotiques comme la néomycine, kanamycine ou G418 aux cellules de mammifère transfectées et exprimant ce gène.

gene Dhfr: Ce gène code pour l'enzyme DHFR (DiHydroFolate Reductase) sous le contrôle du promoteur SV40 et confère une résistance au méthotrexate (MTX). Ce procédé peut être utilisé pour réaliser de l'amplification génique en augmentant la concentration de MTX résultant ainsi d'une augmentation de la production d'anticorps par les cellules transfectées.

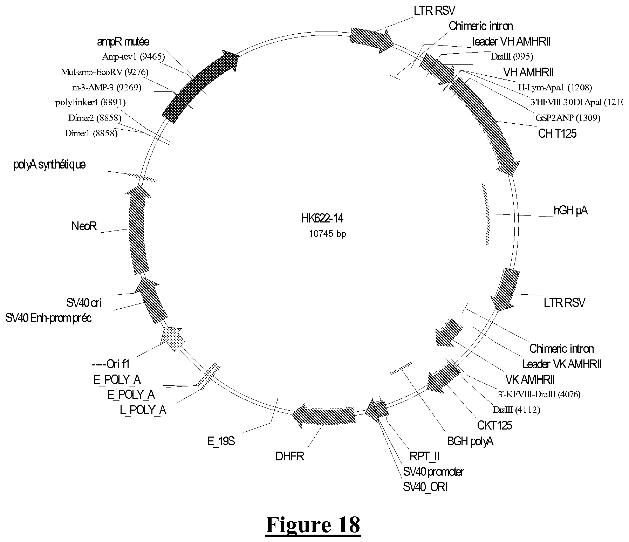

Les figures 18 à 22 présentent les cartes des vecteurs d'expression utilisés pour produire les clones 3C_23, 6B_78, 3C_23K, 4C_35 et 5B_42.

Dans un autre aspect, la présente demande concerne une cellule hôte ou lignée cellulaire transformée par un acide nucléique défini ci-dessus et/ou un vecteur d'expression défini ci-dessus. En particulier, ladite cellule ou lignée cellulaire est caractérisée en ce qu'elle

- présente une apoptose inférieure à 25%,
- est stable au cours des divisions cellulaires, et
- sécrète au moins 14 $\mu$g/ml d'anticorps monoclonal défini précédemment.

La notion de stabilité cellulaire implique que les cellules issue du clonage des cellules clonées issues des cellules contenant au moins un vecteur permettant l'expression d'un anticorps monoclonal selon l'invention sont capables au cours des différentes divisions de conserver leurs propriétés de résistances aux antibiotique et de produire les anticorps monoclonaux.

Dans encore un autre aspect, l'invention concerne une composition pharmaceutique, et notamment vaccinale, comprenant au moins

- un anticorps monoclonal tel que défini dans les revendications, ou
- un acide nucléique tel que défini dans les revendications, ou
- un vecteur tel que défini dans les revendications, ou

en association avec un véhicule pharmaceutiquement acceptable.

Avantageusement, l'invention concerne une composition pharmaceutique, et notamment vaccinale, comprenant au moins un anticorps monoclonal tel que défini dans les revendications, en association avec un véhicule pharmaceutiquement acceptable.

Le dosage de la substance active dépend particulièrement du mode d'administration, et est aisément déterminé par l'homme de métier.

« Un véhicule pharmaceutiquement acceptable » réfère à un matériel non toxique qui est compatible avec un système biologique tel qu'une cellule, une culture cellulaire, un tissu ou un organisme.

Une quantité thérapeutiquement (dose unitaire) efficace peut varier de 0,01 mg/kg à 500 mg/kg, préférablement de 0,1 mg/kg à 500 mg/kg, préférablement de 0,1 mg/kg à 100 mg/kg, préférablement de 0,1 mg/kg à 20 mg/kg, préférablement de 0,1 mg/kg à 10 mg/kg, et plus préférablement de 1 mg/kg à 10 mg/kg, en une ou plusieurs administrations hebdomadaire, pendant plusieurs semaines ou mois.

De même, une quantité thérapeutiquement (dose unitaire) efficace peut varier de 0,2 mg/m$^2$ à 10 g/m$^2$, préférablement de 0,2 mg/m$^2$ à 1 g/m$^2$, préférablement de 2 mg/m$^2$ à 1 g/m$^2$, préférablement de 20 mg/m$^2$ à 1 g/m$^2$, et plus préférablement de 20 mg/m$^2$ à 0,5 g/m$^2$, en une ou plusieurs administrations hebdomadaire, pendant plusieurs semaines ou mois.

La composition pharmaceutique de l'invention peut être administrée notamment par voie intraveineuse, notamment par injection ou par perfusion graduelle, par voie sous-cutanée, par voie systémique, par voie locale au moyen d'infiltrations, per os, ou par voie respiratoire ou pulmonaire au moyen d'aérosol.

Les préparations pour une administration parentérale peuvent inclure des solutions aqueuses ou non-aqueuses stériles, des suspensions ou des émulsions. Des exemples de solvants non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

La forme galénique avantageuse de la composition pharmaceutique de l'invention est administrable par voie orale et comprenant

- un anticorps monoclonal tel que défini dans les revendications, ou
- un acide nucléique tel que défini dans les revendications, ou
- un vecteur d'expression tel que défini dans les revendications, ou

en association avec un excipient, en présence ou non d'un agent de propulsion.

Dans un mode de réalisation de l'invention, l'aérosol se présente sous forme d'un liquide contenant l'anticorps humanisé muté et un excipient. Les excipients sont le plus souvent des alcools, mais tout autre excipient connu de l'homme du métier pourra être utilisé dans le cadre de l'invention. L'aérosol sous forme liquide peut être associé à un gaz propulseur comme les chlorofluorocarbones (CFC) ou hydrofluorocarbones (HFA).

L'aérosol sous forme liquide peut aussi être constitué de microparticules lipidiques et d'un excipient. Dans ce cas, les excipients peuvent être choisis parmi le dipalmitoylphosphatidylcholine de synthèse (DPPC), le lactose ou l'hydroxyethylamidon (HES). Les microparticules sont ensuite administrées à l'aide d'un insufflateur.

Dans un autre mode de réalisation de l'invention, l'aérosol se présente sous forme de poudre. La poudre se compose de particules de taille comprise entre 1 et 10 $\mu$m et de préférence inférieure à 9$\mu$m, ou de préférence inférieure à 5 $\mu$m. A titre d'exemple non limitatif, les méthodes suivantes peuvent être utilisées pour obtenir une poudre sèche : la pulvérisation accompagnée d'une dessiccation par congélation ou la cristallisation par ultrasons, la précipitation dirigée.

L'administration de l'aérosol sera réalisée selon qu'il se présente sous forme liquide ou solide à l'aide d'un nébuliseur qui peut être pneumatique, ultrasonique ou à tamis ou à l'aide d'un aérosol doseur (de liquide pressurisé, mécanique, électrohydrodynamique, thermiques) pour les formulations liquides ou à l'aide d'inhalateur pour les formulations solides. (ReychlerG., Dessanges JF et Vecellio L, Rev. Mal. Respir, 2007 ; 24 :1013-1023).

Selon un autre aspect, la demande concerne un produit comprenant une première préparation pharmaceutique comprenant un anticorps monoclonal défini ci-dessus, et une seconde préparation pharmaceutique comprenant un composé anticancéreux conventionnel, notamment du paclitaxel ou un sel de platine, en particulier de l'oxaloplatine, du cisplatine ou de la carboplatine, en tant que préparation combinée pour une utilisation simultanée, séparée ou séquentielle dans le traitement de patients atteints de pathologie associée au récepteur de type II de l'hormone anti-mullérienne humaine, notamment

le cancer ovarien, en particulier le cancer de l'ovaire métastatique, le cancer séreux, l'hypernéphrome, l'endométrioïde, l'épithélium colloïde.

Selon un autre aspect, la présente description décrit un produit comprenant une première préparation pharmaceutique comprenant un anticorps monoclonal défini ci-dessus, et une seconde préparation pharmaceutique comprenant un composé anticancéreux conventionnel, notamment du paclitaxel ou un sel de platine, en particulier de l'oxaloplatine, du cisplatine ou de la carboplatine, en tant que préparation combinée pour une utilisation simultanée, séparée ou séquentielle dans le traitement de patients atteints de pathologie associée au récepteur de type II de l'hormone anti-mullérienne humaine, notamment

le cancer de la prostate,

le cancer des cellules germinales,

le cancer de l'endomètre,

la tumeur maligne mullérienne mixte de l'utérus,

le leiomyosarcome,

le sarcome stromal de l'endomètre.

Selon un autre aspect, l'invention concerne l'utilisation d'au moins :

- un anticorps monoclonal tel que défini dans les revendications, ou
- un acide nucléique tel que défini dans les revendications, ou
- un vecteur tel que défini dans les revendications, ou
- une cellule telle que définie dans les revendications, s, pour la préparation d'un médicament destiné au traitement ou la prévention d'un cancer ovarien associé au récepteur de type II de l'hormone anti-mullérienne humaine, en particulier le cancer de l'ovaire métastatique, le cancer séreux, l'hypernéphrome, l'endométrioïde, l'épithélium colloïde.

Par traitement, on entend le moyen de soigner une pathologie déclarée, dont les symptômes sont visibles. Par prévention, on entend le moyen d'empêcher ladite pathologie de se déclarer.

Dans un mode de réalisation avantageux, la demande concerne l'utilisation d'un anticorps défini ci-dessus, ou d'un fragment de celui-ci défini ci-dessus, pour le diagnostic et/ou le suivi du cancer ovarien.

Dans un mode de réalisation avantageux, la demande concerne l'utilisation d'un anticorps défini ci-dessus, ou d'un fragment de celui-ci défini ci-dessus, comprenant de plus un anticancéreux conventionnel, notamment du paclitaxel ou un sel de platine, en particulier de l'oxaloplatine, du cisplatine ou de la carboplatine.

Selon un autre aspect, l'invention concerne :

- un anticorps monoclonal tel que défini dans les revendications, ou
- un acide nucléique tel que défini dans les revendications, ou
- un vecteur tel que défini dans les revendications, ou
- une cellule tel que définie dans les revendications, pour son utilisation dans le traitement ou la prévention d'un cancer ovarien associé au récepteur de type II de l'hormone anti-mullérienne humaine, notamment :

  le cancer de l'ovaire métastatique, le cancer séreux, l'hypernéphrome, l'endométrioïde, l'épithélium colloïde,

Dans un mode de réalisation avantageux, l'anticorps monoclonal tel que défini dans les revendications est utilisé pour le diagnostic et/ou le suivi d'un cancer ovarien associé au récepteur de type II de l'hormone anti-mullérienne humaine, notamment :

  le cancer de l'ovaire métastatique, le cancer séreux, l'hypernéphrome, l'endométrioïde, l'épithélium colloïde,

Dans un mode de réalisation avantageux, l'anticorps monoclonal défini ci-dessus ou l'acide nucléique défini ci-dessus ou le vecteur défini ci-dessus ou la cellule définie ci-dessus, comprend de plus un anticancéreux conventionnel, notamment du paclitaxel ou un sel de platine, en particulier de l'oxaloplatine, du cisplatine ou de la carboplatine.

Selon un autre aspect, la demande concerne un kit comprenant au moins :

- un anticorps monoclonal tel défini ci-dessus, ou
- un acide nucléique tel défini ci-dessus, ou
- un vecteur tel défini ci-dessus, ou
- une cellule tel définie ci-dessus,

pour une utilisation dans le diagnostic d'une pathologie associée au récepteur de type II de l'hormone anti-mullérienne humaine, notamment le cancer ovarien.

Selon un autre aspect, la demande concerne un procédé de diagnostic d'un cancer ovarien associé au récepteur de type II de l'hormone anti-mullérienne humaine, sur un échantillon biologique humain, comprenant les étapes suivantes :

  a. marquage d'une biopsie préalablement prélevée sur un patient,
  b. détermination de la présence d'un récepteur de type II de l'hormone anti-mullérienne humaine.

Selon un autre aspect, la demande concerne un procédé de diagnostic d'un cancer ovarien associé au récepteur de type II de l'hormone anti-mullérienne humaine, sur un échantillon biologique humain, comprenant les étapes suivantes :

  a. mise en oeuvre d'une biopsie sur un patient,
  b. marquage de la biopsie,
  c. détermination de la présence d'un récepteur de type II de l'hormone anti-mullérienne humaine,

Le marquage de la biopsie est effectué selon des techniques bien connues de l'homme du métier.

La détermination de la présence du récepteur peut être effectué par des techniques bien connues de l'homme du métier, telles que un dosage immunologique, du binding...

Le marquage de la biopsie est effectué selon des techniques bien connues de l'homme du métier.

La détermination de la présence du récepteur peut être effectué par des techniques bien connues de l'homme du métier, telles que un dosage immunologique, du binding...

Selon un autre aspect, l'invention concerne un procédé de traitement d'un cancer ovarien associé au récepteur de type II de l'hormone anti-mullérienne humaine, sur un échantillon biologique humain, comprenant les étapes suivantes :

a. mise en oeuvre d'une biopsie sur un patient,

b. marquage de la biopsie,

c. détermination de la présence d'un récepteur de type II de l'hormone anti-mullérienne humaine,

d. si la présence d'un récepteur de type II de l'hormone anti-mullérienne humaine est déterminée, traitement du patient par :

    i. un anticorps monoclonal tel que défini dans les revendications, ou

    ii. un acide nucléique tel que défini dans les revendications, ou

    iii. un vecteur tel que défini dans les revendications, ou

    iv. une cellule telle que définie dans les revendications.

**DESCRIPTION DES FIGURES**

La **figure 1** correspond à une représentation schématique d'un anticorps. Les parties noires correspondent aux parties constantes des chaînes lourdes, les parties en gris foncé correspondent à la partie constante de la chaîne légère, les parties en gris clair correspondent à la partie variable de la chaîne lourde, et les parties blanches correspondent à la partie variable de la chaîne légère. -S-S- représente les ponts disulfures établis entre deux cystéines. Les régions CDR et charpentes sont indiquées par des flèches. Les fragments Fab et Fc sont également représentés.

La **figure 2** correspond à une représentation schématique en collier de perles de la séquence d'acides aminés d'une partie variable d'une chaîne légère ou d'une chaîne lourde d'immunoglobuline. Les boules noires correspondent aux acides aminés formant les régions charpentes, et les boules grises correspondent aux acides aminés représentant les CDR.

La **figure 3** correspond à une représentation schématique en collier de perles de la séquence d'acides aminés de la partie variable de la chaîne lourde (figure 3A : acides aminés 1-115, SEQ ID NO : 8) et de la chaîne légère (figure 3B : acides aminés 131-236, SEQ ID NO :2) de l'anticorps 12G4 humanisé avec la numérotation adoptée pour déterminer la position des mutations. Les perles en grisé rayé correspondent à des acides aminés non présents dans la séquence et qui ne sont donc pas comptabilisés dans la numérotation.

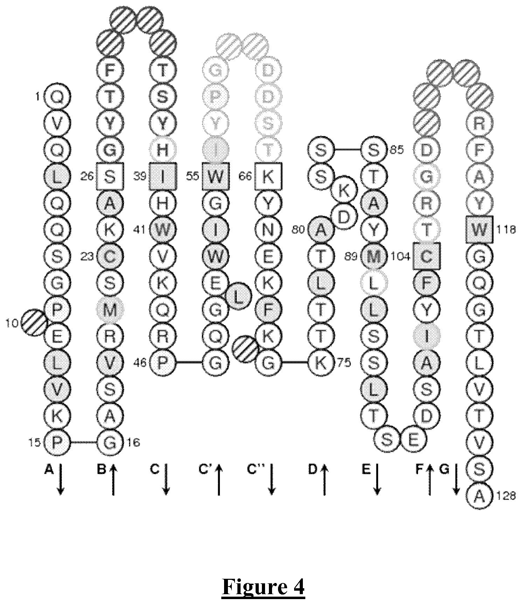

La **figure 4** correspond à une représentation schématique en collier de perles de la séquence d'acides aminés de la partie variable de la chaîne lourde de l'anticorps 12G4 chimérique (SEQ ID NO: 66).

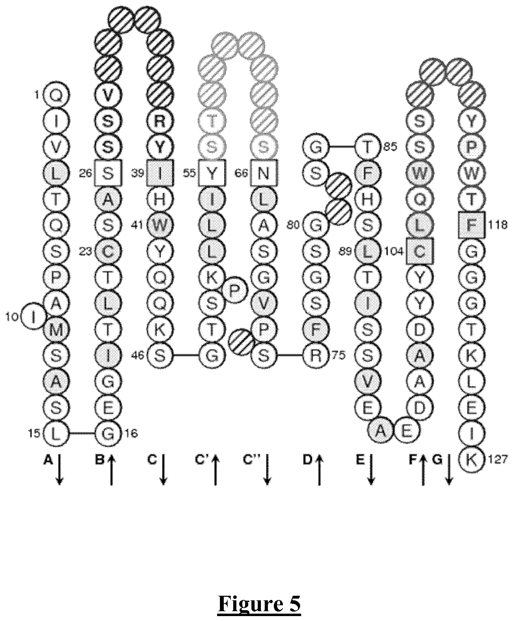

La **figure 5** correspond à une représentation schématique en collier de perles de la séquence d'acides aminés de la partie variable de la chaîne légère de l'anticorps 12G4 chimérique (SEQ ID NO: 62).

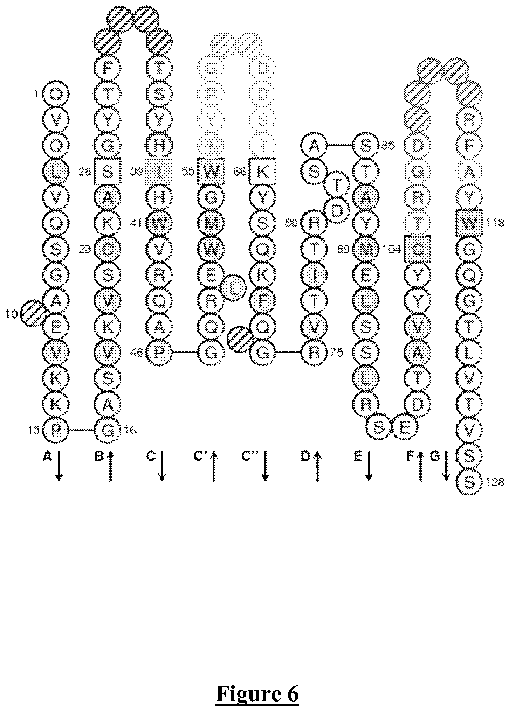

La **figure 6** correspond à une représentation schématique en collier de perles de la séquence d'acides aminés de la partie variable de la chaîne lourde de l'anticorps 12G4 humanisé non muté (SEQ ID NO: 58) et de l'anticorps humanisé 12G4 muté (6B_78; SEQ ID NO: 58).

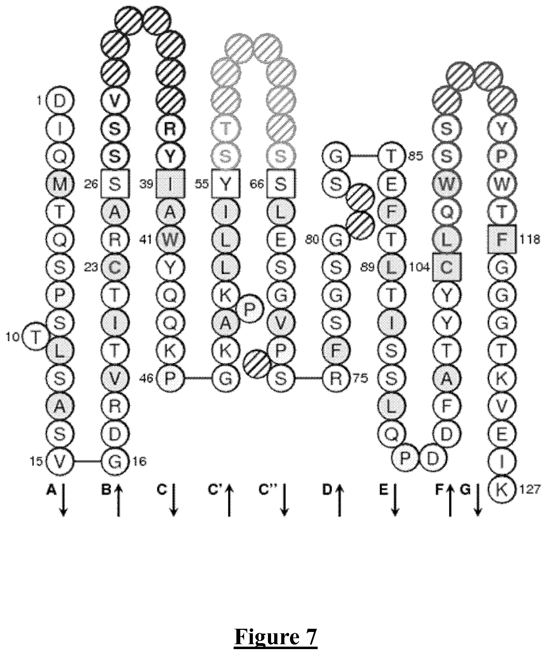

La **figure 7** correspond à une représentation schématique en collier de perles de la séquence d'acides aminés de la partie variable de la chaîne légère de l'anticorps 12G4 humanisé non muté (SEQ ID NO: 54).

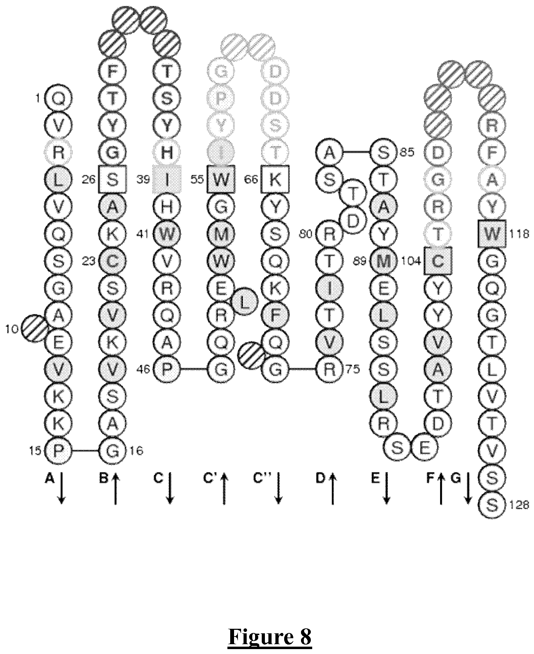

La **figure 8** correspond à une représentation schématique en collier de perles de la séquence d'acides aminés de la partie variable de la chaîne lourde de l'anticorps 12G4 humanisé muté (3C_23; SEQ ID NO: 74).

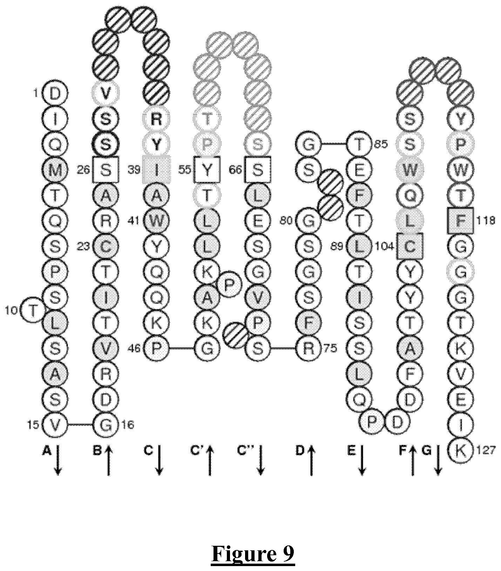

La **figure 9** correspond à une représentation schématique en collier de perles de la séquence d'acides aminés de la partie variable de la chaîne légère de l'anticorps 12G4 humanisé muté (3C_23; SEQ ID NO: 70).

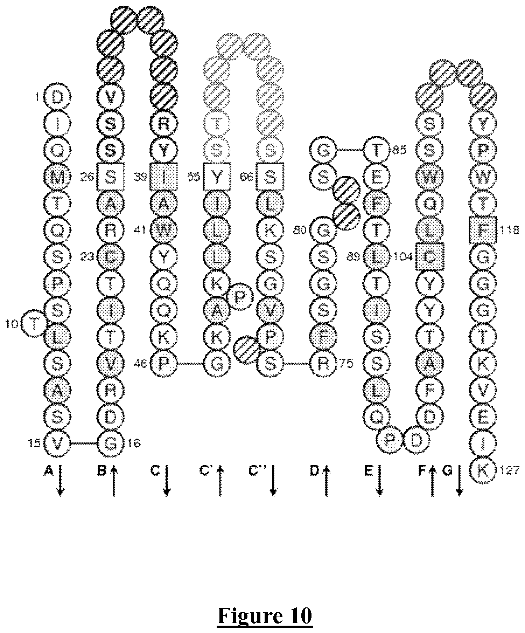

La **figure 10** correspond à une représentation schématique en collier de perles de la séquence d'acides aminés de la partie variable de la chaîne légère de l'anticorps 12G4 humanisé muté (6B_78; SEQ ID NO: 78).

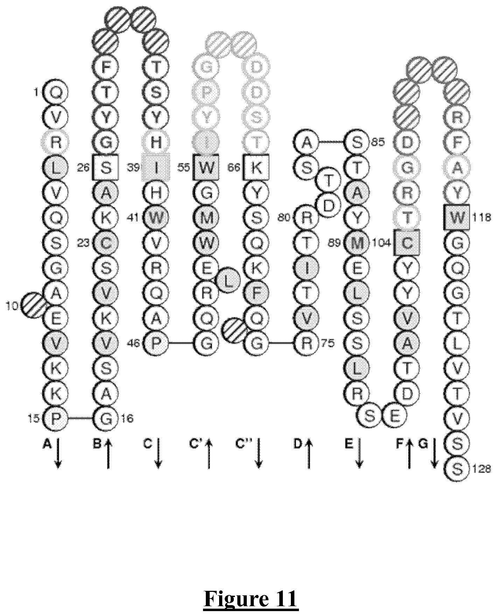

La **figure 11** correspond à une représentation schématique en collier de perles de la séquence d'acides aminés de la partie variable de la chaîne lourde de l'anticorps 12G4 humanisé muté (3C_23K; SEQ ID NO: 86).

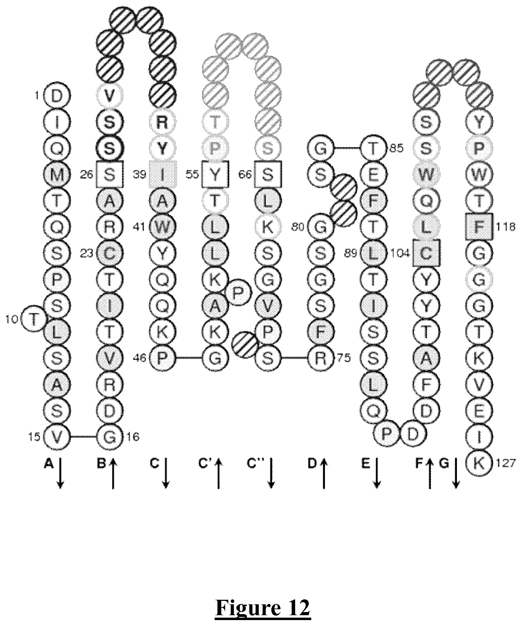

La **figure 12** correspond à une représentation schématique en collier de perles de la séquence d'acides aminés de la partie variable de la chaîne légère de l'anticorps 12G4 humanisé muté (3C_23K; SEQ ID NO: 82).

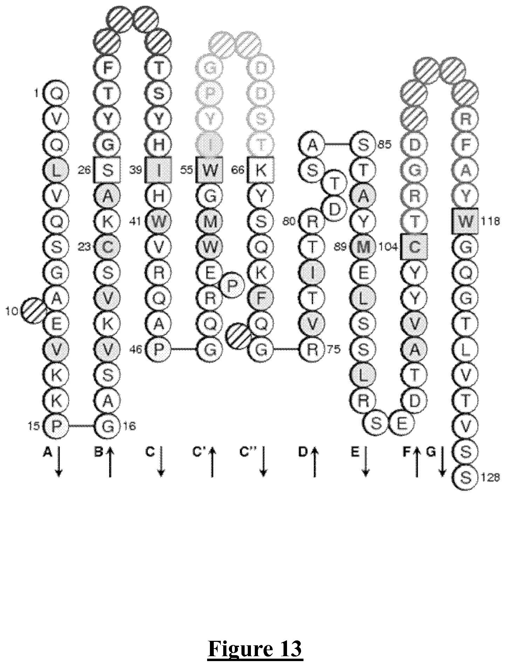

La **figure 13** correspond à une représentation schématique en collier de perles de la séquence d'acides aminés de la partie variable de la chaîne lourde de l'anticorps 12G4 humanisé muté (4C_35; SEQ ID NO: 90).

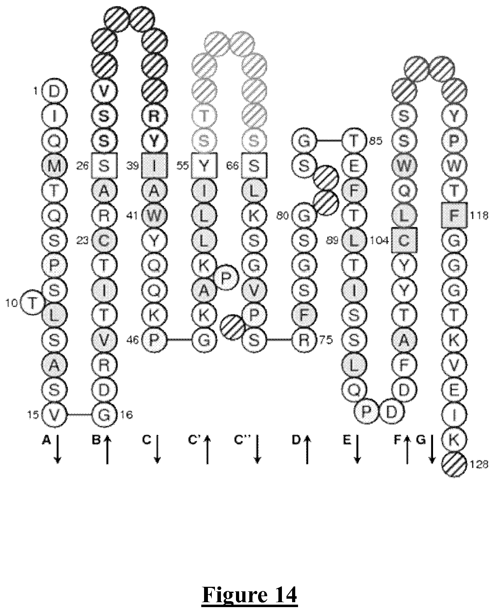

La **figure 14** correspond à une représentation schématique en collier de perles de la séquence d'acides aminés de la partie variable de la chaîne légère de l'anticorps 12G4 humanisé muté (4C_35; SEQ ID NO: 78).

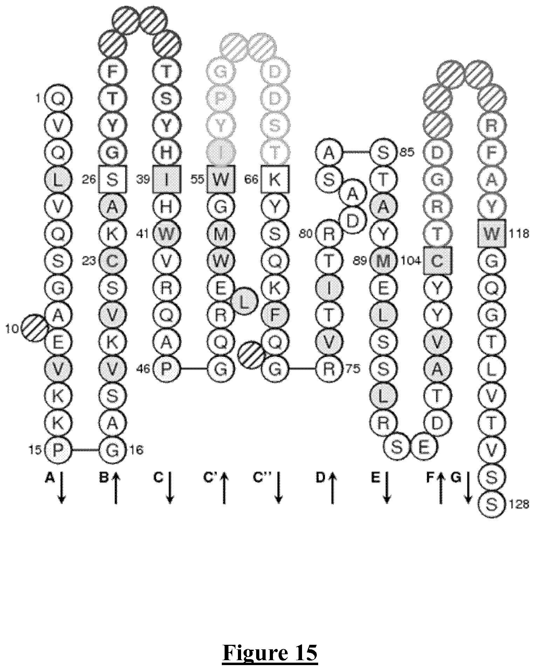

La **figure 15** correspond à une représentation schématique en collier de perles de la séquence d'acides aminés de la partie variable de la chaîne lourde de l'anticorps 12G4 humanisé muté (5B_42; SEQ ID NO: 98).

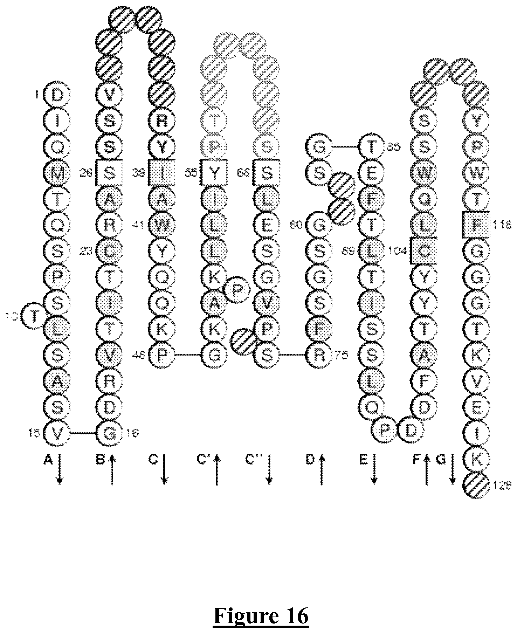

La **figure 16** correspond à une représentation schématique en collier de perles de la séquence d'acides aminés de la partie variable de la chaîne légère de l'anticorps 12G4 humanisé muté (5B_42; SEQ ID NO: 94).

La **figure 17** présente la détermination l'affinité de la liaison de l'anticorps au récepteur AMHR-II dans un test Elisa classique obtenue avec des anticorps mutés (Fab) selon l'invention.

L'axe des abscisses représente la concentration en (Fab) en µg/ml et l'axe des ordonnées représente la DO à 450 nm.

La courbe en pointillé avec des cercles vides blancs représente la liaison de l'anticorps humanisé 12G4 non muté.

La courbe avec des triangles pleins noirs représente la liaison de l'anticorps humanisé 12G4 muté, possédant une mutation dans le CDR (E184K) de la région variable de la chaîne légère (anticorps 6B_78).

La courbe avec des triangles vides blancs représente la liaison de l'anticorps humanisé 12G4 muté, possédant une mutation dans le CDR (S179P) de la région variable de la chaîne légère, une mutation dans la région FR (I177T) de la région variable de la chaîne légère et une mutation dans la région variable de la chaîne lourde (Q3R). (anticorps 3C_23)

La courbe avec des cercles vides blancs représente la liaison de l'anticorps humanisé 12G4 muté, possédant une mutation dans le CDR (E184K) de la région variable de la chaîne légère une mutation dans le CDR (S179P) de la région variable de la chaîne légère, une mutation dans la région FR (I177T) de la région variable de la chaîne légère et une mutation dans la région variable de la chaîne lourde (Q3R). (anticorps 3C_23K)

La courbe avec des cercles pleins noirs représente la liaison de l'anticorps chimérique 12G4 non muté.

La **figure 18** correspond à la représentation schématique du vecteur de clonage H622-14 pour l'anticorps 12G4 chimérique contenant la chaîne lourde où le leader VH AMHR-II est fusionné à la région variable de la chaîne lourde (VH AMHR-II), elle-même fusionnée à la région constante de l'immunoglobuline humaine (CH T125), et la chaîne légère où le leader VK AMHR-II est fusionné à la région variable de la chaîne légère (VK AMHR-II), elle-même fusionnée à la région constante de l'immunoglobuline humaine (CK T125).

Les différents éléments de régulation (promoteurs, introns chimériques, sites de polyadénylation...) ainsi que les gènes de résistance aux antibiotiques et les origines de réplication sont également représentés.

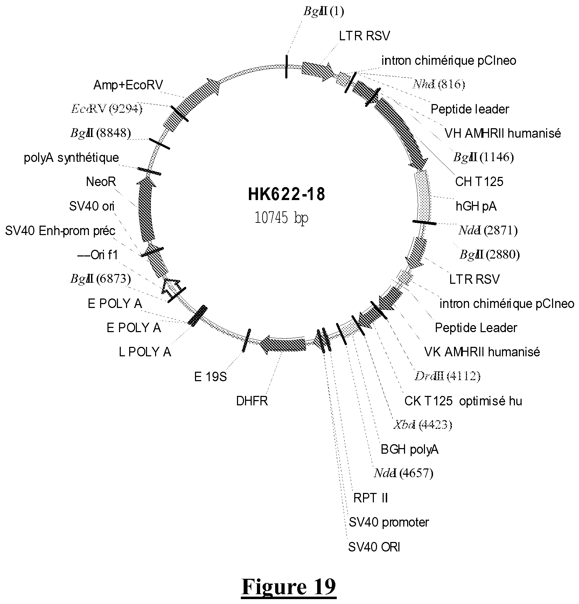

La **figure 19** correspond à la représentation schématique du vecteur de clonage H622-18 pour l'anticorps 12G4 humanisé non muté contenant la chaîne lourde où le leader VH AMHR-II humanisé est fusionné à la région variable de la chaîne lourde (VH AMHR-II humanisé), elle-même fusionnée à la région constante de l'immunoglobuline humaine (CH T125), et la chaîne légère où le leader VK AMHR-II est fusionné à la région variable de la chaîne légère (VK AMHR-II humanisé), elle-même fusionnée à la région constante de l'immunoglobuline humaine (CK T125).

Les différents éléments de régulation (promoteurs, introns chimériques, sites de polyadénylation...) ainsi que les gènes de résistance aux antibiotiques et les origines de réplication sont également représentés.

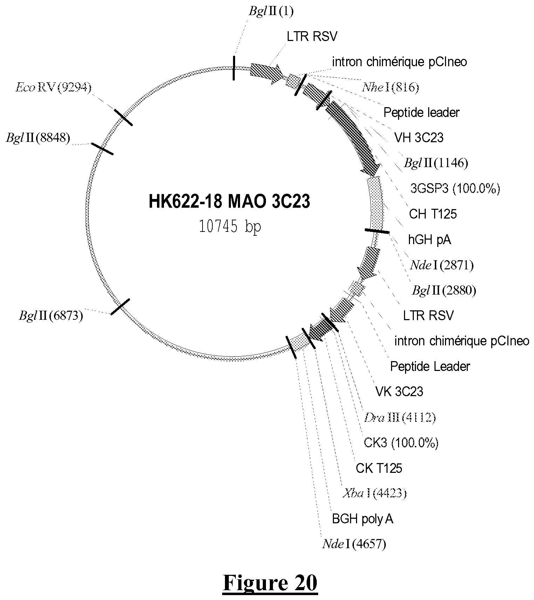

La **figure 20** correspond à la représentation schématique du vecteur de clonage H622-18 MAO 3C23 pour l'anticorps 12G4 humanisé muté 3C_23 contenant la chaîne lourde où le leader VH 3C_23 est fusionné à la région variable de la chaîne lourde (VH 3C_23), elle-même fusionnée à la région constante de l'immunoglobuline humaine (CH T125), et la chaîne légère où le leader VK 3C_23 est fusionné à la région variable de la chaîne légère (VK 3C_23), elle-même fusionnée à la région constante de l'immunoglobuline humaine (CK T125).

Les différents éléments de régulation (promoteurs, introns chimériques, sites de polyadénylation...) ainsi que les gènes de résistance aux antibiotiques et les origines de réplication sont également représentés.

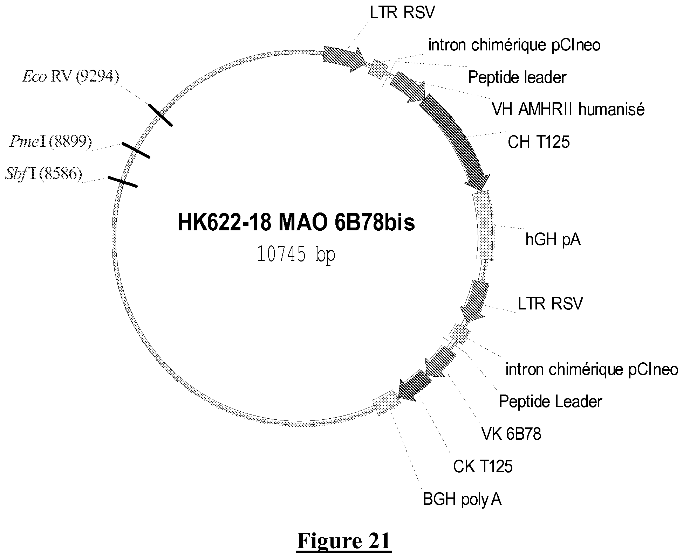

La **figure 21** correspond à la représentation schématique du vecteur de clonage H622-18 MAO 6B_78 pour l'anticorps 12G4 humanisé muté 6B_78 contenant la chaîne lourde où le leader VH AMHR-II humanisé est fusionné à la région variable de la chaîne lourde (VH AMHR-II humanisé), elle-même fusionnée à la région constante de l'immunoglobuline humaine (CH T125), et la chaîne légère où le leader VK 6B_78 est fusionné à la région variable de la chaîne légère (VK 6B_78), elle-même fusionnée à la région constante de l'immunoglobuline humaine (CK T125).

Les différents éléments de régulation (promoteurs, introns chimériques, sites de polyadénylation...) ainsi que les gènes de résistance aux antibiotiques et les origines de réplication sont également représentés.

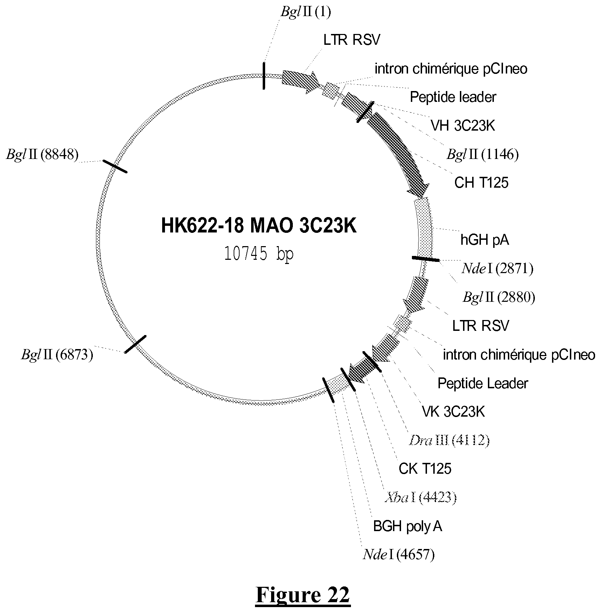

La **figure 22** correspond à la représentation schématique du vecteur de clonage H622-18 MAO 3C_23K pour l'anticorps 12G4 humanisé muté 3C_23K contenant la chaîne lourde où le leader VH 3C_23K est fusionné à la région variable de la chaîne lourde (VH 3C_23K), elle-même fusionnée à la région constante de l'immunoglobuline humaine (CH T125), et la chaîne légère où le leader VK 3C_23K est fusionné à la région variable de la chaîne légère (VK 3C_23K), elle-même fusionnée à la région constante de l'immunoglobuline humaine (CK T125).

Les différents éléments de régulation (promoteurs, introns chimériques, sites de polyadénylation...) ainsi que les gènes de résistance aux antibiotiques et les origines de réplication sont également représentés.

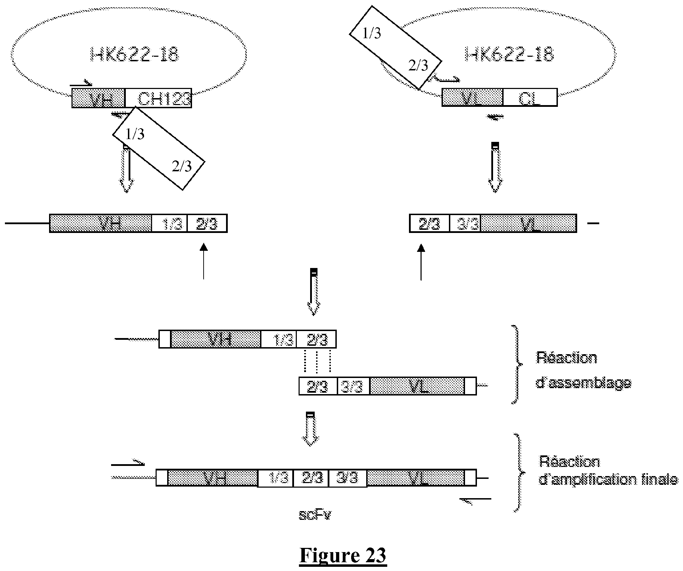

La **figure 23** présente les protocoles schématiques de construction des fragments scFv.

La flèche noire sous la région 2/3 de VH indique la séquence codant les 2/3 N-terminal du lien peptidique.

La flèche noire sous la région 2/3 de VL indique la séquence codant les 2/3 C-terminal du lien peptidique.

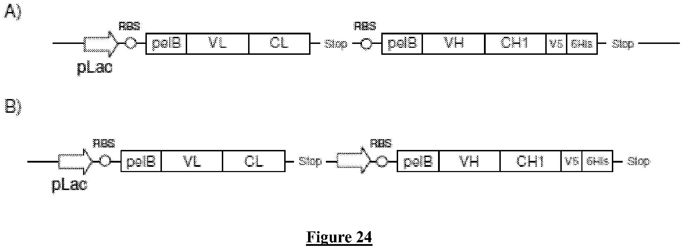

Les **figures 24A et 24B** représentent le sous clonage des séquences nucléotidiques des chaînes légères VL-CL et lourdes VH-CH1 des anticorps mLFB112 et huLFB112 dans les vecteurs d'expression pMG62-Fab.

Figure 24A : mLFB112

Figure 24B : huLFB112

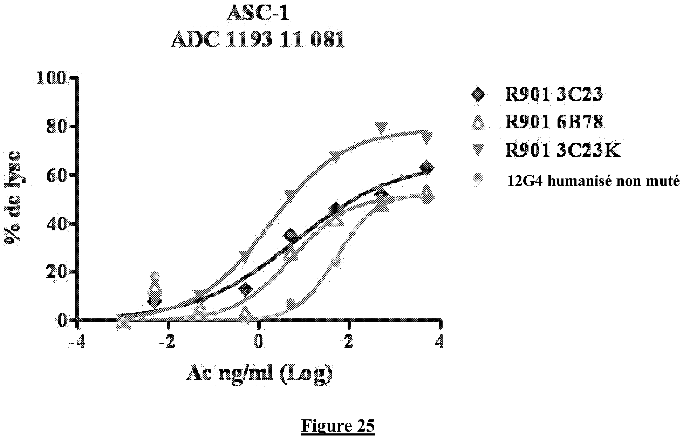

La **figure 25** présente l'activité ADCC des anticorps anti-AMHRII humanisés de l'invention comparée à celle de l'anticorps 12G4 humanisé non muté. Les résultats sont exprimés en pourcentage de lyse de la cellule ASC1 (axe des ordonnées) en fonction de la quantité d'anticorps ajoutée en ng/ml (axe des abcisses). Moy +/- SEM.

La courbe avec losanges représente l'anticorps anti-AMHRII 3C_23 (R901 3C_23), la courbe avec triangles pointe en haut représente l'anticorps anti-AMHRII 6B_78 (R901 6B_78, la courbe avec triangles pointe en bas représente l'anticorps anti-AMHRII 3C_23K (R901 3C_23K), la courbe avec cercles représente l'anticorps anti-AMHRII 12G4 humanisé non muté.

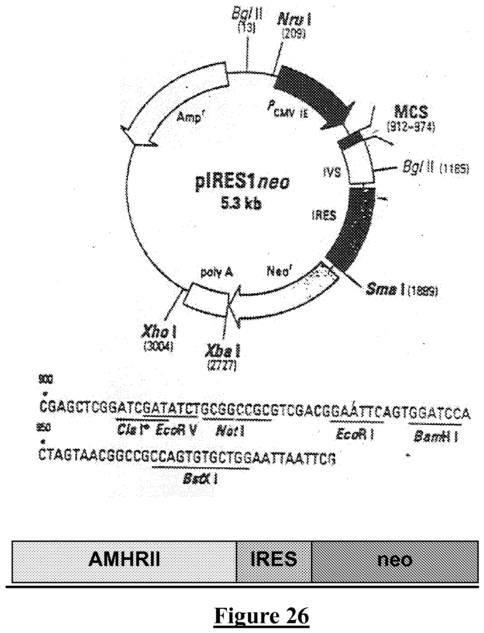

La **figure 26** présente la carte du vecteur d'expression plasmide pIRES-neo utilisé pour la génération de la lignée cov434-AMHRII.

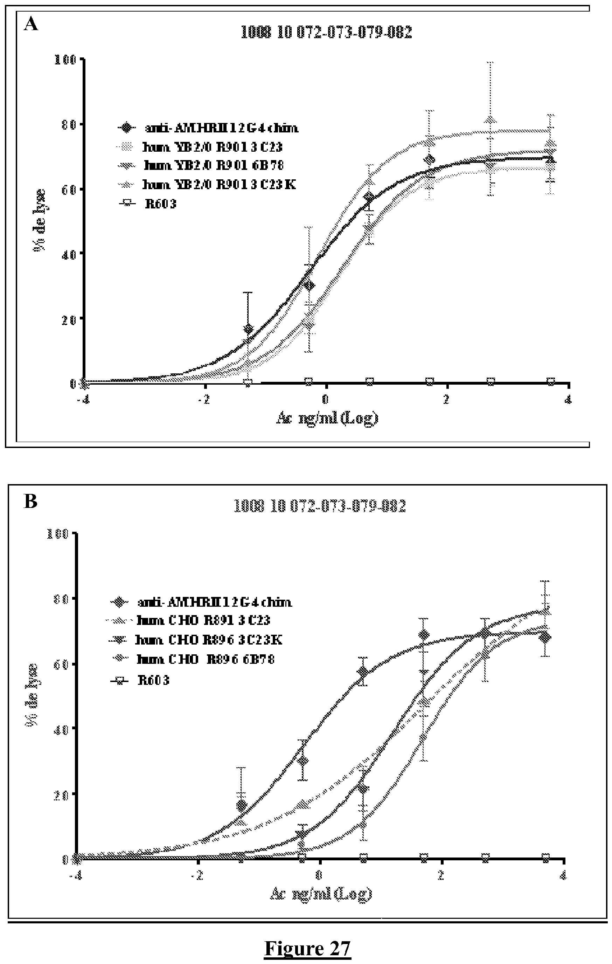

Les **figures 27A et 27B** présentent l'activité ADCC des anticorps anti-AMHRII chimériques et humanisés produits dans les cellules YB2/0 (figure 27A) et les cellules CHO (figure 27B) sur la lignée COV434-AMHRII.

Les résultats sont exprimés en pourcentage de lyse des cellules COV434-AMHRII (axe des ordonnées) en fonction de la quantité d'anticorps ajoutée en ng/ml (axe des abscisses). Moyenne 3 tests +/- SEM.

Figure 27A : la courbe avec losanges représente l'anticorps anti-AMHRII 12G4 chimérique non muté, la courbe avec carrés pleins représente l'anticorps anti-AMHRII YB2/0 3C_23 (R901 3C_23), la courbe avec triangles pointe en bas représente l'anticorps anti-AMHRII YB2/0 6B_78 (R901 6B_78), la courbe avec triangles pointe en haut représente l'anticorps anti-AMHRII YB2/0 3C_23K (R901 3C_23K) et la courbe avec rectangles vides représente l'anticorps anti-CD20 utilisé comme témoin négatif.

Figure 27B: la courbe avec losanges représente l'anticorps anti-AMHRII 12G4 chimérique non muté, la courbe avec triangles pointe en haut représente l'anticorps anti-AMHRII CHO 3C_23 (R901 3C_23), la courbe avec triangles pointe en bas représente l'anticorps anti-AMHRII CHO 3C_23K (R901 3C_23K), la courbe avec cercles représente l'anticorps anti-AMHRII CHO 6B_78 (R901 6B_78)et la courbe avec rectangles vides représente l'anticorps anti-CD20 utilisé comme témoin négatif.

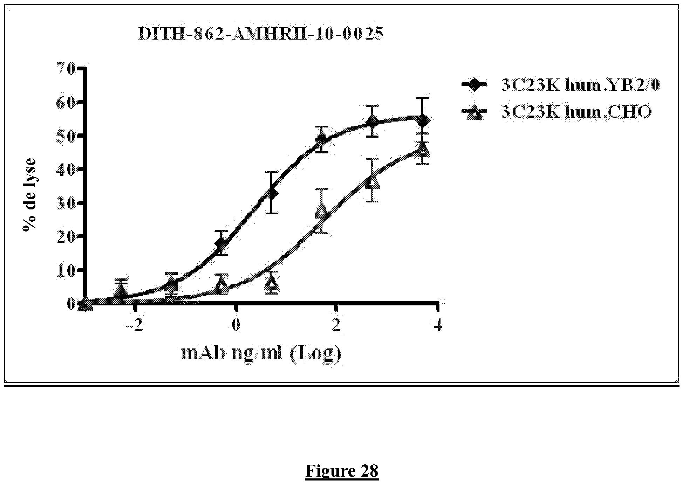

La **figure 28** présente l'activité ADCC des anticorps anti-AMHRII humanisés produits dans les cellules YB2/0 et CHO sur la lignée Asc 1. Les résultats sont exprimés en pourcentage de lyse des cellules Asc 1 (axe des ordonnées) en fonction de la quantité d'anticorps ajoutée en ng/ml (axe des abscisses). Moyenne 3 tests +/- SEM.

La courbe avec losanges représente l'anticorps anti-AMHRII YB2/0 3C_23K, la courbe avec triangles pointe en haut représente l'anticorps anti-AMHRII CHO 3C_23K.

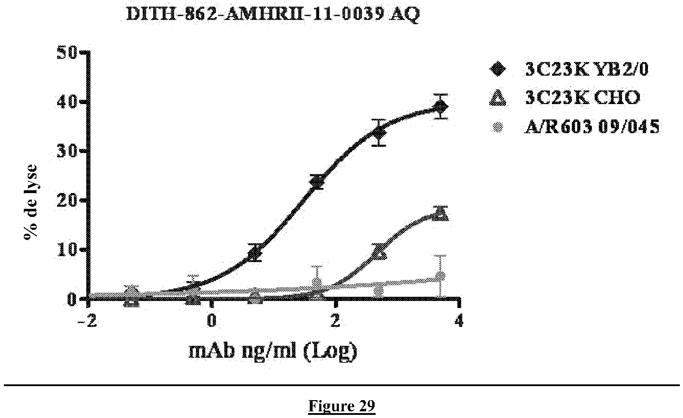

La **figure 29** présente l'activité ADCC des anticorps anti-AMHRII humanisés produits dans YB2/0 et CHO sur la lignée META 2815. Les résultats sont exprimés en pourcentage de lyse des cellules META 2815 (axe des ordonnées) en fonction de la quantité d'anticorps ajoutée en ng/ml (axe des abscisses). Moyenne 3 tests +/- SEM.

La courbe avec losanges représente l'anticorps anti-AMHRII YB2/0 3C_23K, la courbe avec triangles pointe en haut représente l'anticorps anti-AMHRII CHO 3C_23K, la courbe avec cercles représente l'anticorps anti-CD20 utilisé comme témoin négatif (anti-CD20 A/R603 09/045).

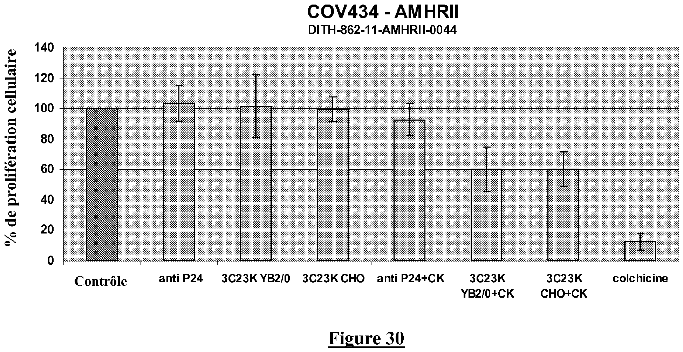

La **figure 30** présente l'effet de l'anticorps anti-AMHRII 3C_23K sur la prolifération des cellules COV434-AMHRII. La valeur 100% correspond à la prolifération des cellules COV434-AMHRII observée sans anticorps (moyenne de 3 test +/- SD).

De gauche à droite, les histogrammes représentent :

Le contrôle sans anticorps, un anticorps antiP24, l'anticorps anti-AMHRII YB2/0 3C_23K, l'anticorps anti-AMHRII CHO 3C_23K, un anticorps antiP24 en présence d'un agent réticulant (CK), l'anticorps anti-AMHRII YB2/0 3C_23K en présence de CK, l'anticorps anti-AMHRII CHO 3C_23K en présence de CK, la colchicine à 1 $\mu$g/ml.

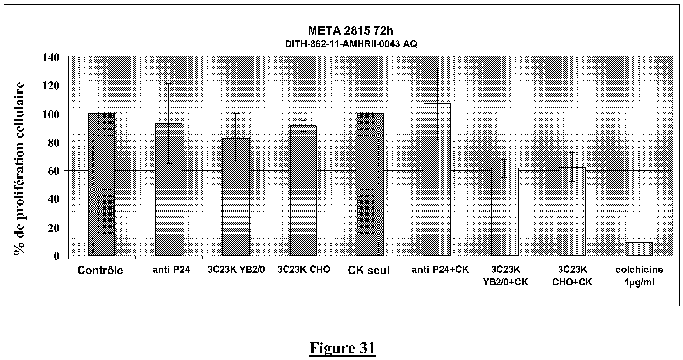

La **figure 31** présente l'effet de l'anticorps anti-AMHRII 3C_23K sur la prolifération des cellules META 2815. La valeur 100% correspond à la prolifération des cellules META 2815 observée sans anticorps (moyenne de 3 test +/- SD).

De gauche à droite, les histogrammes représentent :

Le contrôle sans anticorps, un anticorps antiP24, l'anticorps anti-AMHRII YB2/0 3C_23K, l'anticorps anti-AMHRII CHO 3C_23K, un agent réticulant seul, un anticorps antiP24 en présence d'un agent réticulant (CK), l'anticorps anti-AMHRII YB2/0 3C_23K en présence de CK, l'anticorps anti-AMHRII CHO 3C_23K en présence de CK, la colchicine à 1 $\mu$g/ml.

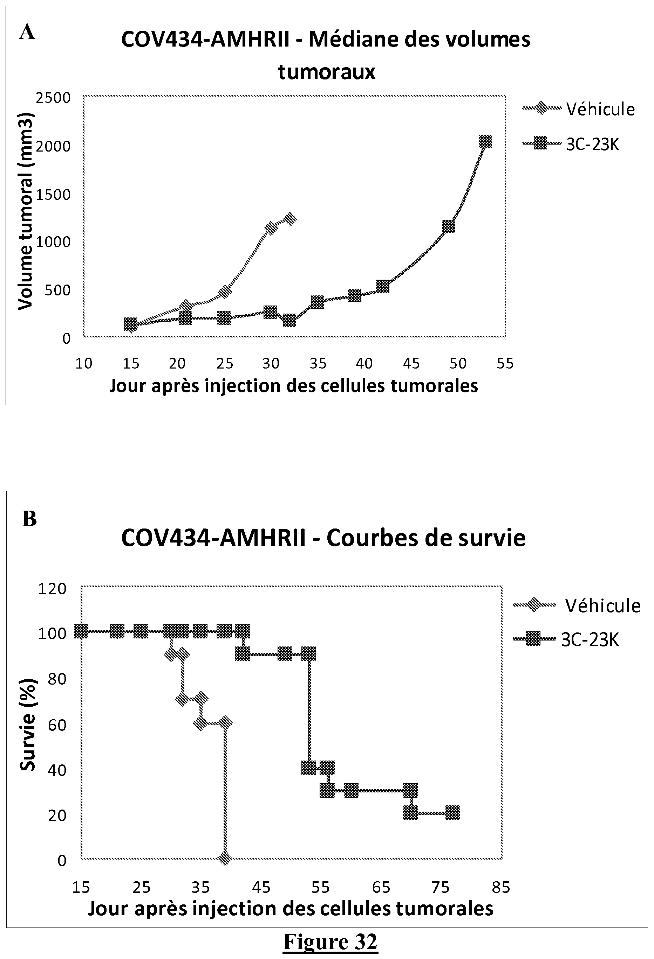

Les **figures 32A et 32B** présentent l'évolution des volumes tumoraux figure 32A) et les courbes de survie (figure 32B) sous l'effet du traitement par 3C23K-YB2/0 avec des injections intrapéritonéales d'anticorps réalisées à 2-3 jours d'intervalle à une dose de 10 mg/kg/inj pour un total de 18 injections (flèches noires) dans le modèle cov434-AMHRII.

Figure 32A :

axe des ordonnées : volumes tumoral en mm$^3$,
axe des abcisses : jours après injection des cellules tumorales.
Courbe avec losanges : véhicule
Courbe avec rectangles : anticorps anti-AMHRII YB2/0 3C_23K.

Figure 32B :

axe des ordonnées : pourcentage de survie
axe des abcisses : jours après injection des cellules tumorales.
Courbe avec losanges : véhicule
Courbe avec rectangles : anticorps anti-AMHRII YB2/0 3C_23K.

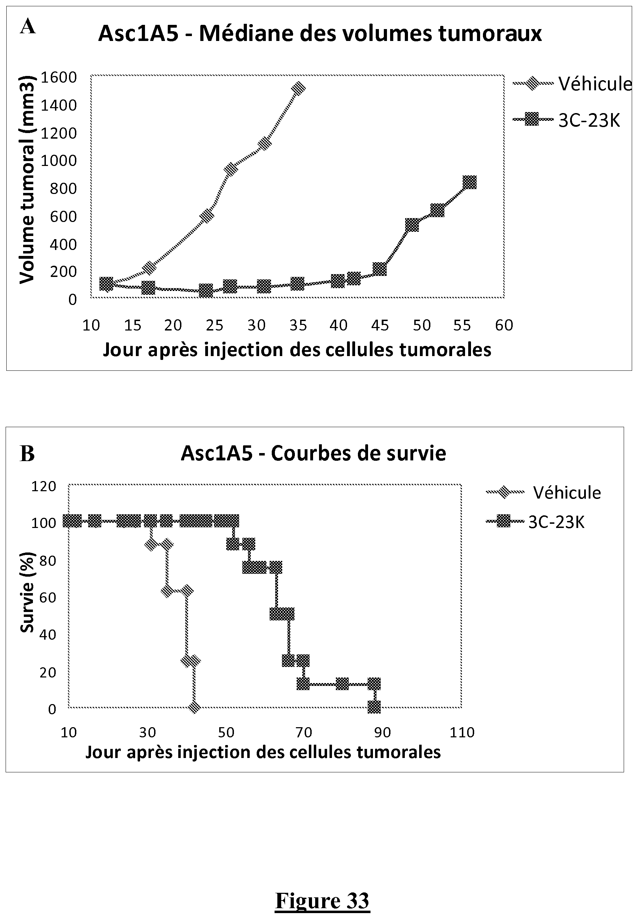

Les **figures 33A et 33B** présentent l'évolution des volumes tumoraux (figure 33A) et les courbes de survie (figure 33B) sous l'effet du traitement par 3C_23K-YB2/0, injections intrapéritonéales d'anticorps réalisées à 2-3 jours d'intervalle à une dose de 10 mg/kg/inj pour un total de 18 injections (flèches noires) dans un modèle Asc1a5.
Figure 33A :

axe des ordonnées : volumes tumoral en mm$^3$,
axe des abcisses : jours après injection des cellules tumorales.
Courbe avec losanges : véhicule
Courbe avec rectangles : anticorps anti-AMHRII YB2/0 3C_23K.

Figure 33B :

axe des ordonnées : pourcentage de survie
axe des abcisses : jours après injection des cellules tumorales.
Courbe avec losanges : véhicule
Courbe avec rectangles : anticorps anti-AMHRII YB2/0 3C_23K.

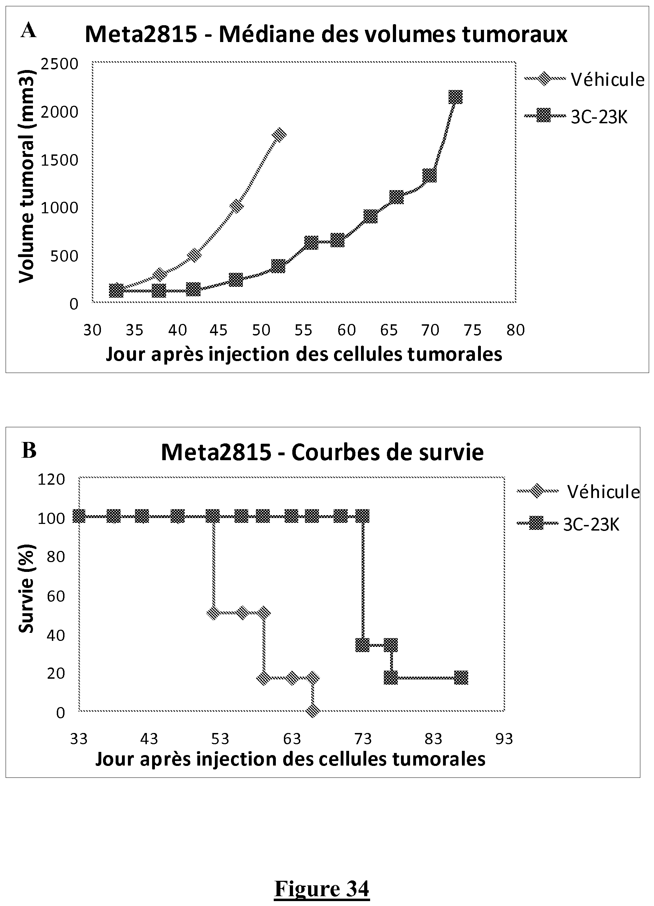

Les **figures 34A et 34B** présentent l'évolution des volumes tumoraux (figure 34A) et courbes de survie (figure 34B) sous l'effet du traitement par 3C_23K-YB2/0, injections intrapéritonéales d'anticorps réalisées à 2-3 jours d'intervalle à une dose de 10 mg/kg/inj pour un total de 18 injections (flèches noires) dans le modèle META 2815.
Figure 34A :

axe des ordonnées : volumes tumoral en mm$^3$,
axe des abcisses : jours après injection des cellules tumorales.
Courbe avec losanges : véhicule
Courbe avec rectangles : anticorps anti-AMHRII YB2/0 3C_23K.

Figure 34B :

axe des ordonnées : pourcentage de survie
axe des abcisses : jours après injection des cellules tumorales.
Courbe avec losanges : véhicule
Courbe avec rectangles : anticorps anti-AMHRII YB2/0 3C_23K.

**EXEMPLES**

**EXEMPLE 1 : Détermination de l'affinité des anticorps anti-AMHR-II**

L'affinité des anticorps pour leur antigène, AMHR-II, est déterminée par technique SPR (Surface Plasmon Resonance) sur BIACore X100 (BIACore, GE Healthcare). Le récepteur AMHR-II recombinant, exprimé sous forme de protéine de fusion avec une région Fc, est immobilisé par un couplage covalent entre ses fonctions amines et les groupements carboxyles du dextran activés en esters de succinimide, présents à la surface de la sensorchip de type CM5. Les groupements COOH de la sensorchip sont activés pendant 7 minutes par une mélange EDC/NHS (0.1 M N-hydroxysuccinimide et 0.1 M 3-(*N*,*N*-dimethylamino)propyl-*N*-ethylcarbodiimide) à un débit de 10 μl/min puis la protéine de fusion AMHR-II/Fc , diluée à 5 μg/ml en tampon acétate de sodium 10 mM, pH 4.0, est injectée à 5μl/min sur la piste 2 de la sensorchip de manière à atteindre 300 RU. Les groupements esters n'ayant pas réagi avec les amines de la protéine de fusion sont désactivés par l'injection d'une solution d'éthanolamine-HCl 1M, pH 8.5 pendant 7 min à un début de 10 μl :min. La piste 1 qui sert de contrôle négatif a été activée et désactivée comme la piste 2.
Toutes les mesures sont effectuées à 25°C. Les anticorps à analyser sont dilués dans le tampon de course HBS-EP (BIACore, GE Healthcare) à des concentrations de 6.25 à 3 333 nM et injectés sur la sensorchip pendant 2 min à un débit de 30 μl/min. L'étape de dissociation est suivie pendant 10 min puis la surface est régénérée par l'injection de tampon Glycine 10 mM, pH 1.5 pendant 30 sec à 10 µl/min.

Les sensorgrammes obtenus sont analysés en utilisant le modèle cinétique 1 : 2 du logiciel BIAevaluation 3.1.

**Résultats**

Les anticorps ont été produits dans des cellules CHO ou YB 2/0 (Tableau I)

TABLEAU I

| Anticorps | Mutations | $k_a$ (M$^{-1}$s$^{-1}$) | $k_d$ (s$^{-1}$) | $K_D$ (nM) | $K_D$ $^{Chimerique}$ /$K_D$ $^{muté}$ |
|---|---|---|---|---|---|
| 12G4 - Chimérique | NA | $3,5.10^3$ | $7,4.10^{-4}$ | 212 | - |
| 6B_78 YB2/0 | VL-E184K | $1,6.10^4$ | $1,3.10^{-3}$ | 82 | 2,6 |
| 3C_23 YB2/0 | VH-Q3R VL-I177T/S179P | $3,6.10^4$ | $3,3.10^{-3}$ | 92 | 2,3 |
| 3C_23K YB2/0 | VH-Q3R VL-I177T/S179P/E184K | $4.10^4$ | $8,6.10^{-4}$ | 21 | 10 |
| 6B_78 CHO | VL-E184K | $1,5.10^4$ | $1,2.10^{-3}$ | 81 | 2,6 |
| 3C_23 CHO | VH-Q3R VL-I177T/S179P | $5.10^4$ | $4,3.10^{-3}$ | 86 | 2,5 |
| 3C_23K CHO | VH-Q3R VL-I177T/S179P/E184K | $4,1.10^4$ | $1.10^{-3}$ | 25 | 8,5 |

Les mutations introduites dans les anticorps 6B_78 et 3C_23 induisent une augmentation de l'affinité pour l'antigène AMHR-II d'un facteur 2,3 à 2,6 par rapport à l'anticorps chimérique (12G4 - chimérique).

Les mutations des deux anticorps 6B_78 et 3C_23 ont un effet synergique; l'introduction de la mutation de l'anticorps 6B_78 dans l'anticorps 3C_23 provoque une augmentation de l'affinité d'un facteur 10.

**EXEMPLE 2 : Détermination de l'affinité de l'anticorps 12G4 murin, chimérique ou humanisé sur des cellules cov434-AMHR-II (peptide épitopique de séquence: GGGGNLTQDRAQVEMQGSR (SEQ ID NO : 101) et GGGGNLTQARGQVEMQGSR (SEQ ID NO : 102) pour le peptide contrôle négatif)**

| | Constante d'association | Constante de dissociation | Constante d'affinité à l'équilibre |
|---|---|---|---|
| 12G4 humanisé | $1.83 \times 10^3$ M$^{-1}$. s$^{-1}$ | $9.62 \times 10^{-3}$ s$^{-1}$ | $5.26 \times 10^{-6}$ M |
| 12G4 chimère | $6.49 \times 10^3$ M$^{-1}$. s$^{-1}$ | $1.53 \times 10^{-3}$ s$^{-1}$ | $2.35 \times 10^{-7}$ M |
| 12G4 murin | | $1.47 \times 10^{-3}$ s$^{-1}$ | de l'ordre de $10^{-7}$ M |

L'affinité de l'anticorps chimérique déterminée sur le récepteur AMHR-II humain est d'environ $10^{-8}$M.

**Exemple 3 : Préparation des anticorps humanisés 12G4 mutés**

L'anticorps murin est sensiblement équivalent à l'anticorps chimérique et présente une forte affinité.

L'anticorps humanisé 12G4 (huLFB112) a été obtenu par greffage des boucles hypervariables CDR de l'anticorps murin 12G4 (mLFB112) sur une charpente protéique de nature humaine ("CDR grafting").

L'anticorps humanisé présente une perte d'affinité non négligeable en comparaison de l'anticorps murin.

L'objectif final est donc d'augmenter l'affinité de l'anticorps humanisé de manière à restaurer les caractéristiques de liaison initiales de l'anticorps murin. Cette optimisation sera réalisée à travers un cycle d'évolution moléculaire par la technologie MutaGen propriétaire de la société Millegen.

**3.1 Construction et validation des outils moléculaires.**

3.1.1. Construction des fragments scFv.

Les séquences nucléotidiques codantes pour les régions variables des chaînes légères (VL) et des chaînes lourdes (VH) des anticorps murin et humanisé ont été amplifiées par PCR à l'aide d'amorces appropriées. Les séquences amplifiées ont ensuite été associées entre elles de manière à générer un fragment d'anticorps recombinant de type scFv. Plusieurs constructions ont ainsi été réalisées : orientation VH-VL ou VL-VH et utilisation de deux liens peptidiques différent (lien peptidique de 15 ou de 18 acides aminés). Au total 8 constructions ont été réalisées, 4 pour l'anticorps murin et 4 pour l'anticorps humanisé. Le principe de construction des fragments scFv est illustré ci-dessous (schéma I). Les séquences codant pour ces scFv ont ensuite été sous-clonés dans le vecteur d'expression phagemidique de MilleGen (pMG58). Ce vecteur permet d'exprimer les fragments d'anticorps de type scFv et de les exposer à la surface d'un bactériophage de type M13 (phage-scFv).

Les séquences nucléotidiques des domaines VH et VL des anticorps murins et humanisé ont été vérifié par séquençage d'ADN.
Le protocole est résumé sur la figure 23.

3.1.2. Expression des scFv à la surface des phages et caractérisation par ELISA.

La quantité de cible fournie (80μg) ne nous permettait pas de tester l'ensemble des 8 constructions réalisées. L'anticorps murin mLFB1 12 exprimé sous la forme d'un scFv est nommé mVH-VL dans la suite du document tandis que l'anticorps humanisé huLFB112 est nommé huVH-VL.

3.1.2.1. Production des phages-scFv

Les bactéries XL1-Blue transformées par les vecteurs pMG58 contenant l'ADN codant pour le scFv mVH-VL d'une part et le scFv huVH-VL d'autre part sont mises en culture à 30°C jusqu'à une DO600nm de 0,5-0,6. Après ajout d'IPTG et infection des bactéries par des phages auxiliaires (M13KO7, New England Biolabs), les cultures sont mises en culture à 26°C pendant une nuit. Le lendemain, les particules phagiques (phages-scFv) sont récupérées dans le surnageant de culture, précipitées à l'aide d'une solution de PEG/NaCl, concentrées (100X) et quantifiées.
Dans notre cas, nous obtenons une concentration de l'ordre de 8 x $10^{11}$ phages/ml pour les deux scFv.

3.1.2.2. Test ELISA-phages.

La fonctionnalité des scFv mVH-VL et huVH-VL produits à la surface des phages a été vérifiée par des tests ELISA direct.

Protocole :

1) Immobilisation de la cible : 100μl/puits de la cible recombinante diluée à 5μg/ml en PBS1X soit 500ng/puits, une nuit à 4°. Utilisation de plaques de microtitration Nunc-Immuno Plate Maxisorp,
2) Saturation : 200μl/puits de PBSIX-Lait écrémé 4%, incubation 2h à 37°C,
3) Liaison : 100μl/puits des solutions de phages-scFv murin et humanisé dilué en PBS1X-Lait 2%-Tween20 0,05%, (gamme de dilution de 2 en 2), incubation 2h à 37°C,
4) Détection : 100μl/puits de l'anticorps anti-phages M13 couplé à la peroxydase (dilutionl/10000, GE Healthcare), incubation 2h à 37°C
5) Révélation : 100μl/puits de TMB
6) Neutralisation : 100μl/puits de H2SO4
7) Mesure des DO à 450nm

Résultats (Tableau II):

Tableau II

| Nombre de phages par puit | mVH-VL | | | huVH-VL | | | Ratio mVH-VL/huVH-VL |
|---|---|---|---|---|---|---|---|
| | + recouvrement | Sans recouvrement | Diff | + recouvrement | Sans recouvrement | Diff | |
| 3,00E+10 | 2,805 | 0,158 | 2,647 | 1,247 | 0,150 | 1,097 | 2,4 |
| 3,00E+10 | 2,193 | 0,085 | 2,107 | 0,689 | 0,096 | 0,593 | 3,6 |

(suite)

| Nombre de phages par puit | mVH-VL | | | huVH-VL | | | Ratio mVH-VL/huVH-VL |
|---|---|---|---|---|---|---|---|
| | + recouvrement | Sans recouvrement | Diff | + recouvrement | Sans recouvrement | Diff | |
| 3,00E+09 | 1,570 | 0,064 | 1,506 | 0,395 | 0,072 | 0,323 | 4,7 |
| 3,00E+09 | 0,946 | 0,059 | 0,887 | 0,226 | 0,056 | 0,170 | 5,2 |
| 3,00E+09 | 0,495 | 0,049 | 0,446 | 0,135 | 0,049 | 0,086 | 5,2 |
| Diff : binding specific (différence entre les puits avec recouvrement et sans recouvrement | | | | | | | |

Le rapport mVH-VL/ huVH-VL a été calculé avec la valeur de binding spécifique (« Diff)

3.1.3 : Construction des fragments Fab

Les séquences nucléotidiques des chaînes légères VL-CL et lourdes VH-CH1 des anticorps mLFB112 et huLFB112 ont été sous-clonés dans les vecteurs d'expression pMG62-Fab (figures 24A et 24B).

Vecteurs d'expression pMG62-Fab.

A) Les deux chaînes VL-CL et VH-CH1 sont exprimées à partir du promoteur pLac en amont de la chaîne légère, la chaîne lourde VH-CH1 est fusionnée à une étiquettes de détection (peptide V5) et une étiquette pour la purification par IMAC (6xHis).
B) Chacune des chaînes légères et lourdes est sous la dépendance d'un promoteur. RBS : Ribosome Binding Site.

## 3.2 Construction et validation des outils moléculaires.

Construction de la banque par MutaGen™.
L'objectif défini pour cette étape était d'obtenir une banque de 5x106 variants avec 1 à 2 mutations en acides aminés par scFv.
L'introduction de mutations au sein des domaines VL et VH de l'anticorps humanisé huLFB112 a été réalisée par la technologie MutaGen™. Au final, nous avons obtenu une banque de grande taille composée d'environ $5 \times 10^7$ clones mutés avec 1 à 5 mutations d'acides aminés par scFv soit 10 fois la diversité prévue initialement.
Pour cela, plusieurs sous-banques ont été construites selon différentes conditions expérimentales : conditions U, M, US et UE définies par différentes amorces nucléotidiques, enzymes mutases utilisées et nombre de réplications. Ces sous-banques sont au nombre de 4 et nommées R20U, 45M, R20US et R20UE. Pour l'ensemble de ces sous banques, un total de 295 séquences a été effectué afin de préciser les différentes caractéristiques de la mutagenèse. Le tableau III ci-dessous présente un aperçu des principales données issues de l'analyse des séquençages.

Tableau III. Analyse des mutations des différentes sous banques.

| | Nom de la banque | | | | |
|---|---|---|---|---|---|
| | R20U | 45M | R20US | R20UEta | TOTAL |
| Taille de la banque | 2,0E+07 | 6,0E+06 | 9,9E+07 | 5,0E+05 | 1,3E+08 |
| Condition | U | M | US | UE | |
| Nombre de séquences | 87 | 67 | 85 | 56 | 295 |
| Analyse des séquences de nucléotides | | | | | |
| Fréquence des mutations par kb | 2,76 | 3,66 | 3,31 | 4,9 | |
| % de délétions | 10% | 20% | 6% | 5% | |
| % d'additions | 2% | - | 0,8% | - | |

(suite)

| Analyse des séquences de nucléotides | | | | | |
|---|---|---|---|---|---|
| % de substitutions | 72% | 80% | 93% | 95% | |
| Fréquence des mutations par kb (sans délétions) | 2,0 | 2,45 | 3,10 | 3,68 | |
| **Analyse des séquences d'acides aminés** | | | | | |
| % de séquences (en poids) (+ mutation silencieuse) | 63% | 46% | 44% | 41% | |
| % de séquences avec décalage du cadre de lecture (+ codon stop) | 13% | 28% | 13% | 27% | |
| % de séquences avec acides aminés mutés | 24% | 25% | 43% | 32% | |
| Nombre de clones scFv avec des acides aminés mutés | 4,8E+06 | 1,5E+06 | 4,3E+07 | 1,6E+05 | 4,9E+07 |
| Nombre de mutations d'acides aminés par scFv | 1,3 | 1,2 | 1,8 | 2,11 | |
| Clones scFv avec 1 acide aminé muté | 65% | 49% | 50% | 33% | 2,5E+07 |
| Clones scFv avec 2 acides aminés mutés | 25% | 34% | 33% | 39% | 1,6E+07 |
| Clones scFv avec 3 acides aminés mutés | 10% | 13% | 8% | 6% | 4,2E+06 |
| Clones scFv avec 4 acides aminés mutés | - | 4% | 6% | 22% | 2,5E+06 |
| Clones scFv avec 5 acides aminés mutés | - | - | 3% | - | 1,2E+06 |

### 3.2. Mise au point des conditions de sélection.

Afin d'évaluer différentes stratégies de sélection, un mélange artificiel entre les phages-scFv murins et humanisé a été réalisé (mélange 1/200, mLFB112/huLFB112), l'objectif étant de simuler le criblage de la banque. Cette simulation de criblage doit permettre de valider différentes conditions de sélection qui ont pour finalité d'amplifier rapidement le clone le plus affin au sein de ce mélange artificiel (c'est à dire le clone murin mLFB112 dans ce cas). Les différentes stratégies évaluées :

   i) Utilisation d'une quantité constante de cible immobilisée au fil des tours de sélection (Cond 1)

   ii) Diminution de la quantité de cible immobilisée au fil des tours de sélection (Cond 2)

   ii) Essai d'une condition « $k_{off}$ » : temps d'incubation long des phages-scFv avec la cible (Cond 3)

Ces différentes conditions ont été réalisées sur 3 tours de sélection. Des séquençages ont été réalisés sur les clones retenus après chaque tour de sélection. Les résultats sont indiqués dans le tableau IV ci -dessous

Tableau IV. Evaluation de trois stratégies de criblage

| Conditions 1 | Bp1 | Bp2 | Bp3 |
|---|---|---|---|
| Recouvrement | 500 ng | 500 ng | 500 ng |
| Nombre de phages scFv utilisés pour le tour de sélection | 1,6E+11 | 1,2E+11 | 2,0E+11 |
| Nombre de phages scFv récupérés à l'issue du tour de sélection | 3,3E+5 | 2,4E+5 | 2,7E+6 |
| % de mLFB112/huLFB112 | 0/100 | 1/99 | 90/10 |
| | | | |
| Conditions 2 | Bp1 | Bp2 | Bp3 |
| Recouvrement | 500 ng | 100 ng | 100 ng |
| Nombre de phages scFv utilisés pour le tour de sélection | 1,6E+11 | 1,2E+11 | 1,8E+11 |
| Nombre de phages scFv récupérés à l'issue du tour de sélection | 3,3E+5 | 2,4E+5 | 2,7E+6 |

(suite)

| Conditions 2 | Bp1 | Bp2 | Bp3 |
|---|---|---|---|
| % de mLFB112/huLFB112 | 0/100 | 1/99 | 30/70 |

| Conditions 3 | Bp1 | Bp2 | Bp3 |
|---|---|---|---|
| Recouvrement | 500 ng | 100 ng $k_{off}$ | 100 ng $k_{off}$ |
| Nombre de phages scFv utilisés pour le tour de sélection | 1,6E+11 | 1,8E+11 | 1,6E+11 |
| Nombre de phages scFv récupérés à l'issue du tour de sélection | 3,3E+5 | 6,7E+5 | 2,0E+6 |
| % de mLFB 112/huLFB 112 | 0/100 | 1/99 | 30/70 |

Au vu de ces résultats, il apparaît que la condition 1 (quantité fixe de cible) est la plus performante pour amplifier le clone de meilleure affinité, le mLFB112 (9 clones sur 10). Utiliser une plus faible quantité de cible (100ng/puits) semble moins appropriée ; seulement 3 clones sur 10 après 3 tours de sélection correspondent au clone de meilleure affinité. De même pour la condition 3 basée sur un temps d'incubation long (« koff selection ») qui ne permet pas d'amplifier suffisamment le clone mLFB1 12. De plus le nombre de phages récupérés pour cette dernière condition à l'issue des 3 tours est peu élevé (2x104 phages). Il nous a donc semblé peu judicieux d'utiliser les conditions 2 et 3 pour un mélange plus diversifié de clones comme c'est le cas pour la banque construite dans le cadre de ce projet.

### 3.3. Criblage primaire (Tours de sélection).

Suite à la mise au point des conditions de criblages, nous avons donc décidé d'utiliser 2 conditions de criblage :

Cond A : 1 $\mu$g/puits de cible pendant 4 tours de sélection puis 2 tours avec 0.5$\mu$g/puits
Cond B : 0.5$\mu$g/puits pendant 6 tours de sélection.

Les résultats obtenus sont présentés dans le tableau V ci-dessous.

Tableau V : Résultats des tours de sélection.

| Conditions A | Tour de sélection 1 | Tour de sélection 2 | Tour de sélection 3 | Tour de sélection 4 | Tour de sélection 5 | Tour de sélection 6 |
|---|---|---|---|---|---|---|
| Recouvrement | 1$\mu$g | 1$\mu$g | 1$\mu$g | 1$\mu$g | 0,5$\mu$g | 0,5$\mu$g |
| Nombre de phages scFv utilisés pour le tour de sélection | 4,8E+11 | 8,0E+11 | 2,8E+11 | 8,0E+11 | 4,5E+11 | 6,0E+11 |
| Nombre de phages scFv récupérés à l'issue du tour de sélection | 2,0E+5 | 1,3E+5 | 2,4E+5 | 7,5E+5 | 7,3E+5 | 1,4E+5 |
| Conditions B | Tour de sélection 1 | Tour de sélection 2 | Tour de sélection 3 | Tour de sélection 4 | Tour de sélection 5 | Tour de sélection 6 |
| Recouvrement | 0,5$\mu$g | 0,5$\mu$g | 0,5$\mu$g | 0,5$\mu$g | 0,5$\mu$g | 0,5$\mu$g |
| Nombre de phages scFv utilisés pour le tour de sélection | 4,8E+11 | 8,0E+11 | 3,2E+11 | 6,4E+11 | 7,5E+10 | 4,2E+11 |
| Nombre de phages scFv récupérés à l'issue du tour de sélection | 2,0E+5 | 8,6E+5 | 5,4E+5 | 1,3E+5 | 2,6E+5 | 6,5E+5 |

A l'issue de ces sélections, les clones obtenus ont été séquencés à partir du 3ème tour de sélection. Les résultats obtenus ont été comparés à ceux obtenus pour la banque de départ (tableau VI).

Tableau VI : Résultats des séquençages.

| | Banque | Tour de sélection 3 | Tour de sélection 4 | Tour de sélection 5 | Tour de sélection 6 |
|---|---|---|---|---|---|
| Nombre de séquences | 295 | 87 | 168 | 114 | 98 |
| % de séquences avec acides aminés mutés | 39% | 26% | 26% | 35% | 20% |
| % de séquences avec décalage du cadre de lecture | 14% | 13% | 27% | 32% | 49% |
| % de séquences (en poids) (+ mutation silencieuse) | 47% | 61% | 48% | 34% | 32% |
| Banque : banque initiale issue du mélange des 4 banques R20U, 45M, R20US, R20Ueta. | | | | | |

L'analyse de ces séquençages a révélé la présence de clones redondants. Au total, sur l'ensemble des clones séquencés, nous avons obtenu 113 clones mutés uniques.

## 3.4. Criblage secondaire (ELISA-phages).

Le criblage secondaire consiste à analyser les clones sélectionnés à l'issue du criblage primaire de manière individuelle. Pour cela, les 113 clones mutés uniques ont été repiqués en plaque de culture (96 puits-1,2ml).

Après production des particules phagiques, les surnageants de culture contenant les phages-scFv ont été utilisés pour effectuer un test de liaison ELISA. La liaison des clones mutés a été évaluée à deux dilutions (½ et ¼ des surnageants contenant les scFv-phages). Les clones murins (mLFB112) et humanisés (huLFB112) construits sous le format scFv-phages ont été utilisés comme références sur chacune des plaques de test. Chacun des clones mutés a été testé 2 fois au minimum.

Les résultats sont exprimés sous la forme de ratio c'est-à-dire les différences de liaison (DO405nm) entre les clones mutés et les références huLFB112 et mLFB1 12.

- Ratio par rapport au scFv humanisé huLFB112 (Ratio / huLFB112)
- Ratio par rapport au scFv murin mLFB1 12 (Ratio / mLFB112)

Sur les 113 clones testés, seuls les meilleurs clones sont présentés ci-dessous.

Le tableau VII ci-après présente les différents clones obtenus et les mutations présentes (position et substitution des acides aminés) dans la chaîne légère et/ou lourde, ainsi que l'affinité de liaison de clones déterminée par Elisa.

Les valeurs indiquées après les substitutions correspondent aux modifications des valeurs valeurs de l'index hydropathique en fonction des différentes substitutions.

Les valeurs d'affinité de liaison correspondent au rapport de l'affinité de liaison de l'anticorps de l'invention pour le récepteur AMHRR-II sur l'affinité de liaison de l'anticorps humanisé 12G4 non muté ou l'affinité de liaison de l'anticorps chimérique 12G4 non muté.

Les valeurs d'affinité de liaison données sont le résultats de la moyenne d'au moins quatre valeurs et les chiffres entre parenthèse correspondent à l'écart type.

Le tableau VII montre qu'avec les substitutions effectuées, bien que celles-ci conduisent à une variation importante de l'index hydropathique, l'affinité de liaison de l'anticorps pour le récepteur est très supérieure à celle de l'anticorps humanisé 12G4 non muté et au moins égale à celle de l'anticorps chimérique 12G4 non muté: ratio AC invention/12G4 chimérique supérieur ou égal à 1.

L'anticorps humanisé muté présente une affinité rétablie voire même supérieure à celle de l'anticorps chimérique ou murin.

TABLEAU VII

| clone | Numérotation VH | Numérotation VL | FIXATION A LA CIBLE AMHRII-Fc DETERMINEE PAR ELISA | |
|---|---|---|---|---|
| | 1 - 115 | 131 - 236 | Ratio AC invention/12G4 humanisé | Ratio AC invention/12G4 chimérique |
| 4C_35 | L45P +3,8_-1,6 | E184K -3,5_-3,9 | 4,3 (0,5) | 1,9 0,4) |

(suite)

| clone | Numérotation VH 1 - 115 | Numérotation VL 131 - 236 | FIXATION A LA CIBLE AMHRII-Fc DETERMINEE PAR ELISA | |
|---|---|---|---|---|
| | | | Ratio AC invention/12G4 humanisé | Ratio AC invention/12G4 chimérique |
| 5B_81 | L45P +3,8_-1,6 | - | 3,5 (0,9) | 1,6 (0,3) |
| 6B_78 | - | E184K -3,5_-3,9 | NT | NT |
| 3C_23 | Q3R -3,5_-4,5 | I177T +4,5_-0,7 S179P -0,8_-1,6 | 2,6(1,1) | 1,2 (0,3) |
| 3C_23K | Q3R -3,5_-4,5 | I177T +4,5_-0,7 S179P -0,8_-1,6 E184K -3,5_-3,9 | NT | NT |
| 5B_42 | T74A -0,7_1,8 | S179P -0,8_-1,6 | NT | NT |
| 4F_196 | Q3E -3,5_-3,5 Q62R -3,5_-4,5 E89D -3,5_-3,5 | - | 3,0 (1,0) | 1,5 (0,3) |
| 6B_87 | Q1E -3,5_-3,5 A24V +1,8_+4,2 | - | 2,1 (0,2) | 1,0 (0,1) |
| 4F_169 | Q6E -3,5_-3,5 T58A -0,7_+1,8 | - | 2,0 (0,7) | 1,0 (0,2) |
| 3D_74 | - | S158P -0,8_-1,6 | 2 (0,7) | 0,8 (0,2) |
| 4C_44 | R87G -3,9_-0,4 | | NT | NT |
| 5A_66 | V67M +4,2_+1,9 | F212S +2,8_-0,8 | 2,2 (0,4) | 1,1 (0,1) |
| 6B_14 | S31G -0,8_-0,4 Q39E -3,5_-3,5 | - | 2,6 0,3) | 1,1 (0,2) |
| 4C_47 | Q3E -3,5_-3,5 S88P -0,8_-1,6 | - | NT | NT |
| 4E_153 | D56N -3,5_-3,5 I70N +4,5_-3,5 F102S +2,8_-0,8 | - | 2,0 (0,4) | 1,0 (0,1) |
| 3C_24 | - | E184G -3,5_-0,4 | NT | NT |
| 5B_18 | Q3E -3,5_-3,5 A9T +1,8_-0,7 A103T +1,8_-0,7 | - | 1,8 (0,2) | 0,9 (0,2) |
| 5B_84 | Q1E -3,5_-3,5 A24G +1,8_-0,4 | - | NT | NT |
| 6B_86 | Q3E -3,5_-3,5 | G179D -0,4_-3,5 | NT | NT |
| 4D_91 | Q1E -3,5_-3,5 V11A +4,2_+1,8 | - | 1,7 (0,1) | 0,8 (0,2) |
| 6B_76 | A40V +1,8_+4,2 | S179P -0,8_-1,6 | 1,7 (0,1) | 0,8 (0,2) |
| 5A_28 | - | Y178H -1,3_-3,2 S179P -0,8_-1,6 | 1,6 (0,6) | 0,8 (0,2) |
| 3D_57 | A76T +1,8_-0,7 A79T +1,8_-0,7 | - | NT | NT |

(suite)

| clone | Numérotation VH 1 - 115 | Numérotation VL 131 - 236 | FIXATION A LA CIBLE AMHRII-Fc DETERMINEE PAR ELISA | |
|---|---|---|---|---|
| | | | Ratio AC invention/12G4 humanisé | Ratio AC invention/12G4 chimérique |
| 6A_80 | A24V +1,8_+4,2 Q62E -3,5_-3,5 | - | 1,5 (0,2) | 1,3 (0,1) |
| 5B_67 | K12R -3,9_-4,5 | - | NT | NT |
| 5B_86 | S31G -0,8_-0,4 Q39E -3,5_-3,5 | I132T +4,5_-0,7 A143T +1,8_-0,7 | NT | NT |
| 5A_73 | A24V +1,8_+4,2 | - | NT | NT |
| 5B_33 | A76T +1,8_-0,7 | - | NT | NT |
| 3B_71 | S114T -0,8_-0,7 | S179P -0,8_-1,6 | NT | NT |
| 3B_87 | - | L175Q +3,8_-3,5 | NT | NT |
| 3D_68 | - | T150A -0,7_1,8 | NT | NT |
| 4E_112 | L110P +3,8_-1,6 | V187A +4,2_+1,8 S192T -0,8_-0,7 | NT | NT |
| 5B_54 | A24T +1,8_-0,7 | - | NT | NT |
| 6A_18 | K13R -3,9_-4,5 | - | NT | NT |
| 3C_40 | - | P224A -1,6_+1,8 | NT | NT |
| 5A_83 | Q62E -3,5_-3,5 S179P -0,8_-1,6 A79T +1,8_-0,7 | - | NT | NT |
| 5A_19 | Q3E -3,5_-3,5 | S182F -0,8_+2,8 | NT | NT |
| 3A_29 | V20A +4,2_+1,8 | - | NT | NT |
| (Q1E, Q3E, Q6E, K19E, Q39E et Q62E: codon TAG suppressé, traduit en E dans les bactéries E.Coli XL1-blue utilisées) NT : non testé | | | | |

### Exemple 4 : Comparaison des clones présentant une amélioration d'affinité

Les clones positifs 3C_23K, 3C_23 et 6B_78 ont été comparés entre eux par détermination de l'affinité de la liaison de l'anticorps au récepteur AMHR-II dans un test Elisa classique obtenue avec des anticorps mutés (Fab) solubles selon l'invention.
Les résultats obtenus sont présentés figure 17.

### Exemple 5 : Etablissement de la lignée transfectée AMHRII cov434-AMHRII

La lignée cov434-AMHRII a été générée par transfection d'un plasmide exprimant l'ADNc codant AMHRII dans la lignée de tumeur de la granulosa cov434 (van den Berg-Bakker, C., et al, 1993. Establishment and characterization of 7 ovarian carcinoma cell lines and one granulosa tumor cell line: Growth features and cytogenetics. International Journal of Cancer 53:613 ; Zhang, H. et al, 2000. Characterization of an immortalized human granulosa cell line (COV434). Molecular Human Reproduction 6:146) n'exprimant pas AMHRII.
Brièvement, l'ADNc de AMHRII a été cloné dans le plasmide commercial pIRES-neo (Clontech - Takara Bio Europe, France ; références 6060-1). Grâce à la séquence IRES, AMHRII et neo sont exprimés sous le contrôle d'un même promoteur CMV (Figure 26).
Cette construction a été transfectée de façon stable dans la lignée de cancer de la granulosa cov434 (agent de transfection Fugene, Roche). Les transfectants obtenus ont été ensuite criblés par cytométrie et par western blot pour l'expression du récepteur AMHRII. Après sous-clonage le clone cellulaire cov434-AMHRII-1F3, contenant un vecteur de type pIRES-neo, a été retenu pour les études *in vitro* et *in vivo*. Cette lignée est dénommée ci-après cov434-AMHRII.

**Exemple 6 : Etablissement de lignées primaires à partir de liquide d'ascite ou de biopsie de patientes atteintes de cancer épithélial ovarien**

Les principale étapes d'établissement des lignées dérivées de prélèvements d'ascites (lignée Asc 1) sont les suivantes :

- J0 : Réception du liquide d'ascite et mise en culture immédiate du prélèvement. Le prélèvement est centrifugé 5 min à 1000 tr/min et le culot repris dans 2mL de milieu pour ensemencement d'un flacon T25 (milieu RPMI 10%FCS).

- J2 - J46: Culture avec observation microscopique régulière. Du milieu neuf est ajouté régulièrement durant cette période. Des lavages PBS sont également effectués tous les 2 jours afin d'éliminer les hématies et les fibroblastes contaminants.
Vers J13 toutes les cellules contaminantes ont disparu et l'on observe un tapis confluant de cellules encore hétérogène (deux types différents).

- J46 : Repiquage : à J46 un tapis homogène de cellules tumorales est observé et les cellules sont alors lavées et repiquées par grattage pour être ré-encemencées dans deux flacons T75.

- J61 : Evaluation de l'expression AMHRII par FACS : après lavage PBS, les cellules sont décollées au grattoir et analysées par cytométrie en flux (marquage AcM 12G4 10$\mu$g/mL, AcII anti-souris FITC).la réalisation des banques est effectuée à ce stade. La lignée AMHRII positive est maintenue en culture pour 10 jours supplémentaires afin de confirmer l'expression du récepteur AMHRII.

- J71 : Confirmation de l'expression AMHRII par FACS.

Les lignées ainsi établies sont entretenues en milieu RPMI 10%FCS, avec un passage par semaine (dilution 1/15ème).
Dans le cas de biopsies (lignée META 2815), la tumeur primaire est tout d'abord entretenue sur souris nude (greffes du prélèvement dans l'espace interscapulaire, 3 passages successifs sur souris) puis la tumeur est prélevée et dilacérée avant reprise dans du milieu de culture. Un protocole identique à celui des prélèvements d'ascites est ensuite appliqué.

**Exemple 7 : Evaluation de l'affinité des différents anticorps humanisés candidats**

Cette étude a été réalisée sur l'anticorps murin 12G4 d'origine ainsi que sur les anticorps humanisés candidats 3C23, 6B78 et 3C23K (de séquence respective 3C_23, 6B_78 et 3C_23K) produits dans YB2/0. L'affinité de ces anticorps a été évaluée sur les cellules cov434-AMHRII.
Brièvement, la détermination du $K_D$ a été effectuée selon la méthode de saturation, par l'addition de doses croissantes d'anticorps radiomarqué à un nombre constant de cellules cov434-AMHRII. Les cellules ($1\times10^6$ dans 50 $\mu$l PBS/BSA 0,5%) ont été incubées (volume final 150 $\mu$l) pendant 1h à 4°C en présence de doses croissantes d'anticorps préalablement marqués à l'iode 125 ($^{125}$I). Pour chaque anticorps, des dilutions de 4 en 4 ont été effectuées en PBS/BSA 0,5% à partir des solutions d'anticorps marqué et non marqué (84,4 $\mu$g/ml). La fixation non spécifique a été évaluée par l'incubation des cellules en présence d'un excès 100 fois molaire d'anticorps non marqué.
Après incubation, les échantillons ont été congelés dans l'azote liquide puis analysés dans un compteur gamma. La fixation spécifique a été déterminée par soustraction de la fixation obtenue en présence de l'excès d'anticorps non marqué.
L'analyse Scatchard, réalisée sur logiciel PRISM, a permis de déterminer les constantes d'affinités présentées dans le tableau VIII.

Tableau VIII : Constante de dissociation ($K_D$) des anticorps anti-AMHRII

|  | 12G4 murin | 3C23 | 6B78 | 3C23K |
|---|---|---|---|---|
| $K_D$ (nM) | 15,41 +/- 0,97 | 7,33 +/-0,44 | 6,68 +/- 0,21 | 5,30 +/- 0,38 |

D'après cette étude, il apparaît que l'anticorps anti-AMHRII humanisé 3C23K possède la meilleure affinité ($K_D$ = 5.3 nM) comparé aux deux autres anticorps candidats 6B78 et 3C23. L'anticorps 3C23K présente aussi une affinité environ trois fois supérieure à celle de l'anticorps murin d'origine 12G4 ($K_D$ = 15,4 nM).

**Exemple 8 : Comparaison de l'activité ADCC des anticorps de l'invention versus l'activité ADCC de l'anticorps 12G4 humanisé non muté.**

**1 MATERIEL ET METHODES**

1.1 *Principe des méthodes*

ADCC

Les cellules cibles ASC1 issues de patientes sont adhérentes et sont préparées la veille du test. Elles sont décollées par la trypsine et incubées en EMS + 5%SVF en plaque fond plat à raison de 50 μl par puits à la concentration de 6 x 10$^5$ cellules/ml. Les plaques sont incubées la nuit à 37°C, 7% CO2.
Le lendemain, les cellules ont adhérées dans le fond du puits. Le surnageant est aspiré et le volume de tampon nécessaire par puits est ajouté pour réaliser le test en présence de NK et d'anticorps.
Les cellules tueuses (cellules NK) sont préalablement purifiées par la technique de déplétion négative développée par la société Miltenyi (Miltenyi Biotec - NK cell isolation kit human réf 130-092-657), à partir de sang périphérique de donneurs sains. La technique ADCC consiste à incuber les cellules NK avec des cellules cibles ASC1 en présence de différentes concentrations de l'anticorps anti-AMHRII humanisés (0.005 à 5000 ng/ml) avec un ratio E/T de 10/1. Après 4 heures d'incubation, l'activité cytotoxique induite par les anticorps anti-AMHRII est mesurée par colorimétrie en dosant dans les surnageants, une enzyme intracellulaire nommée lactate déshydrogénase (LDH) libérée par les cellules cibles lysées (Roche Diagnostics - Cytotoxicity Détection Kit LDH réf 11644793001).

1.2 Eléments d'étude

- Anticorps anti-AMHRII :

  ○ 829 10 054, YB2/0 humanisé, R901 3C23K
  ○ 829 10 050, YB2/0 humanisé, R901 3C23
  ○ 829 10 051, YB2/0 humanisé, R901 6B78
  ○ 632 07 107, anti-AMHRII 12G4 humanisé non muté

- Cellules ASC1 dossier de culture 871 10 063

Les résultats sont présentés dans la figure 25.
Le tableau IX présente les données brutes correspondant à la Figure 25.

Tableau IX:

| Ac ng/ml | Ac ng/ml (Log) | 829 10 050 3C23 YB20 | 829 10051 6B78 YB20 | 829 10 054 3C23K YB20 | 632 07 107 Anti AMHRII hum 1st |
|---|---|---|---|---|---|
| | | % of lysis (ADC 1193 11 081) | | | |
| 0,001 | -3,000 | 0 | 0 | 0 | 0 |
| 0,005 | -2,301 | 8 | 14 | 8 | 18 |
| 0,05 | -1,301 | 9 | 5 | 10 | 2 |
| 0,5 | -0,301 | 13 | 3 | 26 | 0 |
| 5 | 0,699 | 35 | 28 | 51 | 7 |
| 50 | 1,699 | 46 | 42 | 67 | 24 |
| 500 | 2,699 | 52 | 48 | 79 | 50 |

(suite)

| Ac ng/ml | Ac ng/ml (Log) | % of lysis (ADC 1193 11 081) | | | |
|---|---|---|---|---|---|
| | | 829 10 050 3C23 YB20 | 829 10051 6B78 YB20 | 829 10 054 3C23K YB20 | 632 07 107 Anti AMHRII hum 1st |
| 5000 | 3,699 | 63 | 53 | 75 | 50 |

Le tableau X présente les Emax et EC50 obtenus avec les différents anticorps.

Tableau X

| | 82910 050 3C23 YB20 | 82910 051 6B78 YB20 | 829 10 054 3C23K YB20 | 632 07 107 Anti AMHRII hum 1st |
|---|---|---|---|---|
| Emax (% of lysis) | 65,55 | 51,89 | 79,02 | 52,40 |
| EC50 (ng/ml) | 6,298 | 5,383 | 1,704 | 52,35 |

**Exemple 9 : Comparaison de l'activité ADCC des anticorps de l'invention versus l'anticorps 12G4 chimérique**

L'activité ADCC de l'anticorps candidat humanisé 3C_23K (ha12G4 de séquence 3C_23K : mutations VHQ3R (SEQ ID N°:82 (sans leader) ou SEQ ID NO: 84 (avec leader)), et VLI177T/S179P/E184K (SEQ ID NO: 86 (sans leader), ou SEQ ID NO: 88 (avec leader)) a été évaluée.

Brièvement, les cellules effectrices (cellules tueuses NK pour « Natural Killer ») sont préalablement purifiées par la technique de déplétion négative développée par la société Miltenyi (Miltenyi Biotec - NK cell isolation kit human réf 130-092-657), à partir de sang périphérique de donneurs sains et cela après une première étape de purification des cellules mononucléées sur Ficoll.

Le test *in vitro* d'activité ADCC consiste à incuber les cellules NK avec des cellules cibles (lignées cov434-AMHRII, Asc 1 et META 2815), en présence de différentes concentrations d'anticorps anti-AMHRII (anticorps chimérique ch12G4, anticorps humanisés 3C_23K-YB2/0, produit dans YB2/0, et 3C_23K-CHO, produit dans CHO). Le rapport effecteur/cible appliqué est de 15/1. Les anticorps sont dilués en milieu de culture à des concentrations allant de 0.005 à 5000 ng/ml.

Après 4 heures d'incubation, l'activité cytotoxique induite par les anticorps anti-AMHRII est mesurée par colorimétrie en dosant dans les surnageants une enzyme intracellulaire nommée lactate déshydrogénase (LDH) libérée par les cellules cibles lysées (Roche Diagnostics - Cytotoxicity Détection Kit LDH réf 11644793001).

Le pourcentage de lyse est calculé selon la formule suivante :

$$\% \text{ lyse} = [(ER - SR) / (100 - SR)] - [(NC - SR) / (100 - SR)]$$

Avec : ER = release de LDH en présence d'anticorps et de cellules NK
SR = release de LDH spontané de la cellule cible seule
NC = release de LDH en présence de cellules NK et absence d'anticorps.

Les résultats sont exprimés en pourcentage de lyse en fonction de la quantité d'anticorps.

Les valeurs Emax et EC50 sont calculées à l'aide du logiciel PRISM.

Les résultats obtenus sur la lignée cov434-AMHRII sont présentés dans la figure 27. La faible activité de l'anticorps 3C_23K-CHO ne permet pas d'obtenir un plateau dans les conditions du test. Afin de comparer l'efficacité des anticorps le calcul des 50% relatif est dans ce cas effectué, qui représente la quantité d'anticorps 3C_23K-CHO requise pour atteindre 50% du plateau de l'anticorps chimérique (50% relatif = 1). D'après cette évaluation, l'anticorps humanisé possédant la meilleure activité ADCC sur la lignée COV434-AMRHII est l'anticorps 3C_23K (50% relatif : 0.84) produit dans YB2/0. Les anticorps humanisés de séquence 3C_23 et 6B_78 possèdent une valeur de 50% relatif égale à 6.41 et 36.92, respectivement.

Les résultats obtenus sur la lignée Asc 1 sont présentés dans la figure 28. L'anticorps 3C_23K-YB2/0 possède une activité cytotoxique dose dépendante sue les cellules Asc 1 avec un EC50 estimé à 2.24 ng/ml. La faible activité de l'anticorps 3C _23K-CHO ne permet pas d'obtenir un plateau dans les conditions du test. Afin de comparer l'efficacité des deux anticorps, le calcul des 50% relatif est dans ce cas effectué, qui représente la quantité d'anticorps 3C_23K-CHO requise pour atteindre 50% du plateau de l'anticorps 3C_23K-YB2/0 (50% relatif = 1). D'après cette évaluation, l'activité cytotoxique de l'anticorps 3C_23K-YB2/0 est environ 40 fois supérieure à celle de l'anticorps produit dans CHO (50% relatif = 39.4).

De façon similaire, les résultats présentés dans la figure 29 montrent que les anticorps 3C_23K-YB2/0 et 3C_23K-CHO induisent une lyse dose dépendante des cellules META 2815. L'activité cytotoxique de l'anticorps 3C_23K-YB2/0 (EC50 = 30,5ng/ml) est environ 146 fois supérieure à celle de l'anticorps 3C_23K-CHO (EC50 = 466,9 ng/ml).

L'ensemble de ces résultats indiquent que les anticorps anti-AMHRII 3C_23K produits dans YB2/0 ont la capacité d'induire une lyse des cellules exprimant l'antigène AMHRII. La différence d'EC50 entre les anticorps anti-AMHRII-YB2/0 et anti-AMHRII-CHO suggère un avantage particulier à l'anticorps anti-AMHRII-YB2/0 dans des conditions de faible expression antigénique, ou de faible pénétrance de l'anticorps à la tumeur.

### Exemple 10 : Etudes de prolifération cellulaires

L'inhibition de la prolifération cellulaire a été mise en évidence en mesurant la croissance cellulaire au cours du temps en présence ou non des anticorps anti-AMHRII testés.

Brièvement, les cellules cibles (cov434-AMHRII, Asc 1, META2815) sont mises en culture dans des plaques P6 (1 x 10^5 cells/puits) pendant 72h à 37°C, en présence des anticorps anti-AMHRII (10μg/ml) exprimés dans CHO ou YB2/0 et cela avec ou sans agent de réticulation (AffiniPure F(ab')2 Fragment Goat Anti-Human IgG, Fcγ Fragment Spécifié réf : 109-006-008, Jackson Immunoresearch, France). Les cellules sont traitées par la trypsine pendant 5 minutes puis comptées au CEDEX, un compteur de cellules automatique basé sur la viabilité cellulaire (bleu trypan). Un témoin positif d'inhibition de la prolifération est établi en présence de 1μg/ml de colchicine (Réf : C3915, Sigma-Aldrich, France). Un témoin négatif est établi en présence d'un anticorps non relevant (anti-P24). Toutes les dilutions sont faites en milieu de culture (RPMI, 10% SVF). Les résultats sont exprimés en pourcentage de prolifération, la valeur 100% correspondant à la prolifération des cellules observée en l'absence d'anticorps

Les résultats obtenus avec la lignée cov434-AMHRII sont présentés en figure 30.

D'après ces observations les anticorps 3C_23K-YB2/0 et 3C_23K-CHO induisent environ 40% d'inhibition de la prolifération cellulaire des cellules cov434-AMHRII et cela en présence d'un agent de réticulation (CK). Cet effet cytostatique n'est pas observé en présence d'un anticorps non relevant (anticorps p24) tandis que le contrôle positif colchicine (10 μg/ml) induit 88% d'inhibition.

De façon similaire, les résultats présentés dans la figure 31 montrent que les anticorps 3C_23K-YB2/0 et 3C_23K-CHO induisent environ 40% d'inhibition de la prolifération cellulaire sur la lignée META 2815 en présence d'un agent de réticulation (CK).

Cette inhibition de prolifération cellulaire pourrait être la conséquence d'une signalisation cellulaire induite par les anticorps anti-AMHRII sur les lignées cov434-AMHRII et META 2815.

### Exemple 11 : Effet in vivo de l'anticorps 3C 23K-YB2/0 sur des tumeurs COV434-AMHRII

L'efficacité antitumorale de l'anticorps 3C_23K-YB2/0 a été évaluée en traitement tardif sur des souris femelles swiss nude après injection sous-cutanée (s.c.) de cellules tumorale COV434-AMHRII. Les injections (inj) intrapéritonéale (i.p.) d'anticorps ont été réalisées à 2-3 jours d'intervalle à une dose de 10 mg/kg/inj pour un total de 18 injections. Le groupe traité par l'anticorps 3C_23K-YB2/0 a été comparé au groupe traité par le véhicule (PBS).

- Matériel et méthodes

Des souris femelles swiss nude ont été utilisés (Harlan). Au jour 0 de l'expérience, les souris ont reçu une injection sous-cutanée de 7.10^6 cellules tumorales COV434-AMHRII mélangées avec du matrigel (ratio 1 :1). Les animaux ont ensuite été traités par injection i.p. de PBS ou 3C_23K-YB2/0 avec 10 mg/kg/inj à partir du jour 16 (volume tumoraux compris entre 84-270mm3, 3 injections par semaine pendant 6 semaines (18 injections totales).

La mesure du volume tumoral a été réalisée 2 à 3 fois par semaine. Le volume tumoral (TV) a été calculé en utilisant la formule suivante:

$$TV \ (mm3) = (longueur \times largeur \times hauteur)/2,$$

dans laquelle la longueur correspond au plus large diamètre de la tumeur et la largeur au plus petit diamètre de la tumeur.

Les courbes de croissance tumorale ont été tracées en utilisant la moyenne des volumes tumoraux (MTV). Les animaux ont été euthanasiés lorsque le volume individuel de la tumeur avait atteint 2000 mm3. Dans chacun des groupes, les courbes ont été arrêtées lorsque 30% des animaux du groupe avaient été euthanasiées.

L'inhibition de la croissance tumorale (T/C) définie comme le ratio du volume tumoral médian des groupes traités par rapport au groupe contrôle traité véhicule a été calculé comme suit : T/C = (TV médian du groupe traité/TV médian du groupe véhicule) x 100

- T/C supérieur à 42%, le produit est considéré comme inefficace.
- T/C entre 42% et 10%, le produit présente un effet anti-tumoral
- T/C inférieur à 10%, le produit est réellement efficace.

Les différences statistiques entre les différents groupes ont été obtenues avec le test de Kruskal-Wallis, en utilisant la comparaison ANOVA (logiciel Statgraphics centurion XV). Les différences ont été considérées comme significatives si $P<0,05$. Un test logrank, permettant de comparer les paramètres de survie de l'étude, a également été réalisé via ANOVA (logiciel Statgraphics centurion XV). Les différences ont été considérées comme significatives si $P<0,05$.

- Résultats

L'anticorps 3C_23K-YB2/0 montre une activité anti-tumorale puisqu'un retard est observé dans la COV434-AMHRII (figures 32A et 32B).

La comparaison statistique des volumes tumoraux à chaque point de mesure, une fois le traitement initié, montre que l'anticorps 3C_23K-YB2/0 retarde la croissance tumorale (Kruskal-Wallis, via ANOVA). Le rapport T/C calculé entre les groupes traités par le 3C_23K-YB2/0 et véhicule montre une différence significative a tous les points de mesure également une fois le traitement initié. Le test logrank montre également qu'en terme de survie le groupe traité 3C_23K-YB2/0 est statistiquement différent du groupe traité par le véhicule.

Le tableau XI ci-dessous présente l'évolution des volumes tumoraux (ratio traité/contrôle, T/C en %) sous l'effet du traitement par 3C_23K-YB2/0 dans le modèle cov434-AMHRII.

Tableau XI

| Jour de mesure | 15 | 21 | 25 | 30 | 32 |
|---|---|---|---|---|---|
| T/C % | 108 | 60 | 42 | 22 | 13 |

Le tableau XII ci-dessous présente les analyses statistiques obtenues dans le modèle cov434- AMHRII.

Tableau XII

| Jour de mesure | Anova | | | Kruskall wallis | | |
|---|---|---|---|---|---|---|
| | F-ratio | P-value | Sig. | Test | P-value | Sig. |
| 15 | 0,03 | 0,8628 | | 0,00577 | 0,93945 | |
| 21 | 8,34 | 0,0098 | * | 5,67427 | 0,01721 | * |
| 5 | 12,95 | 0,0021 | * | 10,56570 | 0,00115 | * |
| 30 | 34,94 | 0,0000 | * | 13,73030 | 0,00021 | * |
| 32 | 39,04 | 0,0000 | * | 12,90670 | 0,00033 | * |

**Exemple 12 : Effet in vivo de l'anticorps 3C_23K-YB2/0 sur des tumeurs Asc1A5**

L'efficacité antitumorale de l'anticorps 3C_23K-YB2/0 a été évaluée en traitement tardif sur des souris femelles swiss nude après injection sous-cutanée (s.c.) de cellules tumorale Asc1A5 (clone de la lignée Asc 1 d'origine). Les injections (inj) intrapéritonéales (i.p.) d'anticorps ont été réalisées à 2-3 jours d'intervalle à une dose de 10 mg/kg/inj pour un total de 18 injections. Le groupe traité par l'anticorps 3C_23K-YB2/0 a été comparé au groupe traité par le véhicule (PBS).

- Matériel et méthodes

Des souris femelles swiss nude ont été utilisés (Harlan). Au jour 0 de l'expérience, les souris ont reçu une injection sous-cutanée de $7.10^6$ cellules tumorales Asc1A5 mélangées avec du matrigel (ratio 1 :1). Les animaux ont ensuite été traités par injection i.p. de PBS ou 3C_23K-YB2/0 avec 10 mg/kg/inj à partir du jour 12 (volume tumoraux compris entre 40-160mm3, 3 injections par semaine pendant 6 semaines (18 injections totales).

La mesure du volume tumoral a été réalisée 2 à 3 fois par semaine. Le volume tumoral (TV) a été calculé en utilisant la formule suivante :

$$TV \ (mm3) = (longueur \ x \ largeur \ x \ hauteur)/2,$$

dans laquelle la longueur correspond au plus large diamètre de la tumeur et la largeur au plus petit diamètre de la tumeur.

Les courbes de croissance tumorale ont été tracées en utilisant la moyenne des volumes tumoraux (MTV). Les animaux ont été euthanasiés lorsque le volume individuel de la tumeur avait atteint 2000 mm$^3$. Dans chacun des groupes, les courbes ont été arrêtées lorsque 30% des animaux du groupe avaient été euthanasiés.

L'inhibition de la croissance tumorale (T/C) définie comme le ratio du volume tumoral médian des groupes traités par rapport au groupe contrôle traité véhicule a été calculé comme suit : T/C = (TV médian du groupe traité/TV médian du groupe véhicule) x 100

- T/C supérieur à 42%, le produit est considéré comme inefficace.
- T/C entre 42% et 10%, le produit présente un effet anti-tumoral
- T/C inférieur à 10%, le produit est réellement efficace.

Les différences statistiques entre les différents groupes ont été obtenues avec le test de Kruskal-Wallis, en utilisant la comparaison ANOVA (logiciel Statgraphics centurion XV). Les différences ont été considérées comme significatives si P<0,05. Un test logrank, permettant de comparer les paramètres de survie de l'étude, a également été réalisé via ANOVA (logiciel Statgraphics centurion XV). Les différences ont été considérées comme significatives si P<0,05.

- Résultats

L'anticorps 3C_23K-YB2/0 montre une activité anti-tumorale puisqu'un retard est observé dans la croissance tumorale comparativement au groupe traité par le véhicule dans le modèle Asc1a5 (figures 33A et 33B).

La comparaison statistique des volumes tumoraux à chaque point de mesure, une fois le traitement initié, montre que l'anticorps 3C_23K-YB2/0 retarde la croissance tumorale (Kruskal-Wallis, via ANOVA). Le rapport T/C calculé entre les groupes traité par le 3C_23K-YB2/0 et véhicule montre une différence significative à tous les points de mesure également une fois le traitement initié. Le test logrank montre également qu'en terme de survie le groupe traité 3C_23K-YB2/0 est statistiquement différent du groupe traité par le véhicule.

Le tableau XIII ci-dessous présente l'évolution des volumes tumoraux (ratio traité/contrôle, T/C en %) sous l'effet du traitement par 3C_23K-YB2/0 obtenus dans le modèle Asc1a5

Tableau XIII

| Jour de mesure | 12 | 17 | 24 | 27 | 31 | 35 |
|---|---|---|---|---|---|---|
| T/C % | 101 | 33 | 8 | 8 | 7 | 6 |

Le tableau XIV ci-dessous présente les analyses statistiques obtenues dans le modèle Asc1a5.

Tableau XIV

| Jour de mesure | Anova | | | Kruskall wallis | | |
|---|---|---|---|---|---|---|
| | F-ratio | P-value | Sig. | Test | P-value | Sig. |
| 12 | 0,02 | 0,8817 | | 0,0995 | 0,7523 | |
| 17 | 29,19 | 0,0001 | * | 11,3108 | 0,0007 | * |
| 24 | 103,56 | 0 | * | 11,2941 | 0,0007 | * |
| 27 | 32,08 | 0,0001 | * | 11,3108 | 0,0007 | * |
| 31 | 33,37 | 0 | * | 11,3274 | 0,0007 | * |
| 35 | 57,97 | 0 | * | 10,5788 | 0,0011 | * |

**Exemple 13 : Effet in vivo de l'anticorps 3C_23K-YB2/0 sur des tumeurs Meta2815**

L'efficacité antitumorale de l'anticorps 3C_23K-YB2/0 a été évaluée en traitement tardif sur des souris femelles swiss nude après injection sous-cutanée (s.c.) de cellules tumorale Meta 2815. Les injections (inj) intrapéritonéale (i.p.) d'anticorps ont été réalisées à 2-3 jours d'intervalle à une dose de 10 mg/kg/inj pour un total de 18 injections. Le groupe traité par l'anticorps 3C_23K-YB2/0 a été comparé au groupe traité par le véhicule (PBS).

- Matériel et méthodes

Des souris femelles swiss nude ont été utilisés (Harlan). Au jour 0 de l'expérience, les souris ont reçu une injection sous-cutanée de $8.10^6$ cellules tumorales Meta2815. Les animaux ont ensuite été traités par injection i.p. de PBS ou 3C_23K-YB2/0 avec 10 mg/kg/inj à partir du jour 33 (volume tumoraux compris entre 45-240mm3, 3 injections par semaine pendant 6 semaines (18 injections totales).
La mesure du volume tumoral a été réalisée 2 à 3 fois par semaine. Le volume tumoral (TV) a été calculé en utilisant la formule suivante:

$$TV \ (mm3) = (longueur \ x \ largeur \ x \ hauteur)/2,$$

dans laquelle la longueur correspond au plus large diamètre de la tumeur et la largeur au plus petit diamètre de la tumeur. Les courbes de croissance tumorale ont été tracées en utilisant la moyenne des volumes tumoraux (MTV). Les animaux ont été euthanasiés lorsque le volume individuel de la tumeur avait atteint 2000 mm3. Dans chacun des groupes, les courbes ont été arrêtées lorsque 30% des animaux du groupe avaient été euthanasiés.
L'inhibition de la croissance tumorale (T/C) définie comme le ratio du volume tumoral médian des groupes traités par rapport au groupe contrôle traité véhicule a été calculé comme suit : T/C = (TV médian du groupe traité/TV médian du groupe véhicule) x 100

-    T/C supérieur à 42%, le produit est considéré comme inefficace.
-    T/C entre 42% et 10%, le produit présente un effet anti-tumoral
-    T/C inférieur à 10%, le produit est réellement efficace.

Les différences statistiques entre les différents groupes ont été obtenues avec le test de Kruskal-Wallis, en utilisant la comparaison ANOVA (logiciel Statgraphics centurion XV). Les différences ont été considérées comme significatives si P<0,05. Un test logrank, permettant de comparer les paramètres de survie de l'étude, a également été réalisé via ANOVA (logiciel Statgraphics centurion XV). Les différences ont été considérées comme significatives si P<0,05.

- Résultats

L'anticorps 3C_23K-YB2/0 montre une activité anti-tumorale puisqu'un retard est observé dans la croissance tumorale comparativement au groupe traité par le véhicule dans le modèle Meta2815 (figures 34A et 34B).
La comparaison statistique des volumes tumoraux à chaque point de mesure, une fois le traitement initié, montre que l'anticorps 3C_23K-YB2/0 retarde la croissance tumorale (Kruskal-Wallis, via ANOVA). Le rapport T/C calculé entre les groupes traité par le 3C_23K-YB2/0 et véhicule montre une différence significative à tous les points de mesure également une fois le traitement initié. Le test logrank montre également qu'en terme de survie le groupe traité 3C_23K-YB2/0 est statistiquement différent du groupe traité par le véhicule.
Le tableau XV ci-dessous présente l'évolution des volumes tumoraux (ratio traité/contrôle, T/C en %) sous l'effet du traitement par 3C_23K-YB2/0 dans le modèle META 2815

Tableau XV

| Jour de mesure | 33 | 38 | 42 | 47 | 52 |
|---|---|---|---|---|---|
| T/C % | 92 | 38 | 26 | 22 | 21 |

Le tableau XVI présente les analyses statistiques obtenues dans le modèle META 2815

Tableau XVI

| Jour de mesure | Anova | | | Kruskall wallis | | |
|---|---|---|---|---|---|---|
| | F-ratio | P-value | Sig. | Test | P-value | Sig. |
| 33 | 0 | 0,9899 | | 0 | 1 | |
| 38 | 11,21 | 0,0007 | * | 8,30769 | 0,0039 | * |
| 42 | 9,51 | 0,0116 | * | 7,41026 | 0,0064 | * |
| 47 | 12,7 | 0,0052 | * | 8,33684 | 0,0038 | * |
| 52 | 16,14 | 0,0024 | * | 8,30769 | 0,0039 | * |

SEQUENCE LISTING

<110> LFB Biotechnologies

<120> ANTICORPS HUMANISES MUTES DIRIGES CONTRE LE RECEPTEUR DE L'HORMONE ANTI-MULERIENNE DE TYPE II

<130> WOB 10 AC LFB AMHR

<150> FR 10/53712
<151> 2010-05-12

<160> 102

<170> PatentIn version 3.5

<210> 1
<211> 318
<212> DNA
<213> VL sans leader 12G4 humanisé

<220>
<221> CDS
<222> (1)..(318)

<400> 1

EP 2 569 333 B1

```
gac atc cag atg aca cag tcc cca tct acc ctg tct gct tcc gtg gga        48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc        96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg atc tac       144
Ala Trp Tyr Gln Gln Lys Pro Gly Lys Ala Pro Lys Leu Leu Ile Tyr
            35                  40                  45

tca acc tcc tcc ctg gaa tcc ggg gtg ccc agc aga ttc tca ggc agt       192
Ser Thr Ser Ser Leu Glu Ser Gly Val Pro Ser Arg Phe Ser Gly Ser
        50                  55                  60

ggc tcc ggc acc gaa ttc acc ctg acc atc agc tca ctg cag cct gac       240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca       288
Asp Phe Ala Thr Tyr Tyr Cys Leu Gln Trp Ser Ser Tyr Pro Trp Thr
                85                  90                  95

ttc ggc ggc ggc acc aag gtg gag atc aag                               318
Phe Gly Gly Gly Thr Lys Val Glu Ile Lys
            100                 105
```

<210> 2
<211> 106
<212> PRT
<213> VL sans leader 12G4 humanisé

<220>
<223> VL sans leader 12G4 humanisé

<400> 2

36

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Ile Tyr
            35                  40                  45

Ser Thr Ser Ser Leu Glu Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys
            100                 105
```

<210> 3
<211> 384
<212> DNA
<213> Artificial Sequence

<220>
<223> VL avec leader 12G4 humanisé

<220>
<221> CDS
<222> (1)..(384)

<400> 3

```
atg gat atg aga gtg ccc gca cag ctg ctg ggt ctg ctg ctg ctg tgg        48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc        96
Leu Pro Gly Ala Lys Cys Asp Ile Gln Met Thr Gln Ser Pro Ser Thr
            20                  25                  30

ctg tct gct tcc gtg gga gat cgg gtg act atc acc tgc aga gca agc       144
Leu Ser Ala Ser Val Gly Asp Arg Val Thr Ile Thr Cys Arg Ala Ser
        35                  40                  45

tcc tcc gtg agg tac atc gct tgg tac cag cag aag cca gga aag gcc       192
Ser Ser Val Arg Tyr Ile Ala Trp Tyr Gln Gln Lys Pro Gly Lys Ala
    50                  55                  60

cca aag ctg ctg atc tac tca acc tcc tcc ctg gaa tcc ggg gtg ccc       240
Pro Lys Leu Leu Ile Tyr Ser Thr Ser Ser Leu Glu Ser Gly Val Pro
65                  70                  75                  80

agc aga ttc tca ggc agt ggc tcc ggc acc gaa ttc acc ctg acc atc       288
Ser Arg Phe Ser Gly Ser Gly Ser Gly Thr Glu Phe Thr Leu Thr Ile
                    85                  90                  95

agc tca ctg cag cct gac gac ttc gca acc tac tac tgt ctg cag tgg       336
Ser Ser Leu Gln Pro Asp Asp Phe Ala Thr Tyr Tyr Cys Leu Gln Trp
            100                 105                 110

agt agc tac cct tgg aca ttc ggc ggc ggc acc aag gtg gag atc aag       384
Ser Ser Tyr Pro Trp Thr Phe Gly Gly Gly Thr Lys Val Glu Ile Lys
        115                 120                 125
```

<210> 4
<211> 128
<212> PRT
<213> Artificial Sequence

<220>
<223> Synthetic Construct

<400> 4

```
Met Asp Met Arg Val Pro Ala Gln Leu Leu Gly Leu Leu Leu Trp
1               5               10              15

Leu Pro Gly Ala Lys Cys Asp Ile Gln Met Thr Gln Ser Pro Ser Thr
        20              25              30

Leu Ser Ala Ser Val Gly Asp Arg Val Thr Ile Thr Cys Arg Ala Ser
        35              40              45

Ser Ser Val Arg Tyr Ile Ala Trp Tyr Gln Gln Lys Pro Gly Lys Ala
        50              55              60

Pro Lys Leu Leu Ile Tyr Ser Thr Ser Ser Leu Glu Ser Gly Val Pro
65              70              75              80

Ser Arg Phe Ser Gly Ser Gly Ser Gly Thr Glu Phe Thr Leu Thr Ile
                85              90              95

Ser Ser Leu Gln Pro Asp Asp Phe Ala Thr Tyr Tyr Cys Leu Gln Trp
        100             105             110

Ser Ser Tyr Pro Trp Thr Phe Gly Gly Gly Thr Lys Val Glu Ile Lys
        115             120             125
```

<210> 5
<211> 321
<212> DNA
<213> Artificial Sequence

<220>
<223> CL 12G4 humanisé

<220>
<221> CDS
<222> (1)..(321)

<400> 5

```
cgg acc gtc gcc gca cca agt gtc ttc atc ttc ccg cca tct gat gag          48
Arg Thr Val Ala Ala Pro Ser Val Phe Ile Phe Pro Pro Ser Asp Glu
1               5               10              15

cag ttg aaa tct gga act gcc tct gtt gtg tgc ctg ctg aat aac ttc          96
Gln Leu Lys Ser Gly Thr Ala Ser Val Val Cys Leu Leu Asn Asn Phe
                20              25              30

tat ccc aga gag gcc aaa gta cag tgg aag gtg gat aac gcc ctc caa         144
Tyr Pro Arg Glu Ala Lys Val Gln Trp Lys Val Asp Asn Ala Leu Gln
            35              40              45

tcg ggt aac tcc cag gag agt gtc aca gag cag gac agc aag gac agc         192
Ser Gly Asn Ser Gln Glu Ser Val Thr Glu Gln Asp Ser Lys Asp Ser
        50              55              60

acc tac agc ctc agc agc acc ctg acg ctg agc aaa gca gac tac gag         240
Thr Tyr Ser Leu Ser Ser Thr Leu Thr Leu Ser Lys Ala Asp Tyr Glu
65              70              75              80

aaa cac aaa gtc tac gcc tgc gaa gtc acc cat cag ggc ctg agc tcg         288
Lys His Lys Val Tyr Ala Cys Glu Val Thr His Gln Gly Leu Ser Ser
                85              90              95

ccc gtc aca aag agc ttc aac agg gga gag tgt                             321
Pro Val Thr Lys Ser Phe Asn Arg Gly Glu Cys
                100             105
```

<210> 6
<211> 107
<212> PRT
<213> Artificial Sequence

<220>
<223> Synthetic Construct

<400> 6

```
Arg Thr Val Ala Ala Pro Ser Val Phe Ile Phe Pro Pro Ser Asp Glu
1               5               10              15

Gln Leu Lys Ser Gly Thr Ala Ser Val Val Cys Leu Leu Asn Asn Phe
                20              25              30

Tyr Pro Arg Glu Ala Lys Val Gln Trp Lys Val Asp Asn Ala Leu Gln
            35              40              45

Ser Gly Asn Ser Gln Glu Ser Val Thr Glu Gln Asp Ser Lys Asp Ser
        50              55              60

Thr Tyr Ser Leu Ser Ser Thr Leu Thr Leu Ser Lys Ala Asp Tyr Glu
65              70              75              80

Lys His Lys Val Tyr Ala Cys Glu Val Thr His Gln Gly Leu Ser Ser
                85              90              95
```

```
                        Pro Val Thr Lys Ser Phe Asn Arg Gly Glu Cys
                                100                     105
```

<210> 7
<211> 345
<212> DNA
<213> Artificial Sequence

<220>
<223> VH sans leader 12G4 humanisé

<220>
<221> CDS
<222> (1)..(345)

<400> 7

```
cag gtg cag ctg gtg cag agc ggg gcc gag gtg aag aag cct gga gcc    48
Gln Val Gln Leu Val Gln Ser Gly Ala Glu Val Lys Lys Pro Gly Ala
1               5                   10                  15

tca gtg aag gtg agt tgc aag gcc tcc ggt tac acc ttc acc agc tac    96
Ser Val Lys Val Ser Cys Lys Ala Ser Gly Tyr Thr Phe Thr Ser Tyr
            20                  25                  30

cac atc cac tgg gtc aga cag gct ccc ggc cag aga ctg gag tgg atg   144
His Ile His Trp Val Arg Gln Ala Pro Gly Gln Arg Leu Glu Trp Met
            35                  40                  45

ggc tgg atc tac cct gga gat gac tcc acc aag tac tcc cag aag ttc   192
Gly Trp Ile Tyr Pro Gly Asp Asp Ser Thr Lys Tyr Ser Gln Lys Phe
    50                  55                  60

cag ggt cgc gtg acc att acc agg gac acc agc gcc tcc act gcc tac   240
Gln Gly Arg Val Thr Ile Thr Arg Asp Thr Ser Ala Ser Thr Ala Tyr
65                  70                  75                  80

atg gag ctg tct tcc ctg aga tct gag gat acc gca gtc tac tac tgt   288
Met Glu Leu Ser Ser Leu Arg Ser Glu Asp Thr Ala Val Tyr Tyr Cys
                85                  90                  95

aca cgg ggg gac cgc ttt gct tac tgg ggg cag ggc act ctg gtg acc   336
Thr Arg Gly Asp Arg Phe Ala Tyr Trp Gly Gln Gly Thr Leu Val Thr
            100                 105                 110

gtc agc agc                                                        345
Val Ser Ser
        115
```

<210> 8
<211> 115
<212> PRT
<213> Artificial Sequence

<220>
<223> Synthetic Construct

<400> 8

Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Thr Phe Thr Ser Tyr
                20                  25                  30

His Ile His Trp Val Arg Gln Ala Pro Gly Gln Arg Leu Glu Trp Met
            35                  40                  45

Gly Trp Ile Tyr Pro Gly Asp Asp Ser Thr Lys Tyr Ser Gln Lys Phe
        50                  55                  60

Gln Gly Arg Val Thr Ile Thr Arg Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Asp Arg Phe Ala Tyr Trp Gly Gln Gly Thr Leu Val Thr
            100                 105                 110

Val Ser Ser
            115

<210> 9
<211> 402
<212> DNA
<213> Artificial Sequence

<220>
<223> VH avec leader 12G4 humanisé

<220>
<221> CDS
<222> (1)..(402)

<400> 9

```
atg gat tgg aca tgg cga atc ctg ttc ctg gtg gct gcc gca acc ggc      48
Met Asp Trp Thr Trp Arg Ile Leu Phe Leu Val Ala Ala Ala Thr Gly
1               5                   10                  15

gcc cac agc cag gtg cag ctg gtg cag agc ggg gcc gag gtg aag aag      96
Ala His Ser Gln Val Gln Leu Val Gln Ser Gly Ala Glu Val Lys Lys
            20                  25                  30

cct gga gcc tca gtg aag gtg agt tgc aag gcc tcc ggt tac acc ttc     144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga ctg     192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc     240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc     288
Gln Lys Phe Gln Gly Arg Val Thr Ile Thr Arg Asp Thr Ser Ala Ser
                85                  90                  95

act gcc tac atg gag ctg tct tcc ctg aga tct gag gat acc gca gtc     336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act     384
Tyr Tyr Cys Thr Arg Gly Asp Arg Phe Ala Tyr Trp Gly Gln Gly Thr
            115                 120                 125

ctg gtg acc gtc agc agc                                             402
Leu Val Thr Val Ser Ser
        130
```

<210> 10
<211> 134
<212> PRT
<213> Artificial Sequence

<220>
<223> Synthetic Construct

<400> 10

```
Met Asp Trp Thr Trp Arg Ile Leu Phe Leu Val Ala Ala Ala Thr Gly
1               5                   10              15

Ala His Ser Gln Val Gln Leu Val Gln Ser Gly Ala Glu Val Lys Lys
            20                  25              30

Pro Gly Ala Ser Val Lys Val Ser Cys Lys Ala Ser Gly Tyr Thr Phe
            35                  40              45

Thr Ser Tyr His Ile His Trp Val Arg Gln Ala Pro Gly Gln Arg Leu
        50              55              60

Glu Trp Met Gly Trp Ile Tyr Pro Gly Asp Asp Ser Thr Lys Tyr Ser
65                  70              75                  80

Gln Lys Phe Gln Gly Arg Val Thr Ile Thr Arg Asp Thr Ser Ala Ser
            85                  90                  95

Thr Ala Tyr Met Glu Leu Ser Ser Leu Arg Ser Glu Asp Thr Ala Val
            100             105             110

Tyr Tyr Cys Thr Arg Gly Asp Arg Phe Ala Tyr Trp Gly Gln Gly Thr
        115             120             125

Leu Val Thr Val Ser Ser
        130
```

<210> 11
<211> 990
<212> DNA
<213> Artificial Sequence

<220>
<223> CH 12G4 humanisé

<220>
<221> CDS
<222> (1)..(990)

<400> 11

```
gcc agc acc aag ggc cca tcg gtc ttc ccc ctg gca ccc tcc tcc aag        48
Ala Ser Thr Lys Gly Pro Ser Val Phe Pro Leu Ala Pro Ser Ser Lys
1               5               10                  15

agc acc tct ggg ggc aca gcg gcc ctg ggc tgc ctg gtc aag gac tac        96
Ser Thr Ser Gly Gly Thr Ala Ala Leu Gly Cys Leu Val Lys Asp Tyr
            20              25                  30

ttc ccc gaa ccg gtg acg gtg tcg tgg aac tca ggc gcc ctg acc agc       144
Phe Pro Glu Pro Val Thr Val Ser Trp Asn Ser Gly Ala Leu Thr Ser
        35              40                  45

ggc gtg cac acc ttc ccg gct gtc cta cag tcc tca gga ctc tac tcc       192
Gly Val His Thr Phe Pro Ala Val Leu Gln Ser Ser Gly Leu Tyr Ser
    50              55                  60

ctc agc agc gtg gtg acc gtg ccc tcc agc agc ttg ggc acc cag acc       240
Leu Ser Ser Val Val Thr Val Pro Ser Ser Ser Leu Gly Thr Gln Thr
65              70                  75                  80

tac atc tgc aac gtg aat cac aag ccc agc aac acc aag gtg gac aag       288
Tyr Ile Cys Asn Val Asn His Lys Pro Ser Asn Thr Lys Val Asp Lys
                85                  90                  95

aaa gtt gag ccc aaa tct tgt gac aaa act cac aca tgc cca ccg tgc       336
Lys Val Glu Pro Lys Ser Cys Asp Lys Thr His Thr Cys Pro Pro Cys
            100                 105                 110

cca gca cct gaa ctc ctg ggg gga ccg tca gtc ttc ctc ttc ccc cca       384
Pro Ala Pro Glu Leu Leu Gly Gly Pro Ser Val Phe Leu Phe Pro Pro
            115                 120                 125

aaa ccc aag gac acc ctc atg atc tcc cgg acc cct gag gtc aca tgc       432
Lys Pro Lys Asp Thr Leu Met Ile Ser Arg Thr Pro Glu Val Thr Cys
            130                 135                 140

gtg gtg gtg gac gtg agc cac gaa gac cct gag gtc aag ttc aac tgg       480
Val Val Val Asp Val Ser His Glu Asp Pro Glu Val Lys Phe Asn Trp
145                 150                 155                 160

tac gtg gac ggc gtg gag gtg cat aat gcc aag aca aag ccg cgg gag       528
Tyr Val Asp Gly Val Glu Val His Asn Ala Lys Thr Lys Pro Arg Glu
                165                 170                 175

gag cag tac aac agc acg tac cgt gtg gtc agc gtc ctc acc gtc ctg       576
Glu Gln Tyr Asn Ser Thr Tyr Arg Val Val Ser Val Leu Thr Val Leu
                180                 185                 190

cac cag gac tgg ctg aat ggc aag gag tac aag tgc aag gtc tcc aac       624
His Gln Asp Trp Leu Asn Gly Lys Glu Tyr Lys Cys Lys Val Ser Asn
            195                 200                 205

aaa gcc ctc cca gcc ccc atc gag aaa acc atc tcc aaa gcc aaa ggg       672
Lys Ala Leu Pro Ala Pro Ile Glu Lys Thr Ile Ser Lys Ala Lys Gly
    210                 215                 220
```

```
cag ccc cga gaa cca cag gtg tac acc ctg ccc cca tcc cgg gat gag        720
Gln Pro Arg Glu Pro Gln Val Tyr Thr Leu Pro Pro Ser Arg Asp Glu
225             230             235             240

ctg acc aag aac cag gtc agc ctg acc tgc ctg gtc aaa ggc ttc tat        768
Leu Thr Lys Asn Gln Val Ser Leu Thr Cys Leu Val Lys Gly Phe Tyr
            245             250             255

ccc agc gac atc gcc gtg gag tgg gag agc aat ggg cag ccg gag aac        816
Pro Ser Asp Ile Ala Val Glu Trp Glu Ser Asn Gly Gln Pro Glu Asn
            260             265             270

aac tac aag acc acg cct ccc gtg ctg gac tcc gac ggc tcc ttc ttc        864
Asn Tyr Lys Thr Thr Pro Pro Val Leu Asp Ser Asp Gly Ser Phe Phe
            275             280             285

ctc tac agc aag ctc acc gtg gac aag agc agg tgg cag cag ggg aac        912
Leu Tyr Ser Lys Leu Thr Val Asp Lys Ser Arg Trp Gln Gln Gly Asn
            290             295             300

gtc ttc tca tgc tcc gtg atg cat gag gct ctg cac aac cac tac acg        960
Val Phe Ser Cys Ser Val Met His Glu Ala Leu His Asn His Tyr Thr
305             310             315             320

cag aag agc ctc tcc ctg tct ccg ggt aaa                                990
Gln Lys Ser Leu Ser Leu Ser Pro Gly Lys
            325             330
```

<210> 12
<211> 330
<212> PRT
<213> Artificial Sequence

<220>
<223> Synthetic Construct

<400> 12

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110
```

```
Pro Ala Pro Glu Leu Leu Gly Gly Pro Ser Val Phe Leu Phe Pro Pro
        115                 120             125

Lys Pro Lys Asp Thr Leu Met Ile Ser Arg Thr Pro Glu Val Thr Cys
        130             135                 140

Val Val Val Asp Val Ser His Glu Asp Pro Glu Val Lys Phe Asn Trp
145                 150                 155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
        195             200             205

Lys Ala Leu Pro Ala Pro Ile Glu Lys Thr Ile Ser Lys Ala Lys Gly
        210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro Gly Lys
            325             330
```

<210> 13
<211> 318
<212> DNA
<213> Artificial Sequence

<220>

<223> VL sans leader 12G4 chimérique

<220>
<221> CDS
<222> (1)..(318)

<400> 13

| caa | att | gtt | ctc | acc | cag | tct | cca | gca | atc | atg | tct | gca | tct | cta | ggg | 48 |
|-----|-----|-----|-----|-----|-----|-----|-----|-----|-----|-----|-----|-----|-----|-----|-----|----|
| Gln | Ile | Val | Leu | Thr | Gln | Ser | Pro | Ala | Ile | Met | Ser | Ala | Ser | Leu | Gly |    |
| 1   |     |     |     | 5   |     |     |     |     | 10  |     |     |     |     | 15  |     |    |

| gag | ggg | atc | acc | cta | acc | tgc | agt | gcc | agc | tcg | agt | gta | cgt | tac | ata | 96 |
|-----|-----|-----|-----|-----|-----|-----|-----|-----|-----|-----|-----|-----|-----|-----|-----|----|
| Glu | Gly | Ile | Thr | Leu | Thr | Cys | Ser | Ala | Ser | Ser | Ser | Val | Arg | Tyr | Ile |    |
|     |     |     | 20  |     |     |     |     | 25  |     |     |     |     | 30  |     |     |    |

| cac | tgg | tac | cag | cag | aag | tca | ggc | act | tct | ccc | aaa | ctc | ttg | att | tat | 144 |
|-----|-----|-----|-----|-----|-----|-----|-----|-----|-----|-----|-----|-----|-----|-----|-----|-----|
| His | Trp | Tyr | Gln | Gln | Lys | Ser | Gly | Thr | Ser | Pro | Lys | Leu | Leu | Ile | Tyr |     |
|     |     | 35  |     |     |     |     | 40  |     |     |     |     | 45  |     |     |     |     |

| agc | aca | tcc | aac | ctg | gct | tct | gga | gtc | cct | tct | cgc | ttc | agt | ggc | agt | 192 |
|-----|-----|-----|-----|-----|-----|-----|-----|-----|-----|-----|-----|-----|-----|-----|-----|-----|
| Ser | Thr | Ser | Asn | Leu | Ala | Ser | Gly | Val | Pro | Ser | Arg | Phe | Ser | Gly | Ser |     |
|     | 50  |     |     |     |     | 55  |     |     |     |     | 60  |     |     |     |     |     |

| ggg | tct | ggg | acc | ttt | cat | tct | ctc | aca | atc | agc | agt | gtg | gag | gct | gaa | 240 |
|-----|-----|-----|-----|-----|-----|-----|-----|-----|-----|-----|-----|-----|-----|-----|-----|-----|
| Gly | Ser | Gly | Thr | Phe | His | Ser | Leu | Thr | Ile | Ser | Ser | Val | Glu | Ala | Glu |     |
| 65  |     |     |     | 70  |     |     |     |     | 75  |     |     |     |     | 80  |     |     |

| gat | gct | gcc | gat | tat | tac | tgc | ctt | cag | tgg | agt | agt | tat | ccg | tgg | acg | 288 |
|-----|-----|-----|-----|-----|-----|-----|-----|-----|-----|-----|-----|-----|-----|-----|-----|-----|
| Asp | Ala | Ala | Asp | Tyr | Tyr | Cys | Leu | Gln | Trp | Ser | Ser | Tyr | Pro | Trp | Thr |     |
|     |     |     |     | 85  |     |     |     |     | 90  |     |     |     |     | 95  |     |     |

| ttc | ggt | gga | ggc | acc | aag | ctg | gaa | atc | aaa | 318 |
|-----|-----|-----|-----|-----|-----|-----|-----|-----|-----|-----|
| Phe | Gly | Gly | Gly | Thr | Lys | Leu | Glu | Ile | Lys |     |
|     |     |     | 100 |     |     |     |     | 105 |     |     |

<210> 14
<211> 106
<212> PRT
<213> Artificial Sequence

<220>
<223> Synthetic Construct

<400> 14

```
Gln Ile Val Leu Thr Gln Ser Pro Ala Ile Met Ser Ala Ser Leu Gly
1               5                   10                  15

Glu Gly Ile Thr Leu Thr Cys Ser Ala Ser Ser Ser Val Arg Tyr Ile
            20              25                  30

His Trp Tyr Gln Gln Lys Ser Gly Thr Ser Pro Lys Leu Leu Ile Tyr
            35              40                  45

Ser Thr Ser Asn Leu Ala Ser Gly Val Pro Ser Arg Phe Ser Gly Ser
            50              55                  60

Gly Ser Gly Thr Phe His Ser Leu Thr Ile Ser Ser Val Glu Ala Glu
65                  70                  75                  80

Asp Ala Ala Asp Tyr Tyr Cys Leu Gln Trp Ser Ser Tyr Pro Trp Thr
                85                  90                  95

Phe Gly Gly Gly Thr Lys Leu Glu Ile Lys
            100                 105
```

<210> 15
<211> 384
<212> DNA
<213> Artificial Sequence

<220>
<223> VL avec leader 12G4 chimérique

<220>
<221> CDS
<222> (1)..(384)

<400> 15

```
atg gat ttt cag gtg cag att ttc agc ttc ctg cta atc agt gcc tca         48
Met Asp Phe Gln Val Gln Ile Phe Ser Phe Leu Leu Ile Ser Ala Ser
1               5                   10                  15

gtc ata atg tcc aga gga caa att gtt ctc acc cag tct cca gca atc         96
Val Ile Met Ser Arg Gly Gln Ile Val Leu Thr Gln Ser Pro Ala Ile
            20                  25                  30

atg tct gca tct cta ggg gag ggg atc acc cta acc tgc agt gcc agc        144
Met Ser Ala Ser Leu Gly Glu Gly Ile Thr Leu Thr Cys Ser Ala Ser
        35                  40                  45

tcg agt gta cgt tac ata cac tgg tac cag cag aag tca ggc act tct        192
Ser Ser Val Arg Tyr Ile His Trp Tyr Gln Gln Lys Ser Gly Thr Ser
        50                  55                  60

ccc aaa ctc ttg att tat agc aca tcc aac ctg gct tct gga gtc cct        240
Pro Lys Leu Leu Ile Tyr Ser Thr Ser Asn Leu Ala Ser Gly Val Pro
65                  70                  75                  80

tct cgc ttc agt ggc agt ggg tct ggg acc ttt cat tct ctc aca atc        288
Ser Arg Phe Ser Gly Ser Gly Ser Gly Thr Phe His Ser Leu Thr Ile
                85                  90                  95

agc agt gtg gag gct gaa gat gct gcc gat tat tac tgc ctt cag tgg        336
Ser Ser Val Glu Ala Glu Asp Ala Ala Asp Tyr Tyr Cys Leu Gln Trp
                100                 105                 110

agt agt tat ccg tgg acg ttc ggt gga ggc acc aag ctg gaa atc aaa        384
Ser Ser Tyr Pro Trp Thr Phe Gly Gly Gly Thr Lys Leu Glu Ile Lys
        115                 120                 125
```

<210> 16
<211> 128
<212> PRT
<213> Artificial Sequence

<220>
<223> Synthetic Construct

<400> 16

```
Met Asp Phe Gln Val Gln Ile Phe Ser Phe Leu Leu Ile Ser Ala Ser
1               5               10              15

Val Ile Met Ser Arg Gly Gln Ile Val Leu Thr Gln Ser Pro Ala Ile
            20              25              30

Met Ser Ala Ser Leu Gly Glu Gly Ile Thr Leu Thr Cys Ser Ala Ser
        35              40              45

Ser Ser Val Arg Tyr Ile His Trp Tyr Gln Gln Lys Ser Gly Thr Ser
    50              55              60

Pro Lys Leu Leu Ile Tyr Ser Thr Ser Asn Leu Ala Ser Gly Val Pro
65              70              75              80

Ser Arg Phe Ser Gly Ser Gly Ser Gly Thr Phe His Ser Leu Thr Ile
            85              90              95

Ser Ser Val Glu Ala Glu Asp Ala Ala Asp Tyr Tyr Cys Leu Gln Trp
        100             105             110

Ser Ser Tyr Pro Trp Thr Phe Gly Gly Gly Thr Lys Leu Glu Ile Lys
        115             120             125
```

<210> 17
<211> 345
<212> DNA
<213> Artificial Sequence

<220>
<223> VH sans leader 12G4 chimérique

<220>
<221> CDS
<222> (1)..(345)

<400> 17

```
cag gtc cag ctg cag cag tct gga cct gaa ctg gtg aag cct ggg gct    48
Gln Val Gln Leu Gln Gln Ser Gly Pro Glu Leu Val Lys Pro Gly Ala
1               5               10              15

tca gtg agg atg tcc tgc aag gct tct ggc tac acc ttc aca agt tac    96
Ser Val Arg Met Ser Cys Lys Ala Ser Gly Tyr Thr Phe Thr Ser Tyr
            20              25              30

cat ata cac tgg gtg aag cag agg cct gga cag gga ctt gag tgg att   144
His Ile His Trp Val Lys Gln Arg Pro Gly Gln Gly Leu Glu Trp Ile
            35              40              45

gga tgg att tat cct ggc gat gat tct act aaa tac aat gag aag ttc   192
Gly Trp Ile Tyr Pro Gly Asp Asp Ser Thr Lys Tyr Asn Glu Lys Phe
        50              55              60

aag ggc aag acc aca ctg act gca gac aaa tcc tcc agc aca gcc tac   240
Lys Gly Lys Thr Thr Leu Thr Ala Asp Lys Ser Ser Ser Thr Ala Tyr

65                  70              75              80

atg ttg ctc agc agc ctg acc tct gag gac tct gcg atc tat ttc tgt   288
Met Leu Leu Ser Ser Leu Thr Ser Glu Asp Ser Ala Ile Tyr Phe Cys
            85              90              95

aca agg ggg gac cgg ttt gct tac tgg ggc caa ggg act ctg gtc act   336
Thr Arg Gly Asp Arg Phe Ala Tyr Trp Gly Gln Gly Thr Leu Val Thr
            100             105             110

gtc tct gca                                                        345
Val Ser Ala
        115
```

<210> 18
<211> 115
<212> PRT
<213> Artificial Sequence

<220>
<223> Synthetic Construct

<400> 18

```
Gln Val Gln Leu Gln Gln Ser Gly Pro Glu Leu Val Lys Pro Gly Ala
1               5               10              15

Ser Val Arg Met Ser Cys Lys Ala Ser Gly Tyr Thr Phe Thr Ser Tyr
            20              25              30

His Ile His Trp Val Lys Gln Arg Pro Gly Gln Gly Leu Glu Trp Ile
        35              40              45

Gly Trp Ile Tyr Pro Gly Asp Asp Ser Thr Lys Tyr Asn Glu Lys Phe
    50              55              60

Lys Gly Lys Thr Thr Leu Thr Ala Asp Lys Ser Ser Ser Thr Ala Tyr
65              70              75              80

Met Leu Leu Ser Ser Leu Thr Ser Glu Asp Ser Ala Ile Tyr Phe Cys
            85              90              95

Thr Arg Gly Asp Arg Phe Ala Tyr Trp Gly Gln Gly Thr Leu Val Thr
        100             105             110

Val Ser Ala
        115
```

<210> 19
<211> 402
<212> DNA
<213> Artificial Sequence

<220>
<223> VH avec leader 12G4 chimérique

<220>
<221> CDS
<222> (1)..(402)

<400> 19

```
atg cga tgg agc tgg atc ttt ctc ttc ctc ctg tca ata act gca agt        48
Met Arg Trp Ser Trp Ile Phe Leu Phe Leu Leu Ser Ile Thr Ala Ser
1               5                   10                  15

gtc cat tgc cag gtc cag ctg cag cag tct gga cct gaa ctg gtg aag        96
Val His Cys Gln Val Gln Leu Gln Gln Ser Gly Pro Glu Leu Val Lys
                20                  25                  30

cct ggg gct tca gtg agg atg tcc tgc aag gct tct ggc tac acc ttc       144
Pro Gly Ala Ser Val Arg Met Ser Cys Lys Ala Ser Gly Tyr Thr Phe
            35                  40                  45

aca agt tac cat ata cac tgg gtg aag cag agg cct gga cag gga ctt       192
Thr Ser Tyr His Ile His Trp Val Lys Gln Arg Pro Gly Gln Gly Leu
            50                  55                  60

gag tgg att gga tgg att tat cct ggc gat gat tct act aaa tac aat       240
Glu Trp Ile Gly Trp Ile Tyr Pro Gly Asp Asp Ser Thr Lys Tyr Asn
65              70                  75                  80

gag aag ttc aag ggc aag acc aca ctg act gca gac aaa tcc tcc agc       288
Glu Lys Phe Lys Gly Lys Thr Thr Leu Thr Ala Asp Lys Ser Ser Ser
                85                  90                  95

aca gcc tac atg ttg ctc agc agc ctg acc tct gag gac tct gcg atc       336
Thr Ala Tyr Met Leu Leu Ser Ser Leu Thr Ser Glu Asp Ser Ala Ile
            100                 105                 110

tat ttc tgt aca agg ggg gac cgg ttt gct tac tgg ggc caa ggg act       384
Tyr Phe Cys Thr Arg Gly Asp Arg Phe Ala Tyr Trp Gly Gln Gly Thr
            115                 120                 125

ctg gtc act gtc tct gca                                                402
Leu Val Thr Val Ser Ala
            130
```

<210> 20
<211> 134
<212> PRT
<213> Artificial Sequence

<220>
<223> Synthetic Construct

<400> 20

```
Met Arg Trp Ser Trp Ile Phe Leu Phe Leu Leu Ser Ile Thr Ala Ser
1               5                   10                  15

Val His Cys Gln Val Gln Leu Gln Gln Ser Gly Pro Glu Leu Val Lys
                20                  25                  30

Pro Gly Ala Ser Val Arg Met Ser Cys Lys Ala Ser Gly Tyr Thr Phe
            35                  40                  45
```

```
Thr Ser Tyr His Ile His Trp Val Lys Gln Arg Pro Gly Gln Gly Leu
    50                  55                  60

Glu Trp Ile Gly Trp Ile Tyr Pro Gly Asp Asp Ser Thr Lys Tyr Asn
65                  70                  75                      80

Glu Lys Phe Lys Gly Lys Thr Thr Leu Thr Ala Asp Lys Ser Ser Ser
                85                  90                  95

Thr Ala Tyr Met Leu Leu Ser Ser Leu Thr Ser Glu Asp Ser Ala Ile
            100                 105                 110

Tyr Phe Cys Thr Arg Gly Asp Arg Phe Ala Tyr Trp Gly Gln Gly Thr
        115                 120                 125

Leu Val Thr Val Ser Ala
        130
```

<210> 21
<211> 318
<212> DNA
<213> Artificial Sequence

<220>
<223> VL sans leader 3C_23

<220>
<221> CDS
<222> (1)..(318)

<400> 21

```
gac atc cag atg aca cag tcc cca tct acc ctg tct gct tcc gtg gga        48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc        96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg acc tac       144
Ala Trp Tyr Gln Gln Lys Pro Gly Lys Ala Pro Lys Leu Leu Thr Tyr
        35                  40                  45

cca acc tcc tcc ctg gaa tcc ggg gtg ccc agc aga ttc tca ggc agt       192
Pro Thr Ser Ser Leu Glu Ser Gly Val Pro Ser Arg Phe Ser Gly Ser
    50                  55                  60

ggc tcc ggc acc gaa ttc acc ctg acc atc agc tca ctg cag cct gac       240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca       288
Asp Phe Ala Thr Tyr Tyr Cys Leu Gln Trp Ser Ser Tyr Pro Trp Thr
                85                  90                  95

ttc ggc ggc ggc acc aag gtg gag atc aag                               318
Phe Gly Gly Gly Thr Lys Val Glu Ile Lys
            100                 105
```

<210> 22
<211> 106
<212> PRT
<213> Artificial Sequence

<220>
<223> Synthetic Construct

<400> 22

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Thr Tyr
        35                  40                  45

Pro Thr Ser Ser Leu Glu Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
            85                  90                  95

Phe Gly Gly Gly Thr Lys Val Glu Ile Lys
            100                 105
```

<210> 23
<211> 384
<212> DNA
<213> Artificial Sequence

<220>
<223> VL avec leader 3C_23

<220>
<221> CDS
<222> (1)..(384)

<400> 23

```
atg gat atg aga gtg ccc gca cag ctg ctg ggt ctg ctg ctg ctg tgg    48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc    96
Leu Pro Gly Ala Lys Cys Asp Ile Gln Met Thr Gln Ser Pro Ser Thr
            20                  25                  30

ctg tct gct tcc gtg gga gat cgg gtg act atc acc tgc aga gca agc   144
Leu Ser Ala Ser Val Gly Asp Arg Val Thr Ile Thr Cys Arg Ala Ser
        35                  40                  45
```

```
tcc tcc gtg agg tac atc gct tgg tac cag cag aag cca gga aag gcc      192
Ser Ser Val Arg Tyr Ile Ala Trp Tyr Gln Gln Lys Pro Gly Lys Ala
    50                  55                  60

cca aag ctg ctg acc tac cca acc tcc tcc ctg gaa tcc ggg gtg ccc      240
Pro Lys Leu Leu Thr Tyr Pro Thr Ser Ser Leu Glu Ser Gly Val Pro
65                  70                  75                  80

agc aga ttc tca ggc agt ggc tcc ggc acc gaa ttc acc ctg acc atc      288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg      336
Ser Ser Leu Gln Pro Asp Asp Phe Ala Thr Tyr Tyr Cys Leu Gln Trp
            100                 105                 110

agt agc tac cct tgg aca ttc ggc ggc ggc acc aag gtg gag atc aag      384
Ser Ser Tyr Pro Trp Thr Phe Gly Gly Gly Thr Lys Val Glu Ile Lys
        115                 120                 125
```

<210> 24
<211> 128
<212> PRT
<213> Artificial Sequence

<220>
<223> Synthetic Construct

<400> 24


        Met Asp Met Arg Val Pro Ala Gln Leu Leu Gly Leu Leu Leu Leu Trp
        1               5                   10                  15


        Leu Pro Gly Ala Lys Cys Asp Ile Gln Met Thr Gln Ser Pro Ser Thr
                        20                  25                  30


        Leu Ser Ala Ser Val Gly Asp Arg Val Thr Ile Thr Cys Arg Ala Ser
                    35                  40                  45


        Ser Ser Val Arg Tyr Ile Ala Trp Tyr Gln Gln Lys Pro Gly Lys Ala
            50                  55                  60


        Pro Lys Leu Leu Thr Tyr Pro Thr Ser Ser Leu Glu Ser Gly Val Pro
        65                  70                  75                  80


        Ser Arg Phe Ser Gly Ser Gly Ser Gly Thr Glu Phe Thr Leu Thr Ile
                        85                  90                  95


        Ser Ser Leu Gln Pro Asp Asp Phe Ala Thr Tyr Tyr Cys Leu Gln Trp
                    100                 105                 110


        Ser Ser Tyr Pro Trp Thr Phe Gly Gly Gly Thr Lys Val Glu Ile Lys
            115                 120                 125
```

<210> 25
<211> 345
<212> DNA
<213> Artificial Sequence

<220>
<223> VH sans leader 3C_23

<220>
<221> CDS
<222> (1)..(345)

<400> 25

```
cag gtg cgg ctg gtg cag agc ggg gcc gag gtg aag aag cct gga gcc      48
Gln Val Arg Leu Val Gln Ser Gly Ala Glu Val Lys Lys Pro Gly Ala
1               5                   10                  15

tca gtg aag gtg agt tgc aag gcc tcc ggt tac acc ttc acc agc tac      96
Ser Val Lys Val Ser Cys Lys Ala Ser Gly Tyr Thr Phe Thr Ser Tyr
                20                  25                  30

cac atc cac tgg gtc aga cag gct ccc ggc cag aga ctg gag tgg atg     144
His Ile His Trp Val Arg Gln Ala Pro Gly Gln Arg Leu Glu Trp Met
            35                  40                  45

ggc tgg atc tac cct gga gat gac tcc acc aag tac tcc cag aag ttc     192
Gly Trp Ile Tyr Pro Gly Asp Asp Ser Thr Lys Tyr Ser Gln Lys Phe
        50                  55                  60

cag ggt cgc gtg acc att acc agg gac acc agc gcc tcc act gcc tac     240
Gln Gly Arg Val Thr Ile Thr Arg Asp Thr Ser Ala Ser Thr Ala Tyr
65                  70                  75                  80

atg gag ctg tct tcc ctg aga tct gag gat acc gca gtc tac tac tgt     288
Met Glu Leu Ser Ser Leu Arg Ser Glu Asp Thr Ala Val Tyr Tyr Cys
                85                  90                  95

aca cgg ggg gac cgc ttt gct tac tgg ggg cag ggc act ctg gtg acc     336
Thr Arg Gly Asp Arg Phe Ala Tyr Trp Gly Gln Gly Thr Leu Val Thr
                100                 105                 110

gtc tcg agc                                                         345
Val Ser Ser
        115
```

<210> 26
<211> 115
<212> PRT
<213> Artificial Sequence

<220>
<223> Synthetic Construct

<400> 26

```
Gln Val Arg Leu Val Gln Ser Gly Ala Glu Val Lys Lys Pro Gly Ala
1               5                   10              15

Ser Val Lys Val Ser Cys Lys Ala Ser Gly Tyr Thr Phe Thr Ser Tyr
            20                  25                  30

His Ile His Trp Val Arg Gln Ala Pro Gly Gln Arg Leu Glu Trp Met

        35                  40                  45

Gly Trp Ile Tyr Pro Gly Asp Asp Ser Thr Lys Tyr Ser Gln Lys Phe
        50                  55                  60

Gln Gly Arg Val Thr Ile Thr Arg Asp Thr Ser Ala Ser Thr Ala Tyr
65                  70                  75                  80

Met Glu Leu Ser Ser Leu Arg Ser Glu Asp Thr Ala Val Tyr Tyr Cys
            85                  90                  95

Thr Arg Gly Asp Arg Phe Ala Tyr Trp Gly Gln Gly Thr Leu Val Thr
            100                 105                 110

Val Ser Ser
            115
```

<210> 27
<211> 402
<212> DNA
<213> Artificial Sequence

<220>
<223> VH avec leader 3C_23

<220>
<221> CDS
<222> (1)..(402)

<400> 27

```
atg gat tgg aca tgg cga atc ctg ttc ctg gtg gct gcc gca acc ggc          48
Met Asp Trp Thr Trp Arg Ile Leu Phe Leu Val Ala Ala Ala Thr Gly
1               5                   10                  15

gcc cac agc cag gtg cgg ctg gtg cag agc ggg gcc gag gtg aag aag          96
Ala His Ser Gln Val Arg Leu Val Gln Ser Gly Ala Glu Val Lys Lys
            20                  25                  30

cct gga gcc tca gtg aag gtg agt tgc aag gcc tcc ggt tac acc ttc          144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga ctg          192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc          240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc          288
Gln Lys Phe Gln Gly Arg Val Thr Ile Thr Arg Asp Thr Ser Ala Ser
                85                  90                  95

act gcc tac atg gag ctg tct tcc ctg aga tct gag gat acc gca gtc          336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act          384
Tyr Tyr Cys Thr Arg Gly Asp Arg Phe Ala Tyr Trp Gly Gln Gly Thr
            115                 120                 125

ctg gtg acc gtc tcg agc                                                  402
Leu Val Thr Val Ser Ser
            130
```

<210> 28
<211> 134
<212> PRT
<213> Artificial Sequence

<220>
<223> Synthetic Construct

<400> 28

```
Met Asp Trp Thr Trp Arg Ile Leu Phe Leu Val Ala Ala Ala Thr Gly
1               5               10              15

Ala His Ser Gln Val Arg Leu Val Gln Ser Gly Ala Glu Val Lys Lys
            20              25              30

Pro Gly Ala Ser Val Lys Val Ser Cys Lys Ala Ser Gly Tyr Thr Phe
            35              40              45

Thr Ser Tyr His Ile His Trp Val Arg Gln Ala Pro Gly Gln Arg Leu
        50              55              60

Glu Trp Met Gly Trp Ile Tyr Pro Gly Asp Asp Ser Thr Lys Tyr Ser
65              70              75              80

Gln Lys Phe Gln Gly Arg Val Thr Ile Thr Arg Asp Thr Ser Ala Ser
            85              90              95

Thr Ala Tyr Met Glu Leu Ser Ser Leu Arg Ser Glu Asp Thr Ala Val
            100             105             110

Tyr Tyr Cys Thr Arg Gly Asp Arg Phe Ala Tyr Trp Gly Gln Gly Thr
            115             120             125

Leu Val Thr Val Ser Ser
            130
```

<210> 29
<211> 318
<212> DNA
<213> Artificial Sequence

<220>
<223> VL sans leader 6B_78

<220>
<221> CDS
<222> (1)..(318)

<400> 29

```
gac atc cag atg aca cag tcc cca tct acc ctg tct gct tcc gtg gga          48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc          96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg atc tac         144
Ala Trp Tyr Gln Gln Lys Pro Gly Lys Ala Pro Lys Leu Leu Ile Tyr
        35                  40                  45

tca acc tcc tcc ctg aaa tcc ggg gtg ccc agc aga ttc tca ggc agt         192
Ser Thr Ser Ser Leu Lys Ser Gly Val Pro Ser Arg Phe Ser Gly Ser
    50                  55                  60

ggc tcc ggc acc gaa ttc acc ctg acc atc agc tca ctg cag cct gac         240
Gly Ser Gly Thr Glu Phe Thr Leu Thr Ile Ser Ser Leu Gln Pro Asp
65              70                  75                  80

gac ttc gca acc tac tac tgt ctg cag tgg agt agc tac cct tgg aca         288
Asp Phe Ala Thr Tyr Tyr Cys Leu Gln Trp Ser Ser Tyr Pro Trp Thr
                85                  90                  95

ttc ggc ggc ggc acc aag gtg gag atc aag                                 318
Phe Gly Gly Gly Thr Lys Val Glu Ile Lys
            100                 105
```

<210> 30
<211> 106
<212> PRT
<213> Artificial Sequence

<220>
<223> Synthetic Construct

<400> 30

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Ile Tyr
        35                  40                  45

Ser Thr Ser Ser Leu Lys Ser Gly Val Pro Ser Arg Phe Ser Gly Ser
    50                  55                  60

Gly Ser Gly Thr Glu Phe Thr Leu Thr Ile Ser Ser Leu Gln Pro Asp
65              70                  75                  80

Asp Phe Ala Thr Tyr Tyr Cys Leu Gln Trp Ser Ser Tyr Pro Trp Thr
                85                  90                  95
```

64

```
                    Phe Gly Gly Gly Thr Lys Val Glu Ile Lys
                            100                     105
```

<210> 31
<211> 384
<212> DNA
<213> Artificial Sequence

<220>
<223> VL avec leader 6B_78

<220>
<221> CDS
<222> (1)..(384)

<400> 31

```
atg gat atg aga gtg ccc gca cag ctg ctg ggt ctg ctg ctg ctg tgg          48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc          96
Leu Pro Gly Ala Lys Cys Asp Ile Gln Met Thr Gln Ser Pro Ser Thr
                20                  25                  30

ctg tct gct tcc gtg gga gat cgg gtg act atc acc tgc aga gca agc         144
Leu Ser Ala Ser Val Gly Asp Arg Val Thr Ile Thr Cys Arg Ala Ser
            35                  40                  45

tcc tcc gtg agg tac atc gct tgg tac cag cag aag cca gga aag gcc         192
Ser Ser Val Arg Tyr Ile Ala Trp Tyr Gln Gln Lys Pro Gly Lys Ala
        50                  55                  60

cca aag ctg ctg atc tac tca acc tcc tcc ctg aaa tcc ggg gtg ccc         240
Pro Lys Leu Leu Ile Tyr Ser Thr Ser Ser Leu Lys Ser Gly Val Pro
65                  70                  75                  80

agc aga ttc tca ggc agt ggc tcc ggc acc gaa ttc acc ctg acc atc         288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg         336
Ser Ser Leu Gln Pro Asp Asp Phe Ala Thr Tyr Tyr Cys Leu Gln Trp
            100                 105                 110

agt agc tac cct tgg aca ttc ggc ggc ggc acc aag gtg gag atc aag         384
Ser Ser Tyr Pro Trp Thr Phe Gly Gly Gly Thr Lys Val Glu Ile Lys
            115                 120                 125
```

<210> 32
<211> 128
<212> PRT
<213> Artificial Sequence

<220>
<223> Synthetic Construct

<400> 32

```
Met Asp Met Arg Val Pro Ala Gln Leu Leu Gly Leu Leu Leu Trp
1               5               10              15

Leu Pro Gly Ala Lys Cys Asp Ile Gln Met Thr Gln Ser Pro Ser Thr
            20              25              30

Leu Ser Ala Ser Val Gly Asp Arg Val Thr Ile Thr Cys Arg Ala Ser
            35              40              45

Ser Ser Val Arg Tyr Ile Ala Trp Tyr Gln Gln Lys Pro Gly Lys Ala
            50              55              60

Pro Lys Leu Leu Ile Tyr Ser Thr Ser Ser Leu Lys Ser Gly Val Pro
65              70              75              80

Ser Arg Phe Ser Gly Ser Gly Ser Gly Thr Glu Phe Thr Leu Thr Ile
                85              90              95

Ser Ser Leu Gln Pro Asp Asp Phe Ala Thr Tyr Tyr Cys Leu Gln Trp
            100             105             110

Ser Ser Tyr Pro Trp Thr Phe Gly Gly Gly Thr Lys Val Glu Ile Lys
            115             120             125
```

<210> 33
<211> 318
<212> DNA
<213> Artificial Sequence

<220>
<223> VL sans leader 3C_23K

<220>
<221> CDS
<222> (1)..(318)

<400> 33

```
gac atc cag atg aca cag tcc cca tct acc ctg tct gct tcc gtg gga        48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc        96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg acc tac       144
Ala Trp Tyr Gln Gln Lys Pro Gly Lys Ala Pro Lys Leu Leu Thr Tyr
        35                  40                  45

cca acc tcc tcc ctg aaa tcc ggg gtg ccc agc aga ttc tca ggc agt       192
Pro Thr Ser Ser Leu Lys Ser Gly Val Pro Ser Arg Phe Ser Gly Ser
    50                  55                  60

ggc tcc ggc acc gaa ttc acc ctg acc atc agc tca ctg cag cct gac       240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca       288

Asp Phe Ala Thr Tyr Tyr Cys Leu Gln Trp Ser Ser Tyr Pro Trp Thr
                85                  90                  95

ttc ggc ggc ggc acc aag gtg gag atc aag                               318
Phe Gly Gly Gly Thr Lys Val Glu Ile Lys
            100                 105
```

<210> 34
<211> 106
<212> PRT
<213> Artificial Sequence

<220>
<223> Synthetic Construct

<400> 34

```
Asp Ile Gln Met Thr Gln Ser Pro Ser Thr Leu Ser Ala Ser Val Gly
1               5               10              15
```

```
Asp Arg Val Thr Ile Thr Cys Arg Ala Ser Ser Val Arg Tyr Ile
            20              25              30
```

```
Ala Trp Tyr Gln Gln Lys Pro Gly Lys Ala Pro Lys Leu Leu Thr Tyr
        35              40              45
```

```
Pro Thr Ser Ser Leu Lys Ser Gly Val Pro Ser Arg Phe Ser Gly Ser
    50              55              60
```

```
Gly Ser Gly Thr Glu Phe Thr Leu Thr Ile Ser Ser Leu Gln Pro Asp
65              70              75              80
```

```
Asp Phe Ala Thr Tyr Tyr Cys Leu Gln Trp Ser Ser Tyr Pro Trp Thr
            85              90              95
```

```
Phe Gly Gly Gly Thr Lys Val Glu Ile Lys
        100             105
```

<210> 35
<211> 384
<212> DNA
<213> Artificial Sequence

<220>
<223> VL avec leader 3C_23K

<220>
<221> CDS
<222> (1)..(384)

<400> 35

```
atg gat atg aga gtg ccc gca cag ctg ctg ggt ctg ctg ctg ctg tgg      48
Met Asp Met Arg Val Pro Ala Gln Leu Leu Gly Leu Leu Leu Leu Trp
1               5               10              15
```

```
ctg ccc gga gcc aaa tgt gac atc cag atg aca cag tcc cca tct acc      96
```

```
Leu Pro Gly Ala Lys Cys Asp Ile Gln Met Thr Gln Ser Pro Ser Thr
            20              25              30

ctg tct gct tcc gtg gga gat cgg gtg act atc acc tgc aga gca agc   144
Leu Ser Ala Ser Val Gly Asp Arg Val Thr Ile Thr Cys Arg Ala Ser
            35              40              45

tcc tcc gtg agg tac atc gct tgg tac cag cag aag cca gga aag gcc   192
Ser Ser Val Arg Tyr Ile Ala Trp Tyr Gln Gln Lys Pro Gly Lys Ala
            50              55              60

cca aag ctg ctg acc tac cca acc tcc tcc ctg aaa tcc ggg gtg ccc   240
Pro Lys Leu Leu Thr Tyr Pro Thr Ser Ser Leu Lys Ser Gly Val Pro
65              70              75              80

agc aga ttc tca ggc agt ggc tcc ggc acc gaa ttc acc ctg acc atc   288
Ser Arg Phe Ser Gly Ser Gly Ser Gly Thr Glu Phe Thr Leu Thr Ile
                85              90              95

agc tca ctg cag cct gac gac ttc gca acc tac tac tgt ctg cag tgg   336
Ser Ser Leu Gln Pro Asp Asp Phe Ala Thr Tyr Tyr Cys Leu Gln Trp
            100             105             110

agt agc tac cct tgg aca ttc ggc ggc ggc acc aag gtg gag atc aag   384
Ser Ser Tyr Pro Trp Thr Phe Gly Gly Gly Thr Lys Val Glu Ile Lys
            115             120             125
```

<210> 36
<211> 128
<212> PRT
<213> Artificial Sequence

<220>
<223> Synthetic Construct

<400> 36

```
Met Asp Met Arg Val Pro Ala Gln Leu Leu Gly Leu Leu Leu Trp
1               5               10              15

Leu Pro Gly Ala Lys Cys Asp Ile Gln Met Thr Gln Ser Pro Ser Thr
            20              25              30

Leu Ser Ala Ser Val Gly Asp Arg Val Thr Ile Thr Cys Arg Ala Ser
        35              40              45

Ser Ser Val Arg Tyr Ile Ala Trp Tyr Gln Gln Lys Pro Gly Lys Ala
    50              55              60

Pro Lys Leu Leu Thr Tyr Pro Thr Ser Ser Leu Lys Ser Gly Val Pro
65              70              75              80

Ser Arg Phe Ser Gly Ser Gly Ser Gly Thr Glu Phe Thr Leu Thr Ile
            85              90              95

Ser Ser Leu Gln Pro Asp Asp Phe Ala Thr Tyr Tyr Cys Leu Gln Trp
        100             105             110

Ser Ser Tyr Pro Trp Thr Phe Gly Gly Gly Thr Lys Val Glu Ile Lys
        115             120             125
```

<210> 37
<211> 345
<212> DNA
<213> Artificial Sequence

<220>
<223> VH sans leader 3C_23K

<220>
<221> CDS
<222> (1)..(345)

<400> 37

```
cag gtg cgg ctg gtg cag agc ggg gcc gag gtg aag aag cct gga gcc        48
Gln Val Arg Leu Val Gln Ser Gly Ala Glu Val Lys Lys Pro Gly Ala
1               5               10              15

tca gtg aag gtg agt tgc aag gcc tcc ggt tac acc ttc acc agc tac        96
Ser Val Lys Val Ser Cys Lys Ala Ser Gly Tyr Thr Phe Thr Ser Tyr
                20              25              30

cac atc cac tgg gtc aga cag gct ccc ggc cag aga ctg gag tgg atg       144
His Ile His Trp Val Arg Gln Ala Pro Gly Gln Arg Leu Glu Trp Met
            35              40              45

ggc tgg atc tac cct gga gat gac tcc acc aag tac tcc cag aag ttc       192
Gly Trp Ile Tyr Pro Gly Asp Asp Ser Thr Lys Tyr Ser Gln Lys Phe
        50              55              60

cag ggt cgc gtg acc att acc agg gac acc agc gcc tcc act gcc tac       240
Gln Gly Arg Val Thr Ile Thr Arg Asp Thr Ser Ala Ser Thr Ala Tyr
65              70              75              80

atg gag ctg tct tcc ctg aga tct gag gat acc gca gtc tac tac tgt       288
Met Glu Leu Ser Ser Leu Arg Ser Glu Asp Thr Ala Val Tyr Tyr Cys
                85              90              95

aca cgg ggg gac cgc ttt gct tac tgg ggg cag ggc act ctg gtg acc       336
Thr Arg Gly Asp Arg Phe Ala Tyr Trp Gly Gln Gly Thr Leu Val Thr
            100             105             110

gtc tcg agc                                                            345
Val Ser Ser
        115
```

<210> 38
<211> 115
<212> PRT
<213> Artificial Sequence

<220>
<223> Synthetic Construct

<400> 38

```
    Gln Val Arg Leu Val Gln Ser Gly Ala Glu Val Lys Lys Pro Gly Ala
    1               5               10              15
```

```
Ser Val Lys Val Ser Cys Lys Ala Ser Gly Tyr Thr Phe Thr Ser Tyr
            20                  25                  30

His Ile His Trp Val Arg Gln Ala Pro Gly Gln Arg Leu Glu Trp Met
            35                  40                  45

Gly Trp Ile Tyr Pro Gly Asp Asp Ser Thr Lys Tyr Ser Gln Lys Phe
        50                  55                  60

Gln Gly Arg Val Thr Ile Thr Arg Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Asp Arg Phe Ala Tyr Trp Gly Gln Gly Thr Leu Val Thr
            100                 105                 110

Val Ser Ser
            115
```

<210> 39
<211> 402
<212> DNA
<213> Artificial Sequence

<220>
<223> VH avec leader 3C_23K

<220>
<221> CDS
<222> (1)..(402)

<400> 39

```
atg gat tgg aca tgg cga atc ctg ttc ctg gtg gct gcc gca acc ggc          48
Met Asp Trp Thr Trp Arg Ile Leu Phe Leu Val Ala Ala Ala Thr Gly
1               5                   10                  15

gcc cac agc cag gtg cgg ctg gtg cag agc ggg gcc gag gtg aag aag          96
Ala His Ser Gln Val Arg Leu Val Gln Ser Gly Ala Glu Val Lys Lys
            20                  25                  30

cct gga gcc tca gtg aag gtg agt tgc aag gcc tcc ggt tac acc ttc         144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga ctg         192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc         240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc         288
Gln Lys Phe Gln Gly Arg Val Thr Ile Thr Arg Asp Thr Ser Ala Ser


            85                  90                  95

act gcc tac atg gag ctg tct tcc ctg aga tct gag gat acc gca gtc         336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act         384
Tyr Tyr Cys Thr Arg Gly Asp Arg Phe Ala Tyr Trp Gly Gln Gly Thr
            115                 120                 125

ctg gtg acc gtc tcg agc                                                  402
Leu Val Thr Val Ser Ser
            130
```

<210> 40
<211> 134
<212> PRT
<213> Artificial Sequence

<220>
<223> Synthetic Construct

<400> 40

```
Met Asp Trp Thr Trp Arg Ile Leu Phe Leu Val Ala Ala Ala Thr Gly
1               5               10              15

Ala His Ser Gln Val Arg Leu Val Gln Ser Gly Ala Glu Val Lys Lys
            20              25              30

Pro Gly Ala Ser Val Lys Val Ser Cys Lys Ala Ser Gly Tyr Thr Phe
            35              40              45

Thr Ser Tyr His Ile His Trp Val Arg Gln Ala Pro Gly Gln Arg Leu
        50              55              60

Glu Trp Met Gly Trp Ile Tyr Pro Gly Asp Asp Ser Thr Lys Tyr Ser
65              70              75              80

Gln Lys Phe Gln Gly Arg Val Thr Ile Thr Arg Asp Thr Ser Ala Ser
            85              90              95

Thr Ala Tyr Met Glu Leu Ser Ser Leu Arg Ser Glu Asp Thr Ala Val
            100             105             110

Tyr Tyr Cys Thr Arg Gly Asp Arg Phe Ala Tyr Trp Gly Gln Gly Thr
        115             120             125

Leu Val Thr Val Ser Ser
        130
```

<210> 41
<211> 345
<212> DNA
<213> Artificial Sequence

<220>
<223> VH avec leader 4C_35

<220>
<221> CDS
<222> (1)..(345)

<400> 41

```
cag gtg cag ctg gtg cag agc ggg gcc gag gtg aag aag cct gga gcc        48
Gln Val Gln Leu Val Gln Ser Gly Ala Glu Val Lys Lys Pro Gly Ala
1               5               10              15

tca gtg aag gtg agt tgc aag gcc tcc ggt tac acc ttc acc agc tac        96
Ser Val Lys Val Ser Cys Lys Ala Ser Gly Tyr Thr Phe Thr Ser Tyr
            20              25              30

cac atc cac tgg gtc aga cag gct ccc ggc cag aga cca gag tgg atg       144
His Ile His Trp Val Arg Gln Ala Pro Gly Gln Arg Pro Glu Trp Met
            35              40              45

ggc tgg atc tac cct gga gat gac tcc acc aag tac tcc cag aag ttc       192
Gly Trp Ile Tyr Pro Gly Asp Asp Ser Thr Lys Tyr Ser Gln Lys Phe
    50              55              60

cag ggt cgc gtg acc att acc agg gac acc agc gcc tcc act gcc tac       240
Gln Gly Arg Val Thr Ile Thr Arg Asp Thr Ser Ala Ser Thr Ala Tyr
65              70              75              80

atg gag ctg tct tcc ctg aga tct gag gat acc gca gtc tac tac tgt       288
Met Glu Leu Ser Ser Leu Arg Ser Glu Asp Thr Ala Val Tyr Tyr Cys
            85              90              95

aca cgg ggg gac cgc ttt gct tac tgg ggg cag ggc act ctg gtg acc       336
Thr Arg Gly Asp Arg Phe Ala Tyr Trp Gly Gln Gly Thr Leu Val Thr
            100             105             110

gtc agc agc                                                           345
Val Ser Ser
            115
```

<210> 42
<211> 115
<212> PRT
<213> Artificial Sequence

<220>
<223> Synthetic Construct

<400> 42

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Ser Tyr
            20              25              30

His Ile His Trp Val Arg Gln Ala Pro Gly Gln Arg Pro Glu Trp Met
            35              40              45
```

```
Gly Trp Ile Tyr Pro Gly Asp Asp Ser Thr Lys Tyr Ser Gln Lys Phe
    50                  55                  60

Gln Gly Arg Val Thr Ile Thr Arg Asp Thr Ser Ala Ser Thr Ala Tyr
65                  70                  75                      80

Met Glu Leu Ser Ser Leu Arg Ser Glu Asp Thr Ala Val Tyr Tyr Cys
                85                  90                  95

Thr Arg Gly Asp Arg Phe Ala Tyr Trp Gly Gln Gly Thr Leu Val Thr
            100                 105                 110

Val Ser Ser
        115
```

<210> 43
<211> 402
<212> DNA
<213> Artificial Sequence

<220>
<223> VH avec leader 4C_35

<220>
<221> CDS
<222> (1)..(402)

<400> 43

```
atg gat tgg aca tgg cga atc ctg ttc ctg gtg gct gcc gca acc ggc          48
Met Asp Trp Thr Trp Arg Ile Leu Phe Leu Val Ala Ala Ala Thr Gly
1               5               10                  15

gcc cac agc cag gtg cag ctg gtg cag agc ggg gcc gag gtg aag aag          96
Ala His Ser Gln Val Gln Leu Val Gln Ser Gly Ala Glu Val Lys Lys
            20                  25                  30

cct gga gcc tca gtg aag gtg agt tgc aag gcc tcc ggt tac acc ttc          144
Pro Gly Ala Ser Val Lys Val Ser Cys Lys Ala Ser Gly Tyr Thr Phe
        35                  40                  45

acc agc tac cac atc cac tgg gtc aga cag gct ccc ggc cag aga cca          192
Thr Ser Tyr His Ile His Trp Val Arg Gln Ala Pro Gly Gln Arg Pro
    50                  55                  60

gag tgg atg ggc tgg atc tac cct gga gat gac tcc acc aag tac tcc          240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc          288
Gln Lys Phe Gln Gly Arg Val Thr Ile Thr Arg Asp Thr Ser Ala Ser
                85                  90                  95

act gcc tac atg gag ctg tct tcc ctg aga tct gag gat acc gca gtc          336
Thr Ala Tyr Met Glu Leu Ser Ser Leu Arg Ser Glu Asp Thr Ala Val
                100                 105                 110

tac tac tgt aca cgg ggg gac cgc ttt gct tac tgg ggg cag ggc act          384
Tyr Tyr Cys Thr Arg Gly Asp Arg Phe Ala Tyr Trp Gly Gln Gly Thr
            115                 120                 125

ctg gtg acc gtc agc agc                                                   402
Leu Val Thr Val Ser Ser
        130
```

<210> 44
<211> 134
<212> PRT
<213> Artificial Sequence

<220>
<223> Synthetic Construct

<400> 44

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
            35                  40                  45

Thr Ser Tyr His Ile His Trp Val Arg Gln Ala Pro Gly Gln Arg Pro
        50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
            85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser
        130
```

<210> 45
<211> 318
<212> DNA
<213> Artificial Sequence

<220>
<223> VL sans leader 5B_42

<220>
<221> CDS
<222> (1)..(318)

<400> 45

```
gac atc cag atg aca cag tcc cca tct acc ctg tct gct tcc gtg gga        48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc        96
Asp Arg Val Thr Ile Thr Cys Arg Ala Ser Ser Ser Val Arg Tyr Ile
                20                  25                  30

gct tgg tac cag cag aag cca gga aag gcc cca aag ctg ctg atc tac       144
Ala Trp Tyr Gln Gln Lys Pro Gly Lys Ala Pro Lys Leu Leu Ile Tyr
            35                  40                  45

cca acc tcc tcc ctg gaa tcc ggg gtg ccc agc aga ttc tca ggc agt      192
Pro Thr Ser Ser Leu Glu Ser Gly Val Pro Ser Arg Phe Ser Gly Ser
        50                  55                  60

ggc tcc ggc acc gaa ttc acc ctg acc atc agc tca ctg cag cct gac      240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca      288
Asp Phe Ala Thr Tyr Tyr Cys Leu Gln Trp Ser Ser Tyr Pro Trp Thr
                85                  90                  95

ttc ggc ggc ggc acc aag gtg gag atc aag                              318
Phe Gly Gly Gly Thr Lys Val Glu Ile Lys
            100                 105
```

<210> 46
<211> 106
<212> PRT
<213> Artificial Sequence

<220>
<223> Synthetic Construct

<400> 46

```
Asp Ile Gln Met Thr Gln Ser Pro Ser Thr Leu Ser Ala Ser Val Gly
1               5               10              15

Asp Arg Val Thr Ile Thr Cys Arg Ala Ser Ser Ser Val Arg Tyr Ile
            20              25              30

Ala Trp Tyr Gln Gln Lys Pro Gly Lys Ala Pro Lys Leu Leu Ile Tyr
        35              40              45

Pro Thr Ser Ser Leu Glu Ser Gly Val Pro Ser Arg Phe Ser Gly Ser
        50              55              60

Gly Ser Gly Thr Glu Phe Thr Leu Thr Ile Ser Ser Leu Gln Pro Asp
65              70              75              80

Asp Phe Ala Thr Tyr Tyr Cys Leu Gln Trp Ser Ser Tyr Pro Trp Thr
            85              90              95

Phe Gly Gly Gly Thr Lys Val Glu Ile Lys

                100             105
```

<210> 47
<211> 384
<212> DNA
<213> Artificial Sequence

<220>
<223> VL avec leader 5B_42

<220>
<221> CDS
<222> (1)..(384)

<400> 47

80

```
atg gat atg aga gtg ccc gca cag ctg ctg ggt ctg ctg ctg ctg tgg          48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc          96
Leu Pro Gly Ala Lys Cys Asp Ile Gln Met Thr Gln Ser Pro Ser Thr
            20                  25                  30

ctg tct gct tcc gtg gga gat cgg gtg act atc acc tgc aga gca agc          144
Leu Ser Ala Ser Val Gly Asp Arg Val Thr Ile Thr Cys Arg Ala Ser
        35                  40                  45

tcc tcc gtg agg tac atc gct tgg tac cag cag aag cca gga aag gcc          192
Ser Ser Val Arg Tyr Ile Ala Trp Tyr Gln Gln Lys Pro Gly Lys Ala
    50                  55                  60

cca aag ctg ctg atc tac cca acc tcc tcc ctg gaa tcc ggg gtg ccc          240
Pro Lys Leu Leu Ile Tyr Pro Thr Ser Ser Leu Glu Ser Gly Val Pro
65                  70                  75                  80

agc aga ttc tca ggc agt ggc tcc ggc acc gaa ttc acc ctg acc atc          288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg          336
Ser Ser Leu Gln Pro Asp Asp Phe Ala Thr Tyr Tyr Cys Leu Gln Trp
                100                 105                 110

agt agc tac cct tgg aca ttc ggc ggc ggc acc aag gtg gag atc aag          384
Ser Ser Tyr Pro Trp Thr Phe Gly Gly Gly Thr Lys Val Glu Ile Lys
        115                 120                 125
```

<210> 48
<211> 128
<212> PRT
<213> Artificial Sequence

<220>
<223> Synthetic Construct

<400> 48

```
Met Asp Met Arg Val Pro Ala Gln Leu Leu Gly Leu Leu Leu Leu Trp
1               5                   10                  15


Leu Pro Gly Ala Lys Cys Asp Ile Gln Met Thr Gln Ser Pro Ser Thr
```

```
                       20                      25                      30

  Leu Ser Ala Ser Val Gly Asp Arg Val Thr Ile Thr Cys Arg Ala Ser
              35                  40                  45

  Ser Ser Val Arg Tyr Ile Ala Trp Tyr Gln Gln Lys Pro Gly Lys Ala
      50                  55                  60

  Pro Lys Leu Leu Ile Tyr Pro Thr Ser Ser Leu Glu Ser Gly Val Pro
  65                  70                  75                  80

  Ser Arg Phe Ser Gly Ser Gly Ser Gly Thr Glu Phe Thr Leu Thr Ile
                  85                  90                  95

  Ser Ser Leu Gln Pro Asp Asp Phe Ala Thr Tyr Tyr Cys Leu Gln Trp
              100                 105                 110

  Ser Ser Tyr Pro Trp Thr Phe Gly Gly Gly Thr Lys Val Glu Ile Lys
              115                 120                 125
```

<210> 49
<211> 345
<212> DNA
<213> Artificial Sequence

<220>
<223> VH sans leader 5B_42

<220>
<221> CDS
<222> (1)..(345)

<400> 49

```
cag gtg cag ctg gtg cag agc ggg gcc gag gtg aag aag cct gga gcc      48
Gln Val Gln Leu Val Gln Ser Gly Ala Glu Val Lys Lys Pro Gly Ala
1               5                   10                  15

tca gtg aag gtg agt tgc aag gcc tcc ggt tac acc ttc acc agc tac      96
Ser Val Lys Val Ser Cys Lys Ala Ser Gly Tyr Thr Phe Thr Ser Tyr
                20                  25                  30

cac atc cac tgg gtc aga cag gct ccc ggc cag aga ctg gag tgg atg     144
His Ile His Trp Val Arg Gln Ala Pro Gly Gln Arg Leu Glu Trp Met
            35                  40                  45

ggc tgg atc tac cct gga gat gac tcc acc aag tac tcc cag aag ttc     192
Gly Trp Ile Tyr Pro Gly Asp Asp Ser Thr Lys Tyr Ser Gln Lys Phe
        50                  55                  60

cag ggt cgc gtg acc att acc agg gac gcc agc gcc tcc act gcc tac     240
Gln Gly Arg Val Thr Ile Thr Arg Asp Ala Ser Ala Ser Thr Ala Tyr
65                  70                  75                  80

atg gag ctg tct tcc ctg aga tct gag gat acc gca gtc tac tac tgt     288
Met Glu Leu Ser Ser Leu Arg Ser Glu Asp Thr Ala Val Tyr Tyr Cys
                85                  90                  95

aca cgg ggg gac cgc ttt gct tac tgg ggg cag ggc act ctg gtg acc     336
Thr Arg Gly Asp Arg Phe Ala Tyr Trp Gly Gln Gly Thr Leu Val Thr
                100                 105                 110

gtc agc agc                                                         345
Val Ser Ser
            115
```

<210> 50
<211> 115
<212> PRT
<213> Artificial Sequence

<220>
<223> Synthetic Construct

<400> 50

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Ser Tyr
            20              25              30

His Ile His Trp Val Arg Gln Ala Pro Gly Gln Arg Leu Glu Trp Met
        35              40              45

Gly Trp Ile Tyr Pro Gly Asp Asp Ser Thr Lys Tyr Ser Gln Lys Phe
    50              55              60

Gln Gly Arg Val Thr Ile Thr Arg Asp Ala Ser Ala Ser Thr Ala Tyr
65              70              75              80

Met Glu Leu Ser Ser Leu Arg Ser Glu Asp Thr Ala Val Tyr Tyr Cys
            85              90              95

Thr Arg Gly Asp Arg Phe Ala Tyr Trp Gly Gln Gly Thr Leu Val Thr
        100             105             110

Val Ser Ser
        115
```

<210> 51
<211> 402
<212> DNA
<213> Artificial Sequence

<220>
<223> VH avec leader 5B_42

<220>
<221> CDS
<222> (1)..(402)

<400> 51

```
atg gat tgg aca tgg cga atc ctg ttc ctg gtg gct gcc gca acc ggc          48
Met Asp Trp Thr Trp Arg Ile Leu Phe Leu Val Ala Ala Ala Thr Gly
1               5                   10                  15

gcc cac agc cag gtg cag ctg gtg cag agc ggg gcc gag gtg aag aag          96
Ala His Ser Gln Val Gln Leu Val Gln Ser Gly Ala Glu Val Lys Lys
            20                  25                  30

cct gga gcc tca gtg aag gtg agt tgc aag gcc tcc ggt tac acc ttc         144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga ctg         192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc         240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac gcc agc gcc tcc         288
Gln Lys Phe Gln Gly Arg Val Thr Ile Thr Arg Asp Ala Ser Ala Ser
                85                  90                  95

act gcc tac atg gag ctg tct tcc ctg aga tct gag gat acc gca gtc         336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act         384
Tyr Tyr Cys Thr Arg Gly Asp Arg Phe Ala Tyr Trp Gly Gln Gly Thr
            115                 120                 125

ctg gtg acc gtc agc agc                                                  402
Leu Val Thr Val Ser Ser
            130
```

<210> 52
<211> 134
<212> PRT
<213> Artificial Sequence

<220>
<223> Synthetic Construct

<400> 52

```
Met Asp Trp Thr Trp Arg Ile Leu Phe Leu Val Ala Ala Ala Thr Gly
1               5               10                  15

Ala His Ser Gln Val Gln Leu Val Gln Ser Gly Ala Glu Val Lys Lys
            20              25                  30

Pro Gly Ala Ser Val Lys Val Ser Cys Lys Ala Ser Gly Tyr Thr Phe
            35              40                  45

Thr Ser Tyr His Ile His Trp Val Arg Gln Ala Pro Gly Gln Arg Leu
            50              55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Ala Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
            115                 120                 125

Leu Val Thr Val Ser Ser
            130
```

<210> 53
<211> 639
<212> DNA
<213> Artificial Sequence

<220>
<223> Chaîne légère sans leader 12G4 humanisé

<220>
<221> CDS
<222> (1)..(639)

<400> 53

```
gac atc cag atg aca cag tcc cca tct acc ctg tct gct tcc gtg gga          48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc          96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg atc tac         144
Ala Trp Tyr Gln Gln Lys Pro Gly Lys Ala Pro Lys Leu Leu Ile Tyr
            35                  40                  45

tca acc tcc tcc ctg gaa tcc ggg gtg ccc agc aga ttc tca ggc agt         192
Ser Thr Ser Ser Leu Glu Ser Gly Val Pro Ser Arg Phe Ser Gly Ser
        50                  55                  60

ggc tcc ggc acc gaa ttc acc ctg acc atc agc tca ctg cag cct gac         240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca         288
Asp Phe Ala Thr Tyr Tyr Cys Leu Gln Trp Ser Ser Tyr Pro Trp Thr
                85                  90                  95

ttc ggc ggc ggc acc aag gtg gag atc aag cgg acc gtc gcc gca cca         336
Phe Gly Gly Gly Thr Lys Val Glu Ile Lys Arg Thr Val Ala Ala Pro
            100                 105                 110

agt gtc ttc atc ttc ccg cca tct gat gag cag ttg aaa tct gga act         384
Ser Val Phe Ile Phe Pro Pro Ser Asp Glu Gln Leu Lys Ser Gly Thr
            115                 120                 125

gcc tct gtt gtg tgc ctg ctg aat aac ttc tat ccc aga gag gcc aaa         432
```

```
Ala Ser Val Val Cys Leu Leu Asn Asn Phe Tyr Pro Arg Glu Ala Lys
    130                 135                 140

gta cag tgg aag gtg gat aac gcc ctc caa tcg ggt aac tcc cag gag         480
Val Gln Trp Lys Val Asp Asn Ala Leu Gln Ser Gly Asn Ser Gln Glu
145                 150                 155                 160

agt gtc aca gag cag gac agc aag gac agc acc tac agc ctc agc agc         528
Ser Val Thr Glu Gln Asp Ser Lys Asp Ser Thr Tyr Ser Leu Ser Ser
                165                 170                 175

acc ctg acg ctg agc aaa gca gac tac gag aaa cac aaa gtc tac gcc         576
Thr Leu Thr Leu Ser Lys Ala Asp Tyr Glu Lys His Lys Val Tyr Ala
            180                 185                 190

tgc gaa gtc acc cat cag ggc ctg agc tcg ccc gtc aca aag agc ttc         624
Cys Glu Val Thr His Gln Gly Leu Ser Ser Pro Val Thr Lys Ser Phe
            195                 200                 205

aac agg gga gag tgt                                                     639
Asn Arg Gly Glu Cys
            210
```

```
<210> 54
<211> 213
<212> PRT
```

<213> Artificial Sequence

<220>
<223> Synthetic Construct

<400> 54

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Ile Tyr
            35                  40                  45

Ser Thr Ser Ser Leu Glu Ser Gly Val Pro Ser Arg Phe Ser Gly Ser
        50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
            165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
            195                 200                 205

Asn Arg Gly Glu Cys
            210
```

<210> 55
<211> 705
<212> DNA
<213> Artificial Sequence

<220>
<223> Chaîne légère avec leader 12G4 humanisé

<220>
<221> CDS
<222> (1)..(705)

<400> 55

```
atg gat atg aga gtg ccc gca cag ctg ctg ggt ctg ctg ctg ctg tgg      48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc      96
Leu Pro Gly Ala Lys Cys Asp Ile Gln Met Thr Gln Ser Pro Ser Thr
                20                  25                  30

ctg tct gct tcc gtg gga gat cgg gtg act atc acc tgc aga gca agc     144
Leu Ser Ala Ser Val Gly Asp Arg Val Thr Ile Thr Cys Arg Ala Ser
            35                  40                  45

tcc tcc gtg agg tac atc gct tgg tac cag cag aag cca gga aag gcc     192
Ser Ser Val Arg Tyr Ile Ala Trp Tyr Gln Gln Lys Pro Gly Lys Ala
        50                  55                  60

cca aag ctg ctg atc tac tca acc tcc tcc ctg gaa tcc ggg gtg ccc     240
Pro Lys Leu Leu Ile Tyr Ser Thr Ser Ser Leu Glu Ser Gly Val Pro
65                  70                  75                  80

agc aga ttc tca ggc agt ggc tcc ggc acc gaa ttc acc ctg acc atc     288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg     336
Ser Ser Leu Gln Pro Asp Asp Phe Ala Thr Tyr Tyr Cys Leu Gln Trp
```

```
                    100                    105                      110

        agt agc tac cct tgg aca ttc ggc ggc ggc acc aag gtg gag atc aag      384
        Ser Ser Tyr Pro Trp Thr Phe Gly Gly Gly Thr Lys Val Glu Ile Lys
                115                 120                 125

        cgg acc gtc gcc gca cca agt gtc ttc atc ttc ccg cca tct gat gag      432
        Arg Thr Val Ala Ala Pro Ser Val Phe Ile Phe Pro Pro Ser Asp Glu
                130                 135                 140

        cag ttg aaa tct gga act gcc tct gtt gtg tgc ctg ctg aat aac ttc      480
        Gln Leu Lys Ser Gly Thr Ala Ser Val Val Cys Leu Leu Asn Asn Phe
        145                 150                 155                 160

        tat ccc aga gag gcc aaa gta cag tgg aag gtg gat aac gcc ctc caa      528
        Tyr Pro Arg Glu Ala Lys Val Gln Trp Lys Val Asp Asn Ala Leu Gln
                        165                 170                 175

        tcg ggt aac tcc cag gag agt gtc aca gag cag gac agc aag gac agc      576
        Ser Gly Asn Ser Gln Glu Ser Val Thr Glu Gln Asp Ser Lys Asp Ser
                        180                 185                 190

        acc tac agc ctc agc agc acc ctg acg ctg agc aaa gca gac tac gag      624
        Thr Tyr Ser Leu Ser Ser Thr Leu Thr Leu Ser Lys Ala Asp Tyr Glu
                        195                 200                 205

        aaa cac aaa gtc tac gcc tgc gaa gtc acc cat cag ggc ctg agc tcg      672
        Lys His Lys Val Tyr Ala Cys Glu Val Thr His Gln Gly Leu Ser Ser
                210                 215                 220

        ccc gtc aca aag agc ttc aac agg gga gag tgt                          705
        Pro Val Thr Lys Ser Phe Asn Arg Gly Glu Cys
        225                 230                 235
```

<210> 56
<211> 235
<212> PRT
<213> Artificial Sequence

<220>
<223> Synthetic Construct

<400> 56

```
Met Asp Met Arg Val Pro Ala Gln Leu Leu Gly Leu Leu Leu Trp
1               5               10              15

Leu Pro Gly Ala Lys Cys Asp Ile Gln Met Thr Gln Ser Pro Ser Thr
            20              25              30

Leu Ser Ala Ser Val Gly Asp Arg Val Thr Ile Thr Cys Arg Ala Ser
        35              40              45

Ser Ser Val Arg Tyr Ile Ala Trp Tyr Gln Gln Lys Pro Gly Lys Ala
        50              55              60

Pro Lys Leu Leu Ile Tyr Ser Thr Ser Ser Leu Glu Ser Gly Val Pro
65              70              75              80

Ser Arg Phe Ser Gly Ser Gly Ser Gly Thr Glu Phe Thr Leu Thr Ile
            85              90              95

Ser Ser Leu Gln Pro Asp Asp Phe Ala Thr Tyr Tyr Cys Leu Gln Trp
        100             105             110

Ser Ser Tyr Pro Trp Thr Phe Gly Gly Gly Thr Lys Val Glu Ile Lys
        115             120             125

Arg Thr Val Ala Ala Pro Ser Val Phe Ile Phe Pro Pro Ser Asp Glu
    130             135             140

Gln Leu Lys Ser Gly Thr Ala Ser Val Val Cys Leu Leu Asn Asn Phe
145             150             155             160

Tyr Pro Arg Glu Ala Lys Val Gln Trp Lys Val Asp Asn Ala Leu Gln
            165             170             175

Ser Gly Asn Ser Gln Glu Ser Val Thr Glu Gln Asp Ser Lys Asp Ser
        180             185             190

Thr Tyr Ser Leu Ser Ser Thr Leu Thr Leu Ser Lys Ala Asp Tyr Glu
        195             200             205

Lys His Lys Val Tyr Ala Cys Glu Val Thr His Gln Gly Leu Ser Ser
    210             215             220

Pro Val Thr Lys Ser Phe Asn Arg Gly Glu Cys
225             230             235
```

<210> 57
<211> 1335
<212> DNA

91

<213> Artificial Sequence

<220>
<223> Chaîne lourde sans leader 12G4 humanisé

<220>
<221> CDS
<222> (1)..(1335)

<400> 57

```
cag gtg cag ctg gtg cag agc ggg gcc gag gtg aag aag cct gga gcc    48
Gln Val Gln Leu Val Gln Ser Gly Ala Glu Val Lys Lys Pro Gly Ala
1               5                  10                 15

tca gtg aag gtg agt tgc aag gcc tcc ggt tac acc ttc acc agc tac    96
Ser Val Lys Val Ser Cys Lys Ala Ser Gly Tyr Thr Phe Thr Ser Tyr
                20                 25                 30

cac atc cac tgg gtc aga cag gct ccc ggc cag aga ctg gag tgg atg   144
His Ile His Trp Val Arg Gln Ala Pro Gly Gln Arg Leu Glu Trp Met
            35                 40                 45
```

```
ggc tgg atc tac cct gga gat gac tcc acc aag tac tcc cag aag ttc        192
Gly Trp Ile Tyr Pro Gly Asp Asp Ser Thr Lys Tyr Ser Gln Lys Phe
    50              55              60

cag ggt cgc gtg acc att acc agg gac acc agc gcc tcc act gcc tac        240
Gln Gly Arg Val Thr Ile Thr Arg Asp Thr Ser Ala Ser Thr Ala Tyr
65              70              75                      80

atg gag ctg tct tcc ctg aga tct gag gat acc gca gtc tac tac tgt        288
Met Glu Leu Ser Ser Leu Arg Ser Glu Asp Thr Ala Val Tyr Tyr Cys
                85              90                      95

aca cgg ggg gac cgc ttt gct tac tgg ggg cag ggc act ctg gtg acc        336
Thr Arg Gly Asp Arg Phe Ala Tyr Trp Gly Gln Gly Thr Leu Val Thr
            100             105             110

gtc agc agc gcc agc acc aag ggc cca tcg gtc ttc ccc ctg gca ccc        384
Val Ser Ser Ala Ser Thr Lys Gly Pro Ser Val Phe Pro Leu Ala Pro
        115             120             125

tcc tcc aag agc acc tct ggg ggc aca gcg gcc ctg ggc tgc ctg gtc        432
Ser Ser Lys Ser Thr Ser Gly Gly Thr Ala Ala Leu Gly Cys Leu Val
    130             135             140

aag gac tac ttc ccc gaa ccg gtg acg gtg tcg tgg aac tca ggc gcc        480
Lys Asp Tyr Phe Pro Glu Pro Val Thr Val Ser Trp Asn Ser Gly Ala
145             150             155             160

ctg acc agc ggc gtg cac acc ttc ccg gct gtc cta cag tcc tca gga        528
Leu Thr Ser Gly Val His Thr Phe Pro Ala Val Leu Gln Ser Ser Gly
            165             170             175

ctc tac tcc ctc agc agc gtg gtg acc gtg ccc tcc agc agc ttg ggc        576
Leu Tyr Ser Leu Ser Ser Val Val Thr Val Pro Ser Ser Ser Leu Gly
        180             185             190

acc cag acc tac atc tgc aac gtg aat cac aag ccc agc aac acc aag        624
Thr Gln Thr Tyr Ile Cys Asn Val Asn His Lys Pro Ser Asn Thr Lys
        195             200             205

gtg gac aag aaa gtt gag ccc aaa tct tgt gac aaa act cac aca tgc        672
Val Asp Lys Lys Val Glu Pro Lys Ser Cys Asp Lys Thr His Thr Cys
    210             215             220

cca ccg tgc cca gca cct gaa ctc ctg ggg gga ccg tca gtc ttc ctc        720
Pro Pro Cys Pro Ala Pro Glu Leu Leu Gly Gly Pro Ser Val Phe Leu
225             230             235             240

ttc ccc cca aaa ccc aag gac acc ctc atg atc tcc cgg acc cct gag        768
Phe Pro Pro Lys Pro Lys Asp Thr Leu Met Ile Ser Arg Thr Pro Glu
            245             250             255

gtc aca tgc gtg gtg gtg gac gtg agc cac gaa gac cct gag gtc aag        816
Val Thr Cys Val Val Val Asp Val Ser His Glu Asp Pro Glu Val Lys
            260             265             270

ttc aac tgg tac gtg gac ggc gtg gag gtg cat aat gcc aag aca aag        864
Phe Asn Trp Tyr Val Asp Gly Val Glu Val His Asn Ala Lys Thr Lys
            275             280             285

ccg cgg gag gag cag tac aac agc acg tac cgt gtg gtc agc gtc ctc        912
Pro Arg Glu Glu Gln Tyr Asn Ser Thr Tyr Arg Val Val Ser Val Leu
    290             295             300
```

```
acc gtc ctg cac cag gac tgg ctg aat ggc aag gag tac aag tgc aag       960
Thr Val Leu His Gln Asp Trp Leu Asn Gly Lys Glu Tyr Lys Cys Lys
305                 310                 315                 320

gtc tcc aac aaa gcc ctc cca gcc ccc atc gag aaa acc atc tcc aaa      1008
Val Ser Asn Lys Ala Leu Pro Ala Pro Ile Glu Lys Thr Ile Ser Lys
                325                 330                 335

gcc aaa ggg cag ccc cga gaa cca cag gtg tac acc ctg ccc cca tcc      1056
Ala Lys Gly Gln Pro Arg Glu Pro Gln Val Tyr Thr Leu Pro Pro Ser
                340                 345                 350

cgg gat gag ctg acc aag aac cag gtc agc ctg acc tgc ctg gtc aaa      1104
Arg Asp Glu Leu Thr Lys Asn Gln Val Ser Leu Thr Cys Leu Val Lys
                355                 360                 365

ggc ttc tat ccc agc gac atc gcc gtg gag tgg gag agc aat ggg cag      1152
Gly Phe Tyr Pro Ser Asp Ile Ala Val Glu Trp Glu Ser Asn Gly Gln
                370                 375                 380

ccg gag aac aac tac aag acc acg cct ccc gtg ctg gac tcc gac ggc      1200
Pro Glu Asn Asn Tyr Lys Thr Thr Pro Pro Val Leu Asp Ser Asp Gly
385                 390                 395                 400

tcc ttc ttc ctc tac agc aag ctc acc gtg gac aag agc agg tgg cag      1248
Ser Phe Phe Leu Tyr Ser Lys Leu Thr Val Asp Lys Ser Arg Trp Gln
                405                 410                 415

cag ggg aac gtc ttc tca tgc tcc gtg atg cat gag gct ctg cac aac      1296
Gln Gly Asn Val Phe Ser Cys Ser Val Met His Glu Ala Leu His Asn
                420                 425                 430

cac tac acg cag aag agc ctc tcc ctg tct ccg ggt aaa                  1335
His Tyr Thr Gln Lys Ser Leu Ser Leu Ser Pro Gly Lys
                435                 440                 445
```

<210> 58
<211> 445
<212> PRT
<213> Artificial Sequence

<220>
<223> Synthetic Construct

<400> 58

Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Thr Phe Thr Ser Tyr
                20                  25                  30

His Ile His Trp Val Arg Gln Ala Pro Gly Gln Arg Leu Glu Trp Met
            35                  40                  45

Gly Trp Ile Tyr Pro Gly Asp Asp Ser Thr Lys Tyr Ser Gln Lys Phe
        50                  55                  60

Gln Gly Arg Val Thr Ile Thr Arg Asp Thr Ser Ala Ser Thr Ala Tyr
65                  70                  75                  80

```
Met Glu Leu Ser Ser Leu Arg Ser Glu Asp Thr Ala Val Tyr Tyr Cys
                85                    90                      95

Thr Arg Gly Asp Arg Phe Ala Tyr Trp Gly Gln Gly Thr Leu Val Thr
            100                 105                 110

Val Ser Ser Ala Ser Thr Lys Gly Pro Ser Val Phe Pro Leu Ala Pro
            115                 120                 125

Ser Ser Lys Ser Thr Ser Gly Gly Thr Ala Ala Leu Gly Cys Leu Val
        130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
            195                 200                 205

Val Asp Lys Lys Val Glu Pro Lys Ser Cys Asp Lys Thr His Thr Cys
        210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
            245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
            275                 280                 285

Pro Arg Glu Glu Gln Tyr Asn Ser Thr Tyr Arg Val Val Ser Val Leu
            290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335
```

```
Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
            355                 360                 365

Gly Phe Tyr Pro Ser Asp Ile Ala Val Glu Trp Glu Ser Asn Gly Gln
            370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
            435                 440                 445
```

<210> 59
<211> 1392
<212> DNA
<213> Artificial Sequence

<220>
<223> Chaîne lourde avec leader 12G4 humanisé

<220>
<221> CDS
<222> (1)..(1392)

<400> 59

```
atg gat tgg aca tgg cga atc ctg ttc ctg gtg gct gcc gca acc ggc      48
Met Asp Trp Thr Trp Arg Ile Leu Phe Leu Val Ala Ala Ala Thr Gly
1               5                   10                  15

gcc cac agc cag gtg cag ctg gtg cag agc ggg gcc gag gtg aag aag      96
Ala His Ser Gln Val Gln Leu Val Gln Ser Gly Ala Glu Val Lys Lys
                20                  25                  30

cct gga gcc tca gtg aag gtg agt tgc aag gcc tcc ggt tac acc ttc     144
Pro Gly Ala Ser Val Lys Val Ser Cys Lys Ala Ser Gly Tyr Thr Phe
                35                  40                  45

acc agc tac cac atc cac tgg gtc aga cag gct ccc ggc cag aga ctg     192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc     240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc     288
Gln Lys Phe Gln Gly Arg Val Thr Ile Thr Arg Asp Thr Ser Ala Ser
```

```
                        85                    90                    95

act gcc tac atg gag ctg tct tcc ctg aga tct gag gat acc gca gtc        336
Thr Ala Tyr Met Glu Leu Ser Ser Leu Arg Ser Glu Asp Thr Ala Val
            100                    105                   110

tac tac tgt aca cgg ggg gac cgc ttt gct tac tgg ggg cag ggc act        384
Tyr Tyr Cys Thr Arg Gly Asp Arg Phe Ala Tyr Trp Gly Gln Gly Thr
            115                    120                   125

ctg gtg acc gtc agc agc gcc agc acc aag ggc cca tcg gtc ttc ccc        432
Leu Val Thr Val Ser Ser Ala Ser Thr Lys Gly Pro Ser Val Phe Pro
        130                    135                   140

ctg gca ccc tcc tcc aag agc acc tct ggg ggc aca gcg gcc ctg ggc        480
Leu Ala Pro Ser Ser Lys Ser Thr Ser Gly Gly Thr Ala Ala Leu Gly
145                    150                   155                   160

tgc ctg gtc aag gac tac ttc ccc gaa ccg gtg acg gtg tcg tgg aac        528
Cys Leu Val Lys Asp Tyr Phe Pro Glu Pro Val Thr Val Ser Trp Asn
                165                    170                   175

tca ggc gcc ctg acc agc ggc gtg cac acc ttc ccg gct gtc cta cag        576
Ser Gly Ala Leu Thr Ser Gly Val His Thr Phe Pro Ala Val Leu Gln
            180                    185                   190

tcc tca gga ctc tac tcc ctc agc agc gtg gtg acc gtg ccc tcc agc        624
Ser Ser Gly Leu Tyr Ser Leu Ser Ser Val Val Thr Val Pro Ser Ser
            195                    200                   205

agc ttg ggc acc cag acc tac atc tgc aac gtg aat cac aag ccc agc        672
Ser Leu Gly Thr Gln Thr Tyr Ile Cys Asn Val Asn His Lys Pro Ser
        210                    215                   220

aac acc aag gtg gac aag aaa gtt gag ccc aaa tct tgt gac aaa act        720
Asn Thr Lys Val Asp Lys Lys Val Glu Pro Lys Ser Cys Asp Lys Thr
225                    230                   235                   240

cac aca tgc cca ccg tgc cca gca cct gaa ctc ctg ggg gga ccg tca        768
His Thr Cys Pro Pro Cys Pro Ala Pro Glu Leu Leu Gly Gly Pro Ser
                245                    250                   255

gtc ttc ctc ttc ccc cca aaa ccc aag gac acc ctc atg atc tcc cgg        816
Val Phe Leu Phe Pro Pro Lys Pro Lys Asp Thr Leu Met Ile Ser Arg
            260                    265                   270

acc cct gag gtc aca tgc gtg gtg gtg gac gtg agc cac gaa gac cct        864
Thr Pro Glu Val Thr Cys Val Val Val Asp Val Ser His Glu Asp Pro
            275                    280                   285

gag gtc aag ttc aac tgg tac gtg gac ggc gtg gag gtg cat aat gcc        912
Glu Val Lys Phe Asn Trp Tyr Val Asp Gly Val Glu Val His Asn Ala
            290                    295                   300

aag aca aag ccg cgg gag gag cag tac aac agc acg tac cgt gtg gtc        960
Lys Thr Lys Pro Arg Glu Glu Gln Tyr Asn Ser Thr Tyr Arg Val Val
305                    310                   315                   320

agc gtc ctc acc gtc ctg cac cag gac tgg ctg aat ggc aag gag tac       1008
Ser Val Leu Thr Val Leu His Gln Asp Trp Leu Asn Gly Lys Glu Tyr
            325                    330                   335

aag tgc aag gtc tcc aac aaa gcc ctc cca gcc ccc atc gag aaa acc       1056
Lys Cys Lys Val Ser Asn Lys Ala Leu Pro Ala Pro Ile Glu Lys Thr
            340                    345                   350
```

```
atc tcc aaa gcc aaa ggg cag ccc cga gaa cca cag gtg tac acc ctg      1104
Ile Ser Lys Ala Lys Gly Gln Pro Arg Glu Pro Gln Val Tyr Thr Leu
        355             360             365

ccc cca tcc cgg gat gag ctg acc aag aac cag gtc agc ctg acc tgc      1152
Pro Pro Ser Arg Asp Glu Leu Thr Lys Asn Gln Val Ser Leu Thr Cys
        370             375             380

ctg gtc aaa ggc ttc tat ccc agc gac atc gcc gtg gag tgg gag agc      1200
Leu Val Lys Gly Phe Tyr Pro Ser Asp Ile Ala Val Glu Trp Glu Ser
385             390             395             400

aat ggg cag ccg gag aac aac tac aag acc acg cct ccc gtg ctg gac      1248
Asn Gly Gln Pro Glu Asn Asn Tyr Lys Thr Thr Pro Pro Val Leu Asp
                405             410             415

tcc gac ggc tcc ttc ttc ctc tac agc aag ctc acc gtg gac aag agc      1296
Ser Asp Gly Ser Phe Phe Leu Tyr Ser Lys Leu Thr Val Asp Lys Ser
        420             425             430

agg tgg cag cag ggg aac gtc ttc tca tgc tcc gtg atg cat gag gct      1344
Arg Trp Gln Gln Gly Asn Val Phe Ser Cys Ser Val Met His Glu Ala
        435             440             445

ctg cac aac cac tac acg cag aag agc ctc tcc ctg tct ccg ggt aaa      1392
Leu His Asn His Tyr Thr Gln Lys Ser Leu Ser Leu Ser Pro Gly Lys
        450             455             460
```

<210> 60
<211> 464
<212> PRT
<213> Artificial Sequence

<220>
<223> Synthetic Construct

<400> 60

Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu
    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
            85                  90                  95

Thr Ala Tyr Met Glu Leu Ser Ser Leu Arg Ser Glu Asp Thr Ala Val

```
                   100                         105                        110


        Tyr Tyr Cys Thr Arg Gly Asp Arg Phe Ala Tyr Trp Gly Gln Gly Thr
                115                 120                 125


        Leu Val Thr Val Ser Ser Ala Ser Thr Lys Gly Pro Ser Val Phe Pro
                130                 135                 140


        Leu Ala Pro Ser Ser Lys Ser Thr Ser Gly Gly Thr Ala Ala Leu Gly
        145                 150                 155                 160


        Cys Leu Val Lys Asp Tyr Phe Pro Glu Pro Val Thr Val Ser Trp Asn
                        165                 170                 175


        Ser Gly Ala Leu Thr Ser Gly Val His Thr Phe Pro Ala Val Leu Gln
                    180                 185                 190


        Ser Ser Gly Leu Tyr Ser Leu Ser Ser Val Val Thr Val Pro Ser Ser
                    195                 200                 205


        Ser Leu Gly Thr Gln Thr Tyr Ile Cys Asn Val Asn His Lys Pro Ser
                210                 215                 220


        Asn Thr Lys Val Asp Lys Lys Val Glu Pro Lys Ser Cys Asp Lys Thr
        225                 230                 235                 240


        His Thr Cys Pro Pro Cys Pro Ala Pro Glu Leu Leu Gly Gly Pro Ser
                        245                 250                 255


        Val Phe Leu Phe Pro Pro Lys Pro Lys Asp Thr Leu Met Ile Ser Arg
                    260                 265                 270


        Thr Pro Glu Val Thr Cys Val Val Val Asp Val Ser His Glu Asp Pro
                275                 280                 285


        Glu Val Lys Phe Asn Trp Tyr Val Asp Gly Val Glu Val His Asn Ala
                290                 295                 300


        Lys Thr Lys Pro Arg Glu Glu Gln Tyr Asn Ser Thr Tyr Arg Val Val
        305                 310                 315                 320


        Ser Val Leu Thr Val Leu His Gln Asp Trp Leu Asn Gly Lys Glu Tyr
                        325                 330                 335


        Lys Cys Lys Val Ser Asn Lys Ala Leu Pro Ala Pro Ile Glu Lys Thr
                    340                 345                 350


        Ile Ser Lys Ala Lys Gly Gln Pro Arg Glu Pro Gln Val Tyr Thr Leu
                    355                 360                 365
```

```
Pro Pro Ser Arg Asp Glu Leu Thr Lys Asn Gln Val Ser Leu Thr Cys
    370                 375             380

Leu Val Lys Gly Phe Tyr Pro Ser Asp Ile Ala Val Glu Trp Glu Ser
385                 390             395                 400

Asn Gly Gln Pro Glu Asn Asn Tyr Lys Thr Thr Pro Pro Val Leu Asp
                405             410                 415

Ser Asp Gly Ser Phe Phe Leu Tyr Ser Lys Leu Thr Val Asp Lys Ser
            420             425             430

Arg Trp Gln Gln Gly Asn Val Phe Ser Cys Ser Val Met His Glu Ala
        435             440             445

Leu His Asn His Tyr Thr Gln Lys Ser Leu Ser Leu Ser Pro Gly Lys
    450                 455             460
```

<210> 61
<211> 645
<212> DNA
<213> Artificial Sequence

<220>
<223> Chaîne légère sans leader 12G4 chimérique

<220>
<221> CDS
<222> (1)..(645)

<400> 61

```
caa att gtt ctc acc cag tct cca gca atc atg tct gca tct cta ggg    48
Gln Ile Val Leu Thr Gln Ser Pro Ala Ile Met Ser Ala Ser Leu Gly
1               5               10              15

gag ggg atc acc cta acc tgc agt gcc agc tcg agt gta cgt tac ata    96
Glu Gly Ile Thr Leu Thr Cys Ser Ala Ser Ser Ser Val Arg Tyr Ile
            20              25              30

cac tgg tac cag cag aag tca ggc act tct ccc aaa ctc ttg att tat   144
His Trp Tyr Gln Gln Lys Ser Gly Thr Ser Pro Lys Leu Leu Ile Tyr
        35              40              45

agc aca tcc aac ctg gct tct gga gtc cct tct cgc ttc agt ggc agt   192
Ser Thr Ser Asn Leu Ala Ser Gly Val Pro Ser Arg Phe Ser Gly Ser
        50              55              60

ggg tct ggg acc ttt cat tct ctc aca atc agc agt gtg gag gct gaa   240
Gly Ser Gly Thr Phe His Ser Leu Thr Ile Ser Ser Val Glu Ala Glu
65              70              75              80

gat gct gcc gat tat tac tgc ctt cag tgg agt agt tat ccg tgg acg   288
Asp Ala Ala Asp Tyr Tyr Cys Leu Gln Trp Ser Ser Tyr Pro Trp Thr
                85              90              95

ttc ggt gga ggc acc aag ctg gaa atc aaa cga act gtg gct gca cca   336

Phe Gly Gly Gly Thr Lys Leu Glu Ile Lys Arg Thr Val Ala Ala Pro
            100             105             110

agt gtc ttc atc ttc ccg cca tct gat gag cag ttg aaa tct gga act   384
Ser Val Phe Ile Phe Pro Pro Ser Asp Glu Gln Leu Lys Ser Gly Thr
            115             120             125

gcc tct gtt gtg tgc ctg ctg aat aac ttc tat ccc aga gag gcc aaa   432
Ala Ser Val Val Cys Leu Leu Asn Asn Phe Tyr Pro Arg Glu Ala Lys
        130             135             140

gta cag tgg aag gtg gat aac gcc ctc caa tcg ggt aac tcc cag gag   480
Val Gln Trp Lys Val Asp Asn Ala Leu Gln Ser Gly Asn Ser Gln Glu
145             150             155             160

agt gtc aca gag cag gac agc aag gac agc acc tac agc ctc agc agc   528
Ser Val Thr Glu Gln Asp Ser Lys Asp Ser Thr Tyr Ser Leu Ser Ser
                165             170             175

acc ctg acg ctg agc aaa gca gac tac gag aaa cac aaa gtc tac gcc   576
Thr Leu Thr Leu Ser Lys Ala Asp Tyr Glu Lys His Lys Val Tyr Ala
            180             185             190

tgc gaa gtc acc cat cag ggc ctg agc tcg ccc gtc aca aag agc ttc   624
Cys Glu Val Thr His Gln Gly Leu Ser Ser Pro Val Thr Lys Ser Phe
            195             200             205

aac agg gga gag tgt tag tga                                        645
Asn Arg Gly Glu Cys
            210
```

<210> 62
<211> 213
<212> PRT

<213> Artificial Sequence

<220>
<223> Synthetic Construct

<400> 62

```
Gln Ile Val Leu Thr Gln Ser Pro Ala Ile Met Ser Ala Ser Leu Gly
1               5                   10                  15

Glu Gly Ile Thr Leu Thr Cys Ser Ala Ser Ser Ser Val Arg Tyr Ile
            20                  25                  30

His Trp Tyr Gln Gln Lys Ser Gly Thr Ser Pro Lys Leu Leu Ile Tyr
            35                  40                  45

Ser Thr Ser Asn Leu Ala Ser Gly Val Pro Ser Arg Phe Ser Gly Ser
        50                  55                  60

Gly Ser Gly Thr Phe His Ser Leu Thr Ile Ser Ser Val Glu Ala Glu
65                  70                  75                  80

Asp Ala Ala Asp Tyr Tyr Cys Leu Gln Trp Ser Ser Tyr Pro Trp Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
            115                 120                 125

Ala Ser Val Val Cys Leu Leu Asn Asn Phe Tyr Pro Arg Glu Ala Lys
        130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
            195                 200                 205

Asn Arg Gly Glu Cys
            210
```

<210> 63
<211> 711
<212> DNA
<213> Artificial Sequence

<220>
<223> Chaîne légère avec leader 12G4 chimérique

<220>
<221> CDS
<222> (1)..(711)

<400> 63

```
atg gat ttt cag gtg cag att ttc agc ttc ctg cta atc agt gcc tca      48
Met Asp Phe Gln Val Gln Ile Phe Ser Phe Leu Leu Ile Ser Ala Ser
1               5                   10                  15

gtc ata atg tcc aga gga caa att gtt ctc acc cag tct cca gca atc      96
Val Ile Met Ser Arg Gly Gln Ile Val Leu Thr Gln Ser Pro Ala Ile
            20                  25                  30

atg tct gca tct cta ggg gag ggg atc acc cta acc tgc agt gcc agc     144
Met Ser Ala Ser Leu Gly Glu Gly Ile Thr Leu Thr Cys Ser Ala Ser
            35                  40                  45

tcg agt gta cgt tac ata cac tgg tac cag cag aag tca ggc act tct     192
Ser Ser Val Arg Tyr Ile His Trp Tyr Gln Gln Lys Ser Gly Thr Ser
        50                  55                  60

ccc aaa ctc ttg att tat agc aca tcc aac ctg gct tct gga gtc cct     240
Pro Lys Leu Leu Ile Tyr Ser Thr Ser Asn Leu Ala Ser Gly Val Pro
```

65                70                75                80

```
tct cgc ttc agt ggc agt ggg tct ggg acc ttt cat tct ctc aca atc      288
Ser Arg Phe Ser Gly Ser Gly Ser Gly Thr Phe His Ser Leu Thr Ile
                85                    90                    95

agc agt gtg gag gct gaa gat gct gcc gat tat tac tgc ctt cag tgg      336
Ser Ser Val Glu Ala Glu Asp Ala Ala Asp Tyr Tyr Cys Leu Gln Trp
            100                   105                   110

agt agt tat ccg tgg acg ttc ggt gga ggc acc aag ctg gaa atc aaa      384
Ser Ser Tyr Pro Trp Thr Phe Gly Gly Gly Thr Lys Leu Glu Ile Lys
            115                   120                   125

cga act gtg gct gca cca agt gtc ttc atc ttc ccg cca tct gat gag      432
Arg Thr Val Ala Ala Pro Ser Val Phe Ile Phe Pro Pro Ser Asp Glu
        130                   135                   140

cag ttg aaa tct gga act gcc tct gtt gtg tgc ctg ctg aat aac ttc      480
Gln Leu Lys Ser Gly Thr Ala Ser Val Val Cys Leu Leu Asn Asn Phe
145                   150                   155                   160

tat ccc aga gag gcc aaa gta cag tgg aag gtg gat aac gcc ctc caa      528
Tyr Pro Arg Glu Ala Lys Val Gln Trp Lys Val Asp Asn Ala Leu Gln
                165                   170                   175

tcg ggt aac tcc cag gag agt gtc aca gag cag gac agc aag gac agc      576
Ser Gly Asn Ser Gln Glu Ser Val Thr Glu Gln Asp Ser Lys Asp Ser
                180                   185                   190

acc tac agc ctc agc agc acc ctg acg ctg agc aaa gca gac tac gag      624
Thr Tyr Ser Leu Ser Ser Thr Leu Thr Leu Ser Lys Ala Asp Tyr Glu
                195                   200                   205

aaa cac aaa gtc tac gcc tgc gaa gtc acc cat cag ggc ctg agc tcg      672
Lys His Lys Val Tyr Ala Cys Glu Val Thr His Gln Gly Leu Ser Ser
        210                   215                   220

ccc gtc aca aag agc ttc aac agg gga gag tgt tag tga               711
Pro Val Thr Lys Ser Phe Asn Arg Gly Glu Cys
225                   230                   235
```

<210> 64
<211> 235
<212> PRT
<213> Artificial Sequence

<220>
<223> Synthetic Construct

<400> 64

```
Met Asp Phe Gln Val Gln Ile Phe Ser Phe Leu Leu Ile Ser Ala Ser
1               5               10              15

Val Ile Met Ser Arg Gly Gln Ile Val Leu Thr Gln Ser Pro Ala Ile
        20              25              30

Met Ser Ala Ser Leu Gly Glu Gly Ile Thr Leu Thr Cys Ser Ala Ser
        35              40              45

Ser Ser Val Arg Tyr Ile His Trp Tyr Gln Gln Lys Ser Gly Thr Ser
    50              55              60

Pro Lys Leu Leu Ile Tyr Ser Thr Ser Asn Leu Ala Ser Gly Val Pro
65              70              75              80

Ser Arg Phe Ser Gly Ser Gly Ser Gly Thr Phe His Ser Leu Thr Ile
            85              90              95

Ser Ser Val Glu Ala Glu Asp Ala Ala Asp Tyr Tyr Cys Leu Gln Trp
        100             105             110

Ser Ser Tyr Pro Trp Thr Phe Gly Gly Gly Thr Lys Leu Glu Ile Lys
        115             120             125

Arg Thr Val Ala Ala Pro Ser Val Phe Ile Phe Pro Pro Ser Asp Glu
    130             135             140

Gln Leu Lys Ser Gly Thr Ala Ser Val Val Cys Leu Leu Asn Asn Phe
145             150             155             160

Tyr Pro Arg Glu Ala Lys Val Gln Trp Lys Val Asp Asn Ala Leu Gln
            165             170             175

Ser Gly Asn Ser Gln Glu Ser Val Thr Glu Gln Asp Ser Lys Asp Ser
        180             185             190

Thr Tyr Ser Leu Ser Ser Thr Leu Thr Leu Ser Lys Ala Asp Tyr Glu
        195             200             205

Lys His Lys Val Tyr Ala Cys Glu Val Thr His Gln Gly Leu Ser Ser
    210             215             220

Pro Val Thr Lys Ser Phe Asn Arg Gly Glu Cys
225             230             235
```

<210> 65
<211> 1335
<212> DNA

<213> Artificial Sequence

<220>
<223> Chaîne lourde sans leader 12G4 chimérique

<220>
<221> CDS
<222> (1)..(1335)

<400> 65

```
cag gtc cag ctg cag cag tct gga cct gaa ctg gtg aag cct ggg gct      48
Gln Val Gln Leu Gln Gln Ser Gly Pro Glu Leu Val Lys Pro Gly Ala
1               5                   10                  15
```

```
tca gtg agg atg tcc tgc aag gct tct ggc tac acc ttc aca agt tac        96
Ser Val Arg Met Ser Cys Lys Ala Ser Gly Tyr Thr Phe Thr Ser Tyr
            20              25              30

cat ata cac tgg gtg aag cag agg cct gga cag gga ctt gag tgg att       144
His Ile His Trp Val Lys Gln Arg Pro Gly Gln Gly Leu Glu Trp Ile
        35              40              45

gga tgg att tat cct ggc gat gat tct act aaa tac aat gag aag ttc       192
Gly Trp Ile Tyr Pro Gly Asp Asp Ser Thr Lys Tyr Asn Glu Lys Phe
    50              55              60

aag ggc aag acc aca ctg act gca gac aaa tcc tcc agc aca gcc tac       240
Lys Gly Lys Thr Thr Leu Thr Ala Asp Lys Ser Ser Ser Thr Ala Tyr
65              70              75              80

atg ttg ctc agc agc ctg acc tct gag gac tct gcg atc tat ttc tgt       288
Met Leu Leu Ser Ser Leu Thr Ser Glu Asp Ser Ala Ile Tyr Phe Cys
            85              90              95

aca agg ggg gac cgg ttt gct tac tgg ggc caa ggg act ctg gtc act       336
Thr Arg Gly Asp Arg Phe Ala Tyr Trp Gly Gln Gly Thr Leu Val Thr
            100             105             110

gtc tct gca gcc tcc acc aag ggc cca tcg gtc ttc ccc ctg gca ccc       384
Val Ser Ala Ala Ser Thr Lys Gly Pro Ser Val Phe Pro Leu Ala Pro
        115             120             125

tcc tcc aag agc acc tct ggg ggc aca gcg gcc ctg ggc tgc ctg gtc       432
Ser Ser Lys Ser Thr Ser Gly Gly Thr Ala Ala Leu Gly Cys Leu Val
    130             135             140

aag gac tac ttc ccc gaa ccg gtg acg gtg tcg tgg aac tca ggc gcc       480
Lys Asp Tyr Phe Pro Glu Pro Val Thr Val Ser Trp Asn Ser Gly Ala
145             150             155             160

ctg acc agc ggc gtg cac acc ttc ccg gct gtc cta cag tcc tca gga       528
Leu Thr Ser Gly Val His Thr Phe Pro Ala Val Leu Gln Ser Ser Gly
            165             170             175

ctc tac tcc ctc agc agc gtg gtg acc gtg ccc tcc agc agc ttg ggc       576
Leu Tyr Ser Leu Ser Ser Val Val Thr Val Pro Ser Ser Ser Leu Gly
            180             185             190

acc cag acc tac atc tgc aac gtg aat cac aag ccc agc aac acc aag       624
Thr Gln Thr Tyr Ile Cys Asn Val Asn His Lys Pro Ser Asn Thr Lys
        195             200             205

gtg gac aag aaa gtt gag ccc aaa tct tgt gac aaa act cac aca tgc       672
Val Asp Lys Lys Val Glu Pro Lys Ser Cys Asp Lys Thr His Thr Cys
        210             215             220

cca ccg tgc cca gca cct gaa ctc ctg ggg gga ccg tca gtc ttc ctc       720
Pro Pro Cys Pro Ala Pro Glu Leu Leu Gly Gly Pro Ser Val Phe Leu
225             230             235             240

ttc ccc cca aaa ccc aag gac acc ctc atg atc tcc cgg acc cct gag       768
Phe Pro Pro Lys Pro Lys Asp Thr Leu Met Ile Ser Arg Thr Pro Glu
            245             250             255

gtc aca tgc gtg gtg gtg gac gtg agc cac gaa gac cct gag gtc aag       816
Val Thr Cys Val Val Val Asp Val Ser His Glu Asp Pro Glu Val Lys
            260             265             270
```

```
ttc aac tgg tac gtg gac ggc gtg gag gtg cat aat gcc aag aca aag        864
Phe Asn Trp Tyr Val Asp Gly Val Glu Val His Asn Ala Lys Thr Lys
        275             280             285

ccg cgg gag gag cag tac aac agc acg tac cgt gtg gtc agc gtc ctc        912
Pro Arg Glu Glu Gln Tyr Asn Ser Thr Tyr Arg Val Val Ser Val Leu
        290             295             300

acc gtc ctg cac cag gac tgg ctg aat ggc aag gag tac aag tgc aag        960
Thr Val Leu His Gln Asp Trp Leu Asn Gly Lys Glu Tyr Lys Cys Lys
305             310             315             320

gtc tcc aac aaa gcc ctc cca gcc ccc atc gag aaa acc atc tcc aaa       1008
Val Ser Asn Lys Ala Leu Pro Ala Pro Ile Glu Lys Thr Ile Ser Lys
        325             330             335

gcc aaa ggg cag ccc cga gaa cca cag gtg tac acc ctg ccc cca tcc       1056
Ala Lys Gly Gln Pro Arg Glu Pro Gln Val Tyr Thr Leu Pro Pro Ser
        340             345             350

cgg gat gag ctg acc aag aac cag gtc agc ctg acc tgc ctg gtc aaa       1104
Arg Asp Glu Leu Thr Lys Asn Gln Val Ser Leu Thr Cys Leu Val Lys
        355             360             365

ggc ttc tat ccc agc gac atc gcc gtg gag tgg gag agc aat ggg cag       1152
Gly Phe Tyr Pro Ser Asp Ile Ala Val Glu Trp Glu Ser Asn Gly Gln
        370             375             380

ccg gag aac aac tac aag acc acg cct ccc gtg ctg gac tcc gac ggc       1200
Pro Glu Asn Asn Tyr Lys Thr Thr Pro Pro Val Leu Asp Ser Asp Gly
385             390             395             400

tcc ttc ttc ctc tac agc aag ctc acc gtg gac aag agc agg tgg cag       1248
Ser Phe Phe Leu Tyr Ser Lys Leu Thr Val Asp Lys Ser Arg Trp Gln
        405             410             415

cag ggg aac gtc ttc tca tgc tcc gtg atg cat gag gct ctg cac aac       1296
Gln Gly Asn Val Phe Ser Cys Ser Val Met His Glu Ala Leu His Asn
        420             425             430

cac tac acg cag aag agc ctc tcc ctg tct ccg ggt aaa               1335
His Tyr Thr Gln Lys Ser Leu Ser Leu Ser Pro Gly Lys
        435             440             445
```

<210> 66
<211> 445
<212> PRT
<213> Artificial Sequence

<220>
<223> Synthetic Construct

<400> 66

Gln Val Gln Leu Gln Gln Ser Gly Pro Glu Leu Val Lys Pro Gly Ala
1               5               10              15

Ser Val Arg Met Ser Cys Lys Ala Ser Gly Tyr Thr Phe Thr Ser Tyr
        20              25              30

His Ile His Trp Val Lys Gln Arg Pro Gly Gln Gly Leu Glu Trp Ile
        35              40              45

Gly Trp Ile Tyr Pro Gly Asp Asp Ser Thr Lys Tyr Asn Glu Lys Phe
        50              55                  60

Lys Gly Lys Thr Thr Leu Thr Ala Asp Lys Ser Ser Ser Thr Ala Tyr
65              70                  75                  80

Met Leu Leu Ser Ser Leu Thr Ser Glu Asp Ser Ala Ile Tyr Phe Cys
                85                  90                  95

Thr Arg Gly Asp Arg Phe Ala Tyr Trp Gly Gln Gly Thr Leu Val Thr
        100                 105                 110

Val Ser Ala Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
        130                 135                 140

Lys Asp Tyr Phe Pro Glu Pro Val Thr Val Ser Trp Asn Ser Gly Ala
145             150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
        210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
290                 295                 300

113

```
Thr Val Leu His Gln Asp Trp Leu Asn Gly Lys Glu Tyr Lys Cys Lys
305                 310             315                 320


Val Ser Asn Lys Ala Leu Pro Ala Pro Ile Glu Lys Thr Ile Ser Lys
                325             330                 335


Ala Lys Gly Gln Pro Arg Glu Pro Gln Val Tyr Thr Leu Pro Pro Ser
            340             345             350


Arg Asp Glu Leu Thr Lys Asn Gln Val Ser Leu Thr Cys Leu Val Lys
            355             360             365


Gly Phe Tyr Pro Ser Asp Ile Ala Val Glu Trp Glu Ser Asn Gly Gln
    370             375             380


Pro Glu Asn Asn Tyr Lys Thr Thr Pro Pro Val Leu Asp Ser Asp Gly
385             390             395                 400


Ser Phe Phe Leu Tyr Ser Lys Leu Thr Val Asp Lys Ser Arg Trp Gln
            405             410                 415


Gln Gly Asn Val Phe Ser Cys Ser Val Met His Glu Ala Leu His Asn
            420             425             430


His Tyr Thr Gln Lys Ser Leu Ser Leu Ser Pro Gly Lys
        435             440             445
```

<210> 67
<211> 1392
<212> DNA
<213> Artificial Sequence

<220>
<223> Chaîne lourde avec leader 12G4 chimérique

<220>
<221> CDS
<222> (1)..(1392)

<400> 67

```
atg cga tgg agc tgg atc ttt ctc ttc ctc ctg tca ata act gca agt        48
Met Arg Trp Ser Trp Ile Phe Leu Phe Leu Leu Ser Ile Thr Ala Ser
1               5                   10                  15

gtc cat tgc cag gtc cag ctg cag cag tct gga cct gaa ctg gtg aag        96
Val His Cys Gln Val Gln Leu Gln Gln Ser Gly Pro Glu Leu Val Lys
            20                  25                  30

cct ggg gct tca gtg agg atg tcc tgc aag gct tct ggc tac acc ttc       144
Pro Gly Ala Ser Val Arg Met Ser Cys Lys Ala Ser Gly Tyr Thr Phe
        35                  40                  45

aca agt tac cat ata cac tgg gtg aag cag agg cct gga cag gga ctt       192
Thr Ser Tyr His Ile His Trp Val Lys Gln Arg Pro Gly Gln Gly Leu
```

```
              50                      55                      60

gag tgg att gga tgg att tat cct ggc gat gat tct act aaa tac aat      240
Glu Trp Ile Gly Trp Ile Tyr Pro Gly Asp Asp Ser Thr Lys Tyr Asn
65                  70                  75                  80

gag aag ttc aag ggc aag acc aca ctg act gca gac aaa tcc tcc agc      288
Glu Lys Phe Lys Gly Lys Thr Thr Leu Thr Ala Asp Lys Ser Ser Ser
                85                  90                  95

aca gcc tac atg ttg ctc agc agc ctg acc tct gag gac tct gcg atc      336
Thr Ala Tyr Met Leu Leu Ser Ser Leu Thr Ser Glu Asp Ser Ala Ile
            100                 105                 110

tat ttc tgt aca agg ggg gac cgg ttt gct tac tgg ggc caa ggg act      384
Tyr Phe Cys Thr Arg Gly Asp Arg Phe Ala Tyr Trp Gly Gln Gly Thr
            115                 120                 125

ctg gtc act gtc tct gca gcc tcc acc aag ggc cca tcg gtc ttc ccc      432
Leu Val Thr Val Ser Ala Ala Ser Thr Lys Gly Pro Ser Val Phe Pro
            130                 135                 140

ctg gca ccc tcc tcc aag agc acc tct ggg ggc aca gcg gcc ctg ggc      480
Leu Ala Pro Ser Ser Lys Ser Thr Ser Gly Gly Thr Ala Ala Leu Gly
145                 150                 155                 160

tgc ctg gtc aag gac tac ttc ccc gaa ccg gtg acg gtg tcg tgg aac      528
Cys Leu Val Lys Asp Tyr Phe Pro Glu Pro Val Thr Val Ser Trp Asn
                165                 170                 175

tca ggc gcc ctg acc agc ggc gtg cac acc ttc ccg gct gtc cta cag      576
Ser Gly Ala Leu Thr Ser Gly Val His Thr Phe Pro Ala Val Leu Gln
            180                 185                 190

tcc tca gga ctc tac tcc ctc agc agc gtg gtg acc gtg ccc tcc agc      624
Ser Ser Gly Leu Tyr Ser Leu Ser Ser Val Val Thr Val Pro Ser Ser
            195                 200                 205

agc ttg ggc acc cag acc tac atc tgc aac gtg aat cac aag ccc agc      672
Ser Leu Gly Thr Gln Thr Tyr Ile Cys Asn Val Asn His Lys Pro Ser
            210                 215                 220

aac acc aag gtg gac aag aaa gtt gag ccc aaa tct tgt gac aaa act      720
Asn Thr Lys Val Asp Lys Lys Val Glu Pro Lys Ser Cys Asp Lys Thr
225                 230                 235                 240

cac aca tgc cca ccg tgc cca gca cct gaa ctc ctg ggg gga ccg tca      768
His Thr Cys Pro Pro Cys Pro Ala Pro Glu Leu Leu Gly Gly Pro Ser
            245                 250                 255

gtc ttc ctc ttc ccc cca aaa ccc aag gac acc ctc atg atc tcc cgg      816
Val Phe Leu Phe Pro Pro Lys Pro Lys Asp Thr Leu Met Ile Ser Arg
            260                 265                 270

acc cct gag gtc aca tgc gtg gtg gtg gac gtg agc cac gaa gac cct      864
Thr Pro Glu Val Thr Cys Val Val Val Asp Val Ser His Glu Asp Pro
            275                 280                 285

gag gtc aag ttc aac tgg tac gtg gac ggc gtg gag gtg cat aat gcc      912
Glu Val Lys Phe Asn Trp Tyr Val Asp Gly Val Glu Val His Asn Ala
            290                 295                 300

aag aca aag ccg cgg gag gag cag tac aac agc acg tac cgt gtg gtc      960
Lys Thr Lys Pro Arg Glu Glu Gln Tyr Asn Ser Thr Tyr Arg Val Val
305                 310                 315                 320
```

```
agc gtc ctc acc gtc ctg cac cag gac tgg ctg aat ggc aag gag tac       1008
Ser Val Leu Thr Val Leu His Gln Asp Trp Leu Asn Gly Lys Glu Tyr
            325                 330                 335

aag tgc aag gtc tcc aac aaa gcc ctc cca gcc ccc atc gag aaa acc       1056
Lys Cys Lys Val Ser Asn Lys Ala Leu Pro Ala Pro Ile Glu Lys Thr
            340                 345                 350

atc tcc aaa gcc aaa ggg cag ccc cga gaa cca cag gtg tac acc ctg       1104
Ile Ser Lys Ala Lys Gly Gln Pro Arg Glu Pro Gln Val Tyr Thr Leu
            355                 360                 365

ccc cca tcc cgg gat gag ctg acc aag aac cag gtc agc ctg acc tgc       1152
Pro Pro Ser Arg Asp Glu Leu Thr Lys Asn Gln Val Ser Leu Thr Cys
            370                 375                 380

ctg gtc aaa ggc ttc tat ccc agc gac atc gcc gtg gag tgg gag agc       1200
Leu Val Lys Gly Phe Tyr Pro Ser Asp Ile Ala Val Glu Trp Glu Ser
385                 390                 395                 400

aat ggg cag ccg gag aac aac tac aag acc acg cct ccc gtg ctg gac       1248
Asn Gly Gln Pro Glu Asn Asn Tyr Lys Thr Thr Pro Pro Val Leu Asp
                405                 410                 415

tcc gac ggc tcc ttc ttc ctc tac agc aag ctc acc gtg gac aag agc       1296
Ser Asp Gly Ser Phe Phe Leu Tyr Ser Lys Leu Thr Val Asp Lys Ser
            420                 425                 430

agg tgg cag cag ggg aac gtc ttc tca tgc tcc gtg atg cat gag gct       1344
Arg Trp Gln Gln Gly Asn Val Phe Ser Cys Ser Val Met His Glu Ala
            435                 440                 445

ctg cac aac cac tac acg cag aag agc ctc tcc ctg tct ccg ggt aaa       1392
Leu His Asn His Tyr Thr Gln Lys Ser Leu Ser Leu Ser Pro Gly Lys
            450                 455                 460
```

<210> 68
<211> 464
<212> PRT
<213> Artificial Sequence

<220>
<223> Synthetic Construct

<400> 68

```
Met Arg Trp Ser Trp Ile Phe Leu Phe Leu Leu Ser Ile Thr Ala Ser
1               5                   10                  15

Val His Cys Gln Val Gln Leu Gln Gln Ser Gly Pro Glu Leu Val Lys
            20                  25                  30

Pro Gly Ala Ser Val Arg Met Ser Cys Lys Ala Ser Gly Tyr Thr Phe
            35                  40                  45

Thr Ser Tyr His Ile His Trp Val Lys Gln Arg Pro Gly Gln Gly Leu
            50                  55                  60

Glu Trp Ile Gly Trp Ile Tyr Pro Gly Asp Asp Ser Thr Lys Tyr Asn
```

|  | 65 | | | | 70 | | | | 75 | | | | 80 |

Glu Lys Phe Lys Gly Lys Thr Thr Leu Thr Ala Asp Lys Ser Ser Ser
85 90 95

Thr Ala Tyr Met Leu Leu Ser Ser Leu Thr Ser Glu Asp Ser Ala Ile
100 105 110

Tyr Phe Cys Thr Arg Gly Asp Arg Phe Ala Tyr Trp Gly Gln Gly Thr
115 120 125

Leu Val Thr Val Ser Ala Ala Ser Thr Lys Gly Pro Ser Val Phe Pro
130 135 140

Leu Ala Pro Ser Ser Lys Ser Thr Ser Gly Gly Thr Ala Ala Leu Gly
145 150 155 160

Cys Leu Val Lys Asp Tyr Phe Pro Glu Pro Val Thr Val Ser Trp Asn
165 170 175

Ser Gly Ala Leu Thr Ser Gly Val His Thr Phe Pro Ala Val Leu Gln
180 185 190

Ser Ser Gly Leu Tyr Ser Leu Ser Ser Val Val Thr Val Pro Ser Ser
195 200 205

Ser Leu Gly Thr Gln Thr Tyr Ile Cys Asn Val Asn His Lys Pro Ser
210 215 220

Asn Thr Lys Val Asp Lys Lys Val Glu Pro Lys Ser Cys Asp Lys Thr
225 230 235 240

His Thr Cys Pro Pro Cys Pro Ala Pro Glu Leu Leu Gly Gly Pro Ser
245 250 255

Val Phe Leu Phe Pro Pro Lys Pro Lys Asp Thr Leu Met Ile Ser Arg
260 265 270

Thr Pro Glu Val Thr Cys Val Val Val Asp Val Ser His Glu Asp Pro
275 280 285

Glu Val Lys Phe Asn Trp Tyr Val Asp Gly Val Glu Val His Asn Ala
290 295 300

Lys Thr Lys Pro Arg Glu Glu Gln Tyr Asn Ser Thr Tyr Arg Val Val
305 310 315 320

Ser Val Leu Thr Val Leu His Gln Asp Trp Leu Asn Gly Lys Glu Tyr
325 330 335

```
Lys Cys Lys Val Ser Asn Lys Ala Leu Pro Ala Pro Ile Glu Lys Thr
            340                 345             350

Ile Ser Lys Ala Lys Gly Gln Pro Arg Glu Pro Gln Val Tyr Thr Leu
            355                 360             365

Pro Pro Ser Arg Asp Glu Leu Thr Lys Asn Gln Val Ser Leu Thr Cys
            370                 375             380

Leu Val Lys Gly Phe Tyr Pro Ser Asp Ile Ala Val Glu Trp Glu Ser
385                 390                 395             400

Asn Gly Gln Pro Glu Asn Asn Tyr Lys Thr Thr Pro Pro Val Leu Asp
                405                 410             415

Ser Asp Gly Ser Phe Phe Leu Tyr Ser Lys Leu Thr Val Asp Lys Ser
            420                 425             430

Arg Trp Gln Gln Gly Asn Val Phe Ser Cys Ser Val Met His Glu Ala
            435                 440             445

Leu His Asn His Tyr Thr Gln Lys Ser Leu Ser Leu Ser Pro Gly Lys
            450                 455             460
```

<210> 69
<211> 639
<212> DNA
<213> Artificial Sequence

<220>
<223> Chaîne légère sans leader 3C_23

<220>
<221> CDS
<222> (1)..(639)

<400> 69

EP 2 569 333 B1

gac atc cag atg aca cag tcc cca tct acc ctg tct gct tcc gtg gga        48
Asp Ile Gln Met Thr Gln Ser Pro Ser Thr Leu Ser Ala Ser Val Gly
1           5               10              15

gat cgg gtg act atc acc tgc aga gca agc tcc tcc gtg agg tac atc        96
Asp Arg Val Thr Ile Thr Cys Arg Ala Ser Ser Ser Val Arg Tyr Ile
        20              25              30

gct tgg tac cag cag aag cca gga aag gcc cca aag ctg ctg acc tac       144
Ala Trp Tyr Gln Gln Lys Pro Gly Lys Ala Pro Lys Leu Leu Thr Tyr
        35              40              45

cca acc tcc tcc ctg gaa tcc ggg gtg ccc agc aga ttc tca ggc agt       192
Pro Thr Ser Ser Leu Glu Ser Gly Val Pro Ser Arg Phe Ser Gly Ser
    50              55              60

ggc tcc ggc acc gaa ttc acc ctg acc atc agc tca ctg cag cct gac       240
Gly Ser Gly Thr Glu Phe Thr Leu Thr Ile Ser Ser Leu Gln Pro Asp
65              70              75              80

gac ttc gca acc tac tac tgt ctg cag tgg agt agc tac cct tgg aca       288
Asp Phe Ala Thr Tyr Tyr Cys Leu Gln Trp Ser Ser Tyr Pro Trp Thr
            85              90              95

ttc ggc ggc ggc acc aag gtg gag atc aag cgg acc gtc gcc gca cca       336
Phe Gly Gly Gly Thr Lys Val Glu Ile Lys Arg Thr Val Ala Ala Pro
            100             105             110

agt gtc ttc atc ttc ccg cca tct gat gag cag ttg aaa tct gga act       384
Ser Val Phe Ile Phe Pro Pro Ser Asp Glu Gln Leu Lys Ser Gly Thr
            115             120             125

gcc tct gtt gtg tgc ctg ctg aat aac ttc tat ccc aga gag gcc aaa       432
Ala Ser Val Val Cys Leu Leu Asn Asn Phe Tyr Pro Arg Glu Ala Lys
    130             135             140

gta cag tgg aag gtg gat aac gcc ctc caa tcg ggt aac tcc cag gag       480
Val Gln Trp Lys Val Asp Asn Ala Leu Gln Ser Gly Asn Ser Gln Glu
145             150             155             160

agt gtc aca gag cag gac agc aag gac agc acc tac agc ctc agc agc       528
Ser Val Thr Glu Gln Asp Ser Lys Asp Ser Thr Tyr Ser Leu Ser Ser
                165             170             175

acc ctg acg ctg agc aaa gca gac tac gag aaa cac aaa gtc tac gcc       576
Thr Leu Thr Leu Ser Lys Ala Asp Tyr Glu Lys His Lys Val Tyr Ala
            180             185             190

tgc gaa gtc acc cat cag ggc ctg agc tcg ccc gtc aca aag agc ttc       624
Cys Glu Val Thr His Gln Gly Leu Ser Ser Pro Val Thr Lys Ser Phe
            195             200             205

aac agg gga gag tgt                                                    639
Asn Arg Gly Glu Cys
    210

<210> 70
<211> 213
<212> PRT

121

EP 2 569 333 B1

<213> Artificial Sequence

<220>
<223> Synthetic Construct

<400> 70

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Thr Tyr
        35                  40                  45

Pro Thr Ser Ser Leu Glu Ser Gly Val Pro Ser Arg Phe Ser Gly Ser
        50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
            115                 120                 125

Ala Ser Val Val Cys Leu Leu Asn Asn Phe Tyr Pro Arg Glu Ala Lys
        130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
            165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
            195                 200                 205

Asn Arg Gly Glu Cys
            210
```

122

<210> 71
<211> 705
<212> DNA
<213> Artificial Sequence

<220>
<223> Chaîne légère avec leader 3C_23

<220>
<221> CDS
<222> (1)..(705)

<400> 71

```
atg gat atg aga gtg ccc gca cag ctg ctg ggt ctg ctg ctg ctg tgg      48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc      96
Leu Pro Gly Ala Lys Cys Asp Ile Gln Met Thr Gln Ser Pro Ser Thr
                20                  25                  30

ctg tct gct tcc gtg gga gat cgg gtg act atc acc tgc aga gca agc     144
Leu Ser Ala Ser Val Gly Asp Arg Val Thr Ile Thr Cys Arg Ala Ser
```

35                              40                              45

```
tcc tcc gtg agg tac atc gct tgg tac cag cag aag cca gga aag gcc        192
Ser Ser Val Arg Tyr Ile Ala Trp Tyr Gln Gln Lys Pro Gly Lys Ala
    50                  55                  60

cca aag ctg ctg acc tac cca acc tcc tcc ctg gaa tcc ggg gtg ccc        240
Pro Lys Leu Leu Thr Tyr Pro Thr Ser Ser Leu Glu Ser Gly Val Pro
65                  70                  75                  80

agc aga ttc tca ggc agt ggc tcc ggc acc gaa ttc acc ctg acc atc        288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg        336
Ser Ser Leu Gln Pro Asp Asp Phe Ala Thr Tyr Tyr Cys Leu Gln Trp
                100                 105                 110

agt agc tac cct tgg aca ttc ggc ggc ggc acc aag gtg gag atc aag        384
Ser Ser Tyr Pro Trp Thr Phe Gly Gly Gly Thr Lys Val Glu Ile Lys
                115                 120                 125

cgg acc gtc gcc gca cca agt gtc ttc atc ttc ccg cca tct gat gag        432
Arg Thr Val Ala Ala Pro Ser Val Phe Ile Phe Pro Pro Ser Asp Glu
    130                 135                 140

cag ttg aaa tct gga act gcc tct gtt gtg tgc ctg ctg aat aac ttc        480
Gln Leu Lys Ser Gly Thr Ala Ser Val Val Cys Leu Leu Asn Asn Phe
145                 150                 155                 160

tat ccc aga gag gcc aaa gta cag tgg aag gtg gat aac gcc ctc caa        528
Tyr Pro Arg Glu Ala Lys Val Gln Trp Lys Val Asp Asn Ala Leu Gln
                165                 170                 175

tcg ggt aac tcc cag gag agt gtc aca gag cag gac agc aag gac agc        576
Ser Gly Asn Ser Gln Glu Ser Val Thr Glu Gln Asp Ser Lys Asp Ser
                180                 185                 190

acc tac agc ctc agc agc acc ctg acg ctg agc aaa gca gac tac gag        624
Thr Tyr Ser Leu Ser Ser Thr Leu Thr Leu Ser Lys Ala Asp Tyr Glu
                195                 200                 205

aaa cac aaa gtc tac gcc tgc gaa gtc acc cat cag ggc ctg agc tcg        672
Lys His Lys Val Tyr Ala Cys Glu Val Thr His Gln Gly Leu Ser Ser
    210                 215                 220

ccc gtc aca aag agc ttc aac agg gga gag tgt        705
Pro Val Thr Lys Ser Phe Asn Arg Gly Glu Cys
225                 230                 235
```

<210> 72
<211> 235
<212> PRT
<213> Artificial Sequence

<220>
<223> Synthetic Construct

<400> 72

```
Met Asp Met Arg Val Pro Ala Gln Leu Leu Gly Leu Leu Leu Trp
1               5                   10                  15

Leu Pro Gly Ala Lys Cys Asp Ile Gln Met Thr Gln Ser Pro Ser Thr
            20                  25                  30

Leu Ser Ala Ser Val Gly Asp Arg Val Thr Ile Thr Cys Arg Ala Ser
            35                  40                  45

Ser Ser Val Arg Tyr Ile Ala Trp Tyr Gln Gln Lys Pro Gly Lys Ala
        50                  55                  60

Pro Lys Leu Leu Thr Tyr Pro Thr Ser Ser Leu Glu Ser Gly Val Pro
65                  70                  75                  80

Ser Arg Phe Ser Gly Ser Gly Ser Gly Thr Glu Phe Thr Leu Thr Ile
                85                  90                  95

Ser Ser Leu Gln Pro Asp Asp Phe Ala Thr Tyr Tyr Cys Leu Gln Trp
            100                 105                 110

Ser Ser Tyr Pro Trp Thr Phe Gly Gly Gly Thr Lys Val Glu Ile Lys
        115                 120                 125

Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
            165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235
```

<210> 73
<211> 1335
<212> DNA

<213> Artificial Sequence

<220>
<223> Chaîne lourde sans leader 3C_23

<220>
<221> CDS
<222> (1)..(1335)

<400> 73

```
cag gtg cgg ctg gtg cag agc ggg gcc gag gtg aag aag cct gga gcc    48
Gln Val Arg Leu Val Gln Ser Gly Ala Glu Val Lys Lys Pro Gly Ala
1               5                   10                  15

tca gtg aag gtg agt tgc aag gcc tcc ggt tac acc ttc acc agc tac    96
Ser Val Lys Val Ser Cys Lys Ala Ser Gly Tyr Thr Phe Thr Ser Tyr
            20                  25                  30

cac atc cac tgg gtc aga cag gct ccc ggc cag aga ctg gag tgg atg    144
His Ile His Trp Val Arg Gln Ala Pro Gly Gln Arg Leu Glu Trp Met
        35                  40                  45

ggc tgg atc tac cct gga gat gac tcc acc aag tac tcc cag aag ttc    192
Gly Trp Ile Tyr Pro Gly Asp Asp Ser Thr Lys Tyr Ser Gln Lys Phe
    50                  55                  60

cag ggt cgc gtg acc att acc agg gac acc agc gcc tcc act gcc tac    240
Gln Gly Arg Val Thr Ile Thr Arg Asp Thr Ser Ala Ser Thr Ala Tyr
65                  70                  75                  80

atg gag ctg tct tcc ctg aga tct gag gat acc gca gtc tac tac tgt    288
Met Glu Leu Ser Ser Leu Arg Ser Glu Asp Thr Ala Val Tyr Tyr Cys
            85                  90                  95

aca cgg ggg gac cgc ttt gct tac tgg ggg cag ggc act ctg gtg acc    336
Thr Arg Gly Asp Arg Phe Ala Tyr Trp Gly Gln Gly Thr Leu Val Thr
            100                 105                 110

gtc tcg agc gcc agc acc aag ggc cca tcg gtc ttc ccc ctg gca ccc    384
Val Ser Ser Ala Ser Thr Lys Gly Pro Ser Val Phe Pro Leu Ala Pro
        115                 120                 125

tcc tcc aag agc acc tct ggg ggc aca gcg gcc ctg ggc tgc ctg gtc    432
Ser Ser Lys Ser Thr Ser Gly Gly Thr Ala Ala Leu Gly Cys Leu Val
        130                 135                 140

aag gac tac ttc ccc gaa ccg gtg acg gtg tcg tgg aac tca ggc gcc    480
Lys Asp Tyr Phe Pro Glu Pro Val Thr Val Ser Trp Asn Ser Gly Ala
145                 150                 155                 160

ctg acc agc ggc gtg cac acc ttc ccg gct gtc cta cag tcc tca gga    528
Leu Thr Ser Gly Val His Thr Phe Pro Ala Val Leu Gln Ser Ser Gly
                165                 170                 175

ctc tac tcc ctc agc agc gtg gtg acc gtg ccc tcc agc agc ttg ggc    576
Leu Tyr Ser Leu Ser Ser Val Val Thr Val Pro Ser Ser Ser Leu Gly
            180                 185                 190

acc cag acc tac atc tgc aac gtg aat cac aag ccc agc aac acc aag    624
Thr Gln Thr Tyr Ile Cys Asn Val Asn His Lys Pro Ser Asn Thr Lys
        195                 200                 205

gtg gac aag aaa gtt gag ccc aaa tct tgt gac aaa act cac aca tgc    672
Val Asp Lys Lys Val Glu Pro Lys Ser Cys Asp Lys Thr His Thr Cys
        210                 215                 220

cca ccg tgc cca gca cct gaa ctc ctg ggg gga ccg tca gtc ttc ctc    720
Pro Pro Cys Pro Ala Pro Glu Leu Leu Gly Gly Pro Ser Val Phe Leu
225                 230                 235                 240
```

```
ttc ccc cca aaa ccc aag gac acc ctc atg atc tcc cgg acc cct gag        768
Phe Pro Pro Lys Pro Lys Asp Thr Leu Met Ile Ser Arg Thr Pro Glu
            245         250                 255

gtc aca tgc gtg gtg gtg gac gtg agc cac gaa gac cct gag gtc aag        816
Val Thr Cys Val Val Val Asp Val Ser His Glu Asp Pro Glu Val Lys
            260         265                 270

ttc aac tgg tac gtg gac ggc gtg gag gtg cat aat gcc aag aca aag        864
Phe Asn Trp Tyr Val Asp Gly Val Glu Val His Asn Ala Lys Thr Lys
            275         280                 285

ccg cgg gag gag cag tac aac agc acg tac cgt gtg gtc agc gtc ctc        912
Pro Arg Glu Glu Gln Tyr Asn Ser Thr Tyr Arg Val Val Ser Val Leu
            290         295                 300

acc gtc ctg cac cag gac tgg ctg aat ggc aag gag tac aag tgc aag        960
Thr Val Leu His Gln Asp Trp Leu Asn Gly Lys Glu Tyr Lys Cys Lys
305             310                 315                 320

gtc tcc aac aaa gcc ctc cca gcc ccc atc gag aaa acc atc tcc aaa       1008
Val Ser Asn Lys Ala Leu Pro Ala Pro Ile Glu Lys Thr Ile Ser Lys
            325         330                 335

gcc aaa ggg cag ccc cga gaa cca cag gtg tac acc ctg ccc cca tcc       1056
Ala Lys Gly Gln Pro Arg Glu Pro Gln Val Tyr Thr Leu Pro Pro Ser
            340         345                 350

cgg gat gag ctg acc aag aac cag gtc agc ctg acc tgc ctg gtc aaa       1104
Arg Asp Glu Leu Thr Lys Asn Gln Val Ser Leu Thr Cys Leu Val Lys
            355         360                 365

ggc ttc tat ccc agc gac atc gcc gtg gag tgg gag agc aat ggg cag       1152
Gly Phe Tyr Pro Ser Asp Ile Ala Val Glu Trp Glu Ser Asn Gly Gln
            370         375                 380

ccg gag aac aac tac aag acc acg cct ccc gtg ctg gac tcc gac ggc       1200
Pro Glu Asn Asn Tyr Lys Thr Thr Pro Pro Val Leu Asp Ser Asp Gly
385                 390                 395                 400

tcc ttc ttc ctc tac agc aag ctc acc gtg gac aag agc agg tgg cag       1248
Ser Phe Phe Leu Tyr Ser Lys Leu Thr Val Asp Lys Ser Arg Trp Gln
            405                 410                 415

cag ggg aac gtc ttc tca tgc tcc gtg atg cat gag gct ctg cac aac       1296
Gln Gly Asn Val Phe Ser Cys Ser Val Met His Glu Ala Leu His Asn
            420                 425                 430

cac tac acg cag aag agc ctc tcc ctg tct ccg ggt aaa                   1335
His Tyr Thr Gln Lys Ser Leu Ser Leu Ser Pro Gly Lys
            435                 440                 445
```

<210> 74
<211> 445
<212> PRT
<213> Artificial Sequence

<220>
<223> Synthetic Construct

<400> 74

```
Gln Val Arg Leu Val Gln Ser Gly Ala Glu Val Lys Lys Pro Gly Ala
1               5                   10                  15
```

Gln Val Arg Leu Val Gln Ser Gly Ala Glu Val Lys Lys Pro Gly Ala

```
Ser Val Lys Val Ser Cys Lys Ala Ser Gly Tyr Thr Phe Thr Ser Tyr
        20              25              30

His Ile His Trp Val Arg Gln Ala Pro Gly Gln Arg Leu Glu Trp Met
        35              40              45

Gly Trp Ile Tyr Pro Gly Asp Asp Ser Thr Lys Tyr Ser Gln Lys Phe
        50              55              60

Gln Gly Arg Val Thr Ile Thr Arg Asp Thr Ser Ala Ser Thr Ala Tyr
65              70              75              80

Met Glu Leu Ser Ser Leu Arg Ser Glu Asp Thr Ala Val Tyr Tyr Cys
                85              90              95

Thr Arg Gly Asp Arg Phe Ala Tyr Trp Gly Gln Gly Thr Leu Val Thr
            100             105             110

Val Ser Ser Ala Ser Thr Lys Gly Pro Ser Val Phe Pro Leu Ala Pro
        115             120             125

Ser Ser Lys Ser Thr Ser Gly Gly Thr Ala Ala Leu Gly Cys Leu Val
    130             135             140

Lys Asp Tyr Phe Pro Glu Pro Val Thr Val Ser Trp Asn Ser Gly Ala
145             150             155             160

Leu Thr Ser Gly Val His Thr Phe Pro Ala Val Leu Gln Ser Ser Gly
                165             170             175

Leu Tyr Ser Leu Ser Ser Val Val Thr Val Pro Ser Ser Ser Leu Gly
            180             185             190

Thr Gln Thr Tyr Ile Cys Asn Val Asn His Lys Pro Ser Asn Thr Lys
        195             200             205

Val Asp Lys Lys Val Glu Pro Lys Ser Cys Asp Lys Thr His Thr Cys
    210             215             220

Pro Pro Cys Pro Ala Pro Glu Leu Leu Gly Gly Pro Ser Val Phe Leu
225             230             235             240

Phe Pro Pro Lys Pro Lys Asp Thr Leu Met Ile Ser Arg Thr Pro Glu
            245             250             255

Val Thr Cys Val Val Val Asp Val Ser His Glu Asp Pro Glu Val Lys
        260             265             270
```

```
Phe Asn Trp Tyr Val Asp Gly Val Glu Val His Asn Ala Lys Thr Lys
        275             280             285

Pro Arg Glu Glu Gln Tyr Asn Ser Thr Tyr Arg Val Val Ser Val Leu
    290             295             300

Thr Val Leu His Gln Asp Trp Leu Asn Gly Lys Glu Tyr Lys Cys Lys
305             310             315             320

Val Ser Asn Lys Ala Leu Pro Ala Pro Ile Glu Lys Thr Ile Ser Lys
            325             330             335

Ala Lys Gly Gln Pro Arg Glu Pro Gln Val Tyr Thr Leu Pro Pro Ser
        340             345             350

Arg Asp Glu Leu Thr Lys Asn Gln Val Ser Leu Thr Cys Leu Val Lys
    355             360             365

Gly Phe Tyr Pro Ser Asp Ile Ala Val Glu Trp Glu Ser Asn Gly Gln
    370             375             380

Pro Glu Asn Asn Tyr Lys Thr Thr Pro Pro Val Leu Asp Ser Asp Gly
385             390             395             400

Ser Phe Phe Leu Tyr Ser Lys Leu Thr Val Asp Lys Ser Arg Trp Gln
            405             410             415

Gln Gly Asn Val Phe Ser Cys Ser Val Met His Glu Ala Leu His Asn
            420             425             430

His Tyr Thr Gln Lys Ser Leu Ser Leu Ser Pro Gly Lys
        435             440             445
```

<210> 75
<211> 1392
<212> DNA
<213> Artificial Sequence

<220>
<223> Chaîne lourde avec leader 3C_23

<220>
<221> CDS
<222> (1)..(1392)

<400> 75

```
atg gat tgg aca tgg cga atc ctg ttc ctg gtg gct gcc gca acc ggc        48
Met Asp Trp Thr Trp Arg Ile Leu Phe Leu Val Ala Ala Ala Thr Gly
1               5                   10                  15

gcc cac agc cag gtg cgg ctg gtg cag agc ggg gcc gag gtg aag aag        96
Ala His Ser Gln Val Arg Leu Val Gln Ser Gly Ala Glu Val Lys Lys
```

```
              20                    25                    30
cct gga gcc tca gtg aag gtg agt tgc aag gcc tcc ggt tac acc ttc       144
Pro Gly Ala Ser Val Lys Val Ser Cys Lys Ala Ser Gly Tyr Thr Phe
        35                    40                    45

acc agc tac cac atc cac tgg gtc aga cag gct ccc ggc cag aga ctg       192
Thr Ser Tyr His Ile His Trp Val Arg Gln Ala Pro Gly Gln Arg Leu
        50                    55                    60

gag tgg atg ggc tgg atc tac cct gga gat gac tcc acc aag tac tcc       240
Glu Trp Met Gly Trp Ile Tyr Pro Gly Asp Asp Ser Thr Lys Tyr Ser
65                    70                    75                    80

cag aag ttc cag ggt cgc gtg acc att acc agg gac acc agc gcc tcc       288
Gln Lys Phe Gln Gly Arg Val Thr Ile Thr Arg Asp Thr Ser Ala Ser
                85                    90                    95

act gcc tac atg gag ctg tct tcc ctg aga tct gag gat acc gca gtc       336
Thr Ala Tyr Met Glu Leu Ser Ser Leu Arg Ser Glu Asp Thr Ala Val
                100                   105                   110

tac tac tgt aca cgg ggg gac cgc ttt gct tac tgg ggg cag ggc act       384
Tyr Tyr Cys Thr Arg Gly Asp Arg Phe Ala Tyr Trp Gly Gln Gly Thr
                115                   120                   125

ctg gtg acc gtc tcg agc gcc agc acc aag ggc cca tcg gtc ttc ccc       432
Leu Val Thr Val Ser Ser Ala Ser Thr Lys Gly Pro Ser Val Phe Pro
        130                   135                   140

ctg gca ccc tcc tcc aag agc acc tct ggg ggc aca gcg gcc ctg ggc       480
Leu Ala Pro Ser Ser Lys Ser Thr Ser Gly Gly Thr Ala Ala Leu Gly
145                   150                   155                   160

tgc ctg gtc aag gac tac ttc ccc gaa ccg gtg acg gtg tcg tgg aac       528
Cys Leu Val Lys Asp Tyr Phe Pro Glu Pro Val Thr Val Ser Trp Asn
                165                   170                   175

tca ggc gcc ctg acc agc ggc gtg cac acc ttc ccg gct gtc cta cag       576
Ser Gly Ala Leu Thr Ser Gly Val His Thr Phe Pro Ala Val Leu Gln
                180                   185                   190

tcc tca gga ctc tac tcc ctc agc agc gtg gtg acc gtg ccc tcc agc       624
Ser Ser Gly Leu Tyr Ser Leu Ser Ser Val Val Thr Val Pro Ser Ser
                195                   200                   205

agc ttg ggc acc cag acc tac atc tgc aac gtg aat cac aag ccc agc       672
Ser Leu Gly Thr Gln Thr Tyr Ile Cys Asn Val Asn His Lys Pro Ser
        210                   215                   220

aac acc aag gtg gac aag aaa gtt gag ccc aaa tct tgt gac aaa act       720
Asn Thr Lys Val Asp Lys Lys Val Glu Pro Lys Ser Cys Asp Lys Thr
225                   230                   235                   240

cac aca tgc cca ccg tgc cca gca cct gaa ctc ctg ggg gga ccg tca       768
His Thr Cys Pro Pro Cys Pro Ala Pro Glu Leu Leu Gly Gly Pro Ser
                245                   250                   255

gtc ttc ctc ttc ccc cca aaa ccc aag gac acc ctc atg atc tcc cgg       816
Val Phe Leu Phe Pro Pro Lys Pro Lys Asp Thr Leu Met Ile Ser Arg
                260                   265                   270

acc cct gag gtc aca tgc gtg gtg gtg gac gtg agc cac gaa gac cct       864
Thr Pro Glu Val Thr Cys Val Val Val Asp Val Ser His Glu Asp Pro
                275                   280                   285
```

```
gag gtc aag ttc aac tgg tac gtg gac ggc gtg gag gtg cat aat gcc          912
Glu Val Lys Phe Asn Trp Tyr Val Asp Gly Val Glu Val His Asn Ala
    290             295             300

aag aca aag ccg cgg gag gag cag tac aac agc acg tac cgt gtg gtc          960
Lys Thr Lys Pro Arg Glu Glu Gln Tyr Asn Ser Thr Tyr Arg Val Val
305             310             315             320

agc gtc ctc acc gtc ctg cac cag gac tgg ctg aat ggc aag gag tac        1008
Ser Val Leu Thr Val Leu His Gln Asp Trp Leu Asn Gly Lys Glu Tyr
            325             330             335

aag tgc aag gtc tcc aac aaa gcc ctc cca gcc ccc atc gag aaa acc        1056
Lys Cys Lys Val Ser Asn Lys Ala Leu Pro Ala Pro Ile Glu Lys Thr
            340             345             350

atc tcc aaa gcc aaa ggg cag ccc cga gaa cca cag gtg tac acc ctg        1104
Ile Ser Lys Ala Lys Gly Gln Pro Arg Glu Pro Gln Val Tyr Thr Leu
            355             360             365

ccc cca tcc cgg gat gag ctg acc aag aac cag gtc agc ctg acc tgc        1152
Pro Pro Ser Arg Asp Glu Leu Thr Lys Asn Gln Val Ser Leu Thr Cys
    370             375             380

ctg gtc aaa ggc ttc tat ccc agc gac atc gcc gtg gag tgg gag agc        1200
Leu Val Lys Gly Phe Tyr Pro Ser Asp Ile Ala Val Glu Trp Glu Ser
385             390             395             400

aat ggg cag ccg gag aac aac tac aag acc acg cct ccc gtg ctg gac        1248
Asn Gly Gln Pro Glu Asn Asn Tyr Lys Thr Thr Pro Pro Val Leu Asp
            405             410             415

tcc gac ggc tcc ttc ttc ctc tac agc aag ctc acc gtg gac aag agc        1296
Ser Asp Gly Ser Phe Phe Leu Tyr Ser Lys Leu Thr Val Asp Lys Ser
            420             425             430

agg tgg cag cag ggg aac gtc ttc tca tgc tcc gtg atg cat gag gct        1344
Arg Trp Gln Gln Gly Asn Val Phe Ser Cys Ser Val Met His Glu Ala
            435             440             445

ctg cac aac cac tac acg cag aag agc ctc tcc ctg tct ccg ggt aaa        1392
Leu His Asn His Tyr Thr Gln Lys Ser Leu Ser Leu Ser Pro Gly Lys
    450             455             460
```

<210> 76
<211> 464
<212> PRT
<213> Artificial Sequence

<220>
<223> Synthetic Construct

<400> 76

```
Met Asp Trp Thr Trp Arg Ile Leu Phe Leu Val Ala Ala Ala Thr Gly
1               5               10                  15

Ala His Ser Gln Val Arg Leu Val Gln Ser Gly Ala Glu Val Lys Lys
                20              25                  30

Pro Gly Ala Ser Val Lys Val Ser Cys Lys Ala Ser Gly Tyr Thr Phe
```

```
                    35                        40                        45

         Thr Ser Tyr His Ile His Trp Val Arg Gln Ala Pro Gly Gln Arg Leu
             50                      55                  60

         Glu Trp Met Gly Trp Ile Tyr Pro Gly Asp Asp Ser Thr Lys Tyr Ser
         65                      70              75                      80

         Gln Lys Phe Gln Gly Arg Val Thr Ile Thr Arg Asp Thr Ser Ala Ser
                         85                      90                  95

         Thr Ala Tyr Met Glu Leu Ser Ser Leu Arg Ser Glu Asp Thr Ala Val
                     100                     105                 110

         Tyr Tyr Cys Thr Arg Gly Asp Arg Phe Ala Tyr Trp Gly Gln Gly Thr
                     115                     120                 125

         Leu Val Thr Val Ser Ser Ala Ser Thr Lys Gly Pro Ser Val Phe Pro
             130                     135                 140

         Leu Ala Pro Ser Ser Lys Ser Thr Ser Gly Gly Thr Ala Ala Leu Gly
         145                     150                 155                 160

         Cys Leu Val Lys Asp Tyr Phe Pro Glu Pro Val Thr Val Ser Trp Asn
                         165                     170                 175

         Ser Gly Ala Leu Thr Ser Gly Val His Thr Phe Pro Ala Val Leu Gln
                     180                     185                 190

         Ser Ser Gly Leu Tyr Ser Leu Ser Ser Val Val Thr Val Pro Ser Ser
                     195                     200                 205

         Ser Leu Gly Thr Gln Thr Tyr Ile Cys Asn Val Asn His Lys Pro Ser
             210                     215                 220

         Asn Thr Lys Val Asp Lys Lys Val Glu Pro Lys Ser Cys Asp Lys Thr
         225                     230                 235                 240

         His Thr Cys Pro Pro Cys Pro Ala Pro Glu Leu Leu Gly Gly Pro Ser
                         245                     250                 255

         Val Phe Leu Phe Pro Pro Lys Pro Lys Asp Thr Leu Met Ile Ser Arg
                     260                     265                 270

         Thr Pro Glu Val Thr Cys Val Val Val Asp Val Ser His Glu Asp Pro
                     275                     280                 285

         Glu Val Lys Phe Asn Trp Tyr Val Asp Gly Val Glu Val His Asn Ala
             290                     295                 300
```

```
Lys Thr Lys Pro Arg Glu Glu Gln Tyr Asn Ser Thr Tyr Arg Val Val
305             310         315             320

Ser Val Leu Thr Val Leu His Gln Asp Trp Leu Asn Gly Lys Glu Tyr
            325             330             335

Lys Cys Lys Val Ser Asn Lys Ala Leu Pro Ala Pro Ile Glu Lys Thr
            340             345             350

Ile Ser Lys Ala Lys Gly Gln Pro Arg Glu Pro Gln Val Tyr Thr Leu
            355             360             365

Pro Pro Ser Arg Asp Glu Leu Thr Lys Asn Gln Val Ser Leu Thr Cys
    370             375             380

Leu Val Lys Gly Phe Tyr Pro Ser Asp Ile Ala Val Glu Trp Glu Ser
385             390             395             400

Asn Gly Gln Pro Glu Asn Asn Tyr Lys Thr Thr Pro Pro Val Leu Asp
            405             410             415

Ser Asp Gly Ser Phe Phe Leu Tyr Ser Lys Leu Thr Val Asp Lys Ser
            420             425             430

Arg Trp Gln Gln Gly Asn Val Phe Ser Cys Ser Val Met His Glu Ala
            435             440             445

Leu His Asn His Tyr Thr Gln Lys Ser Leu Ser Leu Ser Pro Gly Lys
    450             455             460
```

<210> 77
<211> 639
<212> DNA
<213> Artificial Sequence

<220>
<223> Chaîne légère sans leader 6B_78

<220>
<221> CDS
<222> (1)..(639)

<400> 77

EP 2 569 333 B1

```
gac atc cag atg aca cag tcc cca tct acc ctg tct gct tcc gtg gga          48
Asp Ile Gln Met Thr Gln Ser Pro Ser Thr Leu Ser Ala Ser Val Gly
1                   5                  10                  15

gat cgg gtg act atc acc tgc aga gca agc tcc tcc gtg agg tac atc          96
Asp Arg Val Thr Ile Thr Cys Arg Ala Ser Ser Ser Val Arg Tyr Ile
                20                  25                  30

gct tgg tac cag cag aag cca gga aag gcc cca aag ctg ctg atc tac         144

Ala Trp Tyr Gln Gln Lys Pro Gly Lys Ala Pro Lys Leu Leu Ile Tyr
        35                  40                  45

tca acc tcc tcc ctg aaa tcc ggg gtg ccc agc aga ttc tca ggc agt        192
Ser Thr Ser Ser Leu Lys Ser Gly Val Pro Ser Arg Phe Ser Gly Ser
        50                  55                  60

ggc tcc ggc acc gaa ttc acc ctg acc atc agc tca ctg cag cct gac        240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca        288
Asp Phe Ala Thr Tyr Tyr Cys Leu Gln Trp Ser Ser Tyr Pro Trp Thr
                85                  90                  95

ttc ggc ggc ggc acc aag gtg gag atc aag cgg acc gtc gcc gca cca        336
Phe Gly Gly Gly Thr Lys Val Glu Ile Lys Arg Thr Val Ala Ala Pro
                100                 105                 110

agt gtc ttc atc ttc ccg cca tct gat gag cag ttg aaa tct gga act        384
Ser Val Phe Ile Phe Pro Pro Ser Asp Glu Gln Leu Lys Ser Gly Thr
                115                 120                 125

gcc tct gtt gtg tgc ctg ctg aat aac ttc tat ccc aga gag gcc aaa        432
Ala Ser Val Val Cys Leu Leu Asn Asn Phe Tyr Pro Arg Glu Ala Lys
        130                 135                 140

gta cag tgg aag gtg gat aac gcc ctc caa tcg ggt aac tcc cag gag        480
Val Gln Trp Lys Val Asp Asn Ala Leu Gln Ser Gly Asn Ser Gln Glu
145                 150                 155                 160

agt gtc aca gag cag gac agc aag gac agc acc tac agc ctc agc agc        528
Ser Val Thr Glu Gln Asp Ser Lys Asp Ser Thr Tyr Ser Leu Ser Ser
                165                 170                 175

acc ctg acg ctg agc aaa gca gac tac gag aaa cac aaa gtc tac gcc        576
Thr Leu Thr Leu Ser Lys Ala Asp Tyr Glu Lys His Lys Val Tyr Ala
                180                 185                 190

tgc gaa gtc acc cat cag ggc ctg agc tcg ccc gtc aca aag agc ttc        624
Cys Glu Val Thr His Gln Gly Leu Ser Ser Pro Val Thr Lys Ser Phe
                195                 200                 205

aac agg gga gag tgt                                                     639
Asn Arg Gly Glu Cys
                210
```

<210> 78
<211> 213
<212> PRT
<213> Artificial Sequence

138

<220>
<223> Synthetic Construct

<400> 78

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Ile Tyr
            35                  40                  45

Ser Thr Ser Ser Leu Lys Ser Gly Val Pro Ser Arg Phe Ser Gly Ser
            50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
            115                 120                 125

Ala Ser Val Val Cys Leu Leu Asn Asn Phe Tyr Pro Arg Glu Ala Lys
            130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
            195                 200                 205

Asn Arg Gly Glu Cys
            210
```

<210> 79
<211> 705

<212> DNA
<213> Artificial Sequence

<220>
<223> Chaîne légère avec leader 6B_78

<220>
<221> CDS
<222> (1)..(705)

<400> 79

```
atg gat atg aga gtg ccc gca cag ctg ctg ggt ctg ctg ctg ctg tgg        48
Met Asp Met Arg Val Pro Ala Gln Leu Leu Gly Leu Leu Leu Leu Trp
```

```
                  1                      5                          10                         15

                  ctg ccc gga gcc aaa tgt gac atc cag atg aca cag tcc cca tct acc          96
                  Leu Pro Gly Ala Lys Cys Asp Ile Gln Met Thr Gln Ser Pro Ser Thr
                          20              25                      30

                  ctg tct gct tcc gtg gga gat cgg gtg act atc acc tgc aga gca agc          144
                  Leu Ser Ala Ser Val Gly Asp Arg Val Thr Ile Thr Cys Arg Ala Ser
                          35              40                      45

                  tcc tcc gtg agg tac atc gct tgg tac cag cag aag cca gga aag gcc          192
                  Ser Ser Val Arg Tyr Ile Ala Trp Tyr Gln Gln Lys Pro Gly Lys Ala
                          50              55                      60

                  cca aag ctg ctg atc tac tca acc tcc tcc ctg aaa tcc ggg gtg ccc          240
                  Pro Lys Leu Leu Ile Tyr Ser Thr Ser Ser Leu Lys Ser Gly Val Pro
                  65                      70                      75                      80

                  agc aga ttc tca ggc agt ggc tcc ggc acc gaa ttc acc ctg acc atc          288
                  Ser Arg Phe Ser Gly Ser Gly Ser Gly Thr Glu Phe Thr Leu Thr Ile
                                          85                      90                      95

                  agc tca ctg cag cct gac gac ttc gca acc tac tac tgt ctg cag tgg          336
                  Ser Ser Leu Gln Pro Asp Asp Phe Ala Thr Tyr Tyr Cys Leu Gln Trp
                              100                     105                     110

                  agt agc tac cct tgg aca ttc ggc ggc ggc acc aag gtg gag atc aag          384
                  Ser Ser Tyr Pro Trp Thr Phe Gly Gly Gly Thr Lys Val Glu Ile Lys
                              115                     120                     125

                  cgg acc gtc gcc gca cca agt gtc ttc atc ttc ccg cca tct gat gag          432
                  Arg Thr Val Ala Ala Pro Ser Val Phe Ile Phe Pro Pro Ser Asp Glu
                      130                     135                     140

                  cag ttg aaa tct gga act gcc tct gtt gtg tgc ctg ctg aat aac ttc          480
                  Gln Leu Lys Ser Gly Thr Ala Ser Val Val Cys Leu Leu Asn Asn Phe
                  145                     150                     155                     160

                  tat ccc aga gag gcc aaa gta cag tgg aag gtg gat aac gcc ctc caa          528
                  Tyr Pro Arg Glu Ala Lys Val Gln Trp Lys Val Asp Asn Ala Leu Gln
                              165                     170                     175

                  tcg ggt aac tcc cag gag agt gtc aca gag cag gac agc aag gac agc          576
                  Ser Gly Asn Ser Gln Glu Ser Val Thr Glu Gln Asp Ser Lys Asp Ser
                              180                     185                     190

                  acc tac agc ctc agc agc acc ctg acg ctg agc aaa gca gac tac gag          624
                  Thr Tyr Ser Leu Ser Ser Thr Leu Thr Leu Ser Lys Ala Asp Tyr Glu
                              195                     200                     205

                  aaa cac aaa gtc tac gcc tgc gaa gtc acc cat cag ggc ctg agc tcg          672
                  Lys His Lys Val Tyr Ala Cys Glu Val Thr His Gln Gly Leu Ser Ser
                              210                     215                     220

                  ccc gtc aca aag agc ttc aac agg gga gag tgt                              705
                  Pro Val Thr Lys Ser Phe Asn Arg Gly Glu Cys
                  225                     230                     235
```

<210> 80
<211> 235
<212> PRT

141

<213> Artificial Sequence

<220>
<223> Synthetic Construct

<400> 80

```
Met Asp Met Arg Val Pro Ala Gln Leu Leu Gly Leu Leu Leu Trp
1               5               10              15

Leu Pro Gly Ala Lys Cys Asp Ile Gln Met Thr Gln Ser Pro Ser Thr
            20              25              30

Leu Ser Ala Ser Val Gly Asp Arg Val Thr Ile Thr Cys Arg Ala Ser
        35              40              45

Ser Ser Val Arg Tyr Ile Ala Trp Tyr Gln Gln Lys Pro Gly Lys Ala
    50              55              60

Pro Lys Leu Leu Ile Tyr Ser Thr Ser Ser Leu Lys Ser Gly Val Pro
65              70              75              80

Ser Arg Phe Ser Gly Ser Gly Ser Gly Thr Glu Phe Thr Leu Thr Ile
            85              90              95

Ser Ser Leu Gln Pro Asp Asp Phe Ala Thr Tyr Tyr Cys Leu Gln Trp
        100             105             110

Ser Ser Tyr Pro Trp Thr Phe Gly Gly Gly Thr Lys Val Glu Ile Lys
        115             120             125

Arg Thr Val Ala Ala Pro Ser Val Phe Ile Phe Pro Pro Ser Asp Glu
    130             135             140

Gln Leu Lys Ser Gly Thr Ala Ser Val Val Cys Leu Leu Asn Asn Phe
145             150             155             160

Tyr Pro Arg Glu Ala Lys Val Gln Trp Lys Val Asp Asn Ala Leu Gln
            165             170             175

Ser Gly Asn Ser Gln Glu Ser Val Thr Glu Gln Asp Ser Lys Asp Ser
        180             185             190

Thr Tyr Ser Leu Ser Ser Thr Leu Thr Leu Ser Lys Ala Asp Tyr Glu
        195             200             205

Lys His Lys Val Tyr Ala Cys Glu Val Thr His Gln Gly Leu Ser Ser
    210             215             220

Pro Val Thr Lys Ser Phe Asn Arg Gly Glu Cys
225             230             235
```

<210> 81
<211> 639

<212> DNA
<213> Artificial Sequence

<220>
<223> Chaîne légère sans leader 3C_23K

<220>
<221> CDS
<222> (1)..(639)

<400> 81

```
gac atc cag atg aca cag tcc cca tct acc ctg tct gct tcc gtg gga    48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc    96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg acc tac   144
Ala Trp Tyr Gln Gln Lys Pro Gly Lys Ala Pro Lys Leu Leu Thr Tyr
        35                  40                  45

cca acc tcc tcc ctg aaa tcc ggg gtg ccc agc aga ttc tca ggc agt   192
Pro Thr Ser Ser Leu Lys Ser Gly Val Pro Ser Arg Phe Ser Gly Ser
        50                  55                  60

ggc tcc ggc acc gaa ttc acc ctg acc atc agc tca ctg cag cct gac   240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca   288
Asp Phe Ala Thr Tyr Tyr Cys Leu Gln Trp Ser Ser Tyr Pro Trp Thr
                85                  90                  95

ttc ggc ggc ggc acc aag gtg gag atc aag cgg acc gtc gcc gca cca   336
Phe Gly Gly Gly Thr Lys Val Glu Ile Lys Arg Thr Val Ala Ala Pro
            100                 105                 110

agt gtc ttc atc ttc ccg cca tct gat gag cag ttg aaa tct gga act   384
Ser Val Phe Ile Phe Pro Pro Ser Asp Glu Gln Leu Lys Ser Gly Thr
            115                 120                 125

gcc tct gtt gtg tgc ctg ctg aat aac ttc tat ccc aga gag gcc aaa   432
Ala Ser Val Val Cys Leu Leu Asn Asn Phe Tyr Pro Arg Glu Ala Lys
        130                 135                 140

gta cag tgg aag gtg gat aac gcc ctc caa tcg ggt aac tcc cag gag   480
Val Gln Trp Lys Val Asp Asn Ala Leu Gln Ser Gly Asn Ser Gln Glu
145                 150                 155                 160

agt gtc aca gag cag gac agc aag gac agc acc tac agc ctc agc agc   528
Ser Val Thr Glu Gln Asp Ser Lys Asp Ser Thr Tyr Ser Leu Ser Ser
                165                 170                 175

acc ctg acg ctg agc aaa gca gac tac gag aaa cac aaa gtc tac gcc   576
Thr Leu Thr Leu Ser Lys Ala Asp Tyr Glu Lys His Lys Val Tyr Ala
            180                 185                 190

tgc gaa gtc acc cat cag ggc ctg agc tcg ccc gtc aca aag agc ttc   624
Cys Glu Val Thr His Gln Gly Leu Ser Ser Pro Val Thr Lys Ser Phe
            195                 200                 205

aac agg gga gag tgt                                                639
Asn Arg Gly Glu Cys
            210
```

<210> 82

<211> 213

<212> PRT

<213> Artificial Sequence

EP 2 569 333 B1

<220>
<223> Synthetic Construct

<400> 82

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Thr Tyr
        35                  40                  45

Pro Thr Ser Ser Leu Lys Ser Gly Val Pro Ser Arg Phe Ser Gly Ser
        50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
            115                 120                 125

Ala Ser Val Val Cys Leu Leu Asn Asn Phe Tyr Pro Arg Glu Ala Lys
        130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
            195                 200                 205

Asn Arg Gly Glu Cys
        210
```

<210> 83
<211> 705

146

<212> DNA
<213> Artificial Sequence

<220>
<223> Chaîne légère avec leader 3C_23K

<220>
<221> CDS
<222> (1)..(705)

<400> 83

```
atg gat atg aga gtg ccc gca cag ctg ctg ggt ctg ctg ctg ctg tgg        48
Met Asp Met Arg Val Pro Ala Gln Leu Leu Gly Leu Leu Leu Leu Trp
1               5                   10                  15

ctg ccc gga gcc aaa tgt gac atc cag atg aca cag tcc cca tct acc        96
Leu Pro Gly Ala Lys Cys Asp Ile Gln Met Thr Gln Ser Pro Ser Thr
                20                  25                  30

ctg tct gct tcc gtg gga gat cgg gtg act atc acc tgc aga gca agc       144
Leu Ser Ala Ser Val Gly Asp Arg Val Thr Ile Thr Cys Arg Ala Ser
            35                  40                  45

tcc tcc gtg agg tac atc gct tgg tac cag cag aag cca gga aag gcc       192
Ser Ser Val Arg Tyr Ile Ala Trp Tyr Gln Gln Lys Pro Gly Lys Ala
        50                  55                  60

cca aag ctg ctg acc tac cca acc tcc tcc ctg aaa tcc ggg gtg ccc       240
Pro Lys Leu Leu Thr Tyr Pro Thr Ser Ser Leu Lys Ser Gly Val Pro
65                  70                  75                  80

agc aga ttc tca ggc agt ggc tcc ggc acc gaa ttc acc ctg acc atc       288
Ser Arg Phe Ser Gly Ser Gly Ser Gly Thr Glu Phe Thr Leu Thr Ile
                85                  90                  95

agc tca ctg cag cct gac gac ttc gca acc tac tac tgt ctg cag tgg       336
Ser Ser Leu Gln Pro Asp Asp Phe Ala Thr Tyr Tyr Cys Leu Gln Trp
                100                 105                 110

agt agc tac cct tgg aca ttc ggc ggc ggc acc aag gtg gag atc aag       384
Ser Ser Tyr Pro Trp Thr Phe Gly Gly Gly Thr Lys Val Glu Ile Lys
            115                 120                 125

cgg acc gtc gcc gca cca agt gtc ttc atc ttc ccg cca tct gat gag       432
Arg Thr Val Ala Ala Pro Ser Val Phe Ile Phe Pro Pro Ser Asp Glu
            130                 135                 140

cag ttg aaa tct gga act gcc tct gtt gtg tgc ctg ctg aat aac ttc       480
Gln Leu Lys Ser Gly Thr Ala Ser Val Val Cys Leu Leu Asn Asn Phe
145                 150                 155                 160

tat ccc aga gag gcc aaa gta cag tgg aag gtg gat aac gcc ctc caa       528
Tyr Pro Arg Glu Ala Lys Val Gln Trp Lys Val Asp Asn Ala Leu Gln
                165                 170                 175

tcg ggt aac tcc cag gag agt gtc aca gag cag gac agc aag gac agc       576
```

```
Ser Gly Asn Ser Gln Glu Ser Val Thr Glu Gln Asp Ser Lys Asp Ser
            180                 185             190

acc tac agc ctc agc agc acc ctg acg ctg agc aaa gca gac tac gag      624
Thr Tyr Ser Leu Ser Ser Thr Leu Thr Leu Ser Lys Ala Asp Tyr Glu
            195                 200             205

aaa cac aaa gtc tac gcc tgc gaa gtc acc cat cag ggc ctg agc tcg      672
Lys His Lys Val Tyr Ala Cys Glu Val Thr His Gln Gly Leu Ser Ser
            210                 215             220

ccc gtc aca aag agc ttc aac agg gga gag tgt                          705
Pro Val Thr Lys Ser Phe Asn Arg Gly Glu Cys
225                 230                 235
```

<210> 84
<211> 235
<212> PRT
<213> Artificial Sequence

<220>
<223> Synthetic Construct

<400> 84

```
Met Asp Met Arg Val Pro Ala Gln Leu Leu Gly Leu Leu Leu Trp
1               5                   10                  15

Leu Pro Gly Ala Lys Cys Asp Ile Gln Met Thr Gln Ser Pro Ser Thr
            20                  25                  30

Leu Ser Ala Ser Val Gly Asp Arg Val Thr Ile Thr Cys Arg Ala Ser
        35                  40                  45

Ser Ser Val Arg Tyr Ile Ala Trp Tyr Gln Gln Lys Pro Gly Lys Ala
    50                  55                  60

Pro Lys Leu Leu Thr Tyr Pro Thr Ser Ser Leu Lys Ser Gly Val Pro
65                  70                  75                  80

Ser Arg Phe Ser Gly Ser Gly Ser Gly Thr Glu Phe Thr Leu Thr Ile
            85                  90                  95

Ser Ser Leu Gln Pro Asp Asp Phe Ala Thr Tyr Tyr Cys Leu Gln Trp
        100                 105                 110

Ser Ser Tyr Pro Trp Thr Phe Gly Gly Gly Thr Lys Val Glu Ile Lys
        115                 120                 125

Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
            165                 170                 175

Ser Gly Asn Ser Gln Glu Ser Val Thr Glu Gln Asp Ser Lys Asp Ser
        180                 185                 190

Thr Tyr Ser Leu Ser Ser Thr Leu Thr Leu Ser Lys Ala Asp Tyr Glu
    195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235
```

<210> 85
<211> 1335
<212> DNA

149

<213> Artificial Sequence

<220>
<223> Chaîne lourde sans leader 3C_23K

<220>
<221> CDS
<222> (1)..(1335)

<400> 85

```
cag gtg cgg ctg gtg cag agc ggg gcc gag gtg aag aag cct gga gcc      48
Gln Val Arg Leu Val Gln Ser Gly Ala Glu Val Lys Lys Pro Gly Ala
1               5                   10                  15

tca gtg aag gtg agt tgc aag gcc tcc ggt tac acc ttc acc agc tac      96
Ser Val Lys Val Ser Cys Lys Ala Ser Gly Tyr Thr Phe Thr Ser Tyr
                20                  25                  30

cac atc cac tgg gtc aga cag gct ccc ggc cag aga ctg gag tgg atg      144
His Ile His Trp Val Arg Gln Ala Pro Gly Gln Arg Leu Glu Trp Met
            35                  40                  45

ggc tgg atc tac cct gga gat gac tcc acc aag tac tcc cag aag ttc      192
Gly Trp Ile Tyr Pro Gly Asp Asp Ser Thr Lys Tyr Ser Gln Lys Phe
        50                  55                  60

cag ggt cgc gtg acc att acc agg gac acc agc gcc tcc act gcc tac      240
Gln Gly Arg Val Thr Ile Thr Arg Asp Thr Ser Ala Ser Thr Ala Tyr
65                  70                  75                  80

atg gag ctg tct tcc ctg aga tct gag gat acc gca gtc tac tac tgt      288
Met Glu Leu Ser Ser Leu Arg Ser Glu Asp Thr Ala Val Tyr Tyr Cys
                85                  90                  95

aca cgg ggg gac cgc ttt gct tac tgg ggg cag ggc act ctg gtg acc      336
Thr Arg Gly Asp Arg Phe Ala Tyr Trp Gly Gln Gly Thr Leu Val Thr
                100                 105                 110

gtc tcg agc gcc agc acc aag ggc cca tcg gtc ttc ccc ctg gca ccc      384
Val Ser Ser Ala Ser Thr Lys Gly Pro Ser Val Phe Pro Leu Ala Pro
```

```
                 115                    120                    125

      tcc tcc aag agc acc tct ggg ggc aca gcg gcc ctg ggc tgc ctg gtc      432
      Ser Ser Lys Ser Thr Ser Gly Gly Thr Ala Ala Leu Gly Cys Leu Val
      130                    135                    140

      aag gac tac ttc ccc gaa ccg gtg acg gtg tcg tgg aac tca ggc gcc      480
      Lys Asp Tyr Phe Pro Glu Pro Val Thr Val Ser Trp Asn Ser Gly Ala
      145                    150                    155                    160

      ctg acc agc ggc gtg cac acc ttc ccg gct gtc cta cag tcc tca gga      528
      Leu Thr Ser Gly Val His Thr Phe Pro Ala Val Leu Gln Ser Ser Gly
                       165                    170                    175

      ctc tac tcc ctc agc agc gtg gtg acc gtg ccc tcc agc agc ttg ggc      576
      Leu Tyr Ser Leu Ser Ser Val Val Thr Val Pro Ser Ser Ser Leu Gly
                       180                    185                    190

      acc cag acc tac atc tgc aac gtg aat cac aag ccc agc aac acc aag      624
      Thr Gln Thr Tyr Ile Cys Asn Val Asn His Lys Pro Ser Asn Thr Lys
                       195                    200                    205

      gtg gac aag aaa gtt gag ccc aaa tct tgt gac aaa act cac aca tgc      672
      Val Asp Lys Lys Val Glu Pro Lys Ser Cys Asp Lys Thr His Thr Cys
                       210                    215                    220

      cca ccg tgc cca gca cct gaa ctc ctg ggg gga ccg tca gtc ttc ctc      720
      Pro Pro Cys Pro Ala Pro Glu Leu Leu Gly Gly Pro Ser Val Phe Leu
      225                    230                    235                    240

      ttc ccc cca aaa ccc aag gac acc ctc atg atc tcc cgg acc cct gag      768
      Phe Pro Pro Lys Pro Lys Asp Thr Leu Met Ile Ser Arg Thr Pro Glu
                       245                    250                    255

      gtc aca tgc gtg gtg gtg gac gtg agc cac gaa gac cct gag gtc aag      816
      Val Thr Cys Val Val Val Asp Val Ser His Glu Asp Pro Glu Val Lys
                       260                    265                    270

      ttc aac tgg tac gtg gac ggc gtg gag gtg cat aat gcc aag aca aag      864
      Phe Asn Trp Tyr Val Asp Gly Val Glu Val His Asn Ala Lys Thr Lys
                       275                    280                    285

      ccg cgg gag gag cag tac aac agc acg tac cgt gtg gtc agc gtc ctc      912
      Pro Arg Glu Glu Gln Tyr Asn Ser Thr Tyr Arg Val Val Ser Val Leu
                       290                    295                    300

      acc gtc ctg cac cag gac tgg ctg aat ggc aag gag tac aag tgc aag      960
      Thr Val Leu His Gln Asp Trp Leu Asn Gly Lys Glu Tyr Lys Cys Lys
      305                    310                    315                    320

      gtc tcc aac aaa gcc ctc cca gcc ccc atc gag aaa acc atc tcc aaa     1008
      Val Ser Asn Lys Ala Leu Pro Ala Pro Ile Glu Lys Thr Ile Ser Lys
                       325                    330                    335

      gcc aaa ggg cag ccc cga gaa cca cag gtg tac acc ctg ccc cca tcc     1056
      Ala Lys Gly Gln Pro Arg Glu Pro Gln Val Tyr Thr Leu Pro Pro Ser
                       340                    345                    350

      cgg gat gag ctg acc aag aac cag gtc agc ctg acc tgc ctg gtc aaa     1104
      Arg Asp Glu Leu Thr Lys Asn Gln Val Ser Leu Thr Cys Leu Val Lys
                       355                    360                    365

      ggc ttc tat ccc agc gac atc gcc gtg gag tgg gag agc aat ggg cag     1152
      Gly Phe Tyr Pro Ser Asp Ile Ala Val Glu Trp Glu Ser Asn Gly Gln
                       370                    375                    380
```

```
ccg gag aac aac tac aag acc acg cct ccc gtg ctg gac tcc gac ggc        1200
Pro Glu Asn Asn Tyr Lys Thr Thr Pro Pro Val Leu Asp Ser Asp Gly
385                 390             395                 400

tcc ttc ttc ctc tac agc aag ctc acc gtg gac aag agc agg tgg cag        1248
Ser Phe Phe Leu Tyr Ser Lys Leu Thr Val Asp Lys Ser Arg Trp Gln
                405             410                 415

cag ggg aac gtc ttc tca tgc tcc gtg atg cat gag gct ctg cac aac        1296
Gln Gly Asn Val Phe Ser Cys Ser Val Met His Glu Ala Leu His Asn
                420             425                 430

cac tac acg cag aag agc ctc tcc ctg tct ccg ggt aaa                    1335
His Tyr Thr Gln Lys Ser Leu Ser Leu Ser Pro Gly Lys
            435             440                 445
```

<210> 86
<211> 445
<212> PRT
<213> Artificial Sequence

<220>
<223> Synthetic Construct

<400> 86

Gln Val Arg Leu Val Gln Ser Gly Ala Glu Val Lys Lys Pro Gly Ala
1               5               10              15

Ser Val Lys Val Ser Cys Lys Ala Ser Gly Tyr Thr Phe Thr Ser Tyr
              20              25              30

His Ile His Trp Val Arg Gln Ala Pro Gly Gln Arg Leu Glu Trp Met
          35              40              45

Gly Trp Ile Tyr Pro Gly Asp Asp Ser Thr Lys Tyr Ser Gln Lys Phe
      50              55              60

Gln Gly Arg Val Thr Ile Thr Arg Asp Thr Ser Ala Ser Thr Ala Tyr
65              70              75              80

Met Glu Leu Ser Ser Leu Arg Ser Glu Asp Thr Ala Val Tyr Tyr Cys
              85              90              95

Thr Arg Gly Asp Arg Phe Ala Tyr Trp Gly Gln Gly Thr Leu Val Thr
          100             105             110

Val Ser Ser Ala Ser Thr Lys Gly Pro Ser Val Phe Pro Leu Ala Pro
          115             120             125

Ser Ser Lys Ser Thr Ser Gly Gly Thr Ala Ala Leu Gly Cys Leu Val
          130             135             140

Lys Asp Tyr Phe Pro Glu Pro Val Thr Val Ser Trp Asn Ser Gly Ala

145              150             155             160

Leu Thr Ser Gly Val His Thr Phe Pro Ala Val Leu Gln Ser Ser Gly
            165            170               175

Leu Tyr Ser Leu Ser Ser Val Val Thr Val Pro Ser Ser Ser Leu Gly
            180            185             190

Thr Gln Thr Tyr Ile Cys Asn Val Asn His Lys Pro Ser Asn Thr Lys
            195            200            205

Val Asp Lys Lys Val Glu Pro Lys Ser Cys Asp Lys Thr His Thr Cys
            210            215            220

Pro Pro Cys Pro Ala Pro Glu Leu Leu Gly Gly Pro Ser Val Phe Leu
225               230            235              240

Phe Pro Pro Lys Pro Lys Asp Thr Leu Met Ile Ser Arg Thr Pro Glu
            245            250            255

Val Thr Cys Val Val Val Asp Val Ser His Glu Asp Pro Glu Val Lys
            260            265            270

Phe Asn Trp Tyr Val Asp Gly Val Glu Val His Asn Ala Lys Thr Lys
            275            280            285

Pro Arg Glu Glu Gln Tyr Asn Ser Thr Tyr Arg Val Val Ser Val Leu
            290            295            300

Thr Val Leu His Gln Asp Trp Leu Asn Gly Lys Glu Tyr Lys Cys Lys
305               310            315              320

Val Ser Asn Lys Ala Leu Pro Ala Pro Ile Glu Lys Thr Ile Ser Lys
            325            330            335

Ala Lys Gly Gln Pro Arg Glu Pro Gln Val Tyr Thr Leu Pro Pro Ser
            340            345            350

Arg Asp Glu Leu Thr Lys Asn Gln Val Ser Leu Thr Cys Leu Val Lys
            355            360            365

Gly Phe Tyr Pro Ser Asp Ile Ala Val Glu Trp Glu Ser Asn Gly Gln
            370            375            380

Pro Glu Asn Asn Tyr Lys Thr Thr Pro Pro Val Leu Asp Ser Asp Gly
385               390            395              400

Ser Phe Phe Leu Tyr Ser Lys Leu Thr Val Asp Lys Ser Arg Trp Gln
            405            410            415

```
Gln Gly Asn Val Phe Ser Cys Ser Val Met His Glu Ala Leu His Asn
            420                     425                 430

His Tyr Thr Gln Lys Ser Leu Ser Leu Ser Pro Gly Lys
        435                 440                 445
```

<210> 87
<211> 1392
<212> DNA
<213> Artificial Sequence

<220>
<223> Chaîne lourde avec leader 3C_23K

<220>
<221> CDS
<222> (1)..(1392)

<400> 87

```
atg gat tgg aca tgg cga atc ctg ttc ctg gtg gct gcc gca acc ggc      48
Met Asp Trp Thr Trp Arg Ile Leu Phe Leu Val Ala Ala Ala Thr Gly
1               5                   10                  15

gcc cac agc cag gtg cgg ctg gtg cag agc ggg gcc gag gtg aag aag      96
Ala His Ser Gln Val Arg Leu Val Gln Ser Gly Ala Glu Val Lys Lys
                20                  25                  30

cct gga gcc tca gtg aag gtg agt tgc aag gcc tcc ggt tac acc ttc     144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga ctg     192
Thr Ser Tyr His Ile His Trp Val Arg Gln Ala Pro Gly Gln Arg Leu
        50                  55                  60

gag tgg atg ggc tgg atc tac cct gga gat gac tcc acc aag tac tcc     240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc     288
Gln Lys Phe Gln Gly Arg Val Thr Ile Thr Arg Asp Thr Ser Ala Ser
                85                  90                  95

act gcc tac atg gag ctg tct tcc ctg aga tct gag gat acc gca gtc     336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act     384
Tyr Tyr Cys Thr Arg Gly Asp Arg Phe Ala Tyr Trp Gly Gln Gly Thr
        115                 120                 125

ctg gtg acc gtc tcg agc gcc agc acc aag ggc cca tcg gtc ttc ccc     432
Leu Val Thr Val Ser Ser Ala Ser Thr Lys Gly Pro Ser Val Phe Pro
        130                 135                 140

ctg gca ccc tcc tcc aag agc acc tct ggg ggc aca gcg gcc ctg ggc     480
Leu Ala Pro Ser Ser Lys Ser Thr Ser Gly Gly Thr Ala Ala Leu Gly
145                 150                 155                 160

tgc ctg gtc aag gac tac ttc ccc gaa ccg gtg acg gtg tcg tgg aac     528
```

```
Cys Leu Val Lys Asp Tyr Phe Pro Glu Pro Val Thr Val Ser Trp Asn
            165                 170                 175

tca ggc gcc ctg acc agc ggc gtg cac acc ttc ccg gct gtc cta cag    576
Ser Gly Ala Leu Thr Ser Gly Val His Thr Phe Pro Ala Val Leu Gln
            180                 185                 190

tcc tca gga ctc tac tcc ctc agc agc gtg gtg acc gtg ccc tcc agc    624
Ser Ser Gly Leu Tyr Ser Leu Ser Ser Val Val Thr Val Pro Ser Ser
            195                 200                 205

agc ttg ggc acc cag acc tac atc tgc aac gtg aat cac aag ccc agc    672
Ser Leu Gly Thr Gln Thr Tyr Ile Cys Asn Val Asn His Lys Pro Ser
        210                 215                 220

aac acc aag gtg gac aag aaa gtt gag ccc aaa tct tgt gac aaa act    720
Asn Thr Lys Val Asp Lys Lys Val Glu Pro Lys Ser Cys Asp Lys Thr
225                 230                 235                 240

cac aca tgc cca ccg tgc cca gca cct gaa ctc ctg ggg gga ccg tca    768
His Thr Cys Pro Pro Cys Pro Ala Pro Glu Leu Leu Gly Gly Pro Ser
                245                 250                 255

gtc ttc ctc ttc ccc cca aaa ccc aag gac acc ctc atg atc tcc cgg    816
Val Phe Leu Phe Pro Pro Lys Pro Lys Asp Thr Leu Met Ile Ser Arg
            260                 265                 270

acc cct gag gtc aca tgc gtg gtg gtg gac gtg agc cac gaa gac cct    864
Thr Pro Glu Val Thr Cys Val Val Val Asp Val Ser His Glu Asp Pro
            275                 280                 285

gag gtc aag ttc aac tgg tac gtg gac ggc gtg gag gtg cat aat gcc    912
Glu Val Lys Phe Asn Trp Tyr Val Asp Gly Val Glu Val His Asn Ala
            290                 295                 300

aag aca aag ccg cgg gag gag cag tac aac agc acg tac cgt gtg gtc    960
Lys Thr Lys Pro Arg Glu Glu Gln Tyr Asn Ser Thr Tyr Arg Val Val
305                 310                 315                 320

agc gtc ctc acc gtc ctg cac cag gac tgg ctg aat ggc aag gag tac    1008
Ser Val Leu Thr Val Leu His Gln Asp Trp Leu Asn Gly Lys Glu Tyr
                325                 330                 335

aag tgc aag gtc tcc aac aaa gcc ctc cca gcc ccc atc gag aaa acc    1056
Lys Cys Lys Val Ser Asn Lys Ala Leu Pro Ala Pro Ile Glu Lys Thr
            340                 345                 350

atc tcc aaa gcc aaa ggg cag ccc cga gaa cca cag gtg tac acc ctg    1104
Ile Ser Lys Ala Lys Gly Gln Pro Arg Glu Pro Gln Val Tyr Thr Leu
            355                 360                 365

ccc cca tcc cgg gat gag ctg acc aag aac cag gtc agc ctg acc tgc    1152
Pro Pro Ser Arg Asp Glu Leu Thr Lys Asn Gln Val Ser Leu Thr Cys
        370                 375                 380

ctg gtc aaa ggc ttc tat ccc agc gac atc gcc gtg gag tgg gag agc    1200
Leu Val Lys Gly Phe Tyr Pro Ser Asp Ile Ala Val Glu Trp Glu Ser
385                 390                 395                 400

aat ggg cag ccg gag aac aac tac aag acc acg cct ccc gtg ctg gac    1248
Asn Gly Gln Pro Glu Asn Asn Tyr Lys Thr Thr Pro Pro Val Leu Asp
                405                 410                 415

tcc gac ggc tcc ttc ttc ctc tac agc aag ctc acc gtg gac aag agc    1296
Ser Asp Gly Ser Phe Phe Leu Tyr Ser Lys Leu Thr Val Asp Lys Ser
```

```
              420                    425                    430

    agg tgg cag cag ggg aac gtc ttc tca tgc tcc gtg atg cat gag gct      1344
    Arg Trp Gln Gln Gly Asn Val Phe Ser Cys Ser Val Met His Glu Ala
            435                    440                    445

    ctg cac aac cac tac acg cag aag agc ctc tcc ctg tct ccg ggt aaa      1392
    Leu His Asn His Tyr Thr Gln Lys Ser Leu Ser Leu Ser Pro Gly Lys
            450                    455                    460
```

<210> 88
<211> 464
<212> PRT
<213> Artificial Sequence

<220>
<223> Synthetic Construct

<400> 88

```
Met Asp Trp Thr Trp Arg Ile Leu Phe Leu Val Ala Ala Ala Thr Gly
1               5                   10                  15

Ala His Ser Gln Val Arg Leu Val Gln Ser Gly Ala Glu Val Lys Lys
                20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu
        50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
            115                 120                 125

Leu Val Thr Val Ser Ser Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
                165                 170                 175
```

```
Ser Gly Ala Leu Thr Ser Gly Val His Thr Phe Pro Ala Val Leu Gln
        180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Lys Val Glu Pro Lys Ser Cys Asp Lys Thr
225                 230                 235                 240

His Thr Cys Pro Pro Cys Pro Ala Pro Glu Leu Leu Gly Gly Pro Ser
            245                 250                 255

Val Phe Leu Phe Pro Pro Lys Pro Lys Asp Thr Leu Met Ile Ser Arg
        260                 265                 270

Thr Pro Glu Val Thr Cys Val Val Val Asp Val Ser His Glu Asp Pro
        275                 280                 285

Glu Val Lys Phe Asn Trp Tyr Val Asp Gly Val Glu Val His Asn Ala
        290                 295                 300

Lys Thr Lys Pro Arg Glu Glu Gln Tyr Asn Ser Thr Tyr Arg Val Val
305                 310                 315                 320

Ser Val Leu Thr Val Leu His Gln Asp Trp Leu Asn Gly Lys Glu Tyr
            325                 330                 335

Lys Cys Lys Val Ser Asn Lys Ala Leu Pro Ala Pro Ile Glu Lys Thr
        340                 345                 350

Ile Ser Lys Ala Lys Gly Gln Pro Arg Glu Pro Gln Val Tyr Thr Leu
        355                 360                 365

Pro Pro Ser Arg Asp Glu Leu Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400

Asn Gly Gln Pro Glu Asn Asn Tyr Lys Thr Thr Pro Pro Val Leu Asp
                405                 410                 415

Ser Asp Gly Ser Phe Phe Leu Tyr Ser Lys Leu Thr Val Asp Lys Ser
            420                 425                 430

Arg Trp Gln Gln Gly Asn Val Phe Ser Cys Ser Val Met His Glu Ala
```

```
                  435                    440                       445


        Leu His Asn His Tyr Thr Gln Lys Ser Leu Ser Leu Ser Pro Gly Lys
             450                455                460
```

<210> 89
<211> 1335
<212> DNA
<213> Artificial Sequence

<220>
<223> Chaîne lourde sans leader 4C_35

<220>
<221> CDS
<222> (1)..(1335)

<400> 89

```
cag gtg cag ctg gtg cag agc ggg gcc gag gtg aag aag cct gga gcc        48
Gln Val Gln Leu Val Gln Ser Gly Ala Glu Val Lys Lys Pro Gly Ala
1               5               10              15

tca gtg aag gtg agt tgc aag gcc tcc ggt tac acc ttc acc agc tac        96
Ser Val Lys Val Ser Cys Lys Ala Ser Gly Tyr Thr Phe Thr Ser Tyr
            20              25              30

cac atc cac tgg gtc aga cag gct ccc ggc cag aga cca gag tgg atg       144
His Ile His Trp Val Arg Gln Ala Pro Gly Gln Arg Pro Glu Trp Met
        35              40              45

ggc tgg atc tac cct gga gat gac tcc acc aag tac tcc cag aag ttc       192
Gly Trp Ile Tyr Pro Gly Asp Asp Ser Thr Lys Tyr Ser Gln Lys Phe
    50              55              60

cag ggt cgc gtg acc att acc agg gac acc agc gcc tcc act gcc tac       240
Gln Gly Arg Val Thr Ile Thr Arg Asp Thr Ser Ala Ser Thr Ala Tyr
65              70              75              80

atg gag ctg tct tcc ctg aga tct gag gat acc gca gtc tac tac tgt       288
Met Glu Leu Ser Ser Leu Arg Ser Glu Asp Thr Ala Val Tyr Tyr Cys
                85              90              95

aca cgg ggg gac cgc ttt gct tac tgg ggg cag ggc act ctg gtg acc       336
Thr Arg Gly Asp Arg Phe Ala Tyr Trp Gly Gln Gly Thr Leu Val Thr
            100             105             110

gtc agc agc gcc agc acc aag ggc cca tcg gtc ttc ccc ctg gca ccc       384
Val Ser Ser Ala Ser Thr Lys Gly Pro Ser Val Phe Pro Leu Ala Pro
        115             120             125

tcc tcc aag agc acc tct ggg ggc aca gcg gcc ctg ggc tgc ctg gtc       432
Ser Ser Lys Ser Thr Ser Gly Gly Thr Ala Ala Leu Gly Cys Leu Val
    130             135             140

aag gac tac ttc ccc gaa ccg gtg acg gtg tcg tgg aac tca ggc gcc       480
Lys Asp Tyr Phe Pro Glu Pro Val Thr Val Ser Trp Asn Ser Gly Ala
145             150             155             160

ctg acc agc ggc gtg cac acc ttc ccg gct gtc cta cag tcc tca gga       528
Leu Thr Ser Gly Val His Thr Phe Pro Ala Val Leu Gln Ser Ser Gly
                165             170             175
```

```
ctc tac tcc ctc agc agc gtg gtg acc gtg ccc tcc agc agc ttg ggc        576
Leu Tyr Ser Leu Ser Ser Val Val Thr Val Pro Ser Ser Ser Leu Gly
        180                 185                 190

acc cag acc tac atc tgc aac gtg aat cac aag ccc agc aac acc aag        624
Thr Gln Thr Tyr Ile Cys Asn Val Asn His Lys Pro Ser Asn Thr Lys
        195                 200                 205

gtg gac aag aaa gtt gag ccc aaa tct tgt gac aaa act cac aca tgc        672
Val Asp Lys Lys Val Glu Pro Lys Ser Cys Asp Lys Thr His Thr Cys
210                 215                 220

cca ccg tgc cca gca cct gaa ctc ctg ggg gga ccg tca gtc ttc ctc        720
Pro Pro Cys Pro Ala Pro Glu Leu Leu Gly Gly Pro Ser Val Phe Leu
225                 230                 235                 240

ttc ccc cca aaa ccc aag gac acc ctc atg atc tcc cgg acc cct gag        768
Phe Pro Pro Lys Pro Lys Asp Thr Leu Met Ile Ser Arg Thr Pro Glu
                245                 250                 255

gtc aca tgc gtg gtg gtg gac gtg agc cac gaa gac cct gag gtc aag        816
Val Thr Cys Val Val Val Asp Val Ser His Glu Asp Pro Glu Val Lys
            260                 265                 270

ttc aac tgg tac gtg gac ggc gtg gag gtg cat aat gcc aag aca aag        864
Phe Asn Trp Tyr Val Asp Gly Val Glu Val His Asn Ala Lys Thr Lys
            275                 280                 285

ccg cgg gag gag cag tac aac agc acg tac cgt gtg gtc agc gtc ctc        912
Pro Arg Glu Glu Gln Tyr Asn Ser Thr Tyr Arg Val Val Ser Val Leu
        290                 295                 300

acc gtc ctg cac cag gac tgg ctg aat ggc aag gag tac aag tgc aag        960
Thr Val Leu His Gln Asp Trp Leu Asn Gly Lys Glu Tyr Lys Cys Lys
305                 310                 315                 320

gtc tcc aac aaa gcc ctc cca gcc ccc atc gag aaa acc atc tcc aaa       1008
Val Ser Asn Lys Ala Leu Pro Ala Pro Ile Glu Lys Thr Ile Ser Lys
            325                 330                 335

gcc aaa ggg cag ccc cga gaa cca cag gtg tac acc ctg ccc cca tcc       1056
Ala Lys Gly Gln Pro Arg Glu Pro Gln Val Tyr Thr Leu Pro Pro Ser
            340                 345                 350

cgg gat gag ctg acc aag aac cag gtc agc ctg acc tgc ctg gtc aaa       1104
Arg Asp Glu Leu Thr Lys Asn Gln Val Ser Leu Thr Cys Leu Val Lys
        355                 360                 365

ggc ttc tat ccc agc gac atc gcc gtg gag tgg gag agc aat ggg cag       1152
Gly Phe Tyr Pro Ser Asp Ile Ala Val Glu Trp Glu Ser Asn Gly Gln
        370                 375                 380

ccg gag aac aac tac aag acc acg cct ccc gtg ctg gac tcc gac ggc       1200
Pro Glu Asn Asn Tyr Lys Thr Thr Pro Pro Val Leu Asp Ser Asp Gly
385                 390                 395                 400

tcc ttc ttc ctc tac agc aag ctc acc gtg gac aag agc agg tgg cag       1248
Ser Phe Phe Leu Tyr Ser Lys Leu Thr Val Asp Lys Ser Arg Trp Gln
            405                 410                 415

cag ggg aac gtc ttc tca tgc tcc gtg atg cat gag gct ctg cac aac       1296
Gln Gly Asn Val Phe Ser Cys Ser Val Met His Glu Ala Leu His Asn
            420                 425                 430

cac tac acg cag aag agc ctc tcc ctg tct ccg ggt aaa                   1335
His Tyr Thr Gln Lys Ser Leu Ser Leu Ser Pro Gly Lys
```

```
His Tyr Thr Gln Lys Ser Leu Ser Leu Ser Pro Gly Lys
        435                 440                 445
```

<210> 90
<211> 445
<212> PRT
<213> Artificial Sequence

<220>
<223> Synthetic Construct

<400> 90

```
His Tyr Thr Gln Lys Ser Leu Ser Leu Ser Pro Gly Lys
        435                 440                 445
```

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Ser Tyr
            20              25              30

His Ile His Trp Val Arg Gln Ala Pro Gly Gln Arg Pro Glu Trp Met
            35              40              45

Gly Trp Ile Tyr Pro Gly Asp Asp Ser Thr Lys Tyr Ser Gln Lys Phe
    50              55              60

Gln Gly Arg Val Thr Ile Thr Arg Asp Thr Ser Ala Ser Thr Ala Tyr
65              70              75              80

Met Glu Leu Ser Ser Leu Arg Ser Glu Asp Thr Ala Val Tyr Tyr Cys
            85              90              95

Thr Arg Gly Asp Arg Phe Ala Tyr Trp Gly Gln Gly Thr Leu Val Thr
            100             105             110

Val Ser Ser Ala Ser Thr Lys Gly Pro Ser Val Phe Pro Leu Ala Pro
            115             120             125

Ser Ser Lys Ser Thr Ser Gly Gly Thr Ala Ala Leu Gly Cys Leu Val
    130             135             140

Lys Asp Tyr Phe Pro Glu Pro Val Thr Val Ser Trp Asn Ser Gly Ala
145             150             155             160

Leu Thr Ser Gly Val His Thr Phe Pro Ala Val Leu Gln Ser Ser Gly
            165             170             175

Leu Tyr Ser Leu Ser Ser Val Val Thr Val Pro Ser Ser Ser Leu Gly
            180             185             190

Thr Gln Thr Tyr Ile Cys Asn Val Asn His Lys Pro Ser Asn Thr Lys
            195             200             205
```

```
Val Asp Lys Lys Val Glu Pro Lys Ser Cys Asp Lys Thr His Thr Cys
    210             215             220

Pro Pro Cys Pro Ala Pro Glu Leu Leu Gly Gly Pro Ser Val Phe Leu
225             230             235                 240

Phe Pro Pro Lys Pro Lys Asp Thr Leu Met Ile Ser Arg Thr Pro Glu
            245             250                 255

Val Thr Cys Val Val Val Asp Val Ser His Glu Asp Pro Glu Val Lys
        260             265             270

Phe Asn Trp Tyr Val Asp Gly Val Glu Val His Asn Ala Lys Thr Lys
        275             280             285

Pro Arg Glu Glu Gln Tyr Asn Ser Thr Tyr Arg Val Val Ser Val Leu
    290             295             300

Thr Val Leu His Gln Asp Trp Leu Asn Gly Lys Glu Tyr Lys Cys Lys
305             310             315             320

Val Ser Asn Lys Ala Leu Pro Ala Pro Ile Glu Lys Thr Ile Ser Lys
            325             330             335

Ala Lys Gly Gln Pro Arg Glu Pro Gln Val Tyr Thr Leu Pro Pro Ser
        340             345             350

Arg Asp Glu Leu Thr Lys Asn Gln Val Ser Leu Thr Cys Leu Val Lys
    355             360             365

Gly Phe Tyr Pro Ser Asp Ile Ala Val Glu Trp Glu Ser Asn Gly Gln
    370             375             380

Pro Glu Asn Asn Tyr Lys Thr Thr Pro Pro Val Leu Asp Ser Asp Gly
385             390             395             400

Ser Phe Phe Leu Tyr Ser Lys Leu Thr Val Asp Lys Ser Arg Trp Gln
            405             410             415

Gln Gly Asn Val Phe Ser Cys Ser Val Met His Glu Ala Leu His Asn
        420             425             430

His Tyr Thr Gln Lys Ser Leu Ser Leu Ser Pro Gly Lys
    435             440             445
```

<210> 91
<211> 1392

<212> DNA
<213> Artificial Sequence

<220>
<223> Chaîne lourde avec leader 4C_35

<220>
<221> CDS
<222> (1)..(1392)

<400> 91

```
atg gat tgg aca tgg cga atc ctg ttc ctg gtg gct gcc gca acc ggc     48
Met Asp Trp Thr Trp Arg Ile Leu Phe Leu Val Ala Ala Ala Thr Gly
1               5                   10                  15

gcc cac agc cag gtg cag ctg gtg cag agc ggg gcc gag gtg aag aag     96
Ala His Ser Gln Val Gln Leu Val Gln Ser Gly Ala Glu Val Lys Lys
            20                  25                  30

cct gga gcc tca gtg aag gtg agt tgc aag gcc tcc ggt tac acc ttc    144
Pro Gly Ala Ser Val Lys Val Ser Cys Lys Ala Ser Gly Tyr Thr Phe
            35                  40                  45

acc agc tac cac atc cac tgg gtc aga cag gct ccc ggc cag aga cca    192
Thr Ser Tyr His Ile His Trp Val Arg Gln Ala Pro Gly Gln Arg Pro
        50                  55                  60

gag tgg atg ggc tgg atc tac cct gga gat gac tcc acc aag tac tcc    240
Glu Trp Met Gly Trp Ile Tyr Pro Gly Asp Asp Ser Thr Lys Tyr Ser
65                  70                  75                  80

cag aag ttc cag ggt cgc gtg acc att acc agg gac acc agc gcc tcc    288
Gln Lys Phe Gln Gly Arg Val Thr Ile Thr Arg Asp Thr Ser Ala Ser
                85                  90                  95

act gcc tac atg gag ctg tct tcc ctg aga tct gag gat acc gca gtc    336
Thr Ala Tyr Met Glu Leu Ser Ser Leu Arg Ser Glu Asp Thr Ala Val
            100                 105                 110

tac tac tgt aca cgg ggg gac cgc ttt gct tac tgg ggg cag ggc act    384
Tyr Tyr Cys Thr Arg Gly Asp Arg Phe Ala Tyr Trp Gly Gln Gly Thr
            115                 120                 125

ctg gtg acc gtc agc agc gcc agc acc aag ggc cca tcg gtc ttc ccc    432
Leu Val Thr Val Ser Ser Ala Ser Thr Lys Gly Pro Ser Val Phe Pro
    130                 135                 140

ctg gca ccc tcc tcc aag agc acc tct ggg ggc aca gcg gcc ctg ggc    480
Leu Ala Pro Ser Ser Lys Ser Thr Ser Gly Gly Thr Ala Ala Leu Gly
145                 150                 155                 160

tgc ctg gtc aag gac tac ttc ccc gaa ccg gtg acg gtg tcg tgg aac    528
Cys Leu Val Lys Asp Tyr Phe Pro Glu Pro Val Thr Val Ser Trp Asn
                165                 170                 175

tca ggc gcc ctg acc agc ggc gtg cac acc ttc ccg gct gtc cta cag    576
Ser Gly Ala Leu Thr Ser Gly Val His Thr Phe Pro Ala Val Leu Gln
            180                 185                 190

tcc tca gga ctc tac tcc ctc agc agc gtg gtg acc gtg ccc tcc agc    624
Ser Ser Gly Leu Tyr Ser Leu Ser Ser Val Val Thr Val Pro Ser Ser
            195                 200                 205

agc ttg ggc acc cag acc tac atc tgc aac gtg aat cac aag ccc agc    672
Ser Leu Gly Thr Gln Thr Tyr Ile Cys Asn Val Asn His Lys Pro Ser
    210                 215                 220
```

```
aac acc aag gtg gac aag aaa gtt gag ccc aaa tct tgt gac aaa act        720
Asn Thr Lys Val Asp Lys Lys Val Glu Pro Lys Ser Cys Asp Lys Thr
225             230             235             240

cac aca tgc cca ccg tgc cca gca cct gaa ctc ctg ggg gga ccg tca        768
His Thr Cys Pro Pro Cys Pro Ala Pro Glu Leu Leu Gly Gly Pro Ser
            245             250             255

gtc ttc ctc ttc ccc cca aaa ccc aag gac acc ctc atg atc tcc cgg        816
Val Phe Leu Phe Pro Pro Lys Pro Lys Asp Thr Leu Met Ile Ser Arg
            260             265             270

acc cct gag gtc aca tgc gtg gtg gtg gac gtg agc cac gaa gac cct        864
Thr Pro Glu Val Thr Cys Val Val Val Asp Val Ser His Glu Asp Pro
            275             280             285

gag gtc aag ttc aac tgg tac gtg gac ggc gtg gag gtg cat aat gcc        912
Glu Val Lys Phe Asn Trp Tyr Val Asp Gly Val Glu Val His Asn Ala
    290             295             300

aag aca aag ccg cgg gag gag cag tac aac agc acg tac cgt gtg gtc        960
Lys Thr Lys Pro Arg Glu Glu Gln Tyr Asn Ser Thr Tyr Arg Val Val
305             310             315             320

agc gtc ctc acc gtc ctg cac cag gac tgg ctg aat ggc aag gag tac       1008
Ser Val Leu Thr Val Leu His Gln Asp Trp Leu Asn Gly Lys Glu Tyr
            325             330             335

aag tgc aag gtc tcc aac aaa gcc ctc cca gcc ccc atc gag aaa acc       1056
Lys Cys Lys Val Ser Asn Lys Ala Leu Pro Ala Pro Ile Glu Lys Thr
            340             345             350

atc tcc aaa gcc aaa ggg cag ccc cga gaa cca cag gtg tac acc ctg       1104
Ile Ser Lys Ala Lys Gly Gln Pro Arg Glu Pro Gln Val Tyr Thr Leu
            355             360             365

ccc cca tcc cgg gat gag ctg acc aag aac cag gtc agc ctg acc tgc       1152
Pro Pro Ser Arg Asp Glu Leu Thr Lys Asn Gln Val Ser Leu Thr Cys
            370             375             380

ctg gtc aaa ggc ttc tat ccc agc gac atc gcc gtg gag tgg gag agc       1200
Leu Val Lys Gly Phe Tyr Pro Ser Asp Ile Ala Val Glu Trp Glu Ser
385             390             395             400

aat ggg cag ccg gag aac aac tac aag acc acg cct ccc gtg ctg gac       1248
Asn Gly Gln Pro Glu Asn Asn Tyr Lys Thr Thr Pro Pro Val Leu Asp
            405             410             415

tcc gac ggc tcc ttc ttc ctc tac agc aag ctc acc gtg gac aag agc       1296
Ser Asp Gly Ser Phe Phe Leu Tyr Ser Lys Leu Thr Val Asp Lys Ser
            420             425             430

agg tgg cag cag ggg aac gtc ttc tca tgc tcc gtg atg cat gag gct       1344
Arg Trp Gln Gln Gly Asn Val Phe Ser Cys Ser Val Met His Glu Ala
            435             440             445

ctg cac aac cac tac acg cag aag agc ctc tcc ctg tct ccg ggt aaa       1392
Leu His Asn His Tyr Thr Gln Lys Ser Leu Ser Leu Ser Pro Gly Lys
            450             455             460
```

<210> 92

169

<211> 464
<212> PRT
<213> Artificial Sequence

<220>
<223> Synthetic Construct

<400> 92

<211> 464
<212> PRT
<213> Artificial Sequence

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Pro
    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
            165                 170                 175

Ser Gly Ala Leu Thr Ser Gly Val His Thr Phe Pro Ala Val Leu Gln
        180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Lys Val Glu Pro Lys Ser Cys Asp Lys Thr
225                 230                 235                 240
```

171

His Thr Cys Pro Pro Cys Pro Ala Pro Glu Leu Leu Gly Gly Pro Ser
              245             250                 255

Val Phe Leu Phe Pro Pro Lys Pro Lys Asp Thr Leu Met Ile Ser Arg
              260             265             270

Thr Pro Glu Val Thr Cys Val Val Val Asp Val Ser His Glu Asp Pro
              275             280             285

Glu Val Lys Phe Asn Trp Tyr Val Asp Gly Val Glu Val His Asn Ala
              290             295             300

Lys Thr Lys Pro Arg Glu Glu Gln Tyr Asn Ser Thr Tyr Arg Val Val
305                           310             315                 320

Ser Val Leu Thr Val Leu His Gln Asp Trp Leu Asn Gly Lys Glu Tyr
              325             330             335

Lys Cys Lys Val Ser Asn Lys Ala Leu Pro Ala Pro Ile Glu Lys Thr
              340             345             350

Ile Ser Lys Ala Lys Gly Gln Pro Arg Glu Pro Gln Val Tyr Thr Leu
              355             360             365

Pro Pro Ser Arg Asp Glu Leu Thr Lys Asn Gln Val Ser Leu Thr Cys
              370             375             380

Leu Val Lys Gly Phe Tyr Pro Ser Asp Ile Ala Val Glu Trp Glu Ser
385                           390             395                 400

Asn Gly Gln Pro Glu Asn Asn Tyr Lys Thr Thr Pro Pro Val Leu Asp
              405             410             415

Ser Asp Gly Ser Phe Phe Leu Tyr Ser Lys Leu Thr Val Asp Lys Ser
              420             425             430

Arg Trp Gln Gln Gly Asn Val Phe Ser Cys Ser Val Met His Glu Ala
              435             440             445

Leu His Asn His Tyr Thr Gln Lys Ser Leu Ser Leu Ser Pro Gly Lys
              450             455             460

<210> 93
<211> 639
<212> DNA
<213> Artificial Sequence

<220>

<223> Chaîne légère sans leader 5B_42

<220>
<221> CDS
<222> (1)..(639)

<400> 93

```
gac atc cag atg aca cag tcc cca tct acc ctg tct gct tcc gtg gga          48
Asp Ile Gln Met Thr Gln Ser Pro Ser Thr Leu Ser Ala Ser Val Gly
1               5                   10                  15

gat cgg gtg act atc acc tgc aga gca agc tcc tcc gtg agg tac atc          96
Asp Arg Val Thr Ile Thr Cys Arg Ala Ser Ser Ser Val Arg Tyr Ile
            20                  25                  30

gct tgg tac cag cag aag cca gga aag gcc cca aag ctg ctg atc tac         144
Ala Trp Tyr Gln Gln Lys Pro Gly Lys Ala Pro Lys Leu Leu Ile Tyr
            35                  40                  45

cca acc tcc tcc ctg gaa tcc ggg gtg ccc agc aga ttc tca ggc agt         192
Pro Thr Ser Ser Leu Glu Ser Gly Val Pro Ser Arg Phe Ser Gly Ser
        50                  55                  60

ggc tcc ggc acc gaa ttc acc ctg acc atc agc tca ctg cag cct gac         240
Gly Ser Gly Thr Glu Phe Thr Leu Thr Ile Ser Ser Leu Gln Pro Asp
65                  70                  75                  80

gac ttc gca acc tac tac tgt ctg cag tgg agt agc tac cct tgg aca         288
Asp Phe Ala Thr Tyr Tyr Cys Leu Gln Trp Ser Ser Tyr Pro Trp Thr
                85                  90                  95

ttc ggc ggc ggc acc aag gtg gag atc aag cgg acc gtc gcc gca cca         336
Phe Gly Gly Gly Thr Lys Val Glu Ile Lys Arg Thr Val Ala Ala Pro
            100                 105                 110

agt gtc ttc atc ttc ccg cca tct gat gag cag ttg aaa tct gga act         384
Ser Val Phe Ile Phe Pro Pro Ser Asp Glu Gln Leu Lys Ser Gly Thr
            115                 120                 125

gcc tct gtt gtg tgc ctg ctg aat aac ttc tat ccc aga gag gcc aaa         432
Ala Ser Val Val Cys Leu Leu Asn Asn Phe Tyr Pro Arg Glu Ala Lys
        130                 135                 140

gta cag tgg aag gtg gat aac gcc ctc caa tcg ggt aac tcc cag gag         480
Val Gln Trp Lys Val Asp Asn Ala Leu Gln Ser Gly Asn Ser Gln Glu
145                 150                 155                 160

agt gtc aca gag cag gac agc aag gac agc acc tac agc ctc agc agc         528
Ser Val Thr Glu Gln Asp Ser Lys Asp Ser Thr Tyr Ser Leu Ser Ser
                165                 170                 175

acc ctg acg ctg agc aaa gca gac tac gag aaa cac aaa gtc tac gcc         576
Thr Leu Thr Leu Ser Lys Ala Asp Tyr Glu Lys His Lys Val Tyr Ala
            180                 185                 190

tgc gaa gtc acc cat cag ggc ctg agc tcg ccc gtc aca aag agc ttc         624
Cys Glu Val Thr His Gln Gly Leu Ser Ser Pro Val Thr Lys Ser Phe
            195                 200                 205

aac agg gga gag tgt                                                      639
Asn Arg Gly Glu Cys
            210
```

<210> 94

<211> 213

<212> PRT

<213> Artificial Sequence

<220>
<223> Synthetic Construct

<400> 94

```
        Asp Ile Gln Met Thr Gln Ser Pro Ser Thr Leu Ser Ala Ser Val Gly
        1               5                   10                  15

        Asp Arg Val Thr Ile Thr Cys Arg Ala Ser Ser Ser Val Arg Tyr Ile
                    20                  25                  30

        Ala Trp Tyr Gln Gln Lys Pro Gly Lys Ala Pro Lys Leu Leu Ile Tyr
                    35                  40                  45

        Pro Thr Ser Ser Leu Glu Ser Gly Val Pro Ser Arg Phe Ser Gly Ser
                    50                  55                  60

        Gly Ser Gly Thr Glu Phe Thr Leu Thr Ile Ser Ser Leu Gln Pro Asp
        65                  70                  75                  80

        Asp Phe Ala Thr Tyr Tyr Cys Leu Gln Trp Ser Ser Tyr Pro Trp Thr
                        85                  90                  95

        Phe Gly Gly Gly Thr Lys Val Glu Ile Lys Arg Thr Val Ala Ala Pro
                    100                 105                 110

        Ser Val Phe Ile Phe Pro Pro Ser Asp Glu Gln Leu Lys Ser Gly Thr
                    115                 120                 125

        Ala Ser Val Val Cys Leu Leu Asn Asn Phe Tyr Pro Arg Glu Ala Lys
            130                 135                 140

        Val Gln Trp Lys Val Asp Asn Ala Leu Gln Ser Gly Asn Ser Gln Glu
        145                 150                 155                 160

        Ser Val Thr Glu Gln Asp Ser Lys Asp Ser Thr Tyr Ser Leu Ser Ser
                        165                 170                 175

        Thr Leu Thr Leu Ser Lys Ala Asp Tyr Glu Lys His Lys Val Tyr Ala
                    180                 185                 190

        Cys Glu Val Thr His Gln Gly Leu Ser Ser Pro Val Thr Lys Ser Phe
                    195                 200                 205

        Asn Arg Gly Glu Cys
                210
```

<210> 95

<211> 705
<212> DNA
<213> Artificial Sequence

<220>
<223> Chaîne légère avec leader 5B_42

<220>
<221> CDS
<222> (1)..(705)

<400> 95

```
atg gat atg aga gtg ccc gca cag ctg ctg ggt ctg ctg ctg ctg tgg        48
Met Asp Met Arg Val Pro Ala Gln Leu Leu Gly Leu Leu Leu Leu Trp
1               5               10              15

ctg ccc gga gcc aaa tgt gac atc cag atg aca cag tcc cca tct acc        96
Leu Pro Gly Ala Lys Cys Asp Ile Gln Met Thr Gln Ser Pro Ser Thr
            20              25              30

ctg tct gct tcc gtg gga gat cgg gtg act atc acc tgc aga gca agc        144
Leu Ser Ala Ser Val Gly Asp Arg Val Thr Ile Thr Cys Arg Ala Ser
        35              40              45

tcc tcc gtg agg tac atc gct tgg tac cag cag aag cca gga aag gcc        192
Ser Ser Val Arg Tyr Ile Ala Trp Tyr Gln Gln Lys Pro Gly Lys Ala
        50              55              60

cca aag ctg ctg atc tac cca acc tcc tcc ctg gaa tcc ggg gtg ccc        240
Pro Lys Leu Leu Ile Tyr Pro Thr Ser Ser Leu Glu Ser Gly Val Pro
65              70              75              80

agc aga ttc tca ggc agt ggc tcc ggc acc gaa ttc acc ctg acc atc        288
Ser Arg Phe Ser Gly Ser Gly Ser Gly Thr Glu Phe Thr Leu Thr Ile
            85              90              95

agc tca ctg cag cct gac gac ttc gca acc tac tac tgt ctg cag tgg        336
Ser Ser Leu Gln Pro Asp Asp Phe Ala Thr Tyr Tyr Cys Leu Gln Trp
            100             105             110

agt agc tac cct tgg aca ttc ggc ggc ggc acc aag gtg gag atc aag        384
Ser Ser Tyr Pro Trp Thr Phe Gly Gly Gly Thr Lys Val Glu Ile Lys
            115             120             125

cgg acc gtc gcc gca cca agt gtc ttc atc ttc ccg cca tct gat gag        432
Arg Thr Val Ala Ala Pro Ser Val Phe Ile Phe Pro Pro Ser Asp Glu
    130             135             140

cag ttg aaa tct gga act gcc tct gtt gtg tgc ctg ctg aat aac ttc        480
Gln Leu Lys Ser Gly Thr Ala Ser Val Val Cys Leu Leu Asn Asn Phe
145             150             155             160

tat ccc aga gag gcc aaa gta cag tgg aag gtg gat aac gcc ctc caa        528
Tyr Pro Arg Glu Ala Lys Val Gln Trp Lys Val Asp Asn Ala Leu Gln
            165             170             175

tcg ggt aac tcc cag gag agt gtc aca gag cag gac agc aag gac agc        576
Ser Gly Asn Ser Gln Glu Ser Val Thr Glu Gln Asp Ser Lys Asp Ser
            180             185             190

acc tac agc ctc agc agc acc ctg acg ctg agc aaa gca gac tac gag        624
Thr Tyr Ser Leu Ser Ser Thr Leu Thr Leu Ser Lys Ala Asp Tyr Glu
            195             200             205

aaa cac aaa gtc tac gcc tgc gaa gtc acc cat cag ggc ctg agc tcg        672
Lys His Lys Val Tyr Ala Cys Glu Val Thr His Gln Gly Leu Ser Ser
    210             215             220

ccc gtc aca aag agc ttc aac agg gga gag tgt                            705
Pro Val Thr Lys Ser Phe Asn Arg Gly Glu Cys
225             230             235
```

<210> 96

<211> 235
<212> PRT
<213> Artificial Sequence

<220>
<223> Synthetic Construct

<400> 96

```
Met Asp Met Arg Val Pro Ala Gln Leu Leu Gly Leu Leu Leu Leu Trp
1               5               10              15

Leu Pro Gly Ala Lys Cys Asp Ile Gln Met Thr Gln Ser Pro Ser Thr
            20              25              30

Leu Ser Ala Ser Val Gly Asp Arg Val Thr Ile Thr Cys Arg Ala Ser
        35              40              45

Ser Ser Val Arg Tyr Ile Ala Trp Tyr Gln Gln Lys Pro Gly Lys Ala
    50              55              60

Pro Lys Leu Leu Ile Tyr Pro Thr Ser Ser Leu Glu Ser Gly Val Pro
65              70              75              80

Ser Arg Phe Ser Gly Ser Gly Ser Gly Thr Glu Phe Thr Leu Thr Ile
                85              90              95

Ser Ser Leu Gln Pro Asp Asp Phe Ala Thr Tyr Tyr Cys Leu Gln Trp
            100             105             110

Ser Ser Tyr Pro Trp Thr Phe Gly Gly Gly Thr Lys Val Glu Ile Lys
        115             120             125

Arg Thr Val Ala Ala Pro Ser Val Phe Ile Phe Pro Pro Ser Asp Glu
    130             135             140

Gln Leu Lys Ser Gly Thr Ala Ser Val Val Cys Leu Leu Asn Asn Phe
145             150             155             160

Tyr Pro Arg Glu Ala Lys Val Gln Trp Lys Val Asp Asn Ala Leu Gln
            165             170             175

Ser Gly Asn Ser Gln Glu Ser Val Thr Glu Gln Asp Ser Lys Asp Ser
```

```
            180                    185                    190

    Thr Tyr Ser Leu Ser Ser Thr Leu Thr Leu Ser Lys Ala Asp Tyr Glu
            195                200              205

    Lys His Lys Val Tyr Ala Cys Glu Val Thr His Gln Gly Leu Ser Ser
        210                215              220

    Pro Val Thr Lys Ser Phe Asn Arg Gly Glu Cys
    225                230              235
```

<210> 97
<211> 1335
<212> DNA
<213> Artificial Sequence

<220>
<223> Chaîne lourde sans leader 5B_42

<220>
<221> CDS
<222> (1)..(1335)

<400> 97

```
cag gtg cag ctg gtg cag agc ggg gcc gag gtg aag aag cct gga gcc          48
Gln Val Gln Leu Val Gln Ser Gly Ala Glu Val Lys Lys Pro Gly Ala
1               5               10              15

tca gtg aag gtg agt tgc aag gcc tcc ggt tac acc ttc acc agc tac          96
Ser Val Lys Val Ser Cys Lys Ala Ser Gly Tyr Thr Phe Thr Ser Tyr
                20              25              30

cac atc cac tgg gtc aga cag gct ccc ggc cag aga ctg gag tgg atg         144
His Ile His Trp Val Arg Gln Ala Pro Gly Gln Arg Leu Glu Trp Met
            35              40              45

ggc tgg atc tac cct gga gat gac tcc acc aag tac tcc cag aag ttc         192
Gly Trp Ile Tyr Pro Gly Asp Asp Ser Thr Lys Tyr Ser Gln Lys Phe
        50              55              60

cag ggt cgc gtg acc att acc agg gac gcc agc gcc tcc act gcc tac         240
Gln Gly Arg Val Thr Ile Thr Arg Asp Ala Ser Ala Ser Thr Ala Tyr
65              70              75              80

atg gag ctg tct tcc ctg aga tct gag gat acc gca gtc tac tac tgt         288
Met Glu Leu Ser Ser Leu Arg Ser Glu Asp Thr Ala Val Tyr Tyr Cys
                85              90              95

aca cgg ggg gac cgc ttt gct tac tgg ggg cag ggc act ctg gtg acc         336
Thr Arg Gly Asp Arg Phe Ala Tyr Trp Gly Gln Gly Thr Leu Val Thr
            100             105             110

gtc agc agc gcc agc acc aag ggc cca tcg gtc ttc ccc ctg gca ccc         384
Val Ser Ser Ala Ser Thr Lys Gly Pro Ser Val Phe Pro Leu Ala Pro
        115             120             125

tcc tcc aag agc acc tct ggg ggc aca gcg gcc ctg ggc tgc ctg gtc         432
Ser Ser Lys Ser Thr Ser Gly Gly Thr Ala Ala Leu Gly Cys Leu Val
        130             135             140
```

```
aag gac tac ttc ccc gaa ccg gtg acg gtg tcg tgg aac tca ggc gcc       480
Lys Asp Tyr Phe Pro Glu Pro Val Thr Val Ser Trp Asn Ser Gly Ala
145             150             155             160

ctg acc agc ggc gtg cac acc ttc ccg gct gtc cta cag tcc tca gga       528
Leu Thr Ser Gly Val His Thr Phe Pro Ala Val Leu Gln Ser Ser Gly
                165             170             175

ctc tac tcc ctc agc agc gtg gtg acc gtg ccc tcc agc agc ttg ggc       576
Leu Tyr Ser Leu Ser Ser Val Val Thr Val Pro Ser Ser Ser Leu Gly
                180             185             190

acc cag acc tac atc tgc aac gtg aat cac aag ccc agc aac acc aag       624
Thr Gln Thr Tyr Ile Cys Asn Val Asn His Lys Pro Ser Asn Thr Lys
            195             200             205

gtg gac aag aaa gtt gag ccc aaa tct tgt gac aaa act cac aca tgc       672
Val Asp Lys Lys Val Glu Pro Lys Ser Cys Asp Lys Thr His Thr Cys
        210             215             220

cca ccg tgc cca gca cct gaa ctc ctg ggg gga ccg tca gtc ttc ctc       720
Pro Pro Cys Pro Ala Pro Glu Leu Leu Gly Gly Pro Ser Val Phe Leu
225             230             235             240

ttc ccc cca aaa ccc aag gac acc ctc atg atc tcc cgg acc cct gag       768
Phe Pro Pro Lys Pro Lys Asp Thr Leu Met Ile Ser Arg Thr Pro Glu
                245             250             255

gtc aca tgc gtg gtg gtg gac gtg agc cac gaa gac cct gag gtc aag       816
Val Thr Cys Val Val Val Asp Val Ser His Glu Asp Pro Glu Val Lys
                260             265             270

ttc aac tgg tac gtg gac ggc gtg gag gtg cat aat gcc aag aca aag       864
Phe Asn Trp Tyr Val Asp Gly Val Glu Val His Asn Ala Lys Thr Lys
                275             280             285

ccg cgg gag gag cag tac aac agc acg tac cgt gtg gtc agc gtc ctc       912
Pro Arg Glu Glu Gln Tyr Asn Ser Thr Tyr Arg Val Val Ser Val Leu
        290             295             300

acc gtc ctg cac cag gac tgg ctg aat ggc aag gag tac aag tgc aag       960
Thr Val Leu His Gln Asp Trp Leu Asn Gly Lys Glu Tyr Lys Cys Lys
305             310             315             320

gtc tcc aac aaa gcc ctc cca gcc ccc atc gag aaa acc atc tcc aaa      1008
Val Ser Asn Lys Ala Leu Pro Ala Pro Ile Glu Lys Thr Ile Ser Lys
                325             330             335

gcc aaa ggg cag ccc cga gaa cca cag gtg tac acc ctg ccc cca tcc      1056
Ala Lys Gly Gln Pro Arg Glu Pro Gln Val Tyr Thr Leu Pro Pro Ser
                340             345             350

cgg gat gag ctg acc aag aac cag gtc agc ctg acc tgc ctg gtc aaa      1104
Arg Asp Glu Leu Thr Lys Asn Gln Val Ser Leu Thr Cys Leu Val Lys
                355             360             365

ggc ttc tat ccc agc gac atc gcc gtg gag tgg gag agc aat ggg cag      1152
Gly Phe Tyr Pro Ser Asp Ile Ala Val Glu Trp Glu Ser Asn Gly Gln
                370             375             380

ccg gag aac aac tac aag acc acg cct ccc gtg ctg gac tcc gac ggc      1200
Pro Glu Asn Asn Tyr Lys Thr Thr Pro Pro Val Leu Asp Ser Asp Gly
385             390             395             400

tcc ttc ttc ctc tac agc aag ctc acc gtg gac aag agc agg tgg cag      1248
```

```
Ser Phe Phe Leu Tyr Ser Lys Leu Thr Val Asp Lys Ser Arg Trp Gln
                405                 410                 415

cag ggg aac gtc ttc tca tgc tcc gtg atg cat gag gct ctg cac aac        1296
Gln Gly Asn Val Phe Ser Cys Ser Val Met His Glu Ala Leu His Asn
                420                 425                 430

cac tac acg cag aag agc ctc tcc ctg tct ccg ggt aaa                    1335
His Tyr Thr Gln Lys Ser Leu Ser Leu Ser Pro Gly Lys
                435                 440                 445
```

<210> 98
<211> 445
<212> PRT
<213> Artificial Sequence

<220>
<223> Synthetic Construct

<400> 98

Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Thr Phe Thr Ser Tyr
            20                  25                  30

His Ile His Trp Val Arg Gln Ala Pro Gly Gln Arg Leu Glu Trp Met
            35                  40                  45

Gly Trp Ile Tyr Pro Gly Asp Asp Ser Thr Lys Tyr Ser Gln Lys Phe
    50                  55                  60

Gln Gly Arg Val Thr Ile Thr Arg Asp Ala Ser Ala Ser Thr Ala Tyr
65                  70                  75                  80

Met Glu Leu Ser Ser Leu Arg Ser Glu Asp Thr Ala Val Tyr Tyr Cys
            85                  90                  95

Thr Arg Gly Asp Arg Phe Ala Tyr Trp Gly Gln Gly Thr Leu Val Thr
            100                 105                 110

Val Ser Ser Ala Ser Thr Lys Gly Pro Ser Val Phe Pro Leu Ala Pro
            115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
            165                 170                 175

```
Leu Tyr Ser Leu Ser Ser Val Val Thr Val Pro Ser Ser Ser Leu Gly
            180             185             190

Thr Gln Thr Tyr Ile Cys Asn Val Asn His Lys Pro Ser Asn Thr Lys
            195             200             205

Val Asp Lys Lys Val Glu Pro Lys Ser Cys Asp Lys Thr His Thr Cys
    210             215             220

Pro Pro Cys Pro Ala Pro Glu Leu Leu Gly Gly Pro Ser Val Phe Leu
225             230             235             240

Phe Pro Pro Lys Pro Lys Asp Thr Leu Met Ile Ser Arg Thr Pro Glu
            245             250             255

Val Thr Cys Val Val Val Asp Val Ser His Glu Asp Pro Glu Val Lys
            260             265             270

Phe Asn Trp Tyr Val Asp Gly Val Glu Val His Asn Ala Lys Thr Lys
            275             280             285

Pro Arg Glu Glu Gln Tyr Asn Ser Thr Tyr Arg Val Val Ser Val Leu
    290             295             300

Thr Val Leu His Gln Asp Trp Leu Asn Gly Lys Glu Tyr Lys Cys Lys
305             310             315             320

Val Ser Asn Lys Ala Leu Pro Ala Pro Ile Glu Lys Thr Ile Ser Lys
            325             330             335

Ala Lys Gly Gln Pro Arg Glu Pro Gln Val Tyr Thr Leu Pro Pro Ser
            340             345             350

Arg Asp Glu Leu Thr Lys Asn Gln Val Ser Leu Thr Cys Leu Val Lys
            355             360             365

Gly Phe Tyr Pro Ser Asp Ile Ala Val Glu Trp Glu Ser Asn Gly Gln
    370             375             380

Pro Glu Asn Asn Tyr Lys Thr Thr Pro Pro Val Leu Asp Ser Asp Gly
385             390             395             400

Ser Phe Phe Leu Tyr Ser Lys Leu Thr Val Asp Lys Ser Arg Trp Gln
            405             410             415

Gln Gly Asn Val Phe Ser Cys Ser Val Met His Glu Ala Leu His Asn
            420             425             430
```

```
His Tyr Thr Gln Lys Ser Leu Ser Leu Ser Pro Gly Lys
        435                 440                 445
```

<210> 99
<211> 1392
<212> DNA
<213> Artificial Sequence

<220>
<223> Chaîne lourde avec leader 5B_42

<220>
<221> CDS
<222> (1)..(1392)

<400> 99

```
atg gat tgg aca tgg cga atc ctg ttc ctg gtg gct gcc gca acc ggc        48
Met Asp Trp Thr Trp Arg Ile Leu Phe Leu Val Ala Ala Ala Thr Gly
1               5               10              15

gcc cac agc cag gtg cag ctg gtg cag agc ggg gcc gag gtg aag aag        96
Ala His Ser Gln Val Gln Leu Val Gln Ser Gly Ala Glu Val Lys Lys
            20              25              30

cct gga gcc tca gtg aag gtg agt tgc aag gcc tcc ggt tac acc ttc       144
Pro Gly Ala Ser Val Lys Val Ser Cys Lys Ala Ser Gly Tyr Thr Phe
        35              40              45

acc agc tac cac atc cac tgg gtc aga cag gct ccc ggc cag aga ctg       192
Thr Ser Tyr His Ile His Trp Val Arg Gln Ala Pro Gly Gln Arg Leu
    50              55              60

gag tgg atg ggc tgg atc tac cct gga gat gac tcc acc aag tac tcc       240
Glu Trp Met Gly Trp Ile Tyr Pro Gly Asp Asp Ser Thr Lys Tyr Ser
65              70              75              80

cag aag ttc cag ggt cgc gtg acc att acc agg gac gcc agc gcc tcc       288
Gln Lys Phe Gln Gly Arg Val Thr Ile Thr Arg Asp Ala Ser Ala Ser
            85              90              95

act gcc tac atg gag ctg tct tcc ctg aga tct gag gat acc gca gtc       336
Thr Ala Tyr Met Glu Leu Ser Ser Leu Arg Ser Glu Asp Thr Ala Val
            100             105             110

tac tac tgt aca cgg ggg gac cgc ttt gct tac tgg ggg cag ggc act       384
Tyr Tyr Cys Thr Arg Gly Asp Arg Phe Ala Tyr Trp Gly Gln Gly Thr
        115             120             125

ctg gtg acc gtc agc agc gcc agc acc aag ggc cca tcg gtc ttc ccc       432
Leu Val Thr Val Ser Ser Ala Ser Thr Lys Gly Pro Ser Val Phe Pro
    130             135             140

ctg gca ccc tcc tcc aag agc acc tct ggg ggc aca gcg gcc ctg ggc       480
Leu Ala Pro Ser Ser Lys Ser Thr Ser Gly Gly Thr Ala Ala Leu Gly
145             150             155             160

tgc ctg gtc aag gac tac ttc ccc gaa ccg gtg acg gtg tcg tgg aac       528
Cys Leu Val Lys Asp Tyr Phe Pro Glu Pro Val Thr Val Ser Trp Asn
            165             170             175

tca ggc gcc ctg acc agc ggc gtg cac acc ttc ccg gct gtc cta cag       576
Ser Gly Ala Leu Thr Ser Gly Val His Thr Phe Pro Ala Val Leu Gln
            180             185             190
```

```
tcc tca gga ctc tac tcc ctc agc agc gtg gtg acc gtg ccc tcc agc        624
Ser Ser Gly Leu Tyr Ser Leu Ser Ser Val Val Thr Val Pro Ser Ser
        195             200             205

agc ttg ggc acc cag acc tac atc tgc aac gtg aat cac aag ccc agc        672
Ser Leu Gly Thr Gln Thr Tyr Ile Cys Asn Val Asn His Lys Pro Ser
        210             215             220

aac acc aag gtg gac aag aaa gtt gag ccc aaa tct tgt gac aaa act        720
Asn Thr Lys Val Asp Lys Lys Val Glu Pro Lys Ser Cys Asp Lys Thr
225             230             235             240

cac aca tgc cca ccg tgc cca gca cct gaa ctc ctg ggg gga ccg tca        768
His Thr Cys Pro Pro Cys Pro Ala Pro Glu Leu Leu Gly Gly Pro Ser
            245             250             255

gtc ttc ctc ttc ccc cca aaa ccc aag gac acc ctc atg atc tcc cgg        816
Val Phe Leu Phe Pro Pro Lys Pro Lys Asp Thr Leu Met Ile Ser Arg
        260             265             270

acc cct gag gtc aca tgc gtg gtg gtg gac gtg agc cac gaa gac cct        864
Thr Pro Glu Val Thr Cys Val Val Val Asp Val Ser His Glu Asp Pro
        275             280             285

gag gtc aag ttc aac tgg tac gtg gac ggc gtg gag gtg cat aat gcc        912
Glu Val Lys Phe Asn Trp Tyr Val Asp Gly Val Glu Val His Asn Ala
        290             295             300

aag aca aag ccg cgg gag gag cag tac aac agc acg tac cgt gtg gtc        960
Lys Thr Lys Pro Arg Glu Glu Gln Tyr Asn Ser Thr Tyr Arg Val Val
305             310             315             320

agc gtc ctc acc gtc ctg cac cag gac tgg ctg aat ggc aag gag tac       1008
Ser Val Leu Thr Val Leu His Gln Asp Trp Leu Asn Gly Lys Glu Tyr
            325             330             335

aag tgc aag gtc tcc aac aaa gcc ctc cca gcc ccc atc gag aaa acc       1056
Lys Cys Lys Val Ser Asn Lys Ala Leu Pro Ala Pro Ile Glu Lys Thr
            340             345             350

atc tcc aaa gcc aaa ggg cag ccc cga gaa cca cag gtg tac acc ctg       1104
Ile Ser Lys Ala Lys Gly Gln Pro Arg Glu Pro Gln Val Tyr Thr Leu
            355             360             365

ccc cca tcc cgg gat gag ctg acc aag aac cag gtc agc ctg acc tgc       1152
Pro Pro Ser Arg Asp Glu Leu Thr Lys Asn Gln Val Ser Leu Thr Cys
        370             375             380

ctg gtc aaa ggc ttc tat ccc agc gac atc gcc gtg gag tgg gag agc       1200
Leu Val Lys Gly Phe Tyr Pro Ser Asp Ile Ala Val Glu Trp Glu Ser
385             390             395             400

aat ggg cag ccg gag aac aac tac aag acc acg cct ccc gtg ctg gac       1248
Asn Gly Gln Pro Glu Asn Asn Tyr Lys Thr Thr Pro Pro Val Leu Asp
            405             410             415

tcc gac ggc tcc ttc ttc ctc tac agc aag ctc acc gtg gac aag agc       1296
Ser Asp Gly Ser Phe Phe Leu Tyr Ser Lys Leu Thr Val Asp Lys Ser
            420             425             430

agg tgg cag cag ggg aac gtc ttc tca tgc tcc gtg atg cat gag gct       1344
Arg Trp Gln Gln Gly Asn Val Phe Ser Cys Ser Val Met His Glu Ala
            435             440             445
```

```
ctg cac aac cac tac acg cag aag agc ctc tcc ctg tct ccg ggt aaa        1392
Leu His Asn His Tyr Thr Gln Lys Ser Leu Ser Leu Ser Pro Gly Lys
    450             455             460
```

<210> 100
<211> 464
<212> PRT
<213> Artificial Sequence

<220>
<223> Synthetic Construct

<400> 100

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu
    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Ala Ser Ala Ser
            85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
            165                 170                 175

Ser Gly Ala Leu Thr Ser Gly Val His Thr Phe Pro Ala Val Leu Gln
        180                 185                 190

Ser Ser Gly Leu Tyr Ser Leu Ser Ser Val Val Thr Val Pro Ser Ser
        195                 200                 205
```

Ser Leu Gly Thr Gln Thr Tyr Ile Cys Asn Val Asn His Lys Pro Ser
210 215 220

Asn Thr Lys Val Asp Lys Lys Val Glu Pro Lys Ser Cys Asp Lys Thr
225 230 235 240

His Thr Cys Pro Pro Cys Pro Ala Pro Glu Leu Leu Gly Gly Pro Ser
245 250 255

Val Phe Leu Phe Pro Pro Lys Pro Lys Asp Thr Leu Met Ile Ser Arg
260 265 270

Thr Pro Glu Val Thr Cys Val Val Val Asp Val Ser His Glu Asp Pro
275 280 285

Glu Val Lys Phe Asn Trp Tyr Val Asp Gly Val Glu Val His Asn Ala
290 295 300

Lys Thr Lys Pro Arg Glu Glu Gln Tyr Asn Ser Thr Tyr Arg Val Val
305 310 315 320

Ser Val Leu Thr Val Leu His Gln Asp Trp Leu Asn Gly Lys Glu Tyr
325 330 335

Lys Cys Lys Val Ser Asn Lys Ala Leu Pro Ala Pro Ile Glu Lys Thr
340 345 350

Ile Ser Lys Ala Lys Gly Gln Pro Arg Glu Pro Gln Val Tyr Thr Leu
355 360 365

Pro Pro Ser Arg Asp Glu Leu Thr Lys Asn Gln Val Ser Leu Thr Cys
370 375 380

Leu Val Lys Gly Phe Tyr Pro Ser Asp Ile Ala Val Glu Trp Glu Ser
385 390 395 400

Asn Gly Gln Pro Glu Asn Asn Tyr Lys Thr Thr Pro Pro Val Leu Asp
405 410 415

Ser Asp Gly Ser Phe Phe Leu Tyr Ser Lys Leu Thr Val Asp Lys Ser
420 425 430

Arg Trp Gln Gln Gly Asn Val Phe Ser Cys Ser Val Met His Glu Ala
435 440 445

Leu His Asn His Tyr Thr Gln Lys Ser Leu Ser Leu Ser Pro Gly Lys
450 455 460

190

<210> 101
<211> 19
<212> PRT
<213> Artificial Sequence

<220>
<223> Peptide épitopique

<220>
<221> PEPTIDE
<222> (1)..(19)

<400> 101

```
    Gly Gly Gly Gly Asn Leu Thr Gln Asp Arg Ala Gln Val Glu Met Gln
    1               5                   10                  15
```

Gly Ser Arg

<210> 102
<211> 19
<212> PRT
<213> Artificial Sequence

<220>
<223> Peptide épitopique contrôle négatif

<220>
<221> PEPTIDE
<222> (1)..(19)

<400> 102

```
    Gly Gly Gly Gly Asn Leu Thr Gln Ala Arg Gly Gln Val Glu Met Gln
    1               5                   10                  15


    Gly Ser Arg
```

**Revendications**

1. Anticorps monoclonal 12G4 humanisé muté liant spécifiquement le récepteur de type II de l'hormone anti-mullérienne humaine, possédant :

   a) une chaîne légère constituée de la séquence en acides aminés représentée par :

      - SEQ ID NO: 82 ou SEQ ID NO: 84, et

   b) une chaîne lourde constituée de la séquence en acides aminés représentée par :

      - SEQ ID NO: 86 ou SEQ ID NO: 88.

2. Acide nucléique comprenant ou constitué d'une séquence codant pour l'anticorps de la revendication 1.

3. Acide nucléique selon la revendication 2,
   dans lequel la séquence codant la chaîne légère et la séquence codant la chaîne lourde sont les suivantes :

a) séquence codant la chaîne légère SEQ ID NO: 81 ou SEQ ID NO: 83, et
b) séquence codant la chaîne lourde SEQ ID NO: 85 ou SEQ ID NO: 87.

4. Vecteur d'expression comprenant au moins un acide nucléique selon l'une quelconque des revendications 2 ou 3, ledit acide nucléique étant sous contrôle des éléments permettant son expression et comprenant

   • un premier acide nucléique de séquence: SEQ ID NO 83, ledit premier acide nucléique étant sous contrôle des éléments permettant son expression, et
   • un second acide nucléique de séquences SEQ ID NO 87, ledit second acide nucléique étant sous contrôle des éléments permettant son expression.

5. Cellule hôte ou lignée cellulaire transformée par un acide nucléique selon l'une des revendications 2 et 3 et/ou un vecteur d'expression selon la revendication 4.

6. Composition pharmaceutique, et notamment vaccinale, comprenant au moins

   • un anticorps monoclonal selon la revendication 1, ou
   • un acide nucléique selon l'une quelconque des revendications 2 ou 3, ou
   • un vecteur selon la revendication 4,

   en association avec un véhicule pharmaceutiquement acceptable.

7. Produit comprenant une première préparation pharmaceutique comprenant un anticorps monoclonal selon la revendication 1, et une seconde préparation pharmaceutique comprenant un composé anticancéreux conventionnel, notamment du paclitaxel ou un sel de platine, en particulier de l'oxaloplatine, du cisplatine ou de la carboplatine, en tant que préparation combinée pour une utilisation simultanée, séparée ou séquentielle dans le traitement de patients atteints de cancers ovariens.

8. Anticorps monoclonal selon la revendication 1, ou
   acide nucléique selon l'une quelconque des revendications 2 ou 3, ou
   vecteur selon la revendication 4, ou
   cellule selon la revendication 5,
   pour son utilisation dans le traitement ou la prévention d'un cancer ovarien, en particulier ledit anticorps monoclonal ou ledit acide nucléique ou ledit vecteur ou ladite cellule étant utilisé en association avec un anticancéreux conventionnel, notamment du paclitaxel ou un sel de platine, en particulier de l'oxaloplatine, du cisplatine ou de la carboplatine.

9. Anticorps monoclonal selon la revendication 1 pour son utilisation dans le diagnostic et/ou le suivi d'un cancer ovarien associé au récepteur de type II de l'hormone anti-mullérienne humaine.

10. Procédé de diagnostic d'un cancer ovarien associée au récepteur de type II de l'hormone anti-mullérienne humaine sur un échantillon biologique humain, comprenant les étapes suivantes :

   a. marquage d'une biopsie préalablement prélevée sur un patient à l'aide d'un anticorps monoclonal selon la revendication 1
   b. détermination de la présence d'un récepteur de type II de l'hormone anti-mullérienne humaine.

**Patentansprüche**

1. Mutierter humanisierter monoklonaler Antikörper 12G4, welcher spezifisch den menschlichen Anti-Müller-Hormonrezeptor Typ II bindet, umfassend;

   a) eine leichte Kette, die aus der Aminosäuresequenz besteht, repräsentiert durch:

      S EQ ID NR: 82 oder SEQ ID NR: 84, und

   b) eine schwere Kette, die aus der Aminosäuresequenz besteht, repräsentiert durch:

- SEQ ID NR: 86 oder SEQ ID NR: 88.

**2.** Nukleinsäure, umfassend eine oder bestehend aus einer Sequenz, die für den Antikörper von Anspruch 1 codiert.

**3.** Nukleinsäure nach Anspruch 2,
wobei die Sequenz, welche die leichte Kette codiert, und die Sequenz, welche die schwere Kette codiert, die folgenden sind:

a) die leichte Kette codierende Sequenz SEQ ID NR: 81 oder SEQ ID NR: 83, und
b) die schwere Kette codierende Sequenz SEQ ID NR: 85 oder SEQ ID NR: 87.

**4.** Expressionsvektor, umfassend mindestens eine Nukleinsäure nach einem der Ansprüche 2 oder 3, wobei die Nukleinsäure unter der Kontrolle von Elementen steht, die ihre Expression gestatten und umfassen:

• eine erste Nukleinsäure mit der Sequenz: SEQ ID NR: 83, wobei die erste Nukleinsäure unter der Kontrolle von Elementen steht, die ihre Expression gestatten, und
• eine zweite Nukleinsäure mit der Sequenz; SEQ ID NR: 87, wobei die zweite Nukleinsäure unter der Kontrolle von Elementen steht, die ihre Expression gestatten.

**5.** Wirtszelle oder Zelllinie, transformiert durch eine Nukleinsäure nach einem der Ansprüche 2 oder 3, und/oder einen Expressionsvektor nach Anspruch 4.

**6.** Pharmazeutische Zusammensetzung, und insbesondere vakzinale Zusammensetzung, umfassend mindestens:

• einen monoklonalen Antikörper nach Anspruch 1, oder
• eine Nukleinsäure nach einem der Ansprüche 2 oder 3, oder
• einen Vektor nach Anspruch 4,

in Assoziation mit einem pharmazeutisch annehmbaren Träger.

**7.** Produkt, umfassend eine erste pharmazeutische Zubereitung, umfassend einen monoklonalen Antikörper nach Anspruch 1, und eine zweite pharmazeutische Zubereitung, umfassend eine herkömmliche Anti-Krebs-Verbindung, insbesondere Paclitaxel oder ein Platinsalz, insbesondere Oxaloplatin, Cisplatin oder Carboplatin, als kombinierte Zubereitung zur gleichzeitigen, getrennten oder sequentiellen Verwendung bei der Behandlung von Patientinnen, die an Eierstockkrebs leiden.

**8.** Monoklonaler Antikörper nach Anspruch 1, oder
Nukleinsäure nach einem der Ansprüche 2 oder 3, oder Vektor nach Anspruch 4, oder
Zelle nach Anspruch 5,
zu deren Verwendung bei der Behandlung oder Prävention von Eierstockkrebs, wobei insbesondere der monoklonale Antikörper oder die Nukleinsäure oder der Vektor oder die Zelle in Assoziation mit einer herkömmlichen Anti-Krebs-Verbindung, insbesondere Paclitaxel oder einem Platinsalz, insbesondere Oxaloplatin, Cisplatin oder Carboplatin, verwendet wird.

**9.** Monoklonaler Antikörper nach Anspruch 1, für seine Verwendung bei der Diagnose und/oder der Überwachung von Eierstockkrebs, assoziiert mit dem menschlichen Anti-Müller-Hormonrezeptor Typ II.

**10.** Verfahren zur Diagnose von Eierstockkrebs, assoziiert mit dem menschlichen Anti-Müller-Hormonrezeptor Typ II, in einer menschlichen biologischen Probe, umfassend die folgenden Schritte;

a. Markieren einer zuvor einer Patientin entnommenen Biopsie mit Hilfe eines monoklonalen Antikörpers nach Anspruch 1,
b. Bestimmen des Vorliegens eines menschlichen Anti-Müller-Hormonrezeptors Typ II.

**Claims**

**1.** A mutated humanized 12G4 monoclonal antibody specifically binding the human anti-Müllerian hormone type II receptor, possessing :

a) a light chain consisting of the amino acid sequence represented by :

- SEQ ID NO : 82 or SEQ ID NO ; 84, and

b) a heavy chain consisting of the amino acid sequence represented by :

- SEQ ID NO : 86 or SEQ ID ID NO : 88.

2. Amino acid comprising or consisting of a sequence encoding the antibody of claim 1.

3. Amino acid according to claim 2, in which the sequence encoding the light chain and the sequence encoding the heavy chain are the following :

a) Sequence encoding the light chain SEQ ID ID NO : 81 or SEQ ID NO: 83, and
b) Sequence encoding the heavy chain SEQ ID NO : 85 or SEQ ID NO : 87.

4. Expression vector comprising at least one nucleic acid according to any one of claims 2 or 3, the said nucleic acid being under the control of elements allowing its expression and comprising :

• a first nucleic acid of sequence : SEQ ID NO : 83, said first nucleic acid being under the control of elements allowing its expression, and
• a second nucleic acid of sequence SEQ ID ID NO : 87, said second nucleic acid being under the control of elements allowing its expression.

5. Host cell or cell line transformed with a nucleic acid according to one of claims 2 or 3 and/or an expression vector according to claims 4.

6. Pharmaceutical composition, and in particular vaccine composition, comprising at least:

• a monoclonal antibody according to claim 1, or
• a nucleic acid according to any one of claims 2 or 3, or
• a vector according to claim 4,

in association with a pharmaceutically acceptable vehicle.

7. Product comprising a. first pharmaceutical preparation comprising a monoclonal antibody according to claim 1 and a second pharmaceutical preparation comprising a conventional anti-cancer compound, in particular paclitaxel or a platinum salt, in particular oxaliplatin, cisplatin or carboplatin, as a combined preparation for simultaneous, separate or sequential use in the treatment of patients with ovarian cancers.

8. Monoclonal antibody according to claim 1, or
nucleic acid according to any one of claims 2 or 3, or
vector according to claim 4, or
cell according to claim 5
for use in the treatment or prevention of an ovarian cancer, in particular said monoclonal antibody or said nucleic acid or said vector or said cell being used in association with a conventional anti-cancer agent, in particular paclitaxel or a platinum salt, in particular oxaliplatin, cisplatin or carboplatin.

9. Monoclonal antibody according to claim 1 for use in the diagnosis and/or monitoring of an ovarian cancer associated with the human anti-Müllerian hormone type II receptor.

10. Method for diagnosis of an ovarian cancer associated with human anti-Müllerian hormone type II receptor on a human biological sample, comprising the following steps :

a) labelling of a biopsy previously taken on a patient using a monoclonal antibody according to claim 1
b) determination of the presence of a human anti-Müllerian hormone type II receptor

**Figure 1**

CDRs

**Figure 2**

**Figure 3**

**Figure 4**

**Figure 5**

**Figure 6**

**Figure 7**

**Figure 8**

**Figure 9**

**Figure 10**

**Figure 11**

**Figure 12**

**Figure 13**

**Figure 14**

**Figure 15**

**Figure 16**

**Figure 17**

**Figure 18**

**Figure 19**

**Figure 20**

**Figure 21**

**Figure 22**

**Figure 23**

A)

B)

**Figure 24**

Figure 25

**Figure 26**

**Figure 27**

**Figure 28**

Figure 29

**COV434 - AMHRII**
DITH-862-11-AMHRII-0044

Figure 30

**Figure 31**

**A** COV434-AMHRII - Médiane des volumes tumoraux

Véhicule
3C-23K

**B** COV434-AMHRII - Courbes de survie

Véhicule
3C-23K

Figure 32

**A** — Asc1A5 - Médiane des volumes tumoraux

**B** — Asc1A5 - Courbes de survie

**Figure 33**

**A** Meta2815 - Médiane des volumes tumoraux

**B** Meta2815 - Courbes de survie

**Figure 34**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008053330 A

- FR 1053712

**Littérature non-brevet citée dans la description**

- **LA MARCA A. ; VOLPE A.** The Anti-Mullerian hormone and ovarian cancer. *Human Reproduction Update,* 2007, vol. 13 (3), 265-273
- **FAUCI, BRAUNWALD et al.** Principles of internal medicine. National Cancer Institute cancer.gov / CN-GOF
- **OZOLS R. F. et al.** Focus on epithelial ovarian cancer. *Cancer Cell,* Janvier 2004, vol. 5 (1), 19-24

- **REYCHLERG. ; DESSANGES JF ; VECELLIO L.** *Rev. Mal. Respir,* 2007, vol. 24, 1013-1023
- **VAN DEN BERG-BAKKER et al.** Establishment and characterization of 7 ovarian carcinoma cell lines and one granulosa tumor cell line: Growth features and cytogenetics. *International Journal of Cancer,* 1993, vol. 53, 613
- **ZHANG, H. et al.** Characterization of an immortalized human granulosa cell line (COV434). *Molecular Human Reproduction,* 2000, vol. 6, 146